# EUROPEAN PATENT SPECIFICATION

(11) **EP 1 819 710 B1**
(45) Date of publication and mention of the grant of the patent: **11.03.2009**
(21) Application number: 05814833.9
(22) Date of filing: 30.11.2005
(51) Int. Cl.: C07D 495/14, C07D 495/22, A61K 31/519, A61P 11/06

(54) **NEW PYRIDOTHIENOPYRIMIDINE DERIVATIVES**
NEUE PYRIDOTHIENOPYRIMIDINDERIVATE
NOUVEAUX DERIVES DE PYRIDOTHIENOPYRIMIDINE

(30) Priority: 30.11.2004 ES 200402876
(43) Date of publication of application: 22.08.2007
(73) Proprietor: Laboratorios Almirall, S.A., 08022 Barcelona (ES)
(72) Inventor: PAGES SANTACANA, Lluis, Miquel, E-08029 Barcelona (ES); TALTAVULL MOLL, Joan, E-08028 Barcelona (ES)
(74) Representative: Srinivasan, Ravi Chandran
(86) International application number: PCT/EP2005/012774
(87) International publication number: WO 2006/058724

(56) References cited:
- EP-A- 1 167 367
- US-B1- 6 420 368
- US-B1- 6 495 557
- YU. A. SHARANIN ET. AL.: "Cyclisation of nitriles. XIV. Synthesis and reactions of 1-alkyl-4-cyano-5,6,7,8-tetrahydro-3(2H)-i soquinolinethiones and some of their analogs." JOURNAL OF ORGANIC CHEMISTRY OF THE USSR, vol. 20, no. 11, 1984, pages 2216-2224, XP009054804
- YU. A. SHARANIN ET. AL.: "Cyclisation of nitriles. XV. Synthesis and transformations of 1-benzyl-4-cyano-5,6,7,8-tetrahydro-3(2H)- isoquinolinethione." JOURNAL OF ORGANIC CHEMISTRY OF THE USSR, vol. 20, no. 11, 1984, pages 2225-2230, XP009054803

## Description

The present invention relates to new therapeutically useful pyridothienopyrimidine derivatives, to processes for their preparation and to pharmaceutical compositions containing them. These compounds are potent and selective inhibitors of phosphodiesterase 4 (PDE4) and are thus useful in the treatment, prevention or suppression of pathological conditions, diseases and disorders known to be susceptible of being improved by inhibition of PDE4.

Phosphodiesterases (PDEs) comprise a superfamily of enzymes responsible for the hydrolysis and inactivation of the second messengers cyclic adenosine monophosphate (cAMP) and cyclic guanosine monophosphate (cGMP). Eleven different PDE families have been identified to date (PDE1 to PDE11) which differ in substrate preference, catalytic activity, sensitivity to endogenous activators and inhibitors, and encoding genes.

The PDE4 isoenzyme family exhibits a high affinity for cyclic AMP but has weak affinity for cyclic GMP. Increased cyclic AMP levels caused by PDE4 inhibition are associated with the suppression of cell activation in a wide range of inflammatory and immune cells, including lymphocytes, macrophages, basophils, neutrophils, and eosinophils. Moreover, PDE4 inhibition decreases the release of the cytokine Tumor Necrosis Factor α (TNFα). The biology of PDE4 is described in several recent reviews, for example M. D. Houslay, Prog. Nucleic Acid Res. Mol. Biol. 2001, 69, 249-315; J. E. Souness et al. Immunopharmacol. 2000 47, 127-162; or M. Conti and S. L. Jin, Prog. Nucleic Acid Res. Mol. Biol. 1999, 63, 1-38.

In view of these physiological effects, PDE4 inhibitors of varied chemical structures have been recentlty disclosed for the treatment or prevention of chronic and acute inflammatory diseases and of other pathological conditions, diseases and disorders known to be susceptible to amelioration by inhibition of PDE4. See, for example; US 5449686, US 5710170, WO 98/45268, WO 99/06404, WO 01/57025, WO 01/57036, WO 01/46184, WO 97/05105, WO 96/40636, US 5786354, US 5773467, US 5753666, US 5728712, US 5693659, US 5679696, US 5596013, US 5541219, US 5508300, US 5502072 or H. J. Dyke and J. G. Montana, Exp. Opin. Invest. Drugs 1999, 8, 1301-1325.

A few compounds having the capacity to selectively inhibit phosphodiesterase 4 are in active development. Examples of these compounds are cipamfylline, arofyline, cilomilast, roflumilast, mesopram and pumafentrine.

We have now found that a novel series of pyridothienopyrimidine derivatives are potent and selective inhibitors of PDE4 and are therefore useful in the treatment or prevention of these pathological conditions, diseases and disorders, in particular asthma, chronic obstructive pulmonary disease, rheumatoid arthritis, atopic dermatitis, psoriasis or irritable bowel disease.

The compounds of the present invention can also be used in combination with other drugs known to be effective in the treatment of these diseases. For example, they can be used in combination with steroids or immunosuppressive agents, such as cyclosporin A, rapamycin or T-cell receptor blockers. In this case the administration of the compounds allows a reduction of the dosage of the other drugs, thus preventing the appearance of the undesired side effects associated with both steroids and immunosuppressants.

Like other PDE4 inhibitors (see references above) the compounds of the invention can also be used for blocking the ulcerogenic effects induced by a variety of etiological agents, such as antiinflammatory drugs (steroidal or non-steroidal antiinflammatory agents), stress, ammonia, ethanol and concentrated acids. They can be used alone or in combination with antacids and/or antisecretory drugs in the preventive and/or curative treatment of gastrointestinal pathologies like drug-induced ulcers, peptic ulcers, H. Pylori-related ulcers, esophagitis and gastro-esophageal reflux disease.

They can also be used in the treatment of pathological situations where damage to the cells or tissues is produced through conditions like anoxia or the production of an excess of free radicals. Examples of such beneficial effects are the protection of cardiac tissue after coronary artery occlusion or the prolongation of cell and tissue viability when the compounds of the invention are added to preserving solutions intended for storage of transplant organs or fluids such as blood or sperm. They are also of benefit on tissue repair and wound healing.

The Journal of General Chemistry of the USSR, vol 20, p2216-24 and p2225-30 (1984) describes synthesis of pyridothienopyrimidine compounds. EP-A-1167367 describes synthesis of thienopyrimidine compounds. US 6,420,368 describes synthesis of thienopyrimidine compounds. US 6,495,557 describes synthesis of thienopyrimidine compounds.

Accordingly, the present invention provides the use of a pyridothienopyrimidine derivative of formula (I) 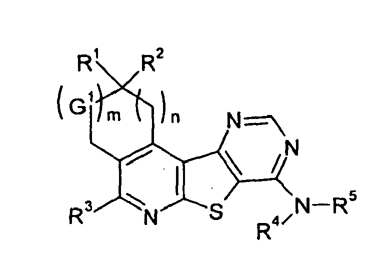 and the pharmaceutically acceptable salts and N-oxides thereof, wherein
G¹ represents a group selected from -CR⁶R⁷- or -NR⁶, R⁶ and R⁷ being independently selected from hydrogen atoms and C₁₋₄ alkyl groups
m and n are integers selected from 0 or 1
R¹ and R² are independently selected from hydrogen atoms and C₁₋₄ alkyl groups
R³ represents a group selected from C₁ to C₂₀ alkyl, amino, mono(C₁ to C₁₀ alkyl)amino, di(C₁ to C₁₀ alkyl)amino, C₅ to C₁₄ aryl, 5- to 14- membered heteroaryl and saturated N-containing 3- to 10- membered heterocyclyl groups bound through the nitrogen atom to the pyridine ring, all of them being optionally substituted by one or more substituents selected from the group consisting of halogen atoms and C₁ to C₂₀ alkyl, (C₁ to C₁₀ alkoxy)(C₁ to C₂₀ alkyl), (C₅ to C₁₄ aryl)(C₁ to C₂₀ alkyl), R⁸OCO-, C₁ to C₁₀ alkoxy, R⁸R⁹N-CO-, -CN, -CF₃, -NR⁸R⁹, -SR⁸ and -SO₂NH₂ groups wherein R⁸ and R⁹ are independently selected from hydrogen atoms and C₁₋₄ alkyl groups
R⁴ and R⁵ are independently selected from the group consisting of hydrogen atoms, C₁ to C₂₀ alkyl groups and groups of formula (II): 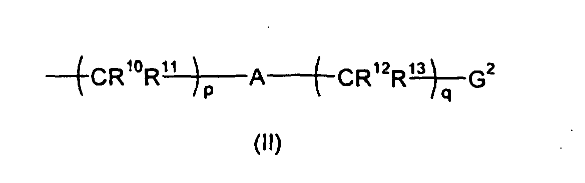 wherein p and q are integers selected from 1, 2 and 3; A is either a direct bond or a group selected from -CONR¹⁴-, -NR¹⁴CO-, -O-, -COO-, -OCO-, -NR¹⁴COO-, -OCONR¹⁴-,-NR¹⁴CONR¹⁵-, -S-, -SO-, -SO₂-, -COS- and -SCO-; and G² is a group selected from C₅ to C₁₄ aryl, 5- to 14- membered heteroaryl or 3- to 10- membered heterocyclyl; wherein the C₁ to C₂₀ alkyl groups and the group G² are optionally substituted by one or more substituents selected from group consisting of halogen atoms and C₁ to C₂₀ alkyl, (C₁ to C₁₀ alkoxy)(C₁ to C₂₀ alkyl), (C₅ to C₁₄ aryl)(C₁ to C₂₀ alkyl), R¹⁶OCO-, hydroxy, C₁ to C₁₀ alkoxy, oxo, R¹⁶R¹⁷NCO-, -CN, -CF₃, -NR¹⁶R¹⁷, -SR¹⁶ and -SO₂NH₂ groups; wherein R¹⁰ to R¹⁷ are independently selected from hydrogen atoms and C₁₋₄ alkyl groups;
in the manufacture of a medicament for the treatment or prevention of a pathological condition or disease which is asthma, chronic obstructive pulmonary disease, rheumatoid arthritis, atopic dermatitis, psoriasis or irritable bowel syndrome.

Among the compounds of formula (I), those wherein R¹ is a C₁₋₄ alkyl group are new. Accordingly further objectives of the present invention are pyridothienopyrimidine derivative of formula (I) 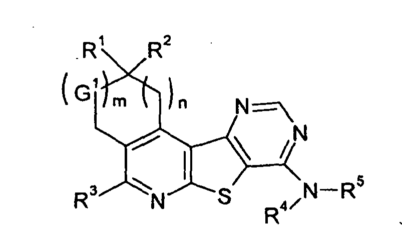 and the pharmaceutically acceptable salts and N-oxides thereof, wherein
G¹ represents a group selected from -CR⁶R⁷- or -NR⁶, R⁶ and R⁷ being independently selected from hydrogen atoms or C₁₋₄ alkyl groups
m and n are integers selected from 0 or 1
R¹ is selected from C₁₋₄ alkyl groups
R² is selected from hydrogen atoms and C₁₋₄ alkyl groups
R³ represents a group selected from C₁ to C₂₀ alkyl, amino, mono(C₁, to C₁₀ alkyl)amino, di(C₁ to C₁₀ alkyl)amino, C₅ to C₁₄ aryl, 5- to 14- membered heteroaryl and saturated N-containing 3- to 10- membered heterocyclyl groups bound through the nitrogen atom to the pyridine ring, all of them being optionally substituted by one or more substituents selected from the group consisting of halogen atoms and C₁ to C₂₀ alkyl, (C₁ to C₁₀ alkoxy)(C₁ to C₂₀ alkyl), (C₅ to C₁₄ aryl)(C₁ to C₂₀ alkyl), R⁸OCO-, C₁ to C₁₀ alkoxy, R⁸R⁹NCO-, -CN, -CF₃, -NR⁸R⁹, -SR⁸ and -SO₂NH₂ groups wherein R⁸ and R⁹ are independently selected from hydrogen atoms or C₁₋₄ alkyl groups
R⁴ and R⁵ are independently selected from the group consisting of hydrogen atoms alkyl groups and groups of formula (II): 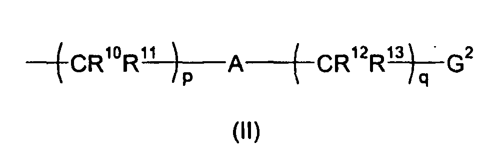 wherein p and q are integers selected from 1, 2 and 3; A is either a direct bond or a group selected from -CONR¹⁴-, -NR¹⁴CO-, -O-, -COO-, -OCO-, -NR¹⁴COO-, -OCONR¹⁴-,-NR¹⁴CONR¹⁵-,-S-, -SO-, -SO₂-, -COS- and -SCO-; and G² is a group selected from C₅ to C₁₄ aryl, 5- to 14- membered heteroaryl or 3- to 10- membered heterocyclyl; wherein the C₁ to C₂₀ alkyl groups and the group G² are optionally substituted by one or more substituents selected from group consisting of halogen atoms and C₁ to C₂₀ alkyl, (C₁ to C₁₀ alkoxy)(C₁ to C₂₀ alkyl), (C₅ to C₁₄ aryl)(C₁ to C₂₀ alkyl), R¹⁶OCO-, hydroxy, C₁ to C₁₀ alkoxy, oxo, R¹⁶R¹⁷NCO-, -CN, -CF₃, -NR¹⁶R¹⁷, -SR¹⁶ and -SO₂NH₂ groups; wherein R¹⁰ to R¹⁷ are independently selected from hydrogen atoms or C₁₋₄ alkyl groups
and the pharmaceutically acceptable salts and N-oxides thereof.

Still further objectives of the present invention are to provide processes for preparing said compounds and pharmaceutical compositions comprising an effective amount of said compounds.

As used herein the term alkyl embraces optionally substituted, linear or branched radicals having 1 to 20 carbon atoms or, preferably 1 to 12 carbon atoms. More preferably alkyl radicals are "lower alkyl" radicals having 1 to 8, preferably 1 to 6 and more preferably 1 to 4 carbon atoms.

Examples include methyl, ethyl, n-propyl, i-propyl, n-butyl, sec-butyl, t-butyl, n-pentyl, 1-methylbutyl, 2-methylbutyl, isopentyl, 1-ethylpropyl, 1,1-dimethylpropyl, 1,2-dimethylpropyl, n-hexyl, 1-ethylbutyl, 2-ethylbutyl, 1,1-dimethylbutyl, 1,2-dimethylbutyl, 1,3-dimethylbutyl, 2,2-dimethylbutyl, 2,3-dimethylbutyl, 2-methylpentyl, 3-methylpentyl and iso-hexyl radicals.

When it is mentioned that alkyl radicals may be optionally subsituted it is meant to include linear or branched alkyl, alkenyl or alkynyl radicals as defined above, which may be unsubstituted or substituted in any position by one or more substituents, for example by 1, 2 or 3 substituents. When two or more substituents are present, each substituent may be the same or different.

A said optionally substituted alkyl group is typically unsubstituted or substituted with 1, 2 or 3 substituents which may be the same or different. The substituents are preferably selected from halogen atoms, preferably fluorine atoms, hydroxy groups and alkoxy groups having from 1 to 4 carbon atoms. Typically, substituents on an alkyl group are themselves unsubstituted. Preferred optionally substituted alkyl groups are unsubstituted or substituted with 1, 2 or 3 fluorine atoms.

As used herein, the term alkoxy (or alkyloxy) embraces optionally substituted, linear or branched oxy-containing radicals each having alkyl portions of 1 to 10 carbon atoms. More preferred alkoxy radicals are "lower alkoxy" radicals having 1 to 8, preferably 1 to 6 and more preferably 1 to 4 carbon atoms.

An alkoxy group is typically unsubstituted or substituted with 1, 2 or 3 substituents which may be the same or different. The substituents are preferably selected from halogen atoms, preferably fluorine atoms, hydroxy groups and alkoxy groups having from 1 to 4 carbon atoms. Typically, the substituents on an alkoxy group are themselves unsubstituted.

Preferred alkoxy radicals include methoxy, ethoxy, n-propoxy, i-propoxy, n-butoxy, sec-butoxy, t-butoxy, trifluoromethoxy, difluoromethoxy, hydroxymethoxy, 2-hydroxyethoxy and 2-hydroxypropoxy.

As used herein, the term monoalkylamino embraces radicals containing an optionally substituted, linear or branched alkyl radicals of 1 to 10 carbon atoms attached to a divalent -NH- radical. More preferred monoalkylamino radicals are "lower monoalkylamino" radicals having 1 to 8, preferably 1 to 6 and more preferably 1 to 4 carbon atoms.

A monoalkylamino group typically contains an alkyl group which is unsubstituted or substituted with 1, 2 or 3 substituents which may be the same or different. The substituents are preferably selected from halogen atoms, preferably fluorine atoms, hydroxy groups and alkoxy groups having from 1 to 4 carbon atoms. Typically, the substitutents on a monoalkylamino group are themselves unsubstituted.

Preferred optionally substituted monoalkylamino radicals include methylamino, ethylamino, n-propylamino, i-propylamino, n-butylamino, sec-butylamino, t-butylamino, trifluoromethylamino, difluoromethylamino, hydroxymethylamino, 2-hydroxyethylamino and 2-hydroxypropylamino.

As used herein, the term dialkylamino embraces radicals containing a trivalent nitrogen atoms with two optionally substituted, linear or branched alkyl radicals of 1 to 10 carbon atoms attached thereto. More preferred dialkylamino radicals are "lower dialkylamino" radicals having 1 to 8, preferably 1 to 6 and more preferably 1 to 4 carbon atoms in each alkyl radical.

A dialkylamino group typically contains two alkyl groups, each of which is unsubstituted or substituted with 1, 2 or 3 substituents which may be the same or different. The substituents are preferably selected from halogen atoms, preferably fluorine atoms, hydroxy groups and alkoxy groups having from 1 to 4 carbon atoms. Typically, the substituents on a dialkylamino group are themselves unsubstituted.

Preferred optionally substituted dialkylamino radicals include dimethylamino, diethylamino, methyl(ethyl)amino, di(n-propyl)amino, n-propyl(methyl)amino, n-propyl(ethyl)amino, di(i-propyl)amino, i-propyl(methyl)amino, i-propyl(ethyl)amino, di(n-butyl)amino, n-butyl(methyl)amino, n-butyl(ethyl)amino, n-butyl(i-propyl)amino, di(sec-butyl)amino, sec-butyl(methyl)amino, sec-butyl(ethyl)amino, sec-butyl(n-propyl)amino, sec-butyl(i-propyl)amino, di(t-butyl)amino, t-butyl(methyl)amino, t-butyl(ethyl)amino, t-butyl(n-propyl)amino, t-butyl(i-propyl)amino, trifluoromethyl(methyl)amino, trifluoromethyl(ethyl)amino, trifluoromethyl(n-propyl)amino, trifluoromethyl(i-propyl)amino, trifluoromethyl(n-butyl)amino, trifluoromethyl(sec-butyl)amino, difluoromethyl(methyl)amino, difluoromethyl(ethyl)amino, difluoromethyl(n-propyl)amino, difluoromethyl(i-propyl)amino, difluoromethyl(n-butyl))amino, difluoromethyl(sec-butyl)amino, difluoromethyl(t-butyl)amino, difluoromethyl(trifluoromethyl)amino, hydroxymethyl(methyl)amino, ethyl(hydroxymethyl)amino, hydroxymethyl(n-propyl)amino, hydroxymethyl(i-propyl)amino, n-butyl(hydroxymethyl)amino, sec-butyl(hydroxymethyl)amino, t-butyl(hydroxymethyl)amino; difluoromethyl(hydroxymethyl)amino, hydroxymethyl(trifluoromethyl)amino, hydroxyethyl(methyl)amino, ethyl(hydroxyethyl)amino, hydroxyethyl(n-propyl)amino, hydroxyethyl(i-propyl)amino, n-butyl(hydroxyethyl)amino, sec-butyl(hydroxyethyl)amino, t-butyl(hydroxyethyl)amino, difluoromethyl(hydroxyethyl)amino, hydroxyethyl(trifluoromethyl)amino, hydroxypropyl(methyl)amino, ethyl(hydroxypropyl)amino, hydroxypropyl(n-propyl)amino, hydroxypropyl(i-propyl)amino, n-butyl(hydroxypropyl)amino, sec-butyl(hydroxypropyl)amino, t-butyl(hydroxypropyl)amino, difluoromethyl(hydroxypropyl)amino, hydroxypropyl(trifluoromethyl)amino.

As used herein, the term aryl radical embraces a C₅-C₁₄ monocyclic or polycyclic aryl radical such as phenyl, naphthyl, anthranyl and phenanthryl. Phenyl is preferred.

A said optionally substituted aryl radical is typically unsubstituted or substituted with 1, 2 or 3 substituents which may be the same or different. The substituents are preferably selected from halogen atoms, preferably fluorine atoms, hydroxy groups, alkoxycarbonyl groups in which the alkyl moiety has from 1 to 4 carbon atoms, hydroxycarbonyl groups, carbamoyl groups, nitro groups, cyano groups, C₁-C₄ alkyl groups, C₁-C₄ alkoxy groups and C₁-C₄ hydroxyalkyl groups. When an aryl radical carries 2 or more substituents, the substituents may be the same or different. Unless otherwise specified, the substituents on an aryl group are typically themselves unsubstituted.

As used herein, the term heteroaryl radical embraces a 5- to 14- membered ring system, preferably a 5- to 10- membered ring system, comprising at least one heteroaromatic ring and containing at least one heteroatom selected from O, S and N. A heteroaryl radical may be a single ring or two or more fused rings wherein at least one ring contains a heteroatom.

A said optionally substituted heteroaryl radical is typically unsubstituted or substituted with 1, 2 or 3 substituents which may be the same or different. The substituents are preferably selected from halogen atoms, preferably fluorine, chlorine or bromine atoms, alkoxycarbonyl groups in which the alkyl moiety has from 1 to 4 carbon atoms, nitro groups, hydroxy groups, C₁-C₄ alkyl groups and C₁-C₄ alkoxy groups. When an heteroaryl radical carries 2 or more substituents, the substituents may be the same or different. Unless otherwise specified, the substituents on a heteroaryl radical are typically themselves unsubstituted.

Examples include pyridyl, pyrazinyl, pyrimidinyl, pyridazinyl, furyl, benzofuranyl, oxadiazolyl, oxazolyl, isoxazolyl, benzoxazolyl, imidazolyl, benzimidazolyl, thiazolyl, thiadiazolyl, thienyl, pyrrolyl, pyridinyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, triazolyl, indolizinyl, indolinyl, isoindolinyl, isoindolyl, imidazolidinyl, pteridinyl, thianthrenyl, pyrazolyl, 2H-pyrazolo[3,4-d]pyrimidinyl, 1H-pyrazolo[3,4-d]pyrimidinyl, thieno[2,3-d] pyrimidnyl and the various pyrrolopyridyl radicals.

Oxadiazolyl, oxazolyl, pyridyl, pyrrolyl, imidazolyl, thiazolyl, thiadiazolyl, thienyl, furanyl, quinolinyl, isoquinolinyl, indolyl, benzoxazolyl, naphthyridinyl, benzofuranyl, pyrazinyl, pyrimidinyl and the various pyrrolopyridyl radicals are preferred.

As used herein, the term heterocyclyl radical embraces a non-aromatic, saturated or unsaturated C₃-C₁₀ carbocyclic ring , such as a 5, 6 or 7 membered radical, in which one or more, for example 1, 2, 3 or 4 of the carbon atoms preferably 1 or 2 of the carbon atoms are replaced by a heteroatom selected from N, O and S. Saturated heterocyclyl radicals are preferred. A heterocyclic radical may be a single ring or two or more fused rings wherein at least one ring contains a heteroatom. When a heterocyclyl radical carries 2 or more substituents, the substituents may be the same or different. A N-containing heterocyclyl radical is an heterocyclyl radical in which at least one carbon atom of the carbocyclyl ring is replaced by a nitrogen atom.

A said optionally substituted heterocyclyl radical is typically unsubstituted or substituted with 1, 2 or 3 substituents which may be the same or different. The substituents are preferably selected from halogen atoms, preferably fluorine atoms, hydroxy groups and alkoxy groups having from 1 to 4 carbon atoms. Typically, the substituents on a heterocyclyl radical are themselves unsubstituted.

Examples of heterocyclic radicals include piperidyl, pyrrolidyl, pyrrolinyl, piperazinyl, morpholinyl, thiomorpholinyl, pyrrolyl, pyrazolinyl, pirazolidinyl, quinuclidinyl, triazolyl, pyrazolyl, tetrazolyl, cromanyl, isocromanyl, imidazolidinyl, imidazolyl, oxiranyl, azaridinyl, 4,5-dihydro-oxazolyl and 3-aza-tetrahydrofuranyl. Prefered heterocyclyl radicals are selected from piperidyl, pyrrolidyl, piperazinyl, morpholinyl and thiomorpholinyl.

Where a heterocyclyl radical carries 2 or more substituents, the substituents may be the same or different.

As used herein, some of the atoms, radicals, moieties, chains and cycles present in the general structures of the invention are "optionally substituted". This means that these atoms, radicals, moieties, chains and cycles can be either unsubstituted or substituted in any poisition by one or more, for example 1, 2, 3 or 4, substituents, whereby the hydrogen atoms bound to the unsubstituted atoms, radicals, moieties, chains and cycles are replaced by chemically acceptable atoms, radicals, moieties, chains and cycles. When two or more substituents are present, each substituent may be the same or different. The substituents are typically themselves unsubstituted.

As used herein, the term halogen atom embraces chlorine, fluorine, bromine and iodine atoms. A halogen atom is typically a fluorine, chlorine or bromine atom, most preferably chlorine or fluorine. The term halo when used as a prefix has the same meaning.

Compounds containing one or more chiral centre may be used in enantiomerically or diastereoisomerically pure form, or in the form of a mixture of isomers.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

As used herein, an N-oxide is formed from the tertiary basic amines or imines present in the molecule, using a convenient oxidising agent.

It is one embodiment of the present invention the use of the compounds of formula (I) wherein G¹ is a group -CR⁶R⁷- wherein R⁶ and R⁷ are independently selected from hydrogen atoms and C₁₋₄ alkyl groups, preferably a group -CH₂- in the manufacture of a medicament for the treatment or prevention of a disorder which is asthma, chronic obstructive pulmonary disease, rheumatoid arthritis, atopic dermatitis, psoriasis or irritable bowel disease.

It is another embodiment of the present invention the use of the compounds of formula (I) wherein R¹ and R² are both methyl groups in the manufacture of a medicament for the treatment or prevention of a disorder which is asthma, chronic obstructive pulmonary disease, rheumatoid arthritis, atopic dermatitis, psoriasis or irritable bowel disease.

It is still another embodiment of the present invention the use of the compounds of formula (I) wherein m and n have both the value of 1; in the manufacture of a medicament for the treatment or prevention of a disorder which is asthma, chronic obstructive pulmonary disease, rheumatoid arthritis, atopic dermatitis, psoriasis or irritable bowel disease.

It is also another embodiment of the present invention the use of the compounds of formula (I) wherein R³ is selected from mono(C₁ to C₁₀ alkyl)amino, di(C₁ to C₁₀ alkyl)amino and saturated N-containing 3- to 10- membered heterocyclyl groups bound through the nitrogen atom to the pyridine ring, all of them being optionally substituted by one or more substituents selected from the group consisting of halogen atoms and C₁ to C₂₀ alkyl, (C₁ to C₁₀ alkoxy)(C₁ to C₂₀ alkyl), (C₅ to C₁₄ aryl)(C₁ to C₂₀ alkyl), R⁸OCO-, C₁ to C₁₀ alkoxy, R⁸R⁹NCO-, -CN, -CF₃, -NR⁸R⁹, -SR⁸ and -SO₂NH₂ groups wherein R⁸ and R⁹ are independently selected from hydrogen atoms and C₁₋₄ alkyl groups; in the manufacture of a medicament for the treatment or prevention of a disorder which is asthma, chronic obstructive pulmonary disease, rheumatoid arthritis, atopic dermatitis, psoriasis or irritable bowel disease.

It is still another embodiment of the present invention the use of the compounds of formula (I) wherein R³ is selected from mono(C₁ to C₁₀ alkyl)amino, di(C₁ to C₁₀ alkyl)amino and saturated N-containing 3- to 10- membered heterocyclyl groups bound through the nitrogen atom to the pyridine ring, all of them being non substituted; in the manufacture of a medicament for the treatment or prevention of a disorder which is asthma, chronic obstructive pulmonary disease, rheumatoid arthritis, atopic dermatitis, psoriasis or irritable bowel disease.

It is still another embodiment of the present invention the use of the compounds of formula (I) wherein R⁴ is a hydrogen atom; in the manufacture of a medicament for the treatment or prevention of a disorder which is asthma, chronic obstructive pulmonary disease, rheumatoid arthritis, atopic dermatitis, psoriasis or irritable bowel disease.

It is another embodiment of the present invention the use of a compound of formula (I) wherein R⁵ is a group of formula (III) 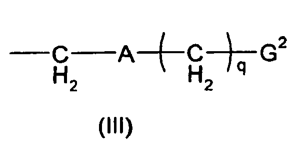 wherein q is an integer selected from 1 or 2, A represents a direct bond or a group - CONH- and G² is a group selected from C₅ to C₁₄ aryl, 5- to 14- membered heteroaryl or 3- to 10- membered heterocyclyl; wherein the group G² is optionally substituted by one or more substituents selected from group consisting of halogen atoms and C₁ to C₂₀ alkyl, (C₁ to C₁₀ alkoxy)(C₁ to C₂₀ alkyl), (C₅ to C₁₄ aryl)(C₁ to C₂₀ alkyl), R¹⁶OCO-, C₁ to C₁₀ alkoxy, R¹⁶R¹⁷NCO-, -CN, -CF₃, -NR¹⁶R¹⁷, -SR¹⁶ and -SO₂NH₂ groups; wherein R¹⁶ and R¹⁷ are independently selected from hydrogen atoms and C₁₋₄ alkyl groups; in the manufacture of a medicament for the treatment or prevention of a disorder which is asthma, chronic obstructive pulmonary disease, rheumatoid arthritis, atopic dermatitis, psoriasis or irritable bowel disease.

It is another embodiment of the present invention to use compounds of formula (I) wherein the group G² are optionally substituted by one or more substituents selected from group consisting of halogen atoms and alkyl, alkoxyalkyl, arylalkyl, R¹⁶OCO-, hydroxy, alkoxy, oxo, R¹⁶R¹⁷N-CO-, -CN, -CF₃, -NR¹⁶R¹⁷, -SR¹⁶ and -SO₂NH₂ groups; wherein R¹⁶ and R¹⁷ are independently selected from hydrogen atoms and C₁₋₄ alkyl groups.

It is yet another embodiment of the present invention to use the compounds of formula (I) wherein R⁵ is a group of formula (III) 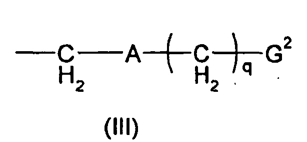 q is an integer selected from 1 or 2, A represents a direct bond or a group -CONH- and G² is optionally substituted by one or more substituents selected from group consisting of halogen atoms and C₁ to C₁₀ alkoxy and R¹⁶OCO- groups; wherein R¹⁶ is selected from hydrogen atoms and C₁₋₄ alkyl groups; in the manufacture of a medicament for the treatment or prevention of a disorder which is asthma, chronic obstructive pulmonary disease, rheumatoid arthritis, atopic dermatitis, psoriasis or irritable bowel disease.

It is a particularly preferred embodiment of the present invention to use the compounds of formula (I) wherein G¹ is a -CH₂- group, R¹ and R² are both methyl groups, m and n have both the value of 1, R³ is selected from mono(C₁ to C₁₀ alkyl)amino, di(C₁ to C₁₀ alkyl)amino and saturated N-containing 3- to 10- membered heterocyclyl groups bound through the nitrogen atom to the pyridine ring, all of them being non substituted, R⁴ is a hydrogen atom and R⁵ is a group of formula (III) 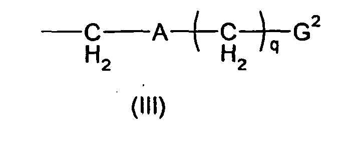 wherein q is an integer selected from 1 or 2, A represents a direct bond or a group - CONH- and G² is a group selected from C₅ to C₁₄ aryl, 5- to 14- membered heteroaryl or 3- to 10- membered heterocyclyl; wherein the group G² is optionally substituted by one or more substituents selected from group consisting of halogen atoms and C₁ to C₈ alkoxy, oxo and R¹⁶OCO- groups; wherein R¹⁶ is as hereinabove defined; in the manufacture of a medicament for the treatment or prevention of a disorder which is asthma, chronic obstructive pulmonary disease, rheumatoid arthritis, atopic dermatitis, psoriasis or irritable bowel disease.

It is another embodiment of the present invention the compounds of formula (I) wherein R¹ is selected from C₁₋₄ alkyl groups and the m, n, G¹, R², R³, R⁴ and R⁵ are as hereinabove defined.

According to another embodiment of the present invention in the compounds of formula (I) R¹ is selected from C₁₋₄ alkyl groups and G¹ is a group -CR⁶R⁷- wherein R⁶ and R⁷ are independently selected from hydrogen atoms and C₁₋₄ alkyl groups, preferably a group - CH₂-.

According to another embodiment of the present invention in the compounds of formula (I) R¹ and R² are both methyl groups.

According to still another embodiment of the present invention in the compounds of formula (I) R¹ is selected from C₁₋₄ alkyl groups and m and n have both the value of 1.

According to still another embodiment of the present invention in the compounds of formula (I) R¹ is selected from C₁₋₄ alkyl groups and R³ is selected from mono(C₁ to C₁₀ alkyl)amino, di(C₁ to C₁₀ alkyl)amino and saturated N-containing 3- to 10- membered heterocyclyl groups bound through the nitrogen atom to the pyridine ring, all of them being optionally substituted by one or more substituents selected from the group consisting of halogen atoms and C₁ to C₂₀ alkyl, (C₁ to C₁₀ alkoxy)(C₁ to C₂₀ alkyl), (C₅ to C₁₄ aryl)(C₁ to C₂₀ alkyl), R⁸OCO-, alkoxy, R⁸R⁹NCO-, -CN, -CF₃, -NR⁸R⁹, -SR⁸ and -SO₂NH₂ groups wherein R⁸ and R⁹ are independently selected from hydrogen atoms or C₁₋₄ alkyl groups. More preferably R³ is selected from mono(C₁ to C₁₀ alkyl)amino, di(C₁ to C₁₀ alkyl)amino and saturated N-containing 3- to 10- membered heterocyclyl groups bound through the nitrogen atom to the pyridine ring, all of them being non substituted.

According to still another embodiment of the present invention in the compounds of formula (I) R¹ is selected from C₁₋₄ alkyl groups and R⁴ is a hydrogen atom.

According to still another embodiment of the present invention in the compounds of formula (I) R¹ is selected from C₁₋₄ alkyl groups and R⁵ is a group of formula (III) 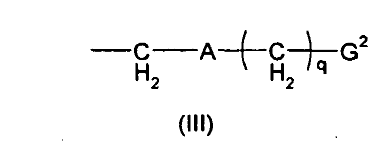 wherein q is an integer selected from 1 or 2, A represents a direct bond or a group - CONH- and G² is a group selected from C₅ to C₁₄ aryl, 5- to 14- membered heteroaryl or 3- to 10- membered heterocyclyl; wherein the group G² is optionally substituted by one or more substituents selected from group consisting of halogen atoms and C₁ to C₂₀ alkyl, (C₁ to C₁₀ alkoxy)(C₁ to C₂₀ alkyl), (C₅ to C₁₄ aryl)(C₁ to C₂₀ alkyl), R¹⁶OCO-, hydroxy, C₁ to C₁₀ alkoxy, oxo, R¹⁶R¹⁷N-CO-, -CN, -CF₃, -NR¹⁶R¹⁷, -SR¹⁶ and -SO₂NH₂ groups; wherein R¹⁶ and R¹⁷ are independently selected from hydrogen atoms or C₁₋₄ alkyl groups.

According to another embodiment of the present invention in the compounds of formula (I) R¹ is selected from C₁₋₄ alkyl groups and R⁵ is a group of formula (III) 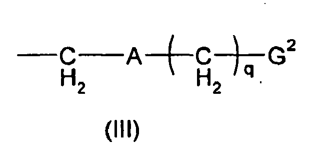 wherein q is an integer selected from 1 or 2, A represents a direct bond or a group - CONH- and G² is optionally substituted by one or more substituents selected from group consisting of halogen atoms and C₁ to C₁₀ alkoxy and R¹⁶OCO- groups; wherein R¹⁶ is selected from hydrogen atoms and C₁₋₄ alkyl groups.

In a particularly preferred embodiment of the present invention in the compounds of formula (I) R¹ is selected from C₁₋₄ alkyl groups, G¹ is a -CH₂- group, R¹ and R² are both methyl groups, m and n have both the value of 1, R³ is selected from mono(C₁ to C₁₀ alkyl)amino, di(C₁ to C₁₀ alkyl)amino and saturated N-containing 3- to 10- membered heterocyclyl groups bound through the nitrogen atom to the pyridine ring, all of them being non substituted, R⁴ is a hydrogen atom and R⁵ is a group of formula (III) 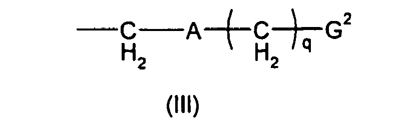 wherein q is an integer selected from 1 or 2, A represents a direct bond or a group - CONH- and G² is a group selected from C₅ to C₁₄ aryl, 5- to 14- membered heteroaryl or 3- to 10- membered heterocyclyl; wherein the group G² is optionally substituted by one or more substituents selected from group consisting of halogen atoms and C₁ to C₁₀ alkoxy, oxo and R¹⁶OCO- groups; wherein R¹⁶ is selected from hydrogen atoms or C₁₋₄ alkyl groups.

Particular individual compounds of the invention include:
2,2-Dimethyl-5-morpholin-4-yl-N-(2-morpholin-4-ylethyl)-1,2,3,4-tetrahydropyrimido[4',5':4,5]thieno[2,3-c]isoquinolin-8-amine
2,2-Dimethyl-5-morpholin-4-yl-N-(pyridin-4-ylmethyl)-1,2,3,4-tetrahydropyrimido[4',5':4,5]thieno[2,3-c]isoquinolin-8-amine
2,2-Dimethyl-5-morpholin-4-yl-N-(pyridin-3-ylmethyl)-1,2,3,4-tetrahydropyrimido[4',5':4,5]thieno[2,3-c]isoquinolin-8-amine
2,2-Dimethyl-5-morpholin-4-yl-N-(pyridin-2-ylmethyl)-1,2,3,4-tetrahydropyrimido[4',5':4,5]thieno[2,3-c]isoquinolin-8-amine
N-(2-Methoxybenzyl)-2,2-dimethyl-5-morpholin-4-yl-1,2,3,4-tetrahydropyrimido[4',5':4,5]thieno[2,3-c]isoquinolin-8-amine
N-(2,3-Dimethoxybenzyl)-2,2-dimethyl-5-morpholin-4-yl-1,2,3,4-tetrahydropyrimido[4',5':4,5]thieno[2,3-c]isoquinolin-8-amine
4-{[(2,2-Dimethyl-5-morpholin-4-yl-1,2,3,4-tetrahydropyrimido[4',5':4,5]thieno[2,3-c]isoquinolin-8-yl)amino]methyl}benzenesulfonamide
2,2-Dimethyl-5-morpholin-4-yl-N-(2-pyridin-2-ylethyl)-1,2,3,4-tetrahydropyrimido[4',5':4,5]thieno[2,3-c]isoquinolin-8-amine
2,2-Dimethyl-5-morpholin-4-yl-N-(1-naphthylmethyl)-1,2,3,4-tetrahydropyrimido[4',5':4,5]thieno[2,3-c]isoquinolin-8-amine
N-(2-Furylmethyl)-2,2-dimethyl-5-morpholin-4-yl-1,2,3,4-tetrahydropyrimido[4',5':4,5]thieno[2,3-c]isoquinolin-8-amine
N-[2-(1H-Imidazol-4-yl)ethyl]-2,2-dimethyl-5-morpholin-4-yl-1,2,3,4-tetrahydropyrimido[4',5':4,5]thieno[2,3-c]isoquinolin-8-amine
1-{3-[(2,2-Dimethyl-5-morpholin-4-yl-1,2,3,4-tetrahydropyrimido[4',5':4,5]thieno[2,3-c]isoquinolin-8-yl)amino]propyl}pyrrolidin-2-one
2,2-Dimethyl-N-(2-morpholin-4-ylethyl)-5-thiomorpholin-4-yl-1,2,3,4-tetrahydropyrimido[4',5':4,5]thieno[2,3-c]isoquinolin-8-amine
2,2-Dimethyl-N-(pyridin-4-ylmethyl)-5-thiomorpholin-4-yl-1,2,3,4-tetrahydropyrimido[4',5':4,5]thieno[2,3-c]isoquinolin-8-amine
2,2-Dimethyl-N-(pyridin-3-ylmethyl)-5-thiomorpholin-4-yl-1,2,3,4-tetrahydropyrimido[4',5':4,5]thieno[2,3-c]isoquinolin-8-amine
2,2-Dimethyl-N-(pyridin-2-ylmethyl)-5-thiomorpholin-4-yl-1,2,3,4-tetrahydropyrimido[4',5':4,5]thieno[2,3-c]isoquinolin-8-amine
N-(2,3-Dimethoxybenzyl)-2,2-dimethyl-5-thiomorpholin-4-yl-1,2,3,4-tetrahydropyrimido[4',5':4,5]thieno[2,3-c]isoquinolin-8-amine
1-{3-[(2,2-Dimethyl-5-thiomorpholin-4-yl-1,2,3,4-tetrahydropyrimido[4',5':4,5]thieno[2,3-c]isoquinolin-8-yl)amino]propyl}pyrrolidin-2-one
Ethyl 4-{2-[(2,2-dimethyl-5-thiomorpholin-4-yl-1,2,3,4-tetrahydropyrimido[4',5':4,5]thieno[2,3-c]isoquinolin-8-yl)amino]ethyl}piperazine-1-carboxylate
2,2-Dimethyl-5-(4-methylpiperazin-1-yl)-N-(2-morpholin-4-ylethyl)-1,2,3,4-tetrahydropyrimido[4',5':4,5]thieno[2,3-c]isoquinolin-8-amine
2,2-Dimethyl-5-(4-methylpiperazin-1-yl)-N-(pyridin-3-ylmethyl)-1,2,3,4-tetrahydropyrimido[4',5':4,5]thieno[2,3-c]isoquinolin-8-amine
2,2-Dimethyl-5-(4-methylpiperazin-1-yl)-N-(pyridin-2-ylmethyl)-1,2,3,4-tetrahydropyrimido[4',5':4,5]thieno[2,3-c]isoquinolin-8-amine
2,2-Dimethyl-N-(2-morpholin-4-ylethyl)-5-pyrrolidin-1-yl-1,2,3,4-tetrahydropyrimido[4',5':4,5]thieno[2,3-c]isoquinolin-8-amine
2,2-Dimethyl-N-(pyridin-4-ylmethyl)-5-pyrrolidin-1-yl-1,2,3,4-tetrahydropyrimido[4',5':4,5]thieno[2,3-c]isoquinolin-8-amine
(2,2-Dimethyl-N-(pyridin-3-ylmethyl)-5-pyrrolidin-1-yl-1,2,3,4-tetrahydropyrimido[4',5':4,5]thieno[2,3-c]isoquinolin-8-amine
2,2-Dimethyl-N-(pyridin-2-ylmethyl)-5-pyrrolidin-1-yl-1,2,3,4-tetrahydropyrimido[4',5':4,5]thieno[2,3-c]isoquinolin-8-amine
N-(2-Methoxybenzyl)-2,2-dimethyl-5-pyrrolidin-1-yl-1,2,3,4-tetrahydropyrimido[4',5':4,5]thieno[2,3-c]isoquinolin-8-amine
N-(2,3-Dimethoxybenzyl)-2,2-dimethyl-5-pyrrolidin-1-yl-1,2,3,4-tetrahydropyrimido[4',5':4,5]thieno[2,3-c]isoquinolin-8-amine
N-(2-Furylmethyl)-2,2-dimethyl-5-pyrrolidin-1-yl-1,2,3,4-tetrahydropyrimido[4',5':4,5]thieno[2,3-c]isoquinolin-8-amine
N-[2-(1H-Imidazol-4-yl)ethyl]-2,2-dimethyl-5-pyrrolidin-1-yl-1,2,3,4-tetrahydropyrimido[4',5':4,5]thieno[2,3-c]isoquinolin-8-amine
1-{3-[(2,2-Dimethyl-5-pyrrolidin-1-yl-1,2,3,4-tetrahydropyrimido[4',5':4,5]thieno[2,3-c]isoquinolin-8-yl)amino]propyl}pyrrolidin-2-one
Ethyl 4-{2-[(2,2-dimethyl-5-pyrrolidin-1-yl-1,2,3,4-tetrahydropyrimido[4',5':4,5]thieno[2,3-c]isoquinolin-8-yl)amino]ethyl}piperazine-1-carboxylate
2,2-Dimethyl-N-(2-piperazin-1-ylethyl)-5-pyrrolidin-1-yl-1,2,3,4-tetrahydropyrimido[4',5':4,5]thieno[2,3-c]isoquinolin-8-amine
2,2-Dimethyl-N-[2-(4-methylpiperazin-1-yl)ethyl]-5-pyrrolidin-1-yl-1,2,3,4-tetrahydropyrimido[4',5':4,5]thieno[2,3-c]isoquinolin-8-amine
N2-(2,2-Dimethyl-5-pyrrolidin-1-yl-1,2,3,4-tetrahydropyrimido[4',5':4,5]thieno[2,3-c]isoquinolin-8-yl)-N1-(tetrahydrofuran-2-ylmethyl)glycinamide
2,2-Dimethyl-5-pyrrolidin-1-yl-N-(quinolin-3-ylmethyl)-1,2,3,4-hydropyrimido[4',5':4,5]thieno[2,3-c]isoquinolin-8-amine
2,2-Dimethyl-5-pyrrolidin-1-yl-N-(isoquinolin-4-ylmethyl)-1,2,3,4-tetrahydropyrimido[4',5':4,5]thieno[2,3-c]isoquinolin-8-amine
N⁵,N⁵,2,2-tetramethyl-N⁸-(2-morpholin-4-ylethyl)-1,2,3,4-tetrahydropyrimido[4',5':4,5]thieno[2,3-c]isoquinoline-5,8-diamine
N⁵, N⁵,2,2-Tetramethyl-N⁸-(pyridin-3-ylmethyl)-1,2,3,4-tetrahydropyrimido[4',5':4,5]thieno[2,3-c]isoquinoline-5,8-diamine
N⁸-(2,3-Dimethoxybenzyl)-N⁵,N⁵,2,2-tetramethyl-1,2,3,4-tetrahydropyrimido[4',5':4,5]thieno[2,3-c]isoquinoline-5,8-diamine
N⁸-[2-(1H-Imidazol]-5-yl)ethyl]-N⁵,N⁵,2,2-tetramethyl-1,2,3,4-tetrahydropyrimido[4',5':4,5]thieno[2,3-c]isoquinoline-5,8-diamine
1-(3-{[5-(Dimethylamino)-2,2-dimethyl-1,2,3,4-tetrahydropyrimido[4',5':4,5]thieno[2,3-c]isoquinolin-8-yl]amino}propyl)pyrrolidin-2-one
Ethyl 4-(2-{[5-(dimethylamino)-2,2-dimethyl-1,2,3,4-tetrahydropyrimido[4',5':4,5]thieno[2,3-c]isoquinolin-8-yl]amino}ethyl)piperazine-1-carboxylate
N⁵,N⁵,2,2-Tetramethyl-N⁸-(2-piperazin-1-ylethyl)-1,2,3,4-tetrahydropyrimido[4',5':4,5]thieno[2,3-c]isoquinoline-5,8-diamine
3-{[5-(Dimethylamino)-2,2-dimethyl-1,2,3,4-tetrahydropyrimido[4',5':4,5]thieno[2,3-c]isoquinolin-8-yl]amino}propanenitrile
8-Ethoxy-N,N,2,2-tetramethyl-1,2,3,4-tetrahydropyrimido[4',5':4,5]thieno[2,3-c]isoquinolin-5-amine
N⁵-Ethyl-N⁵,2,2-trimethyl-N⁸-(2-morpholin-4-ylethyl)-1,2,3,4-tetrahydropyrimido[4',5':4,5]thieno[2,3-c]isoquinoline-5,8-diamine
N⁵-Ethyl-N⁵,2,2-trimethyl-N⁸-(pyridin-4-ylmethyl)-1,2,3,4-tetrahydropyrimido[4',5':4,5]thieno[2,3-c]isoquinoline-5,8-diamine
N⁵-Ethyl-N⁵,2,2-trimethyl-N⁸-(pyridin-3-ylmethyl)-1,2,3,4-tetrahydropyrimido[4',5':4,5]thieno[2,3-c]isoquinoline-5,8-diamine
N⁵-Ethyl-N⁵,2,2-trimethyl-N⁸-(pyridin-2-ylmethyl)-1,2,3,4-terahydropyrimido[4',5':4,5]thieno[2,3-c]isoquinoline-5,8-diamine
N⁵-Ethyl-N⁸-(2-furylmethyl)-N⁵,2,2-trimethyl-1,2,3,4-tetrahydropyrimido[4',5':4,5]thieno[2,3-c]isoquinoline-5,8-diamine
N⁵-Ethyl-N⁸-[2-(1H-imidazol-4-yl)ethyl]-N⁵,2,2-trimethyl-1,2,3,4-tetrahydropyrimido[4',5':4,5]thieno[2,3-c]isoquinoline-5,8-diamine
1-[3-({5-[Ethyl(methyl)amino]-2,2-dimethyl-1,2,3,4-tetrahydropyrimido[4',5':4,5]thieno[2,3-c]isoquinolin-8-yl}amino)propyl]pyrrolidin-2-one
N²-{5-[Ethyl(methyl)amino]-2,2-dimethyl-1,2,3,4-tetrahydropyrimido[4',5':4,5]thieno[2,3-c]isoquinolin-8-yl}-N¹-(tetrahydrofuran-2-ylmethyl)glycinamide
2,2,5-Trimethyl-N-(2-morpholin-4-ylethyl)-1,2,3,4-tetrahydropyrimido[4',5':4,5]thieno[2,3-c]isoquinolin-8-amine
2,2,5-Trimethyl-N-(pyridin-3-ylmethyl)-1,2,3,4-tetrahydropyrimido[4',5':4,5]thieno[2,3-c]isoquinolin-8-amine
1-{3-[(2,2,5-Trimethyl-1,2,3,4-tetrahydropyrimido[4',5':4,5]thieno[2,3-c]isoquinolin-8-yl)amino]propyl}pyrrolidin-2-one
5-Isobutyl-2,2-dimethyl-N-(2-morpholin-4-ylethyl)-1,2,3,4-tetrahydropyrimido[4',5':4,5]thieno[2,3-c]isoquinolin-8-amine
5-Isobutyl-2,2-dimethyl-N-(pyridin-3-ylmethyl)-1,2,3,4-tetrahydropyrimido[4',5':4,5]thieno[2,3-c]isoquinolin-8-amine
1-{3-[(5-Isobutyl-2,2-dimethyl-1,2,3,4-tetrahydropyrimido[4',5':4,5]thieno[2,3-c]isoquinolin-8-yl)amino]propyl}pyrrolidin-2-one
Ethyl 4-{2-[(5-isobutyl-2,2-dimethyl-1,2,3,4-tetrahydropyrimido[4',5':4,5]thieno[2,3-c]isoquinolin-8-yl)amino]ethyl}piperazine-1-carboxylate
5-(2-Furyl)-2,2-dimethyl-N-(2-morpholin-4-ylethyl)-1,2,3,4-tetrahydropyrimido[4',5':4,5]thieno[2,3-c]isoquinolin-8-amine
5-(2-Furyl)-2,2-dimethyl-N-(pyridin-4-ylmethyl)-1,2,3,4-tetrahydropyrimido[4',5':4,5]thieno[2,3-c]isoquinolin-8-amine
5-(2-Furyl)-2,2-dimethyl-N-(pyridin-3-ylmethyl)-1,2,3,4-tetrahydropyrimido[4',5':4,5]thieno[2,3-c]isoquinolin-8-amine
5-(2-Furyl)-2,2-dimethyl-N-(pyridin-2-ylmethyl)-1,2,3,4-tetrahydropyrimido[4',5':4,5]thieno[2,3-c]isoquinolin-8-amine
1-(3-{[5-(2-Furyl)-2,2-dimethyl-1,2,3,4-tetrahydropyrimido[4',5':4,5]thieno[2,3-c]isoquinolin-8-yl]amino}propyl)pyrrolidin-2-one
Ethyl 4-(2-{[5-(2-furyl)-2,2-dimethyl-1,2,3,4-tetrahydropyrimido[4',5':4,5]thieno[2,3-c]isoquinolin-8-yl]amino}ethyl)piperazine-1-carboxylate
5-(3-Furyl)-2,2-dimethyl-N-(2-morpholin-4-ylethyl)-1,2,3,4-tetrahydropyrimido[4',5':4,5]thieno[2,3-c]isoquinolin-8-amine
5-(3-Furyl)-2,2-dimethyl-N-(pyridin-2-ylmethyl)-1,2,3,4-tetrahydropyrimido[4',5':4,5]thieno[2,3-c]isoquinolin-8-amine
5-(3-Furyl)-2,2-dimethyl-N-(pyridin-3-ylmethyl)-1,2,3,4-tetrahydropyrimido[4',5':4,5]thieno[2,3-c]isoquinolin-8-amine
5-(3-Furyl)-2,2-dimethyl-N-(pyridin-4-ylmethyl)-1,2,3,4-tetrahydropyrimido[4',5':4,5]thieno[2,3-c]isoquinolin-8-amine
5-(3-Furyl)-N-[2-(1H-imidazol-4-yl)ethyl]-2,2-dimethyl-1,2,3,4-tetrahydropyrimido[4',5':4,5]thieno[2,3-c]isoquinolin-8-amine
1-(3-{[5-(3-Furyl)-2,2-dimethyl-1,2,3,4-tetrahydropyrimido[4',5':4,5]thieno[2,3-c]isoquinolin-8-yl]amino}propyl)pyrrolidin-2-one
Ethyl 4-(2-{[5-(3-furyl)-2,2-dimethyl-1,2,3,4-tetrahydropyrimido[4',5':4,5]thieno[2,3-c]isoquinolin-8-yl]amino}ethyl)piperazine-1-carboxylate
2,2,3-Trimethyl-5-morpholin-4-yl-N-(2-morpholin-4-ylethyl)-1,2,3,4-tetrahydropyrimido[4',5':4,5]thieno[2,3-c]-2,7-naphthyridin-8-amine
2,2,3-Trimethyl-5-morpholin-4-yl-N-(pyridin-3-ylmethyl)-1,2,3,4-tetrahydropyrimido[4',5':4,5]thieno[2,3-c]-2,7-naphthyridin-8-amine
N-(2-Methoxybenzyl)-2,2,3-trimethyl-5-morpholin-4-yl-1,2,3,4-tetrahydropyrimido[4', 5':4,5]thieno[2,3-c]-2,7-naphthyridin-8-amine
N⁴,N⁴,2,2-Tetramethyl-N⁷-(2-morpholin-4-ylethyl)-2,3-dihydro-1H-cyclopenta[4',5']pyrido[3',2':4,5]thieno[3,2-d]pyrimidin-4,7-diamine
N⁴, N⁴, 2,2-Tetramethyl-N⁷-(pyridin-3-ylmethyl)-2,3-dihydro-1H-cyclopenta[4',5']pyrido[3',2':4,5]thieno[3,2-d]pyirimidin-4,7-diamine
1-(3-{[4-(Dimethylamino)-2,2-dimethyl-2,3-dihydro-1H-cyclopenta[4',5']pyrido[3',2':4,5]thieno[3,2-d]pyrimidin-7-yl]amino}propyl)pyrrolidin-2-one
N⁷-(2-Furylmethyl)-N⁴, N⁴, 2,2-tetramethyl-2,3-dihydro-1H-cyclopenta[4',5']pyrido[3',2':4,5]thieno[3,2-d]pyrimidin-4,7-diamine
2,2-Dimethyl-4-morpholin-4-yl-7-(pyridin-3-ylmethyl)-2,3-dihydro-1H-cyclopenta[4',5']pyrido[3',2':4,5]thieno[3,2-d]pyrimidin-7-amine
N⁴, N⁴,1,1-Tetramethyl-N⁷-(2-morpholin-4-ylethyl)-2,3-dihydro-1H-cyclopenta[4',5']pyrido[3',2':4,5]thieno[3,2-d]pyrimidin-4,7-diamine
N⁴, N⁴,1,1-Tetramethyl-N⁷-(pyridin-3-ylmethyl)-2,3-dihydro-1H-cyclopenta[4',5']pyrido[3',2':4,5]thieno[3,2-d]pyrimidin-4,7-diamine
Ethyl 4-(2-{[4-(dimethylamino)-1,1-dimethyl-2,3-dihydro-1H-cyclopenta[4',5']pyrido[3',2':4,5]thieno[3,2-d]pyrimidin-7-yl]amino}ethyl)pyperazincarboxilate
1-(3-{[4-(Dimehylamino)-1,1-dimethyl-2,3-dihydro-1H-cyclopenta[4',5']pyrido[3',2':4,5]thieno[3,2-d]pyrimidin-7-yl]amino}propyl)pyrrolidin-2-one
N⁷-[2-(1H-Imidazol-4-yl)ethyl]-N⁴,N⁴,1,1-tetramethyl-2,3-dihydro-1H-cyclopenta[4',5']pyrido[3',2':4,5]thieno[3,2-d]pyrimidin-4,7-diamine
N⁷-(2-Furylmethyl)-N⁴,N⁴,1,1-tetramethyl-2,3-dihydro-1H-cyclopenta[4',5']pyrido[3',2':4,5]thieno[3,2-d]pyrimidin-4,7-diamine
N⁷-(2,3-Dimethoxybenzyl)-N⁴,N⁴,1,1-tetramethyl-2,3-dihydro-1H-cyclopenta[4',5']pyrido[3',2':4,5]thieno[3,2-d]pyrimidin-4,7-diamine
1,1-Dimethyl-4-morpholin-4-yl-N⁷-(2-morpholin-4-ylethyl)-2,3-dihydro-1H-cyclopenta[4',5']pyrido[3',2':4,5]thieno[3,2-d]pyrimidin-7-amine
1,1-Dimethyl-4-morpholin-4-yl-N7-(pyridin-3-ylmethyl)-2,3-dihydro-1H-cyclopenta[4',5']pyrido[3',2':4,5]thieno[3,2-d]pyrimidin-7-amine
1-(3-{[4-(Morpholin-4-yl)-1,1-dimethyl-2,3-dihydro-1H-cyclopenta[4',5']pyrido[3',2':4,5]thieno[3,2-d]pyrimidin-7-yl]amino}propyl)pyrrolidin-2-one
2,2-Dimethyl-7-(2-morpholin-4-ylethyl)-4-pyrrolidin-1-yl-2,3-dihydro-1H-cyclopenta[4',5']pyrido[3',2':4,5]thieno[3,2-d]pyrimidin-7-amine
2,2-Dimethyl-7-(pyridin-3-ylmethyl)-4-pyrrolidin-1-yl-2,3-dihydro-1H-cyclopenta[4',5']pyrido[3',2':4,5]thieno[3,2-d]pyrimidin-7-amine
2-[(2,2-Dimethyl-4-pyrrolidin-1-yl-2,3-dihydro-1H-cyclopenta[4',5']pyrido[3',2':4,5]thieno[3,2-d]pyrimidin-7-yl)(2-morpholin-4-ylethyl)amino]ethanol
2-[(2,2-Dimethyl-5-pyrrolidin-1-yl-1,2,3,4-tetrahydropyrimido[4',5':4,5]thieno[2,3-c]isoquinolin-8-yl)-(2-morpholin-4-ylethyl)amino]ethanol
N,N,2,2-Tetramethyl-4-pyrrolidin-1-yl-2,3-dihydro-1H-cyclopenta[4',5']pyrido[3',2':4,5]thieno[3,2-d]pyrimidin-7-amine
2,2-Dimethyl-N-(2-morpholin-4-ylethyl)-N-(pyridin-3-ylmethyl)-4-pyrrolidin-1-yl-2,3-dihydro-1H-cyclopenta[4',5']pyrido[3',2':4,5]thieno[3,2-d]pyrimidin-7-amine
N⁴-Ethyl-N⁴,2,2-trimethyl-N⁷-(2-morpholin-4-ylethyl)-2,3-dihydro-1H-cyclopenta[4',5']pyrido[3',2':4,5]thieno[3,2-d]pyrimidin-4,7-diamine
N⁴-Ethyl-N⁴,2,2-trimethyl-N⁷-(pyridin-3-ylmethyl)-2,3-dihydro-1H-cyclopenta[4',5']pyrido[3',2':4,5]thieno[3,2-d]pyrimidin-4,7-diamine
1-[3-({4-[Ethyl(methyl)amino]-2,2-dimethyl-2,3-dihydro-1H-cyclopenta[4',5']pyrido[3',2':4,5]thieno[3,2-d]pyrimidin-7-yl}amino)propyl]pyrrolidin-2-one
N⁷-(2,3-Dimethoxybenzyl)-N⁴-ethyl-N⁴,2,2-trimethyl-2,3-dihydro-1H-cyclopenta[4',5']pyrido[3',2':4,5]thieno[3,2-d]pyrimidin-4,7-diamine
2-[{4-[Ethyl(methyl)amino+-2,2-dimethyl-2,3-dihydro-1H-cyclopenta[4',5']pyrido[3',2':4,5]thieno[3,2-d]pyrimidin-7-yl}(2-morpholin-4-ylethyl)amino]ethanol
N⁴-Ethyl-N⁴,2,2-trimethyl-N⁷-(2-morpholin-4-ylethyl)-N⁷-(pyridin-3-ylmethyl)-2,3-dihydro-1H-cyclopenta[4',5']pyrido[3',2':4,5]thieno[3,2-d]pyrimidin-4,7-diamine
2,2-Dimethyl-4-morpholin-4-yl-N-(2-morpholin-4-ylethyl)-2,3-dihydro-1H-cyclopenta[4',5']pyrido[3',2':4,5]thieno[3,2-d]pyrimidin-7-amine
2-[(2,2-Dimethyl-4-morpholin-4-yl-2,3-dihydro-1H-cyclopenta[4',5']pyrido[3',2':4,5]thieno[3,2-d]pyrimidin-7-yl)(2-morpholin-4-ylethyl)amino]ethanol
N-(2,3-Dimethoxybenzyl)-2,2-dimethyl-4-morpholin-4-yl-2,3-dihydro-1H-cyclopenta[4',5']pyrido[3',2':4,5]thieno[3,2-d]pyrimidin-7-amine
1-{3-[(2,2-Dimethyl-4-morpholin-4-yl-2,3-dihydro-1H-cyclopenta[4',5']pyrido[3',2':4,5]thieno[3,2-d]pyrimidin-7-yl)amino]propyl}pyrrolidin-2-one
2-[(2,2-Dimethyl-5-morpholin-4-yl-1,2,3,4-tetrahydropyrimido[4',5':4,5]thieno[2,3-c]isoquinolin-8-yl)(2-morpholin-4-ylethyl)amino]ethanol
2-[(2,2-Dimethyl-5-morpholin-4yl-1,2,3,4-tetrahydropyrimido[4',5':4,5]thieno[2,3-c]isoquinolin-8-yl)(pyridin-3-ylmethyl)amino]ethanol
N⁵,2,2-Trimethyl-N⁸-(2-morpholin-4-ylethyl)-1,2,3,4-tetrahydropyrimido[4',5':4,5]thieno[2,3-c]isoquinoline-5,8-diamine
N⁵,2,2-Trimethyl-N⁸-(pyridin-3-ylmethyl)-1,2,3,4-tetrahydropyrimido[4',5':4,5]thieno[2,3-c]isoquinoline-5,8-diamine
1-(3-{[2,2-Dimethyl-5-(methylamino)-1,2,3,4-tetrahydropyrimido[4',5':4,5]thieno[2,3-c]isoquinolin-8-yl]amino}propyl)pyrrolidin-2-one

Of outstanding interest are:
2,2-Dimethyl-5-morpholin-4-yl-N-(2-morpholin-4-ylethyl)-1,2,3,4-tetrahydropyrimido[4',5':4,5]thieno[2,3-c]isoquinolin-8-amine
2,2-Dimethyl-N-(2-morpholin-4-ylethyl)-5-pyrrolidin-1-yl-1,2,3,4-tetrahydropyrimido[4',5':4,5]thieno[2,3-c]isoquinolin-8-amine
2,2-Dimethyl-N-(pyridin-4-ylmethyl)-5-pyrrolidin-1-yl-1,2,3,4-tetrahydropyrimido[4',5':4,5]thieno[2,3-c]isoquinolin-8-amine
2,2-Dimethyl-N-(pyridin-3-ylmethyl)-5-pyrrolidin-1-yl-1,2,3,4-tetrahydropyrimido[4',5':4,5]thieno[2,3-c]isoquinolin-8-amine
   and pharmaceutically acceptable salts thereof.

According to a further feature of the present invention, the compounds of formula (I) may be prepared by one of the processes described below.

Compounds **Ia** wherein R³ is a monosubstituted, disubstituted or unsubstituted amino group may be obtained as shown in Scheme 1.

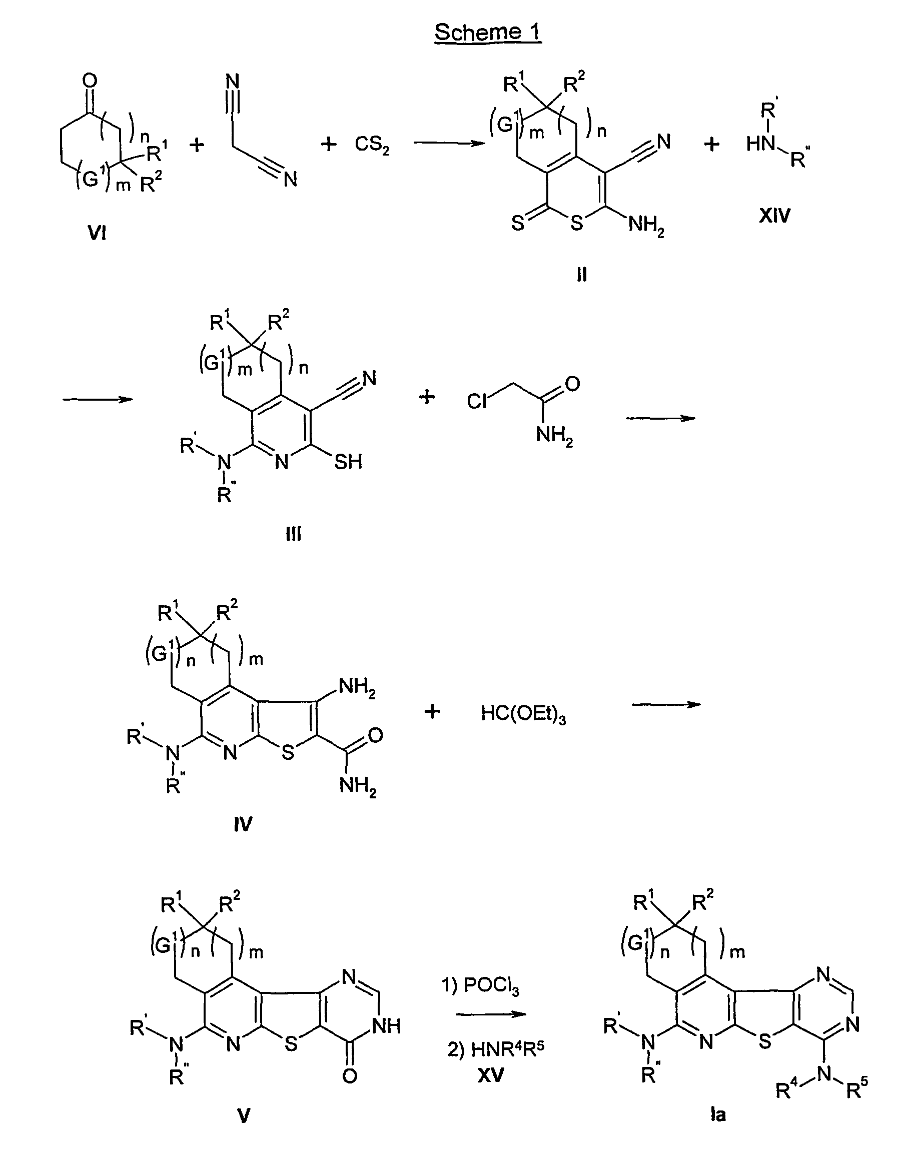

A ketone of formula **VI**, wherein G¹, m, n, R¹ and R² are as hereinbefore defined, is condensed with malononitrile in the presence of carbon disulfide to yield the heterocycle of formula II, according to the method described by E.G. Paronikyan and A.S. Noravyan at Chem. Heterocycl. Compd (NY), 1999, 35(7), 799-803. Ketones **VI** are commercially available or prepared according to the methods described at C. Ainsworth Org. Synth., 1959, 39, 536, J.Cologne, A.Varagnat Bull. Soc. Chim. France, 1964, 10, 2499-504, and E.M. Kosower, T.S.Sorensen, **1963**, *28*, 687.

Reaction of compound II with an amine HNR'R" of formula **XIV**, wherein R' and R" are as hereinbefore defined, yields the pyridine derivative III, as described by K.Gewald et al at J.Prakt.Chem., 1973, 315(4), 679-689.

Subsequent cyclocondensation of compound **III** with 2-chloroacetamide in the presence of a base such as potasium carbonate affords the thienopyridine compound **IV**, according to C.Peinador et al J.Het.Chem., 1992, 29, 1693 or C.Peinador et al Bioorg. Med. Chem., 1998, 6, 1911.

The pyridothienopyrimidine derivative V is sinthesized by cyclisation of intermediate **IV** with a orthoformate derivative HC(OR⁶)₃, wherein R⁶ is a C₁₋₄ alkyl group, as described at C.Peinador et al Bioorg.Med.Chem., 1998, 6, 1911, or formic acid or a reactive derivative thereof. The reactive derivative of the formic acid is preferably the acid halide, orthoester or anhydride. The reaction can be carried out in a solvent, preferably a polar aprotic solvent, such as N,N-dimethylformamide, dioxane, acetone or tetrahydrofuran, in the presence of an organic base, preferably an amine base, such as triethylamine and at a temperature from 15°C to 40°C. The reaction can also be carried out in the absence of a solvent, in which case an excess of formic acid or reactive derivative of formic acid is used and the mixture is heated at a temperature from 40°C to its boiling point.

The corresponding chloroimine derivative of **V** is sinthesized using phosphorous oxychloride as solvent, and the resulting intermediate is reacted with an amine of formula **XV**, wherein R⁴ and R⁵ are as hereinbefore defined, to give the desired final compound **Ia**.

When the defined groups R', R" and R¹ to R⁶ are susceptible to chemical reaction under the conditions of the hereinbefore described processes or are incompatible with said processes, conventional protecting groups may be used in accordance with standard practice, for example see T. W. Greene and P. G. M. Wuts in 'Protective Groups in Organic Chemistry', 3rd Edition, John Wiley & Sons (1999). It may be that deprotection will form the last step in the synthesis of compounds of formula **I.**

Another route for the obtention of compounds **Ib** is shown in Scheme 2.

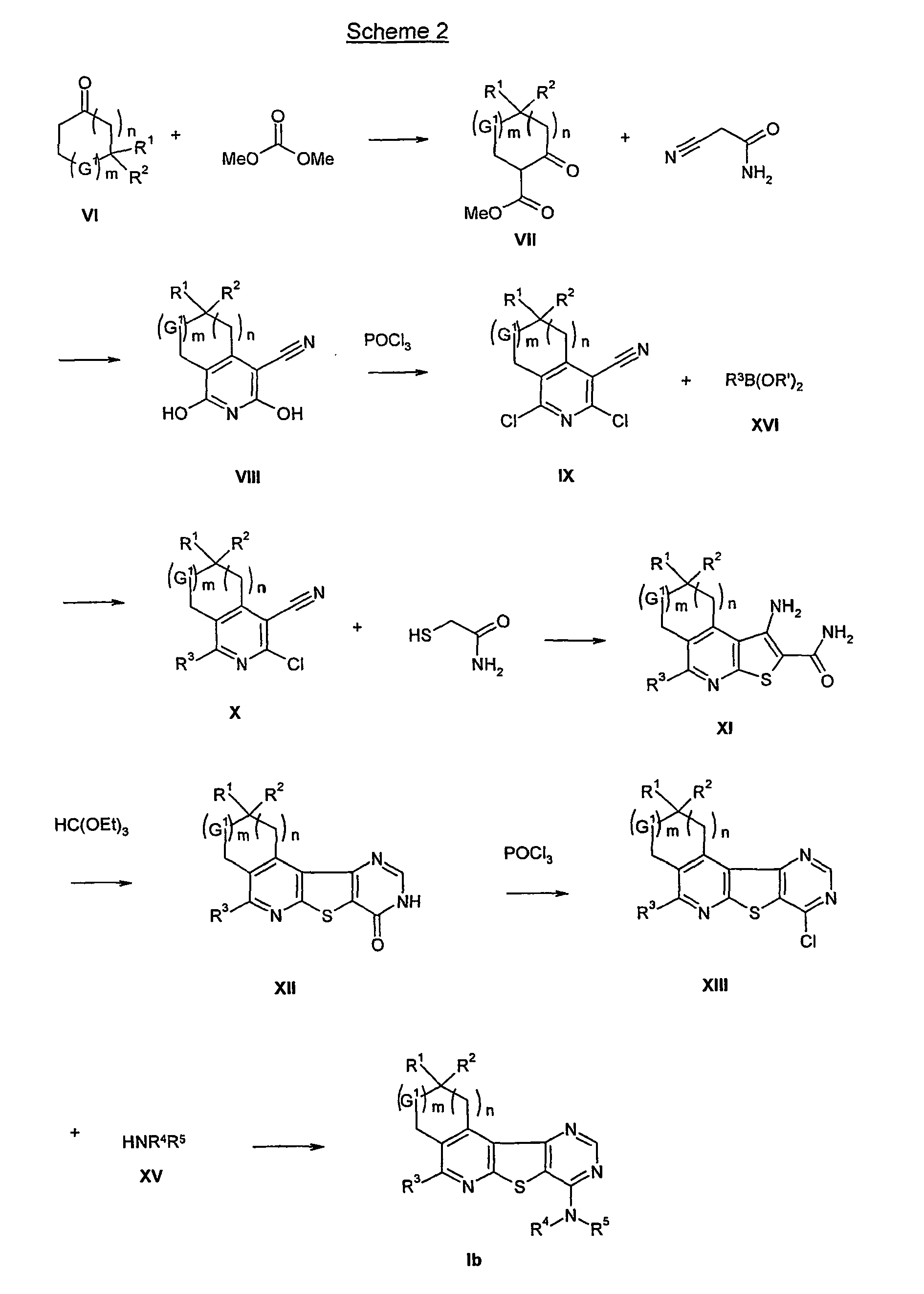

Ketone **VI,** wherein G¹, m, n, R¹ and R² are as hereinbefore defined, reacts with dimethyl carbonate in the presence of a strong base such as sodium hydride in tetrahydrofurane to yield the diketone **VII**, according to the method described by L.A.Paquette at J.Org.Chem., 1991, 56, 6199. Ketones **VI** are commercially available or prepared according to the methods described at C. Ainsworth Org.Synth., 1959, 39, 536, J.Cologne, A.Varagnat Bull. Soc. Chim. France, 1964, 10, 2499-504, and E.M. Kosower, T.S.Sorensen, 1963, 28, 687.

Reaction of compound **VII** with cyanoacetamide in methanol under refluxing conditions with the presence of potassium hydroxide yields the pyridine derivative **VIII**, as described by E.Wenkert et al. at J.Am.Chem.Soc., 1965, 87, 5461. The same reference applies for the conversion of **VIII** to the 1,6-dichloropyridine derivative **IX** by reaction with phosphorous oxychloride without solvent at 150-170°C in a sealed tube.

**IX** is converted to **X** under classical Suzuki coupling conditions by reaction with a boronic acid of a lower alkyl boronate of formula **XVI** in the presence of potassium carbonate and tetrakis(triphenylphosphine)palladium(0) under reflux of dioxane, where the boronic acids R³B(OH)₂ or their corresponding boronates are commercially available or sinthesized by common methodology, being R³ as hereinbefore defined.

Subsequent cyclocondensation of compound **X** with 2-mercaptoacetamide in the presence of a base such as potasium carbonate affords the thienopyridine compound **XI,** according to Santilli, A. A.; Kim, D. H.; Wanser, S. V.; J Heterocycl Chem, 1971, 8, 445 or Schneller, S. W.; Clough, F. W.; J Heterocycl Chem, 1975, 12, 513.

The pyridothienopyrimidine derivative **XII** is sinthesized by cyclisation of intermediate **XI** with a orthoformate derivative HC(OR⁶)₃, wherein R⁵ is a C₁₋₄ alkyl group, as described at C.Peinador et al Bioorg.Med.Chem., 1998, 6, 1911, or formic acid or a reactive derivative thereof. The reactive derivative of formic acid is preferably the acid halide, orthoester or anhydride. The reaction can be carried out in a solvent, preferably a polar aprotic solvent, such as N,N-dimethylformamide, dioxane, acetone or tetrahydrofuran, in the presence of an organic base, preferably an amine base, such as triethylamine and at a temperature from 15°C to 40°C. The reaction can also be carried out in the absence of a solvent, in which case an excess of formic acid or reactive derivative of the formic acid is used and the mixture is heated at a temperature from 40°C to its boiling point.

The corresponding chloroimine derivative **XIII** is sinthesized using phosphorous oxychloride as solvent, and the resulting intermediate is reacted with an amine of formula **XV**, wherein R⁴ and R⁵ are as hereinbefore defined, to give the desired final compound **Ib.**

The pharmaceutically acceptable salts of the compounds of the present invention represented by formula **Ia** and **Ib** may be acid addition salts or alkali addition salts. Examples of the acid addition salts include those formed with a mineral acid such as, for example, hydrochloric, hydrobromic, hydroiodic, sulfaric, nitric, phosphoric, or with an organic acid such as, for example, acetic, maleic, fumaric, citric, oxalic, succinic, tartaric, malic, mandelic, methanesulfonic, and p-toluenesulfonic. Examples of the alkali addition salts include inorganic salts such as, for example sodium, potassium, calcium and ammonium salts and organic alkali salts such as, for example, ethylenediamine, ethanolamine, *N,N*-dialkylenethanolamine, triethanolamine and basic amino acid salts

The compounds of the present invention represented by the above described formula (**Ia** and **Ib**) may include enantiomers depending on their asymmetry or diastereoisomers. The single isomers and mixtures of the isomers fall within the scope of the present invention.

The compounds of formulae **VI, XIV, XV** and **XVI** are known compounds or can be prepared by analogy with known methods.

### PHARMACOLOGICAL ACTIVITY

### PDE4 Assay Procedure

Compounds to be tested were resuspended in DMSO at a stock concentration of 1 mM. The compounds were tested at different concentrations varying from 10 µM to 10 pM to calculate an IC₅₀. These dilutions were done in 96-well plates. In some cases, plates containing diluted compounds were frozen before being assayed. In these cases, the plates were thawed at room temperature and stirred for 15 minutes.

Ten microliters of the diluted compounds were poured into a "low binding" assay plate. Eighty microliters of reaction mixture containing 50 mM Tris pH 7.5, 8.3 mM MgCl₂, 1.7 mM EGTA, and 15 nM [3H]-cAMP were added to each well. The reaction was initiated by adding ten microliters of a solution containing PDE4. The plate was then incubated under stirring for 1 hour at room temperature. After incubation the reaction was stopped with 50 microlitres of SPA beads, and the reaction was allowed to incubate for another 20 minutes at room temperature before measuring radioactivity using standard instrumentation.

The reaction mixture was prepared by adding 90 ml of H₂O to 10 ml of 10X assay buffer (500 mM Tris pH 7.5, 83 mM MgCl₂, 17 mM EGTA), and 40 microlitres 1 µCi/µL [3H]-cAMP. SPA beads solution was prepared by adding 500 mg to 28 ml H₂O for a final concentration of 20 mg/ml beads and 18 mM zinc sulphate.

The results are shown in Table 1.

| **Example** | **IC**₅₀ **PDE4 (nM)** |
|---|---|
| 1 | 2.9 |
| 3 | 3.6 |
| 16 | 3.0 |
| 23 | 8.1 |
| 24 | 14 |
| 25 | 1.5 |
| 43 | 7.9 |
| 54 | 10 |
| 60 | 4.8 |
| 62 | 3.8 |
| 68 | 2.9 |
| 79 | 4.6 |

It can be seen from Table 1 that the compounds of formula (**I**) are potent inhibitors of phosphodiesterase 4 (PDE 4). Preferred pyridothienopyrimidine derivatives of the invention possess an IC₅₀ value for the inhibition of PDE4 (determined as defined above) of less than 100 nM, preferably less than 50 nM and most preferably less than 30 nM.

The compounds are also capable of blocking the production of some pro-inflammatory cytokines such as, for example, TNFα. Thus, they can be used in the treatment of allergic, inflammatory and immunological diseases, as well as those diseases or conditions where the blockade of pro-inflammatory cytokines or the selective inhibition of PDE 4 could be of benefit.

These disease states include asthma, chronic obstructive pulmonary disease, allergic rhinitis, rheumatoid arthritis, osteoarthritis, osteoporosis, bone-formation disorders, glomerulonephritis, multiple sclerosis, ankylosing spondylitis, Graves ophtalmopathy, myasthenia gravis, diabetes insipidus, graft rejection, gastrointestinal disorders such as ulcerative colitis or Crohn disease, septic shock, adult distress respiratory syndrome, and skin diseases such as atopic dermatitis, contact dermatitis, acute dermatomyositis and psoriasis. They can also be used as improvers of cerebrovascular function as well as in the treatment of other CNS related diseases such as dementia, Alzheimer's disease, depression, and as nootropic agents.

The compounds of the present invention are also of benefit when administered in combination with other drugs such as steroids and immunosuppressive agents, such as cyclosporin A, rapamycin or T-cell receptor blockers. In this case the administration of the compounds allows a reduction of the dosage of the other drugs, thus preventing the appearance of the undesired side effects associated with both steroids and immunosuppressants. The compounds of the invention have also shown their efficacy in blocking, after preventive and/or curative treatment, the erosive and ulcerogenic effects induced by a variety of etiological agents, such as antiinflammatory drugs (steroidal or non-steroidal antiinflammatory agents), stress, ammonia, ethanol and concentrated acids.

They can be used alone or in combination with antacids and/or antisecretory drugs in the preventive and/or curative treatment of gastrointestinal pathologies like drug-induced ulcers, peptic ulcers, H. Pylori-related ulcers, esophagitis and gastro-esophageal reflux disease. They can also be used in the treatment of pathological situations where damage to the cells or tissues is produced through conditions like anoxia or the production of an excess of free radicals. Examples of such beneficial effects are the protection of cardiac tissue after coronary artery occlusion or the prolongation of cell and tissue viability when the compounds of the invention are added to preserving solutions intended for storage of transplant organs or fluids such as blood or sperm. They are also of benefit on tissue repair and wound healing.

Accordingly, the present invention provides a pyridothienopyrimidine derivatives of the invention for use in treating a subject afflicted with a pathological condition or disease which is asthma, chronic obstructive pulmonary disease, rheumatoid arthritis, atopic dermatitis, psoriasis or irritable bowel syndrome.

The present invention also provides a combination product comprising: (i) a compound according to any one of claims 12 to 23; and (ii) another compound selected from (a) steroids, (b) immunosuppressive agents, (c) T-cell receptor blockers and (d) antiinflammatory drugs; for simultaneous, separate or sequential use in the treatment of the human or animal body.

The present invention also provides pharmaceutical compositions which comprise, as an active ingredient, at least a pyridothienopyrimidine derivative of formula (I) or a pharmaceutically acceptable salt thereof in association with a pharmaceutically acceptable excipient such as a carrier or diluent. The active ingredient may comprise 0.001 % to 99% by weight, preferably 0.01% to 90% by weight, of the composition depending upon the nature of the formulation and whether further dilution is to be made prior to application. Preferably the compositions are made up in a form suitable for oral, topical, nasal, rectal, percutaneous or injectable administration.

The pharmaceutically acceptable excipients which are admixed with the active compound, or salts of such compound, to form the compositions of this invention are well-known per se and the actual excipients used depend inter alia on the intended method of administering the compositions.

Compositions for oral administration may take the form of tablets, retard tablets, sublingual tablets, capsules, inhalation aerosols, inhalation solutions, dry powder inhalation, or liquid preparations, such as mixtures, elixirs, syrups or suspensions, all containing the compound of the invention; such preparations may be made by methods well-known in the art.

The diluents which may be used in the preparation of the compositions include those liquid and solid diluents which are compatible with the active ingredient, together with colouring or flavouring agents, if desired. Tablets or capsules may conveniently contain between 2 and 500 mg of active ingredient or the equivalent amount of a salt thereof.

The liquid composition adapted for oral use may be in the form of solutions or suspensions. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent.

Compositions for parenteral injection may be prepared from soluble salts, which may or may not be freeze-dried and which may be dissolved in pyrogen free aqueous media or other appropriate parenteral injection fluid.

Compositions for topical administration may take the form of ointments, creams or lotions, all containing the compound of the invention; such preparations may be made by methods well-known in the art.

Effective doses are normally in the range of 10-600 mg of active ingredient per day. Daily dosage may be administered in one or more treatments, preferably from 1 to 4 treatments, per day.

The syntheses of the compounds of the invention and of the intermediates for use therein are illustrated by the following Examples (including Preparation Examples (Preparations 1 to 63)) which do not limit the scope of the invention in any way.

¹H Nuclear Magnetic Resonance Spectra were recorded on a Varian Gemini 300 spectrometer.

Low Resolution Mass Spectra (m/z) were recorded on a Micromass ZMD mass spectrometer using ESI ionization.

Melting points were recorded using a Perkin Elmer DSC-7 apparatus.

The chromatographic separations were obtained using a Waters 2690 system equipped with a Symmetry C18 (2.1 x 10 mm, 3.5 mM) column. The mobile phase was formic acid (0.4 mL), ammonia (0.1 mL), methanol (500 mL) and acetonitrile (500 mL) (B) and formic acid (0.46 mL), ammonia (0.115 mL) and water (1000 mL) (A): initially from 0% to 95% of B in 20 min, and then 4 min. with 95% of B. The reequilibration time between two injections was 5 min. The flow rate was 0.4 mL/min. The injection volume was 5 microliter. Diode array chromatograms were collected at 210 nM.

### PREPARATION EXAMPLES

### PREPARATION 1

### 3,3-Dimethylcyclohexanone

To a suspension of copper(I) cyanide (2.46g, 27.5 mmol) cooled at 0°C a 3.0M solution of methyl magnesium bromide (18.25 ml, 54.8 mmol) is dropwise added. Once the addition is completed, the reaction mixture is stirred for 30 min more at 0°C and then cooled to -78°C. A solution of 3-methyl-2-cyclohexen-1-one (1.0g, 9.07 mmol) in ethyl ether (15 ml) is then dropwise added. When the addition is over, the reaction mixture is stirred between -40 °C and -20 °C for two hours. Finally, an aqueous solution of phosphate buffer (pH=7.2, 90 ml) is carefully added to quench the reaction, followed by saturated solution of ammonium chloride (35 ml). The system is allowed to reach room temperature and the two phases separated. The aqueous phase is extracted twice with ethyl ether and the organic phases washed with brine, dried over magnesium sulphate, filtered and the solvents evaporated under vacuum. 1.08 g of the desired final compound, as an orange oil, is obtained, pure enough so as to be used in the next synthetic step without further purification. Yield = 94%
¹H NMR (200 MHz, CDCl₃) δ ppm 0.98 (s, 6H) 1.59 (m, 2H) 1.89 (m, 2H) 2.16 (s, 2H) 2.28 (t, J=6.62 Hz, 2H)

### PREPARATION 2

### 3-Amino-6,6-dimethyl-1-thioxo-5,6,7,8-tetrahydro-1H-isothiochromene-4-carbonitrile

2,2-Dimethylcyclohexanone (1.15g, 9.07 mmol, see Preparation 1) is solved in methanol (1.10 ml) and carbon disulfide (1.10 ml, 18.2 mmol) is added in one portion. Malononitrile (0.60g, 9.07 mmol) is added portionwise and, finally, triethylamine is added (0.44 ml). The reaction mixture is stirred at room temperature for 48h. The solvent is evaporated under vacuum and 0.84g of 2-(3,3-dimethylcyclohexylidene)malononitrile were isolated by flash chromatography, eluting first with CH₂Cl₂ and next with the mixture of solvents. This intermediate compound was solved in methanol (0.56ml) and carbon disulfide (2 equivalents) and triethylamine (0.35 eq.) were added. After 48h stirring at room temperature, a solid is filtered and washed with methanol. It weighs 0.45g and its
¹HNMR is consistent with the final product. From the methanolic phase, another 0.5g of the final compound were isolated by flash chromatography, eluting with CH₂Cl₂: MeOH 95:5. Global yield= 42%.
¹H NMR (200 MHz, CDCl₃) δ ppm 1.01 (s, 6H) 1.57 (m, 2H) 2.52 (s, 2H) 2.76 (t, J=6.62 Hz, 2H) 5.67 (s, 2H)

### PREPARATION 3

### 3-Mercapto-6,6-dimethyl-1-morpholin-4-yl-5,6,7,8-tetrahydroisoquinoline-4-carbonitrile

The product resulting from preparation 2 (0.94g, 3.75 mmol) is suspended in ethanol (4.5 ml) and morpholine (1.86 ml, 21.4 mmol) is added. The reaction mixture is refluxed under nitrogen overnight. Then the system is allowed to reach room temperature and the reaction mixture is left in an ice bath for two hours. The solid formed is filtrated and washed twice with ethanol. After drying, 0.35g of the final compound are obtained as a dark solid, pure enough to perform the next step. Yield= 31%.
¹H NMR (200 MHz, CDCl₃) δ ppm 1.01 (s, 6H) 1.5 (t, J=6.99 Hz, 2H) 2.2 (m, 1 H) 2.47 (t, J=6.99, 2H) 2.6 (s, 2H) 3.3 (m, 4H) 3.9 (m, 4H)

### PREPARATION 4

### 1-Amino-8,8-dimethyl-5-morpholin-4-yl-6,7,8,9-tetrahydrothieno[2,3-c]isoquinoline-2-carboxamide

To a suspension of 3-mercapto-6,6-dimethy)-1-morpholin-4-yl-5,6,7,8-tetrahydroisoquinoline-4-carbonitrile (0.35g, 1.15 mmol, see Preparation 3) in ethanol (15 ml), potasium carbonate (0.38g, 2.77 mmol) and 2-chloroacetamide (0.12g, 1.27 mmol) are added, and the reaction mixture is then refluxed for 3h. The solvent is evaporated under vacuum and water is added to the residue: the precipitated solid is filtered and dried. It weighs 0.31g and its ¹H-RMN is consistent with the desired product. Yield= 74%. ¹H NMR (200 MHz, CDCl₃) δ ppm 1.6 (s, 6H) 2.7 (t, J=6.99 Hz, 2H) 3.0 (s, 2H) 3.2 (m, 4H) 3.4 (m, 2H) 3.85 (m, 6H) 5.25 (s, 2H) 6.25 (s, 2H)

### PREPARATION 5

### 2,2-Dimethyl-5-morpholin-4-yl-1,2,3,4-tetrahydropyrimido[4',5':4,5]thieno[2,3-c]isoquinolin-8(9H)-one

1-Amino-8, 8-dimethyl-5-morpholin-4-yl-8,9-dihydro-6H-pyrano[4,3-d]thieno[2,3-b]pyridine-2-carboxamide (0.31g, 0.85 mmol), see Preparation 4) is supended in ethyl orthoformate (6 ml) and p-toluensulfonic acid hydrate(0.02g, 0.1 mmol) is added. This mixture is heated under reflux overnight. Then the reaction mixture is allowed to reach room temperature and left in an ice bath for two hours. The precipitated formed is filtered and washed with ethyl ether. After drying it weighs 0.15g and its ¹H-RMN is consistent with the desired compound. Yield= 47%.
¹H NMR (200 MHz, CDCl₃) δ ppm 1.1 (s, 6H) 1.6 (t, *J*=6.99 Hz, 2H) 1.8 (m, 4H) 3.3 (m, 4H) 3.35 (s, 1 H) 3.85 (m, 4H) 8.1 (s, 1 H)

### PREPARATION 6

### 8-Chloro-2,2-dimethyl-5-morpholin-4-yl-1,2,3,4-tetrahydropyrimido[4',5':4,5]thieno[2,3-c]isoquinoline

The final product of preparation 5 (0.15g, 0.40 mmol) is suspended in phosphorous oxychloride (2 ml) and heated to reflux for 90 min. The excess of phosphorous oxychloride is evaporated under vacuum and the residue redissolved between chloroform and a cooled solution of 2N NaOH. The aqueous phase is extracted twice with chloroform and the organic phases are washed with water and brine, dried over magnesium sulfate, filtered and the solvent evaporated. 0.16g of a brownish solid is obtained, whose ¹HNMR is consistent with the desired final product. Quantitative yield.
¹H NMR (200 MHz, CDCl₃) δ ppm 1.1 (s, 6H) 1.6 (t, *J*=6.99 Hz, 2H) 2.8 (t, *J*=6.99 Hz, 2H) 3.4 (m, 6H) 3.9 (m, 4H) 9.05 (s, 1 H)

### PREPARATION 7

### 3-Mercapto-6,6-dimethyl-1-thiomorpholin-4-yl-5,6,7,8-tetrahydroisoquinoline-4-carbonitrile

The product resulting from preparation 2 (3.0g, 11.98 mmol) is suspended in ethanol (15 ml) and thiomorpholine (6.87 ml, 68.3 mmol) is added. The reaction mixture is refluxed under nitrogen overnight. The solvent is evaporated and the residue is passed through a flash chromatography column, eluting first with dichloromethane and then with the mixture CH₂Cl₂:MeOH 9:1. 3.69g of a mixture 55:45 of the final compound and starting material, respectively, are obtained. The next synthetic step is performed with this mixture. ¹H NMR (200 MHz, CDCl₃) δ ppm 1.01 (s, 6H) 1.5 (t, *J*=6.99 Hz, 2H) 2.2 (m, 1 H) 2.47 (t, *J*=6.99, 2H) 2.6 (s, 2H) 3.3 (m, 4H) 3.9 (m, 4H)

### PREPARATION 8

### 1-Amino-8,8-dimethyl-5-thiomorpholin-4-yl-6,7,8,9-tetrahydrothieno[2,3-c]isoquinoline-2-carboxamide

To a suspension of 3-mercapto-6,6-dimethyl-1-thiomorpholin-4-yl-5,6,7,8-tetrahydroisoquinoline-4-carbonitrile (3.69g, mixture of products, see Preparation 7) in ethanol (175 ml), potasium carbonate (3.83g, 27.7 mmol) and 2-chloroacetamide (1.19g, 12.7 mmol) are added, and the reaction mixture is then refluxed for 18h. The solvent is evaporated under vacuum and water is added to the residue: the precipitated solid is filtered and dried. It is then purified by flash chromatography, eluting with CH₂Cl₂:MeOH 95:5. 0.85g of the final compound are isolated. Its ¹H-RMN is consistent with the proposed structure. Yield (calculated from the starting material of Preparation 35)= 20%.
¹H NMR (200 MHz, CDCl₃) δ ppm 1.1 (s, 6H) 1.5 (m, 2H) 2.7 (t, *J*=6.99 Hz, 2H) 2.8 (m, 4H) 3.0 (s, 2H) 3.4 (m, 4H) 5.25 (s, 2H) 6.45 (s, 2H)

### PREPARATION 9

### 2,2-Dimethyl-6-thiomorpholin-4-yl-1,2,3,4-tetrahydropyrimido[4',5':4,5]thieno[2,3-c]isoquinolin-8(9H)-one

1-Amino-8,8-dimethyl-5-thiomorpholin-4-yl-8,9-dihydro-6H-pyrano[4,3-d]thieno[2,3-b]pyridine-2-carboxamide (0.85g, 2.26 mmol, see Preparation 8) is supended in ethyl orthoformate (17 ml) and p-toluensulfonic acid hydrate(0.05g, 0.23 mmol) is added. This mixture is heated under reflux for 3h. Then the reaction mixture is allowed to reach room temperature and left in an ice bath for two hours. The precipitated formed is filtered and washed with ethyl ether. After drying it weighs 0.46g and its¹H-RMN is consistent with the desired compound. Yield= 53%.
¹H NMR (200 MHz, CDCl₃) δ ppm 1.1 (s, 6H) 1.6 (t, *J*=6.99 Hz, 2H) 2.7 (m, 2H) 2.9 (m, 4H) 3.35 (s, 2H) 3.55 (m, 4H) 8.25 (s, 1 H)

### PREPARATION 10

### 8-Chloro-2,2-dimethyl-5-thiomorpholin-4-yl-1,2,3,4-tetrahydropyrimido[4',5':4,5]thieno[2,3-c]isoquinoline

The final product of preparation 9 (0.46g, 1.18 mmol) is suspended in phosphorous oxychloride (10 ml) and heated to reflux for 90 min. The excess of phosphorous oxychloride is evaporated under vacuum and the residue redissolved between chloroform and a cooled solution of 2N NaOH. The aqueous phase is extracted twice with chloroform and the organic phases are washed with water and brine, dried over magnesium sulfate, filtered and the solvent evaporated. 0.48g of a solid is obtained, whose ¹HNMR is consistent with the desired final product. Quantitative yield.
¹H NMR (200 MHz, CDCl₃) δ ppm 1.15 (s, 6H) 1.6 (t, *J*=6.99 Hz, 2H) 2.75 (t, *J*=6.99 Hz, 2H) 2.85 (m, 4H) 3.4 (s, 2H) 3.6 (m, 4H) 9.05 (s, 1 H)

### PREPARATION 11

### 3-Mercapto-6,6-dimethyl-1-(4-methylpiperazin-1-yl)-5,6,7,8-tetrahydroisoquinoline-4-carbonitrile

The product resulting from preparation 2 (1.0g, 3.99 mmol) is suspended in ethanol (7 ml) and N-methylpiperazine (2.53 ml, 22.7 mmol) is added. The reaction mixture is refluxed under nitrogen overnight. The solvent is evaporated and the residue is passed through a flash chromatography column, eluting first with dichloromethane and then with the mixture CH₂Cl₂:MeOH 95:5. 0.9g of the final compound are obtained.
Yield=71%.
¹H NMR (300 MHz, CHLOROFORM-D) δ ppm 1.10 (s,6H) 1.50 (m, 2H) 2.5 (m, 2H) 2.60 (s, 2H) 2.65 (s, 3H) 2.80 (bs, 1 H) 2.95 (m, 4H) 3.55 (m, 4H)

### PREPARATION 12

### 1-Amino-8,8-dimethyl-5-(4-methylpiperazine-1-yl)-6,7,8,9-tetrahydrothieno[2,3-c]isoquinoline-2-carboxamide

To a suspension of 3-mercapto-6,6-dimethyl-1-(4-methylpiperazin-1-yl)-5,6,7,8-tetrahydroisoquinoline-4-carbonitrile (0.9g, see Preparation 11) in ethanol (50 ml), potasium carbonate (0.83g, 5.97 mmol) and 2-chloroacetamide (0.29g, 3.12 mmol) are added, and the reaction mixture is then refluxed for 6h and then left overnight at room temperature. The solvent is evaporated under vacuum and water is added to the residue: the precipitated solid is filtered and dried. 0.95g of the final compound are isolated. Its ¹H-RMN is consistent with the proposed structure. Yield = 90%.
¹H NMR (200 MHz, CDCl₃) δ ppm 1.1 (s, 6H) 1.5 (t, *J*=6.99 Hz, 2H) 2.40 (s, 3H) 2.65 (m, 6H) 3.0 (s, 2H) 3.30 (m, 4H) 5.25 (s, 2H) 6.45 (s, 2H)

### PREPARATION 13

### 2,2-Dimethyl-5-(4-methylpiperazin-1-yl)-1,2,3,4-tetrahydropyrimido[4',5':4,5]thieno[2,3-c]isoquinolin-8(9H)-one

1-Amino-8,8-dimethyl-5-(4-methylpiperazine-1-yl)-6,7,8,9-tetrahydrothieno[2,3-c]isoquinoline-2-carboxamide (0.95g, 2.55 mmol, see Preparation 12) is supended in ethyl orthoformate (20 ml) and p-toluensulfonic acid hydrate(0.05g, 0.26 mmol) is added. This mixture is heated under reflux for 4h. Then the reaction mixture is allowed to reach room temperature and left in an ice bath for two hours, but no solid precipitates. The excess of ethyl ortoformate is evaporated under vacuum and the residue is solved in chloroform. This organic phase is washed with water, aqueous solution of potassium carbonate 4M and brine. After drying with magnesium sulfate, the organic phase is filtered and evaporated. A brownish semisolid is obtained, which weighs 0.80g. Although with certain excess of ethyl ortoformate, ¹H-RMN is consistent with the desired compound. Yield= 82%.
¹H NMR (200 MHz, CDCl₃) δ ppm 1.1 (s, 6H) 1.6 (t, *J*=6.99 Hz, 2H) 2.40 (s, 3H) 2.60 (m, 6H) 2.7 (t, *J*=6.99 Hz, 2H) 3.35 (m, 4H) 6.80 (s, 1 H) 8.45 (s, 1 H)

### PREPARATION 14

### 8-Chloro-2,2-dimethyl-5-(4-methylpiperazin-yl)-1,2,3,4-tetrahydropyrimido[4',5':4,5]thieno[2,3-c]isoquinoline

The final product of preparation 13 (0.80g, 2.10 mmol) is suspended in phosphorous oxychloride (20 ml) and heated to reflux for 90 min. The excess of phosphorous oxychloride is evaporated under vacuum and the residue redissolved between chloroform and a cooled solution of 2N NaOH. The aqueous phase is extracted twice with chloroform and the organic phases are washed with water and brine, dried over magnesium sulfate, filtered and the solvent evaporated. 0.90g of a black oil is obtained, which is purified by flash chromatography, eluting with CH₂Cl₂:MeOH 95:5. 0.26g of the final compound are isolated. The ¹HNMR is consistent with the desired final product. Yield= 31%.
¹H NMR (200 MHz, CDCl₃) δ ppm 1.15 (s, 6H) 1.6 (t, *J*=6.99 Hz, 2H) 2.40 (s, 3H) 2.65 (m, 4H) 2.75 (t, *J*=6.99 Hz, 2H) 3.45 (m, 6H) 9.05 (s, 1 H)

### PREPARATION 15

### 3-Mercapto-6,6-dimethyl-1-pyrrolidin-1-yl-5,6,7,8-tetrahydroisoquinoline-4-carbonitrile

The product resulting from preparation 2 (2.20g, 8.79 mmol) is suspended in ethanol (14 ml) and pyrrolidine (4.18 ml, 50.1 mmol) is added. The reaction mixture is refluxed under nitrogen for 3h. The solvent is evaporated and the residue is passed through a flash chromatography column, eluting first with dichloromethane and then with the mixture CH₂Cl₂:MeOH 95:5. 1.93g of the final compound are obtained.
Yield= 76%.
¹H NMR (300 MHz, CHLOROFORM-D) δ ppm 1.10 (s,6H) 1.50 (m, 2H) 2.1 (m, 6H) 2.5 (bs, 1 H) 3.5 (m, 4H) 3.8 (s, 2H)

### PREPARATION 16

### 1-Amino-8,8-dimethyl-5-pyrrolidin-1-yl-6,7,8,9-tetrahydrothieno[2,3-c]isoquinoline-2-carboxamide

To a suspension of 3-mercapto-6,6-dimethyl-1-pyrrolidin-yl-5,6,7,8-tetrahydroisoquinoline-4-carbonitrile (1.93g, 6.71 mmol, see Preparation 15) in ethanol (100 ml), potasium carbonate (1.95g, 14.1 mmol) and 2-chloroacetamide (0.69g, 7.39 mmol) are added, and the reaction mixture is then refluxed for 4h and then left overnight at room temperature. The solvent is evaporated under vacuum and water is added to the residue: the precipitated solid is filtered and dried. 1.70g of the final compound are isolated. Its ¹H-RMN is consistent with the proposed structure. Yield = 71%.
¹H NMR (200 MHz, CDCl₃) δ ppm 1.1 (s, 6H) 1.5 (t, *J*=6.99 Hz, 2H) 1.90 (m, 4H) 2.65 (t, *J*=6.99 Hz, 2H) 3.50 (s, 2H) 3.60 (m, 4H) 5.15 (s, 2H) 6.45 (s, 2H)

### PREPARATION 17

### 2,2-Dimethyl-5-pyrrolidin-1-yl-1,2,3,4-tetrahydropyrimido[4',5':4,5]thieno[2,3-c]isoquinolin-8(9H)-one

1-Amino-8,8-dimethyl-5-pyrrolidin-1-yl-6,7,8,9-tetrahydrothieno[2,3-c]isoquinoline-2-carboxamide (1.70g, 4.93 mmol, see Preparation 16) is supended in ethyl orthoformate (35 ml) and p-toluensulfonic acid hydrate(0.07g, 0.35 mmol) is added. This mixture is heated under reflux for 4h. Then the reaction mixture is allowed to reach room temperature and left overnight at +5°C. 0.65g of a black solid is filtered, but its ¹HNMR is not consistent with the final structure and it is rejected. The excess of ethyl ortoformate is evaporated under vacuum and the residue is purified by flash chromatography, eluting first with dichloromethane and then with CH₂Cl₂:MeOH 98:2. 0.69g of the desired final compound are isolted. Yield= 39%.
1 H NMR (200 MHz, CDCl₃) δ ppm 1.1 (s, 6H) 1.6 (t, *J*=6.99 Hz, 2H) 2.0 (m, 4H) 2.8 (t, *J*=6.99 Hz, 2H) 3.30 (s, 2H) 3.70 (m, 4H) 8.20 (s, 1 H) 12.4 (bs, 1 H)

### PREPARATION 18

### 8-Chloro-2,2-dimethyl-5-pyrrolidin-yl-1,2,3,4-tetrahydropyrimido[4',5':4,5]thieno[2,3-c]isoquinoline

The final product of preparation 17 (0.69g, 1.94 mmol) is suspended in phosphorous oxychloride (10 ml) and heated to reflux for 90 min. The excess of phosphorous oxychloride is evaporated under vacuum and the residue redissolved between chloroform and a cooled solution of 2N NaOH. The aqueous phase is extracted twice with chloroform and the organic phases are washed with water and brine, dried over magnesium sulfate, filtered and the solvent evaporated. 0.60g of the final compound are obtained. The ¹HNMR is consistent with the desired final product. Yield= 83%.
¹H NMR (200 MHz, CDCl₃) δ ppm 1.15 (s, 6H) 1.6 (t, *J*=6.99 Hz, 2H) 2.0 (m, 4H) 2.80 (t, *J*=6.99 Hz, 2H) 3.35 (s, 2H) 3.75 (m, 4H) 8.90 (s, 1 H)

### PREPARATION 19

### 3-Mercapto-1-dimethylamino-6,6-dimethyl-5,6,7,8-tetrahydroisoquinoline-4-carbonitrile

The product resulting from preparation 2 (4.0g, 16.0 mmol) is suspended in ethanol (4.2 ml) and dimethylamine (16.26ml, 5.6M solution in EtOH, 91.0 mmol) is added. The reaction mixture is heated overnight at 85°C under nitrogen in a pressure vessel. The solvent is evaporated and the residue is passed through a flash chromatography column, eluting with the mixture CH₂Cl₂:MeOH 98:2. 1.54g of the final compound are obtained.
Yield= 37%.
¹H NMR (300 MHz, CHLOROFORM-D) δ ppm 1.05 (s,6H) 1.50 (t, *J*=6.56 Hz, 2 H) 1.65 (bs, 1 H) 2.5 (t, *J*=6.56 Hz, 2 H) 2.6 (s, 2H) 3.1 (s, 6H)

### PREPARATION 20

### 1-Amino-5-dimethylamino-8,8-dimethyl-6,7,8,9-tetrahydrothieno[2,3-c]isoquinoline-2-carboxamide

To a suspension of 3-mercapto-1-dimethylamino-6,6-dimethyl-5,6,7,8-tetrahydroisoquinoline-4-carbonitrile (1.54g, 5.84mmol, see Preparation 19) in ethanol (100 ml), potasium carbonate (1.94g, 14.0 mmol) and 2-chloroacetamide (0.60g, 6.43 mmol) are added, and the reaction mixture is then refluxed overnight. The solvent is evaporated under vacuum and water is added to the residue. After extraction with chloroform, the organic phase is washed with water and brine, dried over magnesium sulfate, filtered and evaporated under vacuum.1.84g of the final compound are isolated.
Its ¹H-RMN is consistent with the proposed structure. Yield = 99%.
¹H NMR (200 MHz, CDCl₃) δ ppm 1.1 (s, 6H) 1.5 (t, *J*=6.99 Hz, 2H) 2.65 (t, *J*=6.99 Hz, 2H) 2.95 (s, 6H) 3.00 (s, 2H) 5.20 (s, 2H) 6.45 (s, 2H)

### PREPARATION 21

### 5-Dimethylamino-2,2-dimethyl-1-yl-1,2,3,4-tetrahydropyrimido[4',5':4,5]thieno[2,3-c]isoquinolin-8(9H)-one

1-Amino-5-dimethylamino-8,8-dimethyl-6,7,8,9-tetrahydrothieno[2,3-c]isoquinoline-2-carboxamide (1.84g, 5.78 mmol, see Preparation 20) is supended in ethyl orthoformate (50 ml) and p-toluensulfonic acid hydrate (0.12g, 0.58 mmol) is added. This mixture is heated under reflux for 4h. Then the reaction mixture is allowed to reach room temperature and left overnight at +5°C. 1.20g of a solid is filtered, and its ¹HNMR is consistent with the final structure. The organic phase is evaporated under vacuum and the residue is purified by flash chromatography, eluting first with dichloromethane and then with CH₂Cl₂:MeOH 98:2. Additional 0.46g of the desired final compound are isolted. Global yield= 87%.
¹H NMR (200 MHz, CDCl₃) δ ppm 1.1 (s, 6H) 1.6 (t, *J*=6.99 Hz, 2H) 2.8 (t, *J*=6.99 Hz, 2H) 3.0 (s, 6H) 3.35 (s, 2H) 8.20 (s, 1 H) 12.1 (bs, 1 H)

### PREPARATION 22

### 8-Chloro-5-dimethylamino-2,2-dimethyl-1,2,3,4-tetrahydropyrimido[4',5':4,5]thieno[2,3-c]isoquinoline

The final product of preparation 21 (1.20g, 3.65 mmol) is suspended in phosphorous oxychloride (15 ml) and heated to reflux for 90 min. The excess of phosphorous oxychloride is evaporated under vacuum and the residue redissolved between chloroform and a cooled solution of 2N NaOH. The aqueous phase is extracted twice with chloroform and the organic phases are washed with water and brine, dried over magnesium sulfate, filtered and the solvent evaporated. 1.27g of the final compound are obtained. The ¹HNMR is consistent with the desired final product. Quantitative yield.
¹H NMR (200 MHz, CDCl₃) δ ppm 1.1 (s, 6H) 1.6 (t, *J*=6.99 Hz, 2H) 2.8 (t, *J*=6.99 Hz, 2H) 3.0 (s, 6H) 3.40 (s, 2H) 9.01 (s, 1 H)

### PREPARATION 23

### 8-Chloro-2,2,3-trimethyl-5-morpholin-4-yl-1,2,3,4-tetrahydropyrimido[4',5':4,5]thieno[2,3-c]-2,7-naphthyridine

2,2,3-Trimethyl-5-morpholin-4-yl-1,2,3,4-tetrahydropyrimido[4',5':4,5]thieno[2,3-c]-2,7-naphthyridin-8(9*H*)-one (0.3g, 0.78 mmol, commercially available at Pharmeks Ltd., ref.nr. PHAR010756) is suspended in phosphorous oxychloride (7 ml) and the mixture is refluxed for 90 minutes. The excess of POCl₃ is evaporated under vacuum and the residue is redissolved between NaOH 2N and chloroform. The aqueous phase is extracted twice with chloroform. The organic phase is washed with water and brine, dried over magnesium sulfate, filtered and evaporated. 0.27g of a brownish oil is obtained, pure enough to perform the following synthetic step. Yield= 87%
¹H NMR (300 MHz, CHLOROFORM-D) δ ppm 1.25 (s, 6H) 2.45 (s, 3H) 3.35 (m, 4H) 3.55 (s, 2H) 3.75 (s, 2H) 3.90 (m, 4H) 9.0 (s, 1H)

### PREPARATION 24

### 2-Chloro-N-(tetrahydrofuran-2-ylmethyl)-acetamide

A solution of chloroacetyl chloride (2.0 ml, 25.1 mmol) in dichloromethane (20 ml) is dropwise added to an ice-cooled solution of tetrahydrofurfurylamine (2.59 ml, 25.1 mmol) and trimethylamine (5.24ml, 37.7 mmol) in dichloromethane (60 ml). Once the addition is over, the reaction mixture is allowed to stir at room temperature for 4h. This organic solution is washed twice with water and then with brine, dried over magnesium sulphate, filtered and evaporated under vacuum. 3.39g of the desired final product are obtained, pure enough to perform the next synthetic step. Yield= 76%.
1 H NMR (200 MHz, CHLOROFORM-D) δ ppm 1.55 (m, 1 H) 1.98 (m, 3H) 3.25 (m, 1 H) 3.6 (m, 1 H) 3.8 (m, 1 H) 3.90 (m, 1 H) 4.05 (m, 1 H) 4.10 (s, 2H) 6.90 (bs, 1 H).

### PREPARATION 25

### 2-Amino-N-(tetahydrofuran-2-ylmethyl)-acetamide hydrochloride

2-Chloro-N-(tetrahydrofuran-2-ylmethyl)-acetamide (0.50g, 2.81 mmol, see Preparation 24) is dissolved in ethanol saturated with ammonia (15 ml) and heated to 80°C in a sealed tube for 3.5h. Once at room temperature, the solvent is evaporated and the residue (0.53g, orange solid) contains the final product, pure enough to perform the following synthetic step. Yield= 97%.
LRMS: m/z 158 (M+1)⁺

### PREPARATION 26

### 1-(Ethylmethylamino)-3-mercapto-6,6-dimethyl-5,6,7,8-tetrahydroisoquinoline-4-carbonitrile

The product resulting from preparation 2 (2.20g, 8.79 mmol) is suspended in ethanol (12 ml) and ethylmethylamine (4.52 ml, 52.7 mmol) is added. The reaction mixture is refluxed under nitrogen for 6h. The solvent is evaporated and the residue is passed through a flash chromatography column, eluting with the mixture CH₂Cl₂:MeOH 98:2. 1.20g of the final compound are obtained.
Yield= 50%.
¹H NMR (300 MHz, CHLOROFORM-D) δ ppm 1.10 (s, 6H) 1.25 (t, 3H) 1.45-1.60 (m, 4H) 2.5 (m, 2H) 2.6 (s, 2H) 3.0 (s, 2H) 3.35 (q, 2H)

### PREPARATION 27

### 1-Amino-8,8-dimethyl-5-ethylmethylamino-6,7,8,9-tetrahydrothieno[2,3-c]isoquinoline-2-carboxamide

To a suspension of 1-(ethylmethylamino)-3-mercapto-6,6-dimethyl-5,6,7,8-tetrahydroisoquinoline-4-carbonitrile (1.20g, 4.36 mmol, see Preparation 27) in ethanol (25 ml), potasium carbonate (1.45g, 10.5 mmol) and 2-chloroacetamide (0.45g, 4.79 mmol) are added, and the reaction mixture is then refluxed for 18h and then left overnight at room temperature. The solvent is evaporated under vacuum and water is added to the residue. After extraction with chloroform, the organic phase is washed with water and brine, dried over magnesium sulphate, filtered and evaporated under vacuum. The residue is purified by flash chromatography eluting with CH₂Cl₂/MeOH 98:2. 1.04g of the final compound are isolated. Its ¹H-RMN is consistent with the proposed structure. Yield = 72%.
¹H NMR (200 MHz, CDCl₃) δ ppm 1.10 (s, 6H) 1.20 (t, 3H) 1.5 (m, 2H) 2.65 (t, 2H) 2.90 (s, 3H) 3.0 (s, 2H) 3.20 (q, 2H) 5.20 (bs, 1 H) 6.45 (bs, 2H)

### PREPARATION 28

### 2,2-Dimethyl-5-ethylmethylamine-1,2,3,4-tetrahydropyrimido[4',5':4,5]thieno[2,3-c]isoquinolin-8(9H)-one

1-Amino-8,8-dimethyl-5-ethylmethylamino-6,7,8,9-tetrahydrothieno[2,3-c]isoquinoline-2-carboxamide (1.04g, 3.13 mmol, see Preparation 28) is supended in ethyl orthoformate (30 ml) and p-toluensulfonic acid hydrate(0.06g, 0.31 mmol) is added. This mixture is heated under reflux for 18h. Then the reaction mixture is allowed to reach room temperature and left overnight at +5°C. 0.62g of a white solid is filtered, washed with Et₂O and its ¹HNMR is consistent with the final structure. Yield= 58%.
¹H NMR (200 MHz, CDCl₃) δ ppm 1.05 (s, 6H) 1.20 (t, 3H) 1.55 (t, 2H) 2.7 (t, 2H) 2.90 (s, 3H) 3.30 (m, 5H) 8.3 0 (s, 1H)

### PREPARATION 29

### 8-Chloro-2,2-dimethyl-5-ethylmethylamino-1,2,3,4-tetrahydropyrimido[4',5':4,5]thieno[2,3-c]isoquinoline

The final product of preparation 29 (0.62g, 1.81 mmol) is suspended in phosphorous oxychloride (10 ml) and heated to reflux for 2h. The excess of phosphorous oxychloride is evaporated under vacuum and the residue redissolved between chloroform and a cooled solution of 2N NaOH. The aqueous phase is extracted twice with chloroform and the organic phases are washed with water and brine, dried over magnesium sulfate, filtered and the solvent evaporated. 0.64g of the final compound are obtained. The ¹HNMR is consistent with the desired final product. Yield= 98%.
¹H NMR (200 MHz, CDCl₃) δ ppm 1.15 (s, 6H) 1.25 (t, 2H) 1.6 (m, 3H) 2.75 (t, 2H) 3.05 (s, 3H) 3.4 (s, 2H) 3.42 (q, 2H) 9.00 (s, 1H)

### PREPARATION 30

### Methyl 4,4-dimethyl-2-oxo-cyclohexanecarboxylate

3,3-Dimethylcyclohexan-1-one (3.0g, 23.8 mmol, synthesized according to M.G. Organ et al. J.Am. Chem. Soc., 1994, 116, 3312-3323) reacts with dimethylcarbonate (10.02 ml, 119 mmol) in tetrahydrofurane (75 ml) in the presence of sodium hydride (60%, 1.96g, 49.0 mmol) according to the method described in L.A.Paquette J.Org.Chem., 1991, 56, 6199 to yield 4.70g (100%) of the desired final product, pure enough to perform the next synthetic step.
¹H NMR (200 MHz, CDCl₃) δ ppm 0.09 (s, 6H) 1.40 (t, 2H) 1.85 (t, 1H) 2.1 (s, 2H) 2.25 (t, 2H) 3.75 (s, 3H)

### PREPARATION 31

### 1,3-Dihydroxy-6,6-dimethyl-5,6,7,8-tetrahydroisoquinoline-4-carbonitrile

Methyl 4,4-dimethyl-2-oxo-cyclohexanecarboxylate (4.38g, 23.8 mmol, see Preparation 30) reacts with cyanoacetamide (2.00g, 23.8 mmol) in methanol (20 ml) in the presence of potassium hydroxide (85%, 1.40g, 25.0 mmol) according to the method described at E.Wenkert J.Am.Chem.Soc., 1965, 87, 5461 to yield 2.75g (yield=53%) of the desired final product.
¹H NMR (300 MHz, DMSO-D6) d ppm 0.9 (s, 6 H) 1.4 (t, *J*=6.6 Hz, 2 H) 2.3 (t, *J*=6.6 Hz, 2 H) 2.4 (s, 2 H) 2.5 (m, 2 H)

### PREPARATION 32

### 1,3-Dichloro-6,6-dimethyl-5,6,7,8-tetrahydroisoquinoline-4-carbonitrile

1,3-Dihydroxy-6,6-dimethyl-5,6,7,8-tetrahydroisoquinoline-4-carbonitrile (2.75g, 12.6 mmol, see Preparation 31) reacts with phosphorous oxychloride (8 ml) according to the method described at E.Wenkert J.Am.Chem.Soc., 1965, 87, 5461 to yield 1.97g (yield=61 %) of the desired final product.
¹H NMR (300 MHz, DMSO-D6) d ppm 1.10 (s, 6 H) 1.65 (t, 2 H) 2.70 (s, 2 H) 2.80 (m, 2 H)

### PREPARATION 33

### 3-Chloro-1,6,6-trimethyl-5,6,7,8-tetrahydroisoquinoline-4-carbonitrile

Palladium triphenylphosphine (0.23g, 0.19mmol) and potassium carbonate (0.81g, 5.88mmol) are suspended in dioxane (10ml) and 1,3-dichloro-6,6-dimethyl-5,6,7,8-tetrahydroisoquinoline-4-carbonitrile (0.5g, 1.96mmol, see Preparation 32) is added. Methylboronic acid (0.12g, 1.96 mmol) is dropwise added at room temperature under nitrogen atmosphere, and the reaction mixture is refluxed for 48h. The solvent is evaporated under reduced pressure and the residue is purified by flash chromatography, eluting with CH₂Cl₂/hexane 7:3. 0.21g of the desired final product are obtained. Yield= 45%.
¹H NMR (300 MHz, DMSO-D6) d ppm 1.10 (s, 6 H) 1.65 (t, 2 H) 2.5 (s, 3H) 2.65 (t, 2 H) 2.70 (s, 2 H)

### PREPARATION 34

### 1-Amino-5,8,8-trimethyl-6,7,8,9-tetrahydrothieno[2,3-c]isoquinoline-2-carboxamide

3-Chloro-1,6,6-trimethyl-5,6,7,8-tetrahydroisoquinoline-4-carbonitrile (0.21g, 0.89 mmol, see Preparation 33) is solved in ethanol (5 ml) and potassium carbonate (0.30g, 2.14 mmol) and thioacetamide (0.81 ml, 0.89 mmol) are added. This reaction mixture is refluxed overnight under nitrogen. Additional 0.2 equivalents of thioacetamide are added and the reflux is followed for another 4h. Once the solvent is evaporated under reduced pressure, the residue is suspended in water and extracted with chloroform. The organic phase was washed with water and brine, dried over magnesium sulphate, filtered and evaporated to dryness. 0.26g of the desired final product are obtained, pure enough to perform the followin sinthetic step. Yield= 100%.
¹H NMR (300 MHz, DMSO-D6) d ppm 1.10 (s, 6 H) 1.66 (t, 2 H) 2.55 (s, 3H) 2.72 (t, 2 H) 3.03 (s, 2 H) 5.32 (bs, 2H) 6.46 (bs, 2H)

### PREPARATION 35

### 2,2,5-Trimethyl-1,2,3,4-tetrahydropyrimido[4',5':4,5]thieno[2,3-c]isoquinolin-8(9H)-one

1-Amino-5,8,8-trimethyl-6,7,8,9-tetrahydrothieno[2,3-c]isoquinoline-2-carboxamide (0.26g, 0.89 mmol, see Preparation 34) is supended in ethyl orthoformate (10 ml) and p-toluensulfonic acid hydrate(0.02g, 0.089 mmol) is added. This mixture is heated under reflux for 3h. Then the reaction mixture is allowed to reach room temperature and left overnight at +5°C. 0.16g of a white solid is filtered, washed with Et₂O and its ¹HNMR is consistent with the final structure. Yield= 60%.
¹H NMR (200 MHz, CDCl₃) δ ppm 1.08 (s, 6H) 1.71 (t, 2H) 2.62 (s, 3H) 2.79 (t, 2H) 3.39 (s, 2H) 8.11 (s, 1H)

### PREPARATION 36

### 8-Chloro-2,2,5-trimethyl-1,2,3,4-tetrahydropyrimido[4',5':4,5]thieno[2,3-c]isoquinoline

The final product of preparation 35 (0.16g, 0.53 mmol) is suspended in phosphorous oxychloride (5 ml) and heated to reflux for 2h. The excess of phosphorous oxychloride is evaporated under vacuum and the residue redissolved between dichloromethane and a solution of 2N K₂CO₃. The aqueous phase is extracted twice with dichloromethane and the organic phases are washed with water and brine, dried over magnesium sulfate, filtered and the solvent evaporated. 0.17g of the final compound are obtained. The ¹HNMR is consistent with the desired final product. Yield= 100%.
¹H NMR (200 MHz, CDCl₃) δ ppm 1.10 (s, 6H) 1.75 (t, 2H) 2.68 (s, 3H) 2.83 (t, 2H) 3.47 (s, 2H) 9.10 (s, 1 H)

### PREPARATION 37

### 3-Chloro-1-isobutyl-6,6-dimethyl-5,6,7,8-tetrahydroisoquinoline-4-carbonitrile

Obtained (47%) from the title compound of Preparation 32 and isobutyl boronic acid following the experimental procedure described in Preparation 33.
¹H NMR (200 MHz, CDCl₃) δ ppm 0.95 (d, 6H) 1.01 (s, 6H) 1.59 (s, 2H) 1.63 (t, 2H) 2.20 (m, 1H) 2.65 (d, 2H) 2.72 (s, 2H)

### PREPARATION 38

### 1-Amino-5-isobutyl-8,8-dimethyl-6,7,8,9-tetrahydrothieno[2,3-c]isoquinoline-2-carboxamide

Obtained (99%) from the title compound of Preparation 37 and thioacetamide following the experimental procedure described in Preparation 34.
¹H NMR (200 MHz, CDCl₃) δ ppm 0.97 (d, 6H) 1.05 (s, 6H) 1.63 (t, 2H) 2.21 (m, 1H) 2.72 (d, 2H) 2.80 (t, 2H) 3.04 (s, 2H) 5.32 (bs, 2H) 6.47 (bs, 2H)

### PREPARATION 39

### 2,2-Dimethyl-5-isobutyl-1,2,3,4-tetrahydropyrimido[4',5':4,5]thieno[2,3-c]isoquinolin-8(9H)-one

Obtained (72%) from the title compound of Preparation 38 and ethyl orthoformate following the experimental procedure described in Preparation 35.
¹H NMR (200 MHz, CDCl₃)δ ppm 1.00 (d, 6H) 1.01 (s, 6H) 1.70 (t, 2H) 2.29 (m, 1H) 2.80 (d, 2H) 2.88 (t, 2H) 3.42 (s, 2H) 8.29 (s, 1 H) 12.57 (bs, 1 H)

### PREPARATION 40

### 8-Chloro-2,2-dimethyl-5-isobutyl-1,2,3,4-tetrahydropyrimido[4',5':4,5]thieno[2,3-c]isoquinoline

Obtained (68%) from the title compound of Preparation 39 and phosphorous oxychloride following the experimental procedure described in Preparation 36.
¹H NMR (200 MHz, CDCl₃) δ ppm 1.02 (d, 6H) 1.10 (s, 6H) 1.72 (t, 2H) 2.30 (m, 1 H) 2.83 (d, 2H) 2.90 (t, 2H) 3.47 (s, 2H) 9.09 (s, 1 H)

### PREPARATION 41

### 3-Chloro-1-furan-2-yl-6,6-trimethyl-5,6,7,8-tetrahydroisoquinoline-4-carbonitrile

Obtained (42%) from the title compound of Preparation 32 and 2-furanboronic acid following the experimental procedure described in Preparation 33.
¹H NMR (200 MHz, CDCl₃) δ ppm 1.06 (s, 6H) 1.67 (t, 2H) 2.77 (s, 2H) 3.06 (t, 2H) 6.60 (m, 1 H) 7.24 (m, 1 H) 7.67 (s, 1 H)

### PREPARATION 42

### 1-Amino-5-furan-2-yl-8,8-dimethyl-6,7,8,9-tetrahydrothieno[2,3-c]isoquinoline-2-carboxamide

Obtained (92%) from the title compound of Preparation 41 and thioacetamide following the experimental procedure described in Preparation 34.
¹H NMR (200 MHz, CDCl₃) δ ppm 1.10 (s, 6H) 1.67 (t, 2H) 3.07 (t, 2H) 3.10 (s, 2H) 5.35 (bs, 2H) 6.51 (bs, 2H) 6.59 (d, 1 H) 7.07 (d, 1H) 7.65 (s, 1 H)

### PREPARATION 43

### 5-Furan-2-yl 2,2-dimethyl-1,2,3,4-tetrahydropyrimido[4',5':4,5]thieno[2,3-c]isoquinolin-8(9H)-one

Obtained (97%) from the title compound of Preparation 42 and ethyl orthoformate following the experimental procedure described in Preparation 35.
¹H NMR (200 MHz, CDCl₃) δ ppm 1.06 (s, 6H) 1.67 (t, 2H) 3.10 (t, 2H) 3.44 (s, 2H) 6.74 (d, 1 H) 7.28(d, 1 H) 7.98 (d, 1 H) 8.42 (s, 1 H)

### PREPARATION 44

### 8-Chloro-5-(2-furyl)-2,2-dimethyl-1,2,3,4-tetrahydropyrimido[4',5':4,5]thieno[2,3-c]isoquinoline

Obtained (66%) from the title compound of Preparation 43 and phosphorous oxychloride following the experimental procedure described in Preparation 36.
¹H NMR (200 MHz, CDCl₃) δ ppm 1.15 (s, 6H) 1.75 (t, 2H) 3.18 (t, 2H) 3.53 (s, 2H) 6.64 (d, 1H) 7.23 (d, 1H) 7.72 (s, 1H) 9.11 (s, 1 H)

### PREPARATION 45

### 3-Chloro-1-furan-3-yl-6,6-trimethyl-5,6,7,8-tetrahydroisoquinoline-4-carbonitrile

Obtained (61 %) from the title compound of Preparation 32 and 3-furanboronic acid following the experimental procedure described in Preparation 33.
¹H NMR (200 MHz, CDCl₃) δ ppm 1.06 (s, 6H) 1.68 (t, 2H) 2.78 (s, 2H) 2.83 (t, 2H) 7.07 (d, 1 H) 7.52 (m, 1H) 7.99 (s, 1 H)

### PREPARATION 46

### 1-Amino-5-furan-3-yl-8,8-dimethyl-6,7,8,9-tetrahydrothieno[2,3-c]isoquinoline-2-carboxamide

Obtained (93%) from the title compound of Preparation 45 and thioacetamide following the experimental procedure described in Preparation 34.
¹H NMR (200 MHz, CDCl₃) δ ppm 1.09 (s, 6H) 1.66 (t, 2H) 2.90 (t, 2H) 3.10 (s, 2H) 5.354 (bs, 2H) 6.51 (bs, 2H) 7.04 (s, 1 H) 7.52 (s, 1 H) 7.91 (s, 1 H)

### PREPARATION 47

### 5-Furan-3-yl 2,2-dimethyl-1,2,3,4-tetrahydropyrimido[4',5':4,5]thieno[2,3-c]isoquinolin-8(9H)-one

Obtained (73%) from the title compound of Preparation 46 and ethyl orthoformate following the experimental procedure described in Preparation 35.
¹H NMR (200 MHz, CDCl₃) δ ppm 1.12 (s, 6H) 1.71 (t, 2H) 2.97 (t, 2H) 3.47 (s, 2H) 7.11 (s, 1H) 7.55 (s, 1 H) 7.98 (s, 1 H) 8.13 (s, 1 H)

### PREPARATION 48

### 8-Chloro-5-(3-furyl)-2,2-dimethyl-1,2,3,4-tetrahydropyrimido[4',5':4,5]thieno[2,3-c]isoquinoline

Obtained (85%) from the title compound of Preparation 47 and phosphorous oxychloride following the experimental procedure described in Preparation 36.
¹H NMR (200 MHz, CDCl₃) δ ppm 1.15 (s, 6H) 1.75 (t, 2H) 3.01 (t, 2H) 3.53 (s, 2H) 7.16 (s, 1H) 7.57 (s, 1 H) 8.03 (s, 1 H) 9.11 (s, 1 H)

### PREPARATION 49

### 2-(3,3-Dimethylcyclopentyliden)malononitrile

In a Dean-Stark apparatus are refluxed overnight 3,3-dimethylcyclopentanone (6.18g, 55.09 mmol, synthesized according to L.A.Paquette et al. J.Am.Chem.Soc., 1987, 109, 5731-5740), malononitrile (5.46g, 82.64 mmol), ammonium acetate (2.76g, 35.81 mmol) and acetic acid (5.50 ml) in toluene (80 ml). Once again at room temperature, the liquid phase is decanted and washed with water and brine, dried with magnesium sulphate, filtered and evaporated under reduced pressure. 7.32g of the final product are obtained as an oil and pure enough to perform the next synthetic step. ¹HNMR in agreement with the proposed structure. Yield= 83%
¹H NMR (200 MHz, CDCl₃) δ ppm 1.10 (s, 6H) 1.75 (t, 2H) 2.60 (s, 2H) 2.90 (t, 2H)

### PREPARATION 50

### 3-Amino-6,6-dimethyl-1-thioxo-1,5,6,7-tetrahydrocyclopenta[c]thiopyran-4-carbonitrile

2-(3,3-Dimethylcyclopentyliden)malononitrile (7.32g, 45.69 mmol, see Preparation 49) is solved in dimethylformamide (18.75 ml) and carbon disulfide (13.62 ml, 0.29 mol) is added in one portion. Finally, triethylamine is added (0.48 ml, 3.44 mmol). The reaction mixture is stirred at room temperature for 20h. Water is added and the precipitated solid is filtered and washed with methanol. It weighs 3.51g and its ¹HNMR is consistent with the final product. From the aqueous phase, by extractions with ethyl ether, another 2.92g of the final compound were isolated, pure enough to perform the next synthetic step.Global
yield= 60%.
¹H NMR (200 MHz, CDCl₃) δ ppm 1.20 (s, 6H) 1.70 (s, 2H) 1.80 (s, 2H) 3.00 (bs, 1 H) 8.25 (bs, 1H)

### PREPARATION 51

### 1-Dimethylamino-3-mercapto-6,6-dimethyl-6,7-dihydro-5H-[2]pyridin-4-carbonitrile

The product resulting from preparation 50 (3.51g, 14.85 mmol) is suspended in ethanol (3.9 ml) and dimethylamine (15.2 ml, 5.6M solution in EtOH, 84.7 mmol) is added. The reaction mixture is heated overnight at 85°C under nitrogen in a pressure vessel. The solvent is evaporated and the residue is passed through a flash chromatography column, eluting with the mixture CH₂Cl₂:MeOH 98:2. 1.21g of the final compound are obtained.
Yield= 33%.
¹H NMR (300 ,MHz, CHLOROFORM-D) δ ppm 1.10 (s,6H) 1.60 (bs, 1H) 2.70 (s, 2H) 3.10 (s, 2H) 3.2 (s, 6H)

### PREPARATION 52

### 1-Amino-5-dimethylamino-7,7-dimethyl-6H-7,8-dihydrocyclopenta[d]thieno[2,3-b]pyridin-2-carboxamide

To a suspension of 1-dimethylamino-3-mercapto-6,6-dimethyl-6,7-dihydro-5H-[2]pyridin-4-carbonitrile(1.21g, 4.89 mmol, see Preparation 51) in ethanol (50 ml), potasium carbonate (1.35g, 9.78 mmol) and 2-chloroacetamide (0.50g, 5.38 mmol) are added, and the reaction mixture is then refluxed overnight. The solvent is evaporated under vacuum and water is added to the residue. After extraction with ethyl acetate, the organic phase is washed with water and brine, dried over magnesium sulfate, filtered and evaporated under vacuum.1.04g of the final compound are isolated. Its ¹H-RMN is consistent with the proposed structure. Yield = 99%.
¹H NMR (200 MHz, CDCl₃) δ ppm 1.20 (s, 6H) 2.85 (s, 2H) 3.00 (s, 2H) 3.10 (s, 6H) 5.25 (s, 2H) 6.15 (s, 2H)

### PREPARATION 53

### 4-Dimethylamino-2,2-dimethyl-2,3-dihydro-1H-cyclopenta[4',5']pyrido[3',2':4,5]thieno[3,2-d]pyrimidin-7(8H)-one

1-Amino-5-dimethylamino-7,7-dimethyl-6H-7,8-dihydrocyclopenta[d]thieno[2,3-b]pyridin-2-carboxamide (1.04g, 3.42 mmol, see Preparation 52) is supended in ethyl orthoformate (23 ml) and p-toluensulfonic acid hydrate (74 mg, 0.39 mmol) is added. This mixture is heated under reflux for 24h. Then the reaction mixture is allowed to reach room temperature and left overnight at +5°C. 0.24g of a solid is filtered, and its ¹HNMR is consistent with the final structure. The organic phase is evaporated under vacuum and the residue is purified by flash chromatography, eluting first with dichloromethane/methanol 99.5:0.5 and then with CH₂Cl₂/MeOH 98:2. Additional 0.58g of the desired final compound are isolted. Global yield= 76%.
¹H NMR (200 MHz, CDCl₃) δ ppm 1.2 (s, 6H) 2.95 (s, 2H) 3.20 (s, 6H) 3.25 (s, 2H) 8.25 (s, 1 H) 12.1 (bs, 1 H)

### PREPARATION 54

### 7-Chloro-N,N,2,2-tetramethyl-2,3-dihydro-1H-cyclopenta[4',5']pyrido[3',2':4,5]thieno[3,2-d]pyrimidin-4-amine

The final product of preparation 53 (0.82g, 2.61 mmol) is suspended in phosphorous oxychloride (14 ml) and heated to reflux for 3h. The excess of phosphorous oxychloride is evaporated under vacuum and the residue redissolved between ethyl acetate and a cooled solution of 2N NaOH. The aqueous phase is extracted twice with ethyl acetate and the organic phases are washed with water and brine, dried over magnesium sulfate, filtered and the solvent evaporated. 0.51g of the final compound are obtained. The ¹HNMR is consistent with the desired final product. Yield= 59%.
¹H NMR (200 MHz, CDCl₃) δ ppm 1.20 (s, 6H) 3.0 (s, 2H) 3.25 (s, 6H) 3.30 (s, 2H) 8.95 (s, 1 H)

### PREPARATION 55

### 3-Mercapto-6,6-dimethyl-1-morpholin-4-yl-6,7-dihydro-5H-[2]pyridin-4-carbonitril

The product resulting from preparation 50 (2.92g, 12.35 mmol) is suspended in ethanol (18 ml) and morpholine (6.13g, 70.4 mmol) is added. The reaction mixture is heated overnight at 100°C under nitrogen. The solvent is evaporated and the residue is passed through a flash chromatography column, eluting with the mixture CH₂Cl₂/MeOH 98:2. 1.70g of the final compound are obtained.
Yield= 67%.
¹H NMR (300 MHz, CHLOROFORM-D) δ ppm 1.20 (s,6H) 2.70 (s, 2H) 2.75 (s, 2H) 3.60 (m, 4H) 3.80 (m, 4H)

### PREPARATION 56

### 1-Amino-5-(1-morfolino)-7,7-dimethyl-6H-7,8-dihydrocyclopenta[d]thieno[2,3-b]pyridin-2-carboxamide

To a suspension of 3-mercapto-6,6-dimethyl-1-morpholin-4-yl-6,7-dihydro-5H-[2]pyridin-4-carbonitrile (3.62g, 12.51 mmol, see Preparation 55) in ethanol (80 ml), potasium carbonate (3.46g, 25.03 mmol) and 2-chloroacetamide (1.28g, 13.69 mmol) are added, and the reaction mixture is then refluxed for 48h. The solvent is evaporated under vacuum and water is added to the residue. After usual work-up with dichloromethane, 1.57g of a solid is obtained and its ¹H-RMN is consistent with the desired product. Yield= 36%.
LRMS: m/z 347 (M+1)⁺

### PREPARATION 57

### 2,2-Dimethyl-4-(1-morpholino)-2,3-dihydro-1H-cyclopenta[4',5']pyrido[3',2':4,5]thieno[3,2-d]pyrimidin-7(8H)-one

1-Amino-5-(1-morfolino)-7,7-dimethyl-6H-7,8-dihydrocyclopenta[d]thieno[2,3-b]pyridin-2-carboxamide (1.57g, 4.53 mmol, see Preparation 56) is supended in ethyl orthoformate (30 ml) and p-toluensulfonic acid hydrate(0.10g, 0.53 mmol) is added. This mixture is heated under reflux overnight. Then the solvent is evaporated to dryness. The residue is purified by flash chromatography, eluting with CH₂Cl₂/MeOH 98:2. 0.19g are obtained and its ¹H-RMN is consistent with the desired compound. Yield= 12%.
¹H NMR (300 MHz, CHLOROFORM-D) δ ppm 1.20 (s, 6H) 2.80 (s, 2H) 3.25 (s, 2H) 3.50 (m, 4H) 3.75 (m, 4H) 8.20 (s, 1H)

### PREPARATION 58

### 7-Chloro-2,2-dimethyl-4-morpholin-4-yl-2,3-dihydro-1H-cyclopenta[4',5']pyrido[3',2':4,5]thieno[3,2-d]pyrimidine

The final product of preparation 57 (0.19g, 0.53 mmol) is suspended in phosphorous oxychloride (3 ml) and heated to reflux for 4h. The excess of phosphorous oxychloride is evaporated under vacuum and the residue redissolved between ethyl acetate and a cooled solution of 2N NaOH. The aqueous phase is extracted twice with ethyl acetate and the organic phases are washed with water and brine, dried over magnesium sulfate, filtered and the solvent evaporated. 0.11g of a brownish solid is obtained, whose ¹HNMR is consistent with the desired final product. Yield= 55%.
¹H NMR (200 MHz, CDCl₃) δ ppm 1.20 (s, 6H) 2.80 (s, 2H) 3.30 (s, 2H) 3.65 (m, 4H) 3.85 (m, 4H) 8.95 (s, 1 H)

### PREPARATION 59

### 2-(2,2-Dimethylcyclopentiliden)malononitrile

In a Dean-Stark apparatus are refluxed overnight 2,2-dimethylcyclopentanone (5.07g, 45.20 mmol, commercially available at Aldrich nr.31,147-5), malononitrile (4.48g, 67.8 mmol), ammonium acetate (2.26g, 29.32 mmol) and acetic acid (4.5 ml) in toluene (67 ml). The liquid phase is decanted and washed with water and brine, dried with magnesium sulphate, filtered and evaporated under reduced pressure. 6.59g of the final product are obtained as an oil and pure enough to perform the next synthetic step. ¹HNMR in agreement with the proposed structure. Yield= 91%
¹H NMR (200 MHz, CDCl₃) δ ppm 1.40 (s, 6H) 1.80 (m, 4H) 2.90 (t, 2H)

### PREPARATION 60

### 3-Amino-5,5-dimethyl-1-thioxo-1,5,6,7-tetrahydrocyctopenta[c]thiopyran-4-carbonitrile

2-(2,2-Dimethylcyclopentyliden)malononitrile (6.59g, 41.13 mmol, see Preparation 59) is solved in dimethylformamide (17 ml) and carbon disulfide (12.26 ml, 0.26 mol) is added in one portion. Finally, triethylamine is added (0.43 ml, 3.09 mmol). The reaction mixture is stirred at room temperature for 20h. Water is added and the precipitated solid is filtered and washed with methanol. It weighs 4.95g and its ¹HNMR is consistent with the final product. From the aqueous phase, by extractions with ethyl ether, another 4.48g of the final compound were isolated, pure enough to perform the next synthetic step.Global
yield= 97%.
¹H NMR (200 MHz, CDCl₃) δ ppm 1.45 (s, 6H) 1.90 (t, 2H) 2.85 (t, 2H) 6.30 (bs, 1 H)

### PREPARATION 61

### 1-Dimethylamino-3-mercapto-5,5-dimethyl-6,7-dihydro-5H-[2]-pyridin-4-carbonitrile

The product resulting from preparation 60 (4.95g, 20.94 mmol) is suspended in ethanol (5.5 ml) and dimethylamine (21.5 ml, 5.6M solution in EtOH, 119.36 mmol) is added. The reaction mixture is heated overnight at 85°C under nitrogen in a pressure vessel. The solvent is evaporated and the residue is passed through a flash chromatography column, eluting first with the mixture CH₂Cl₂/MeOH 99:1 and then with CH₂Cl₂/MeOH 98:2. 1.67g of the final compound are obtained.
Yield= 32%.
¹H NMR (300 MHz, CHLOROFORM-D) δ ppm 1.40 (s,6H) 1.90 (t, 2H) 2.90 (t, 2H) 3.25 (s, 6H)

### PREPARATION 62

### 1-Amino-5-dimethylamino-8,8-dimethyl-7,8-di hydro-6H-cyclopenta[d]thieno[2,3-b]pyridine-2-carboxamide

To a suspension of 1-dimethylamino-3-mercapto-5,5-dimethyl-6,7-dihydro-5H-[2]-pyridin-4-carbonitrile(1.67g, 6.75 mmol, see Preparation 61) in ethanol (68 ml), potasium carbonate (1.87g, 7.43 mmol) and 2-chloroacetamide (0.69g, 7.43 mmol) are added, and the reaction mixture is then refluxed overnight. The solvent is evaporated under vacuum and water is added to the residue. After extraction with ethyl acetate, the organic phase is washed with water and brine, dried over magnesium sulfate, filtered and evaporated under vacuum.1.70g of the final compound are isolated. Its ¹H-RMN is consistent with the proposed structure. Yield = 83%.
¹H NMR (200 MHz, CDCl₃) δ ppm 1.50 (s, 6H) 2.00 (t, 2H) 3.00 (t, 2H) 3.10 (s, 6H) 5.40 (bs, 2H) 6.45 (bs, 2H)

### PREPARATION 63

### 4-Dimethylamino-1,1-dimethyl-2,3-dihydro-1H-cyclopenta[4',5']pyrido[3',2':4,5]thieno[3,2-d]pyrimidin-7(8H)-one

1-Amino-5-dimethylamino-8,8-dimethyl-7,8-dihydro-6H-cyclopenta[d]thieno[2,3-b]pyridin-2-carboxamide (1.70g, 5.58 mmol, see Preparation 62) is supended in ethyl orthoformate (38 ml) and p-toluensulfonic acid hydrate (0.12g, 0.63 mmol) is added. This mixture is heated under reflux for 24h. Then the reaction mixture is allowed to reach room temperature and left overnight at +5°C. 1.40g of a solid is filtered, washed with ethyl ether, and its ¹HNMR is consistent with the final structure. Yield= 80%.
¹H NMR (200 MHz, CDCl₃) δ ppm 1.60 (s, 6H) 1.95 (t, 2H) 3.10 (s, 6H) 3.10 (t, 2H) 8.30 (s, 1 H) 12.65 (bs, 1 H)

### PREPARATION 64

### 7-Chloro-N,N,1,1-tetramethyl-2,3-dihydro-1H-cyclopenta[4',5']pyrido[3',2':4,5]thieno[3,2-d]pyrimidine-4-amine

The final product of preparation 63 (1.40g, 4.45 mmol) is suspended in phosphorous oxychloride (23 ml) and heated to reflux for 4h. The excess of phosphorous oxychloride is evaporated under vacuum and the residue redissolved between ethyl acetate and a cooled solution of 2N NaOH. The aqueous phase is extracted twice with ethyl acetate and the organic phases are washed with water and brine, dried over magnesium sulfate, filtered and the solvent evaporated. 1.25g of the final compound are obtained. The ¹HNMR is consistent with the desired final product. Yield= 84%.
¹H NMR (200 MHz, CDCl₃) δ ppm 1.70 (s, 6H) 2.05 (t, 2H) 3.15 (t, 2H) 3.25 (s, 6H) 8.95 (s, 1 H)

### PREPARATION 65

### 3-Mercapto-5,5-dimethyl-1-morpholin-4-yl-6,7-dihydro-5H-[2]-pyridin-4-carbonitrile

The product resulting from preparation 60 (4.48g, 18.95 mmol) is suspended in ethanol (27 ml) and morpholine (9.41g, 108.02 mmol) is added. The reaction mixture is heated overnight at 100°C under nitrogen. The solvent is evaporated and the residue is passed through a flash chromatography column, eluting with CH₂Cl₂/MeOH 98:2. 4.95g of the final compound are obtained.
Yield= 90%.
¹H NMR (300 MHz, CHLOROFORM-D) δ ppm 1.40 (s,6H) 1.90 (t, 2H) 2.80 (t, 2H) 3.15 (m, 4H) 3.90 (m, 4H) 6.30 (bs, 1 H)

### PREPARATION 66

### 1-Amino-8,8-dimethyl-5-morpholin-4-yl-7,8-dihydro-6H-cyclopenta[d]thieno[2,3-b]pyridin-2-carboxamide

To a suspension of 3-mercapto-5,5-dimethyl-1-morpholin-6,7-dihydro-5H-[2]-pyridin-4-carbonitrile(4.95g, 17.10 mmol, see Preparation 65) in ethanol (170 ml), potasium carbonate (4.73g, 34.2 mmol) and 2-chloroacetamide (1.76g, 18.81 mmol) are added, and the reaction mixture is then refluxed overnight. The solvent is evaporated under vacuum and water is added to the residue. After extraction with ethyl acetate, the organic phase is washed with water and brine, dried over magnesium sulfate, filtered and evaporated under vacuum.1.93g of the final compound are isolated. Its ¹H-RMN is consistent with the proposed structure. Yield = 33%.
¹H NMR (200 MHz, CDCl₃) δ ppm 1.50 (s, 6H) 2.05 (t, 2H) 2.85 (t, 2H) 3.40 (m, 4H) 3.85 (m, 4H) 5.30 (bs, 2H) 6.45 (bs, 2H)

### PREPARATION 67

### 1,1-Dimethyl-4-morpholin-4-yl-2,3-dihydro-1H-cyclopenta[4',5']pyrido[3',2':4,5]thieno[3,2-d]pyrimidin-7(8H)-one

1-Amino-8,8-dimethyl-5-morpholin-7,8-dihydro-6H-cyclopenta[d]thieno[2,3-b]pyridin-2-carboxamide (1.93g, 5.57 mmol, see Preparation 66) is supended in ethyl orthoformate (38 ml) and p-toluensulfonic acid hydrate (0.12g, 0.63 mmol) is added. This mixture is heated under reflux for 24h. Then the reaction mixture is allowed to reach room temperature and left overnight at +5°C. 0.17g of a light brown solid is filtered, washed with ethyl ether, but its ¹HNMR is not consistent with the final structure and it is rejected. The filtrated liquid is evaporated to dryness and passed through a flash chromatography column, eluting first with CH₂Cl₂/MeOH 99.5:0.5 and then with CH₂Cl₂/MeOH 98:2. 0.43g of the final compound are obtained. Yield= 22%.
¹H NMR (200 MHz, CDCl₃) δ ppm 1.70 (s, 6H) 2.05 (t, 2H) 2.95 (t, 2H) 3.50 (m, 4H) 3.85 (m, 4H) 8.25 (s, 1 H) 13.05 (bs, 1 H)

### PREPARATION 68

### 7-Chloro-1,1-dimethyl-4-morpholin-4-yl-2,3-dihydro-1H-cyclopenta[4',5']pyrido[3',2':4,5]thieno[3,2-d]pyrimidine

The final product of preparation 67 (0.43g, 1.21 mmol) is suspended in phosphorous oxychloride (6 ml) and heated to reflux for 5h. The excess of phosphorous oxychloride is evaporated under vacuum and the residue redissolved between ethyl acetate and a cooled solution of 2N NaOH. The aqueous phase is extracted twice with ethyl acetate and the organic phases are washed with water and brine, dried over magnesium sulfate, filtered and the solvent evaporated. 0.43g of the final compound are obtained. The ¹HNMR is consistent with the desired final product. Yield= 96%.
¹H NMR (200 MHz, CDCl₃) δ ppm 1.70 (s, 6H) 2.05 (t, 2H) 3.00 (t, 2H) 3.65 (m, 4H) 3.85 (m, 4H) 9.00 (s, 1 H)

### PREPARATION 69

### 3-Mercapto-6,6-dimethyl-1-pyrrolidin-1-yl-6,7-dihydro-5H-[2]pyridin-4-carbonitrile

The product resulting from preparation 50 (5.0g, 21.15 mmol) is suspended in ethanol (19 ml) and pyrrolidine (10.0 ml, 120.6 mmol) is added. The reaction mixture is heated overnight at 85°C under nitrogen. The solvent is evaporated and the residue is passed through a flash chromatography column, eluting first with the mixture CH₂Cl₂:MeOH 98:2 and then with CH₂Cl₂:MeOH 95:5. 2.79g of the final compound are obtained.
Yield= 48%.
**LL-X00175-24**
¹H NMR (300 MHz, CHLOROFORM-D) δ ppm 1.10 (s,6H) 1.60 (bs, 1H) 2.10 (m, 4H) 2.70 (s,2H) 2.90 (s, 2H) 3.7 (t, 4H)

### PREPARATION 70

### 1-Amino-7,7-dimethyl-5-pyrrolidin-1-yl-7,8-dihydro-6H-cyclopenta[d]thieno[2,3-b]pyridin-2-carboxamide

To a suspension of 3-mercapto-6,6-dimethyl-1-pyrrolidin-1-yl-6,7-dihydro-5H-[2]pyridin-4-carbonitrile (2.55g, 9.33 mmol, see Preparation 69) in ethanol (95 ml), potasium carbonate (2.58g, 18.66 mmol) and 2-chloroacetamide (0.96g, 10.26 mmol) are added, and the reaction mixture is then refluxed overnight. The solvent is evaporated under vacuum and water is added to the residue. After extraction with dichloromethane, the organic phase is washed with water and brine, dried over magnesium sulfate, filtered and evaporated under vacuum. 0.96g of the final compound are isolated. Its ¹H-RMN is consistent with the proposed structure. Yield = 31%.
**LL-X00175-33**
¹H NMR (200 MHz, CDCl₃) δ ppm 1.20 (s, 6H) 1.95 (m, 4H) 3.00 (s, 4H) 3.70 (t, 4H) 5.25 (s, 2H) 6.15 (s, 2H)

### PREPARATION 71

### 2,2-Dimethyl-4-pyrrolidin-1-yl-2,3-dihydro-1H-cyclopenta[4',5']pyrido[3',2':4,5]thieno[3,2-d]pyrimidin-7(8H)-one

1-Amino-7,7-dimethyl-5-pyrrolidin-1-yl-7,8-dihydro-6H-cyclopenta[d]thieno[2,3-b]pyridin-2-carboxamide (0.96g, 2.91 mmol, see Preparation 70) is supended in ethyl orthoformate (19.7 ml) and p-toluensulfonic acid hydrate (60 mg, 0.32 mmol) is added. This mixture is heated under reflux for 18h. Then the reaction mixture is allowed to reach room temperature and left overnight at +5°C. 0.58g of a solid is filtered, and its ¹HNMR is consistent with the final structure. The organic phase is evaporated under vacuum and the residue is purified by flash chromatography, eluting with dichloromethane/methanol 98:2. Additional 70 mg of the desired final compound are isolated. Global yield= 66%. **LL-X00175-35**
¹H NMR (200 MHz, CDCl₃) δ ppm 1.2 (s, 6H) 1.9 (m, 4H) 3.05 (s, 2H) 3.15 (s,2H) 3.70 (m, 4H) 8.25 (s, 1 H) 12.1 (bs, 1 H)

### PREPARATION 72

### 7-Chloro-2,2-dimethyl-4-pyrrolidin-1-yl-2,3-dihydro-1H-cyclopenta[4',5']pyrido[3',2':4,5]thieno[3,2-d]pyrimidine

The final product of preparation 71 (0.65g, 1.91 mmol) is suspended in phosphorous oxychloride (10 ml) and heated to reflux for 4h. The excess of phosphorous oxychloride is evaporated under vacuum and the residue redissolved between dichloromethane and a cooled solution of 8N NaOH. The aqueous phase is extracted twice with dichloromethane and the organic phases are washed with water and brine, dried over magnesium sulfate, filtered and the solvent evaporated. 0.53g of the final compound are obtained as a brownish solid. The ¹HNMR is consistent with the desired final product. Yield= 88%.
**LL-X00175-37**
¹H NMR (200 MHz, CDCl₃) δ ppm 1.20 (s, 6H) 2.00 (m, 4H) 3.05 (s, 2H) 3.25 (s, 2H) 3.90 (m, 4H) 8.95 (s, 1 H)

### PREPARATION 73

### 1-(Ethylmethylamino)-3-mercapto-6,6-dimethyl-6,7-dihydro-5H-[2]pyridin-4-carbonitrile

The product resulting from preparation 50 (3.0g, 12.69 mmol) is suspended in ethanol (17 ml) and ethylmethylamine (6.21 ml, 72.33 mmol) is added. The reaction mixture is heated overnight at 85°C under nitrogen in a pressure vessel. The solvent is evaporated and the residue is passed through a flash chromatography column, eluting with the mixture CH₂Cl₂:MeOH 98:2. 1.28g of the final compound are obtained.
Yield= 39%.
**LL-X00175-50**
¹H NMR (300 MHz, CHLOROFORM-D) δ ppm 1.10 (s,6H) 1.25 (t, 3H) 2.65 (s, 2H) 2.90 (bs, 1 H) 3.10 (s,2H) 3.18 (s, 3H) 3.5 (q, 2H)

### PREPARATION 74

### 1-Amino-7,7-dimethyl-5-ethylmethylamino-7,8-dihydro-6H-cyclopenta[d]thieno[2,3-b]pyridin-2-carboxamide

To a suspension of 1-(ethylmethylamino)-3-mercapto-6,6-dimethyl-6,7-dihydro-5H-[2]pyridin-4-carbonitrile (1.28g, 4.9 mmol, see Preparation 73) in ethanol (50 ml), potasium carbonate (1.35g, 9.8 mmol) and 2-chloroacetamide (0.50g, 5.39 mmol) are added, and the reaction mixture is then refluxed overnight. The solvent is evaporated under vacuum and water is added to the residue. A solid is formed, which is filtered and washed with Et₂O. 0.60g of the final compound are isolated. After extraction of the aqueous phase with ethyl ether, the organic phase is washed with water and brine, dried over magnesium sulfate, filtered and evaporated under vacuum. 0.5g more of the final compound are isolated. Its ¹H-RMN is consistent with the proposed structure. Yield = 71%. **LL-X00175-52**
¹H NMR (200 MHz, CDCl₃) δ ppm 1.20 (m, 9H) 2.80 (s, 2H) 3.00 (s, 2H) 3.10 (s, 3H) 3.50 (q, 2H) 5.25 (s, 2H) 6.15 (s, 2H)

### PREPARATION 75

### 2,2-Dimethyl-4-ethylmethylamino-2,3-dihydro-1H-cyclopenta[4',5']pyrido[3',2':4,5]thieno[3,2-d]pyrimidin-7(8H)-one

1-Amino-7,7-dimethyl-5-ethylmethylamino-7,8-dihydro-6H-cyclopenta[d]thieno[2,3-b]pyridin-2-carboxamide (1.10g, 3.45 mmol, see Preparation 74) is supended in ethyl orthoformate (23.3 ml) and p-toluensulfonic acid hydrate (70 mg, 0.37 mmol) is added. This mixture is heated under reflux for 18h. The organic phase is evaporated under vacuum and the residue is purified by flash chromatography, eluting with dichloromethane/methanol 98:2. 0.69g of the desired final compound are isolated. Global
yield= 61%.
**LL-X00175-55**
¹H NMR (200 MHz, CDCl₃) δ ppm 1.2 (m, 9H) 2.90 (s, 2H) 3.20 (s, 3H) 3.30 (s,2H) 3.60 (q, 2H) 8.25 (s, 1 H)

### PREPARATION 76

### 7-Chloro-2,2-dimethyl-4-ethylmethylamino-2,3-dihydro-1H-cyclopenta[4',5']pyrido[3',2':4,5]thieno[3,2-d]pyrimidine

The final product of preparation 75 (0.69g, 2.10 mmol) is suspended in phosphorous oxychloride (11 ml) and heated to reflux for 4h. The excess of phosphorous oxychloride is evaporated under vacuum and the residue redissolved between dichloromethane and a cooled solution of 8N NaOH. The aqueous phase is extracted twice with ethyl acetate and the organic phases are washed with water and brine, dried over magnesium sulfate, filtered and the solvent evaporated. 0.64g of the final compound are obtained as a brownish solid. The ¹HNMR is consistent with the desired final product.
Yield= 88%.
**LL-X00175-56**
¹H NMR (200 MHz, CDCl₃) δ ppm 1.50 (s, 6H) 1.60 (t, 3H) 3.15 (s, 2H) 3.60 (m, 5H) 4.00 (q, 2H) 9.2 (s, 1 H)

### PREPARATION 77

### 1-(Benzylmethylamino)-3-mercapto-6,6-dimethyl-5,6,7,8-tetrahydro-isoquinoline-4-carbonitrile

The product resulting from preparation 50 (2.80g, 11.2 mmol) is suspended in ethanol (10 ml) and benzylmethylamine (8.65 ml, 67 mmol) is added. The reaction mixture is heated for 48h at 90°C under nitrogen in a pressure vessel. The solvent is evaporated and the residue is passed through a flash chromatography column, eluting first with dichloromethane and then with the mixture CH₂Cl₂:MeOH 98:2. 1.45g of the final compound are obtained.
Yield= 38%.
**JT-X00248-40**
LRMS: m/z 338 (M+1)⁺

### PREPARATION 78

### 1-Amino-5-benzylmethytamino-7,7-dimethyl-7,8-dihydro-6H-cyclopenta[d]thieno[2,3-b]pyridin-2-carboxamide

To a suspension of 1-(benzylmethylamino)-3-mercapto-6,6-dimethyl-5,6,7,8-tetrahydro-isoquinoline-4-carbonitrile (1.45g, 4.3 mmol, see Preparation 77) in ethanol (70 ml), potasium carbonate (1.25 g, 9.02 mmol) and 2-chloroacetamide (0.44g, 4.73 mmol) are added, and the reaction mixture is then refluxed overnight under nitrogen. The solvent is evaporated under vacuum and water is added to the residue. After extraction of the aqueous phase with ethyl acetate, the organic phase is washed with water and brine, .dried over magnesium sulfate, filtered and evaporated under vacuum. 1.70g of the final compound are isolated. Its ¹H-RMN is consistent with the proposed structure. Yield = 100%.
**JT-X00248-41**
¹H NMR (200 MHz, CDCl₃) δ ppm 1.10 (s, 6H) 1.55 (t, 2H) 2.75 (t, 2H) 2.80 (s, 3H) 3.00 (s, 2H) 4.40 (s, 2H) 5.35 (bs, 2H) 6.5 (bs, 2H) 7.40 (m, 5H)

### PREPARATION 79

### 2,2-Dimethyl-4-benzylmethylamino-2,3-dihydro-1H-cyclopenta[4',5']pyrido[3',2':4,5]thieno[3,2-d]pyrimidin-7(8H)-one

1-Amino-5-benzylmethylamino-7,7-dimethyl-7,8-dihydro-6H-cyclopenta[d]thieno[2,3-b]pyridin-2-carboxamide (1.69g, 4.30 mmol, see Preparation 78) is supended in ethyl orthoformate (20 ml) and p-toluensulfonic acid hydrate (81 mg, 0.43 mmol) is added. This mixture is heated under reflux for 18h. The organic phase is evaporated under vacuum and the residue is purified by flash chromatography, eluting with dichloromethane/methanol 98:2. 1.26g of the desired final compound are isolated. Global yield= 73%.
**JT-X00248-44**
¹H NMR (200 MHz, CDCl₃) δ ppm 1.1 (s, 6H) 1.6 (m, 2H) 2.80 (t, 2H) 2.90 (s, 3H) 3.40 (s, 2H) 4.50 (s, 2H) 7.40 (m, 5H) 8.25 (s, 1 H) 12.4 (bs, 1 H)

### PREPARATION 80

### 7-Chloro-4-benzylmethylamino-2,2-dimethyl-2,3-dihydro-1H-cyclopenta[4',5']pyrido[3',2':4,5]thieno[3,2-d]pyrimidine

The final product of preparation 79 (1.26g, 3.11 mmol) is suspended in phosphorous oxychloride (8 ml) and heated at 100°C for 90 min. The excess of phosphorous oxychloride is evaporated under vacuum and the residue redissolved between chloroform and water. The aqueous phase is extracted twice with chloroform and the organic phases are washed with water and brine, dried over magnesium sulfate, filtered and the solvent evaporated. 1.27g of the final compound are obtained as a brownish solid. The ¹HNMR is consistent with the desired final product. Yield= 96%.
**JT-X00248-46**
¹H NMR (200 MHz, CDCl₃) δ ppm 1.10 (s, 6H) 1.60 (t, 2H) 2.80 (m, 2H) 3.05 (s, 3H) 3.40 (s, 2H) 4.70 (s, 2H) 7.40 (m, 5H) 9.05 (s, 1 H)

### PREPARATION 81

### N⁵-Benzyl-N⁵,2,2-trimethyl-N⁸-(2-morpholin-4-ylethyl)-1,2,3,4-tetrahydropyrimido [4',5':4,5]thieno[2,3-c]isoquinoline-5,8-diamine

7-Chloro-4-benzylmethylamino-2,2-dimethyl-2,3-dihydro-1 H-cyclopenta[4',5']pyrido[3',2':4,5]thieno[3,2-d]pyrimidine (0.25g, 0.59 mmol, see Preparation 80) is suspended in ethanol (15 ml) and (2-morpholin-4-ylethyl)amine (465 µl, 3.55 mmol) is added. The mixture is refluxed overnight and then allowed to cool to room temperature. The solvent is evaporated under reduced pressure and the residue is purified by flash chromatography eluting first with dichloromethane/methanol 98:2 and then with dichloromethane/methanol 95:5. 203 mg of the final product have been isolated. Its ¹HNMR is consistent with the desired final compound. Yield= 67%.
**JT-X00248-48**
¹H NMR (300 MHz, CHLOROFORM-D) δ ppm 1.1 (s, 6 H) 1.6 (t, *J*=6.3 Hz, 2 H) 2.6 (s, 4 H) 2.7 (s, 2 H) 2.8 (t, *J*=6.6 Hz, 2 H) 2.9 (s, 3 H) 3.5 (s, 2 H) 3.7 (m, *J*=14.0 Hz, 6 H) 4.5 (s, 2 H) 5.5 (s, 1 H) 7.4 (m, 5 H) 8.7 (s, 1 H)

### PREPARATION 82

### N⁵-Benzyl-N⁵,2,2-trimethyl-N⁸-(pyridin-3-ylmethyl)-1,2,3,4-tetrahydropyrimido[4',5':4,5] thieno[2,3-c]isoquinoline-5,8-diamine

Obtained (53%) from the title compound of Preparation 80 and (pyridine-3-ylmethyl)amine following the experimental procedure described in Preparation 81.
**JT-X00248-50**
¹H NMR (300 MHz, CHLOROFORM-D) δ ppm 1.1 (s, 6 H) 1.6 (t, *J*=6.0 Hz, 2 H) 2.8 (m, 2 H) 2.9 (s, 2 H) 3.5 (s, 3 H) 4.5 (s, 3 H) 4.9 (d, *J*=5.2 Hz, 2 H) 5.1 (t, *J*=5.8 Hz, 1 H) 7.3 (m, 5 H) 7.8 (d, *J*=7.7 Hz, 1 H) 8.6 (d, *J*=4.4 Hz, 1 H) 8.7 (s, 1 H) 8.8 (s, 1 H)

### PREPARATION 83

### 1-[3-({5-[Benzyl(methyl)amino]-2,2-dimethyl-1,2,3,4-tetrahydropyrimido[4',5':4,5]thieno[2,3-c] isoquinolin-8-yl}amino)propyl]pyrrolidin-2-one

Obtained (70%) from the title compound of Preparation 80 and 1-(3-aminopropyl)pyrrolidin-2-one following the experimental procedure described in Preparation 81.
**JT-X00248-49**
¹H NMR (300 MHz, CHLOROFORM-D) δ ppm 1.1 (s, 6 H) 1.6 (m, 2 H) 1.9 (m, 2 H) 2.1 (m, 2 H) 2.5 (t, *J*=8.1 Hz, 2 H) 2.8 (t, *J*=6.5 Hz, 2 H) 2.9 (s, 3 H) 3.4 (m, 6 H) 3.7 (q, *J*=6.2 Hz, 2 H) 4.5 (s, 2 H) 6.3 (s, 1 H) 7.4 (m, 5 H) 8.7 (s, 1 H)

### EXAMPLES

### EXAMPLE 1

### 2,2-Dimethyl-5-morpholin-4-yl-N-(2-morpholin-4-ylethyl)-1,2,3,4-tetrahydropyrimido[4',5':4,5]thieno[2,3-c]isoquinolin-8-amine

**8-Chloro-2,2-dimethyl-5-morpholin-4-yl-1,2,3,4-**tetrahydropyrimido[4',5':4,5]thieno[2,3-c]isoquinoline (0.16g, 0.40 mmol, see Preparation 6) is suspended in ethanol (10 ml) and (2-morpholin-4-ylethyl)amine (0.27 ml, 2.02 mmol) is added. The mixture is refluxed overnight and then allowed to cool to room temperature. At +5°C a precipitate is formed, which is filtrated and washed with ethanol and ethyl ether. Once dried, it weighs 0.12g and its ¹HNMR is consistent with the desired final compound. Yield= 62%. m.p. 210.2-210.9°C
¹H NMR (300 MHz, DMSO-D6) δ ppm 1.07 (s, 6 H) 1.55 (t, *J*=6.10 Hz, 2 H) 1.55 (t, *J*=6.10 Hz, 2 H) 2.36 (s, 1 H) 2.45 (m, 2 H) 2.56 (t, *J*=6.87 Hz, 2 H) 2.75 (t, *J*=6.10 Hz, 2 H) 3.20 (m, 4 H) 3.43 (m, 1 H) 3.57 (m, 2 H) 3.63 (m, 2 H) 3.78 (m, 4 H) 7.64 (t, *J*=5.49 Hz, 1 H) 8.59 (s, 1 H)

### EXAMPLE 2

### 2,2-Dimethyl-5-morpholin-4-yl-N-(pyridin-4-ylmethyl)-1,2,3,4-tetrahydropyrimido[4',5':4,5]thieno[2,3-c]isoquinolin-8-amine

Obtained (45%) from the title compound of Preparation 6 and (pyridin-4-ylmethyl)amine following the experimental procedure described in Example 1. m.p. 239.9-240.8°C
¹H NMR (300 MHz, DMSO-D6) δ ppm 1.06 (s, 6 H) 1.54 (t, *J*=5.49 Hz, 2 H) 2.76 (m, 2 H) 3.22 (s, 4 H) 3.38 (m, 2H) 3.78 (s, 4 H) 4.76 (d, *J*=5.49 Hz, 2 H) 7.33 (d, *J*=4.88 Hz, 2 H) 8.37 (t, *J*=5.49 Hz, 1 H) 8.49 (d, *J*=5.49 Hz, 2 H) 8.56 (s, 1 H)

### EXAMPLE 3

### 2,2-Dimethyl-5-morpholin-4-yl-N-(pyridin-3-ylmethyl)-1,2,3,4-tetrahydropyrimido[4',5':4,5]thieno[2,3-c]isoquinolin-8-amine

Obtained (84%) from the title compound of Preparation 6 and (pyridin-3-ylmethyl)amine following the experimental procedure described in Example 1. m.p. 248.8-249.3°C
¹H NMR (300 MHz, DMSO-D6) δ ppm 1.07 (s, 6 H) 1.55 (t, *J*=5.80 Hz, 2 H) 2.76 (s, 2 H) 3.21 (s, 4 H) 3.36 (m, 2 H) 3.78 (s, 4 H) 4.77 (d, *J*=5.80 Hz, 2 H) 7.36 (m, 1 H) 7.77 (d, *J*=7.94 Hz, 1 H) 8.32 (d, *J*=6.10 Hz, 1 H) 8.46 (d, *J*=4.88 Hz, 1 H) 8.61 (m, 2 H)

### EXAMPLE 4

### 2,2-Dimethyl-5-morpholin-4-yl-N-(pyridin-2-ylmethyl)-1,2,3,4-tetrahydropyrimido[4',5':4,5]thieno[2,3-c]isoquinolin-8-amine

Obtained (82%) from the title compound of Preparation 6 and (pyridin-2-ylmethyl)amine following the experimental procedure described in Example 1.
m.p. 217.9-218.2°C
¹H NMR (300 MHz, CHLOROFORM-D) δ ppm 1.14 (s, 6 H) 1.60 (m, 2 H) 2.78 (t, *J*=6.46 Hz, 2 H) 3.31 (m, 4 H) 3.47 (s, 2 H) 3.90 (m, 4 H) 4.97 (d, *J*=4.67 Hz, 2 H) 6.26 (d, *J*=4.67 Hz, 1 H) 7.25 (dd, *J*=6.32, 3.85 Hz, 1 H) 7.37 (d, *J*=7.69 Hz, 1 H) 7.70 (m, 1 H) 8.63 (d, *J*=5.77 Hz, 1 H) 8.77 (s, 1 H)

### EXAMPLE 5

### N-(2-Methoxybenzyl)-2,2-dimethyl-5-morpholin-4-yl-1,2,3,4-tetrahydropyrimido[4',5':4,5]thieno[2,3-c]isoquinolin-8-amine

Obtained (90%) from the title compound of Preparation 6 and (2-methoxybenzyl)amine following the experimental procedure described in Example 1. m.p. 212.4-213.1°C
¹H NMR (300 MHz, CHLOROFORM-D) δ ppm 1.13 (s, 6 H) 1.60 (m, 4 H) 2.77 (t, *J*=6.46 Hz, 2 H) 3.29 (m, 4 H) 3.45 (s, 2 H) 3.88 (m, 2 H) 3.91 (s, 3H) 4.88 (d, *J*=5.77 Hz, 2 H) 5.31 (t, *J*=5.77 Hz, 1 H) 6.94 (t, *J*=7.28 Hz, 2 H) 7.30 (m, 1 H) 7.39 (m, 1 H) 8.75 (s, 1 H)

### EXAMPLE 6

### N-(2,3-Dimethoxybenzyl)-2,2-dimethyl-5-morpholin-4-yl-1,2,3,4-tetrahydropyrimido[4',5':4,5]thieno[2,3-c]isoquinolin-8-amine

Obtained (92%) from the title compound of Preparation 6 and (2,3-dimethoxybenzyl)amine following the experimental procedure described in Example 1. m.p. 219.0-220.1°C
¹H NMR (300 MHz, DMSO-D6) δ ppm 1.07 (s, 6 H) 1.55 (t, *J*=6.10 Hz, 2 H) 2.75 (d, *J*=5.19 Hz, 2 H) 3.21 (s, 4 H) 3.35 (m, 6 H) 3.79 (s, 3H) 3.81 (s, 3 H) 4.76 (d, *J*=5.49 Hz, 2 H) 6.84 (d, *J*=7.02 Hz, 1 H) 6.97 (m, 2 H) 8.16 (t, *J*=5.49 Hz, 1 H) 8.56 (s, 1 H)

### EXAMPLE 7

### 4-{[(2,2-Dimethyl-5-morpholin-4-yl-1,2,3,4-tetrahydropyrimido[4',5':4,5]thieno[2,3-c]isoquinolin-8-yl)amino]methyl}benzenesulfonamide

Obtained (51%) from the title compound of Preparation 6 and (4-methylamino)benzenesulfonamide following the experimental procedure described in Example 1. m.p. 258.2-259.5°C
¹H NMR (300 MHz, DMSO-D6) δ ppm 1.07 (s, 6 H) 1.56 (m, 2 H) 2.76 (s, 2 H) 3.21 (s, 4 H) 3.36 (m, 2 H) 3.78 (s, 4 H) 4.80 (d, *J*=5.77 Hz, 2 H) 7.31 (s, 2 H) 7.51 (d, *J*=8.24 Hz, 2 H) 7.77 (d, *J*=8.52 Hz, 2 H) 8.38 (s, 1 H) 8.57 (s, 1 H)

### EXAMPLE 8

### 2,2-Dimethyl-5-morpholin-4-yl-N-(2-pyridin-2-ylethyl)-1,2,3,4-tetrahydropyrimido[4',5':4,5]thieno[2,3-c]isoquinolin-8-amine

Obtained (83%) from the title compound of Preparation 6 and (2-pyridin-2-ylethyl)amine following the experimental procedure described in Example 1.
m.p. 237.9-239.2°C
¹H NMR (300 MHz, CHLOROFORM-D) δ ppm 1.14 (m, 6 H) 1.60 (m, 4 H) 2.77 (s, 2 H) 3.19 (t, *J*=6.18 Hz, 2 H) 3.31 (m, 4 H) 3.90 (m, 4 H) 4.06 (m, 2 H) 6.33 (m, 1 H) 7.19 (t, *J*=7.28 Hz, 2 H) 7.63 (m, 1 H) 8.62 (d, *J*=1.10 Hz, 1 H) 8.73 (s, 1 H)

### EXAMPLE 9

### 2,2-Dimethyl-5-morpholin-4-yl-N-(1-naphthylmethyl)-1,2,3,4-tetrahydropyrimido[4',5':4,5]thieno[2,3-c]isoquinolin-8-amine

Obtained (40%) from the title compound of Preparation 6 and (1-naphthylmethyl)amine following the experimental procedure described in Example 1.
m.p. 266.1-267.3°C
¹H NMR (300 MHz, CHLOROFORM-D) δ ppm 1.14 (s, 6 H) 1.60 (m, 2 H) 2.77 (t, *J*=6.18 Hz, 2 H) 3.28 (m, 4 H) 3.48 (s, 2 H) 3.88 (m, 4 H) 4.92 (s, 1 H) 5.33 (d, *J*=5.22 Hz, 2 H) 7.52 (m, 4 H) 7.90 (m, 2 H) 8.11 (dd, *J*=6.32, 3.30 Hz, 1 H) 8.84 (s, 1 H)

### EXAMPLE 10

### N-(2-Furylmethyl)-2,2-dimethyl-5-morpholin-4-yl-1,2,3,4-tetrahydropyrimido[4',5':4,5]thieno[2,3-c]isoquinolin-8-amine

Obtained (62%) from the title compound of Preparation 6 and (2-furylmethyl)amine following the experimental procedure described in Example 1.
m. p. 209.6-211.0°C
¹H NMR (300 MHz, CHLOROFORM-D) δ ppm 1.14 (s, 6 H) 1.60 (m, 2 H) 2.77 (d, *J*=6.32 Hz, 2 H) 3.31 (m, 4 H) 3.48 (m, 2 H) 3.90 (m, 4 H) 4.89 (d, *J*=5.49 Hz, 2 H) 4.97 (m, 1 H) 6.37 (m, 2 H) 7.42 (d, *J*=1.10 Hz, 1 H) 8.78 (s, 1H)

### EXAMPLE 11

### N-[2-(1H-Imidazol-4-yl)ethyl]-2,2-dimethyl-5-morpholin-4-yl-1,2,3,4-tetrahydropyrimido[4',5':4,5]thieno[2,3-c]isoquinolin-8-amine

Obtained (33%) from the title compound of Preparation 6 and 2-(1H-imidazol-4-yl)-ethylamine following the experimental procedure described in Example 1. m.p. 222.7-224.1°C
¹H NMR (400 MHz, DMSO-D6) d ppm 1.1 (s, 6 H) 1.6 (t, *J*=6.1 Hz, 2 H) 2.7 (d, *J*=6.3 Hz, 2 H) 2.9 (m, 2 H) 3.2 (m, 5 H) 3.4 (d, *J*=9.0 Hz, 2 H) 3.8 (m, 6 H) 6.9 (s, 1 H) 7.5 (s, 1 H) 7.8 (t, *J*=5.5 Hz, 1 H) 8.6 (s, 1 H)

### EXAMPLE 12

### 1-{3-[(2,2-Dimethyl-5-morpholin-4-yl-1,2,3,4-tetrahydropyrimido[4',5':4,5]thieno[2,3-c]isoquinolin-8-yl)amino]propyl}pyrrolidin-2-one

Obtained (81 %) from the title compound of Preparation 6 and 1-(3-aminopropyl)-pyrrolidin-2-one following the experimental procedure described in Example 1. m.p. 209.6-210.2°C
¹H NMR (400 MHz, CHLOROFORM-D) d ppm 1.1 (s, 6 H) 1.6 (m, 2 H) 1.9 (m, 2 H) 2.1 (m, 2 H) 2.5 (t, *J*=8.2 Hz, 2 H) 2.8 (t, *J*=6.5 Hz, 2 H) 3.3 (m, 4 H) 3.5 (m, 6 H) 3.7 (q, *J*=6.1 Hz, 2 H) 3.9 (m, 4 H) 6.3 (t, *J*=6.1 Hz, 1 H) 8.7 (s, 1 H)

### EXAMPLE 13

### 2,2-Dimethyl-N-(2-morpholin-4-ylethyl)-5-thiomorpholin-4-yl-1,2,3,4-tetrahydropyrimido[4',5':4,5]thieno[2,3-c]isoquinolin-8-amine

8-Chloro-2,2-dimethyl-5-thiomorpholin-4-yl-1,2,3,4-tetrahydropyrimido[4',5':4,5]thieno[2,3-c]isoquinoline (0.07g, 0.17 mmol, see Preparation 10) is suspended in ethanol (5 ml) and (2-morpholin-4-ylethyl)amine (0.11 ml, 0.86 mmol) is added. The mixture is refluxed overnight and then allowed to cool to room temperature. At +5°C a precipitate is formed, which is filtrated and washed with ethanol and ethyl ether. Once dried, it weighs 0.05g and its ¹HNMR is consistent with the desired final compound.
Yield= 55%.
m.p. 188.2-189.3°C
¹H NMR (300 MHz, CHLOROFORM-D) δ ppm 1.13 (s, 6 H) 1.60 (m, 2 H) 2.56 (d, *J*=4.67 Hz, 2 H) 2.73 (q, *J*=6.50 Hz, 4 H) 2.85 (m, 4 H) 3.45 (s, 2 H) 3.54 (m, 4 H) 3.74 (m, 8 H) 5.56 (m, 1 H) 8.73 (m, 1 H)

### EXAMPLE 14

### 2,2-Dimethyl-N-(pyridin-4-ylmethyl)-5-thiomorpholin-4-yl-1,2,3,4-tetrahydropyrimido[4',5':4,5]thieno[2,3-c]isoquinolin-8-amine

Obtained (46%) from the title compound of Preparation 10 and (pyridine-4-ylmethyl)amine following the experimental procedure described in Example 13. m.p. 236.8-237.4°C
1 H NMR (400 MHz, CHLOROFORM-D) d ppm 1.1 (m, 6 H) 1.6 (m, 2 H) 2.7 (t, *J*=6.3 Hz, 2 H) 2.8 (m, 4 H) 3.5 (s, 2 H) 3.5 (m, 4 H) 4.9 (d, *J*=5.9 Hz, 2 H) 5.1 (t, *J*=6.1 Hz, 1 H) 7.3 (m, 2 H) 8.6 (m, 2 H) 8.7 (s, 1 H)

### EXAMPLE 15

### 2,2-Dimethyl-N-(pyridin-3-ylmethyl)-5-thiomorpholin-4-yl-1,2,3,4-tetrahydropyrimido[4',5':4,5]thieno[2,3-c]isoquinolin-8-amine

Obtained (43%) from the title compound of Preparation 10 and (pyridin-3-ylmethyl)amine following the experimental procedure described in Example 13. m.p. 257.1-258.0°C
¹H NMR (300 MHz, CHLOROFORM-D) δ ppm 1.13 (s, 6 H) 1.60 (m, 2 H) 2.73 (d, *J*=6.04 Hz, 2 H) 2.84 (m, 4 H) 3.45 (s, 2 H) 3.54 (m, 4 H) 4.93 (d, *J*=5.77 Hz, 2 H) 5.02 (s, 1 H) 7.29 (m, 1 H) 7.76 (m, 1 H) 8.56 (dd, *J*=4.67, 1.65 Hz, 1 H) 8.69 (d, *J*=1.65 Hz, 1 H) 8.76 (s, 1 H)

### EXAMPLE 16

### 2,2-Dimethyl-N-(pyridin-2-ylmethyl)-5-thiomorpholin-4-yl-1,2,3,4-tetrahydropyrimido[4',5':4,5]thieno[2,3-c]isoquinolin-8-amine

Obtained (84%) from the title compound of Preparation 10 and 1-(3-aminopropyl)pyrrolidin-2-one following the experimental procedure described in Example 13.
m.p. 224.7-225.8°C
¹H NMR (400 MHz, CHLOROFORM-D) d ppm 1.1 (s, 6 H) 1.6 (m, 2 H) 2.7 (t, *J*=6.3 Hz, 2 H) 2.8 (m, 4 H) 3.5 (s, 2 H) 3.5 (m, 4 H) 5.0 (d, *J*=4.7 Hz, 2 H) 6.3 (t, *J*=4.5 Hz, 1 H) 7.2 (m, 1 H) 7.4 (d, *J*=7.4 Hz, 1 H) 7.7 (t, *J*=7.6 Hz, 1 H) 8.6 (d, *J*=4.3 Hz, 1 H) 8.8 (s, 1 H)

### EXAMPLE 17

### N-(2,3-Dimethoxybenzyl)-2,2-dimethyl-5-thiomorpholin-4-yl-1,2,3,4-tetrahydropyrimido[4',5':4,5]thieno[2,3-c]isoquinolin-8-amine

Obtained (70%) from the title compound of Preparation 10 and (2,3-dimethoxybenzyl)amine following the experimental procedure described in Example 13. m.p. 112.5-113.9°C
¹H NMR (300 MHz, CHLOROFORM-D) δ ppm 1.12 (s, 6 H) 1.59 (m, 2 H) 2.73 (m, 2 H) 2.84 (m, 4 H) 3.45 (s, 2 H) 3.53 (m, 4 H) 3.89 (s, 3 H) 3.93 (s, 3 H) 4.90 (d, *J*=5.77 Hz, 2 H) 5.17 (s, 1 H) 6.90 (dd, *J*=7.42, 2.47 Hz, 1 H) 7.02 (m, 2 H) 8.75 (s, 1 H)

### EXAMPLE 18

### 1-{3-[(2,2-Dimethyl-5-thiomorpholin-4-yl-1,2,3,4-tetrahydropyrimido[4',5':4,5]thieno[2,3-c]isoquinolin-8-yl)amino]propyl}pyrrolidin-2-one

Obtained (84%) from the title compound of Preparation 10 and 1-(3-aminopropyl)pyrrolidin-2-one following the experimental procedure described in Example 13.
m.p. 219.2-220.7°C
¹H NMR (400 MHz, CHLOROFORM-D) d ppm 1.2 (m, 6 H) 1.6 (m, 2 H) 1.9 (d, *J*=5.1 Hz, 2 H) 2.1 (m, 2 H) 2.5 (t, *J*=8.0 Hz, 2 H) 2.7 (t, *J*=6.3 Hz, 2 H) 2.8 (dd, *J*=4.9, 2.5 Hz, 4 H) 3.4 (m, 6 H) 3.5 (m, 4 H) 3.7 (q, *J*=6.3 Hz, 2 H) 6.3 (m, 1 H) 8.7 (d, *J*=2.7 Hz, 1 H)

### EXAMPLE 19

### Ethyl 4-{2-[(2,2-dimethyl-5-thiomorpholin-4-yl-1,2,3,4-tetrahydropyrimido[4',5':4,5]thieno[2,3-c]isoquinolin-8-yl)amino]ethyl)piperazine-1-carboxylate

Obtained (41 %) from the title compound of Preparation 10 and ethyl 4(2-aminoethyl)piperazine-1-carboxylate following the experimental procedure described in Example 13.
m.p. 182.9-183.8°C
¹H NMR (400 MHz, CHLOROFORM-D) d ppm 1.1 (d, *J*=21.1 Hz, 6 H) 1.2 (t, 3 H) 1.6 (m, 4 H) 2.5 (s, 4 H) 2.7 (q, *J*=6.1 Hz, 4 H) 2.8 (m, 4 H) 3.5 (m, 2 H) 3.5 (d, *J*=14.9 Hz, 6 H) 3.7 (q, *J*=5.2 Hz, 2 H) 4.2 (q, *J*=7.2 Hz, 2 H) 5.5 (m, *J*=4.7 Hz, 1 H) 8.7 (s, 1 H)

### EXAMPLE 20

### 2,2-Dimethyl-5-(4-methylpiperazin-1-yl)-N-(2-morpholin-4-ylethyl)-1,2,3,4-tetrahydropyrimido[4',5':4,5]thieno[2,3-c]isoquinolin-8-amine

8-Chloro-2,2-dimethyl-5-(4-methylpiperazin-yl)-1,2,3,4-tetrahydropyrimido[4',5':4,5]thieno[2,3-c]isoquinoline (0.08g, 0.20 mmol, see Preparation 14) is suspended in ethanol (5 ml) and (2-morpholin-4-ylethyl)amine (0.13 ml, 1.00 mmol) is added. The mixture is refluxed overnight and then allowed to cool to room temperature. The solvent is evaporated under pressure and the residue is purified by flash chromatography eluting with CH₂Cl₂/MeOH 95:5. 0.05g of the desired final product are isolated. Its ¹HNMR is consistent with the desired final compound. Yield= 50%.
m.p. 150-150.8°C
¹H NMR (300 MHz, CHLOROFORM-D) d ppm 1.1 (s, 6 H) 1.6 (m, 2 H) 2.4 (s, 3 H) 2.5 (m, 3 H) 2.6 (s, 4 H) 2.7 (m, 4 H) 3.3 (m, 4 H) 3.4 (s, 2 H) 3.7 (m, 7 H) 5.5 (m, *J*=4.0, 4.0 Hz, 1 H) 8.7 (s, 1 H).

### EXAMPLE 21

### 2,2-Dimethyl-5-(4-methylpiperazin-1-yl)-N-(pyridin-3-ylmethyl)-1,2,3,4-tetrahydropyrimido[4',5':4,5]thieno[2,3-c]isoquinolin-8-amine

Obtained (46%) from the title compound of Preparation 14 and (pyridin-3-ylmethyl)amine following the experimental procedure described in Example 20.
m.p. 210.0-211.6°C
1 H NMR (300 MHz, CHLOROFORM-D) δ ppm 1.13 (s, 6H) 1.59 (t, *J*=6.26 Hz, 2 H) 2.42 (s, 2H) 2.67 (d, *J*=4.27 Hz, 4 H) 2.76 (t, *J*=6.26 Hz, 2 H) 3.38 (m, 4 H) 3.45 (s, 3 H) 4.92 (d, *J*=5.80 Hz, 2 H) 5.13 (t, *J*=5.80 Hz, 1 H) 7.28 (m, 1 H) 7.75 (m, 1 H) 8.55 (dd, *J*=4.73, 1.68 Hz, 1 H) 8.68 (d, *J*=1.83 Hz, 1 H) 8.75 (s, 1 H)

### EXAMPLE 22

### 2,2-Dimethyl-5-(4-methylpiperazin-1-yl)-N-(pyridin-2-ylmethyl)-1,2,3,4-tetrahydropyrimido[4',5':4,5]thieno[2,3-c]isoquinolin-8-amine

Obtained (80%) from the title compound of Preparation 14 and (pyridin-2-ylmethyl)amine following the experimental procedure described in Example 20.
m.p. 200.2-201.8°C
¹H NMR (300 MHz, CHLOROFORM-D) δ ppm 1.16 (m, 6 H) 1.59 (m, 2 H) 2.45 (s, 3 H) 2.75 (m, 6 H) 3.48 (m, 6 H) 4.97 (d, *J*=4.88 Hz, 2 H) 6.25 (d, *J*=5.80 Hz, 1 H) 7.28 (m, 1 H) 7.35 (s, 1 H) 7.70 (m, 1 H) 8.64 (s, 1 H) 8.78 (m, 1 H)

### EXAMPLE 23

### 2,2-Dimethyl-N-(2-morpholin-4.-ylethyl)-5-pyrrolidin-1-yl-1,2,3,4-tetrahydropyrimido[4',5':4,5]thieno[2,3-c]isoquinolin-8-amine

8-Chloro-2,2-dimethyl-5-pyrrolidin-1-yl-1,2,3,4-tetrahydropyrimido[4',5':4,5]thieno[2,3-c]isoquinoline (0.08g, 0.21 mmol, see Preparation 18) is suspended in ethanol (5 ml) and (2-morpholin-4-ylethyl)amine (0.14 ml, 1.07 mmol) is added. The mixture is refluxed overnight and then allowed to cool to room temperature. After evaporation of the solvent under vacuum, the residue is purified by flash chromatography, eluting first with dichloromethane and then with CH₂Cl₂:MeOH 95:5. 0.05g of the desired final compound are isolated. Yield= 50%.
m.p. 173.3-174.0°C
¹H NMR (300 MHz, DMSO-D6) δ ppm 1.06 (s, 6 H) 1.50 (t, *J*=6.10 Hz, 2 H) 1.90 (m, 4 H) 2.44 (m, 4 H) 2.56 (m, 2 H) 2.77 (t, *J*=5.95 Hz, 2 H) 3.30 (s, 2 H) 3.60 (m, 10 H) 7.41 (t, *J*=5.49 Hz, 1 H) 8.52 (s, 1 H)

### EXAMPLE 24

### 2,2-Dimethyl-N-(pyridin-4-ylmethyl)-5-pyrrolidin-1-yl-1,2,3,4-tetrahydropyrimido[4',5':4,5]thieno[2,3-c]isoquinolin-8-amine

Obtained (28%) from the title compound of Preparation 18 and (pyridin-4-ylmethyl)amine following the experimental procedure described in Example 23.
¹H NMR (300 MHz, DMSO-D6) δ ppm 1.06 (s, 6 H) 1.51 (t, *J*=5.80 Hz, 2 H) 1.90 (m, 4 H) 2.78 (t, *J*=6.41 Hz, 2 H) 3.30 (m, 2 H) 3.62 (t, *J*=5.65 Hz, 4 H) 4.74 (d, *J*=5.49 Hz, 2 H) 7.32 (d, *J*=5.80 Hz, 2 H) 8.16 (t, *J*=5.80 Hz, 1 H) 8.49 (m, *J*=5.80 Hz, 3 H)

### EXAMPLE 25

### (2,2-Dimethyl-N-(pyridin-3-ylmethyl)-5-pyrrolidin-1-yl-1,2,3,4-tetrahydropyrimido[4',5':4,5]thieno[2,3-c]isoquinolin-8-amine

Obtained (42%) from the title compound of Preparation 18 and (pyridin-3-ylmethyl)amine following the experimental procedure described in Example 23.
¹H NMR (300 MHz, DMSO-D6) □ppm 1.06 (s, 6 H) 1.50 (t, *J*=6.26 Hz, 2 H) 1.89 (m, 4 H) 2.77 (t, *J*=6.10 Hz, 2 H) 3.30 (m, 2 H) 3.60 (m, 4 H) 4.74 (d, *J*=5.80 Hz, 2 H) 7.35 (dd, *J*=7.78, 4.73 Hz, 1 H) 7.75 (m, 1 H) 8.13 (t, *J*=5.95 Hz, 1 H) 8.45 (dd, *J*=4.88, 1.53 Hz, 1 H) 8.53 (s, 1 H) 8.60 (d, *J*=1.53 Hz, 1 H)

### EXAMPLE 26

### 2,2-Dimethyl-N-(pyridin-2-ylmethyl)-5-pyrrolidin-1-yl-1,2,3,4-tetrahydropyrimido[4',5':4,5]thieno[2,3-c]isoquinolin-8-amine

Obtained (58%) from the title compound of Preparation 18 and (pyridin-2-ylmethyl)amine following the experimental procedure described in Example 23.
m.p. 211.7-212.6°C
¹H NMR (300 MHz, DMSO-D6) δ ppm 1.09 (m, 6 H) 1.51 (s, 2 H) 1.90 (s, 4 H) 2.78 (m, 2 H) 3.31 (m, 2 H) 3.62 (s, 4 H) 4.81 (d, *J*=6.04 Hz, 2 H) 7.28 (m, 2 H) 7.73 (m, 1 H) 8.14 (d, *J*=6.32 Hz, 1 H) 8.50 (m, 2 H)

### EXAMPLE 27

### N-(2-Methoxybenzyl)-2,2-dimethyl-5-pyrrolidin-1-yl-1,2,3,4-tetrahydropyrimido[4',5':4,5]thieno[2,3-c]isoquinolin-8-amine

Obtained (66%) from the title compound of Preparation 18 and 2-methoxybenzylamine following the experimental procedure described in Example 23.
m.p. 186.4-188.9°C
¹H NMR (300 MHz, DMSO-D6) δ ppm 1.06 (s, 6 H) 1.50 (t, *J*=6.56 Hz, 2 H) 1.89 (m, 4 H) 2.77 (t, *J*=5.95 Hz, 2 H) 3.31 (m, 2 H) 3.61 (t, *J*=6.10 Hz, 4 H) 3.85 (s, 3 H) 4.69 (d, *J*=5.49 Hz, 2 H) 6.86 (t, *J*=7.78 Hz, 1 H) 7.00 (d, *J*=7.63 Hz, 1 H) 7.13 (m, 1 H) 7.22 (m, 1 H) 7.92 (t, *J*=5.80 Hz, 1 H) 8.48 (s, 1 H)

### EXAMPLE 28

### N-(2,3-Dimethoxybenzyl)-2,2-dimethyl-5-pyrrolidin-1-yl-1,2,3,4-tetrahydropyrimido[4',5':4,5]thieno[2,3-c]isoquinolin-8-amine

Obtained (33%) from the title compound of Preparation 18 and 2,3-dimethoxybenzylamine following the experimental procedure described in Example 23. m.p. 193.5-195.0°C
¹H NMR (300 MHz, DMSO-D6) δ ppm 1.06 (s, 6 H) 1.51 (d, *J*=1.22 Hz, 2 H) 1.90 (m, 4 H) 2.77 (m, 2 H) 3.32 (m, 2 H) 3.61 (s, 4 H) 3.79 (s, 3 H) 3.80 (s, 3 H) 4.74 (d, *J*=5.80 Hz, 2 H) 6.82 (m, 1 H) 6.96 (m, 2 H) 7.95 (s, 1 H) 8.49 (s, 1 H)

### EXAMPLE 29

### N-(2-Furylmethyl)-2,2-dimethyl-5-pyrrolidin-1-yl-1,2,3,4-tetrahydropyrimido[4',5':4,5]thieno[2,3-c]isoquinolin-8-amine

Obtained (27%) from the title compound of Preparation 18 and (2-furylmethyl)amine following the experimental procedure described in Example 23.
m.p. 201.0-201.6°C
¹H NMR (400 MHz, CHLOROFORM-D) d ppm 1.1 (s, 6 H) 1.6 (m, 7 H) 2.0 (m, 4 H) 2.8 (t, *J*=6.4 Hz, 3 H) 3.3 (s, 2 H) 3.7 (m, 4 H) 8.9 (s, 1 H)

### EXAMPLE 30

### N-[2-(1H-Imidazol-4-yl)ethyl]-2,2-dimethyl-5-pyrrolidin-1-yl-1,2,3,4-tetrahydropyrimido[4',5':4,5]thieno[2,3-c]isoquinolin-8-amine

Obtained (93%) from the title compound of Preparation 18 and 2-(1 H-imidazol-4-yl)ethylamine following the experimental procedure described in Example 23.
m.p. 192.0-192.7°C
¹H NMR (400 MHz, DMSO-D6) d ppm 1.1 (s, 6 H) 1.5 (t, *J*=6.0 Hz, 2 H) 1.9 (m, 4 H) 2.8 (d, *J*=6.2 Hz, 2 H) 2.8 (s, 2 H) 3.3 (m, 4 H) 3.6 (m, 4 H) 3.7 (m, 2 H) 7.5 (s, 1 H) 7.6 (t, *J*=5.4 Hz, 1 H) 8.5 (s, 1 H)

### EXAMPLE 31

### 1-{3-[(2,2-Dimethyl-5-pyrrolidin-1-yl-1,2,3,4-tetrahydropyrimido[4',5':4,5]thieno[2,3-c]isoquinolin-8-yl)amino]propyl}pyrrolidin-2-one

Obtained (49%) from the title compound of Preparation 18 and 1-(3-aminopropyl)pyrrolidin-2-one following the experimental procedure described in Example 23.
m.p. 199.6-200.5°C
¹H NMR (400 MHz, CHLOROFORM-D) d ppm 1.1 (m, 6 H) 1.6 (m, 3 H) 1.9 (dd, *J*=12.2, 6.0 Hz, 2 H) 1.9 (m, 4 H) 2.0 (m, 2 H) 2.5 (m, 2 H) 2.8 (t, *J*=6.4 Hz, 2 H) 3.4 (m, 5 H) 3.6 (m, 6 H) 6.0 (t, *J*=6.0 Hz, 1 H) 8.7 (s, 1 H)

### EXAMPLE 32

### Ethyl 4-{2-[(2,2-dimethyl-5-pyrrolidin-1-yl-1,2,3,4-tetrahydropyrimido[4',5':4,5]thieno[2,3-c]isoquinolin-8-yl)amino]ethyl}piperazine-1-carboxylate

Obtained (89%) from the title compound of Preparation 18 and ethyl 4-(2-aminoethyl)-piperazine-1-carboxylate following the experimental procedure described in Example 23.
m.p. 76.2-77.8°C
¹H NMR (400 MHz, CHLOROFORM-D) d ppm 1.1 (d, *J*=6.6 Hz, 6 H) 1.3 (m, 3 H) 1.6 (m, 3 H) 2.0 (m, 4 H) 2.5 (m, 4 H) 2.7 (t, *J*=6.0 Hz, 2 H) 2.8 (t, *J*=6.4 Hz, 2 H) 3.4 (s, 2 H) 3.5 (m, 4 H) 3.7 (m, 5 H) 4.1 (m, 2 H) 5.4 (t, *J*=4.6 Hz, 1 H) 8.7 (s, 1 H)

### EXAMPLE 33

### 2,2-Dimethyl-N-(2-piperazin-1-ylethyl)-5-pyrrolidin-1-yl-1,2,3,4-tetrahydropyrimido[4',5':4,5]thieno[2,3-c]isoquinolin-8-amine

Ethyl 4-12-[(2,2-dimethyl-5-pyrrolidin-1-yl-1,2,3,4-tetrahydropyrimido[4',5':4,5]thieno[2,3-c]isoquinolin-8-yl)amino]ethyl}piperazine-1-carboxylate (0.07g, 0.13 mmol, see Example 32) is dissolved in isopropanol (3 ml). Potassium hydroxide 85% (0.09 g, 1.3 mmol) is added and the reaction mixture is refluxed overnight. The solvent is evaporated under reduced pressure and the residue is fractioned between water and chloroform. After the usual work-up, the organic residue is purified through a flash-chromatography column, eluting first with CH₂Cl₂/MeOH 95:5 and then with CH₂Cl₂/MeOH 9:1. 0.02g of the desired final product are obtained. Yield= 36%
LRMS: m/z 466 (M+1)⁺

### EXAMPLE 34

### 2,2-Dimethyl-N-[2-(4-methylpiperazin-1-yl)ethyl]-5-pyrrolidin-1-yl-1,2,3,4-tetrahydropyrimido[4',5':4,5]thieno[2,3-c]isoquinolin-8-amine

Ethyl 4-{2-[(2,2-dimethyl-5-pyrrolidin-1-yl-1,2,3,4-tetrahydropyrimido[4',5':4,5]thieno[2,3-c]isoquinolin-8-yl)amino]ethyl}piperazine-1-carboxylate (0.08g, 0.14 mmol, see Example 32) dissolved in tetrahydrofuran (2 ml) is dropwise added at room temperature to a suspension of lithium aluminium hydride (0.01 g, 0.29 mmol) in tetrahydrofuran (1 ml). After 3h refluxing, water (50 µl), NaOH 2N (200 µl) and water (50 µl) are succsessively added. The inorganic salts are filtered through Celite^{®} , the solvent is evaporated under reduced pressure and the residue fractioned between water and chloroform. After the usual work-up, the residue is is purified through a flash-chromatography column, eluting first with CH₂Cl₂/MeOH 95:5 and then with CH₂Cl₂/MeOH 9:1. 0.02g of the desired final product are obtained. Yield= 34%
LRMS: m/z 480 (M+1)⁺

### EXAMPLE 35

### N2-(2,2-Dimethyl-5-pyrrolidin-1-yl-1,2,3,4-tetrahydropyrimido[4',5':4,5]thieno[2,3-c]isoquinolin-8-yl)-N1-(tetrahydrofuran-2-ylmethyl)glycinamide

Obtained (29%) from the title compound of Preparation 18 and the title compound of Preparation 25 following the experimental procedure described in Example 23.
m.p. 209.7-211.3°C
¹H NMR (400 MHz, DMSO-D6) d ppm 1.1 (m, 6 H) 1.5 (m, 4 H) 1.8 (m, 5 H) 2.8 (t, *J*=6.2 Hz, 2 H) 3.2 (t, *J*=5.8 Hz, 1 H) 3.3 (s, 4 H) 3.4 (d, *J*=7.0 Hz, 1 H) 3.6 (m, 4 H) 3.7 (d, *J*=6.2 Hz, 1 H) 3.8 (t, *J*=6.2 Hz, 1 H) 4.1 (d, *J*=6.2 Hz, 2 H) 7.7 (s, 1 H) 8.0 (m, 1 H) 8.5 (s, 1 H)

### EXAMPLE 36

### 2,2-Dimethyl-5-pyrrolidin-1-yl-N-(qui nolin-3-ylmethyl)-1,2,3,4-hydropyrimido[4',5':4,5]thieno[2,3-c]isoquinolin-8-amine

Obtained (54%) from the title compound of Preparation 18 and quinolin-3-ylmethylamine following the experimental procedure described in Example 23.
m.p.226.1-226.9°C
¹H NMR (400 MHz, DMSO-D6) d ppm 1.1 (s, 6 H) 1.5 (t, *J*=6.2 Hz, 2 H) 1.9 (m, 4 H) 2.8 (t, *J*=6.0 Hz, 2 H) 3.3 (d, *J*=4.1 Hz, 2 H) 3.6 (m, 4 H) 4.9 (d, *J*=6.2 Hz, 2 H) 7.6 (t, *J*=7.5 Hz, 1 H) 7.7 (m, 1 H) 8.0 (m, 2 H) 8.2 (m, 2 H) 8.5 (s, 1 H) 9.0 (d, *J*=2.1 Hz, 1 H)

### EXAMPLE 37

### 2,2-Dimethyl-5-pyrrolidin-1-yl-N-(isoquinolin-4-ylmethyl)-1,2,3,4-tetrahydropyrimido[4',5':4,5]thieno[2,3-c]isoquinolin-8-amine

Obtained (62%) from the title compound of Preparation 18 and isoquinolin-4-ylmethylamine following the experimental procedure described in Example 23.
m. p.275.9-276.7°C
¹H NMR (400 MHz, DMSO-D6) d ppm 1.1 (s, 6 H) 1.3 (m, 2 H) 1.5 (t, *J*=6.5 Hz, 2 H) 1.9 (s, 4 H) 2.8 (m, 2 H) 3.6 (s, 4 H) 5.2 (d, *J*=5.9 Hz, 2 H) 7.7 (m, 1 H) 7.8 (m, 1 H) 8.1 (s, 1 H) 8.2 (d, *J*=8.2 Hz, 1 H) 8.3 (d, *J*=8.2 Hz, 1 H) 8.5 (s, 1 H) 8.6 (s, 1 H) 9.3 (s, 1 H)

### EXAMPLE 38

### N⁵,N⁵,2,2-tetramethyl-N⁸-(2-morpholin-4-ylethyl)-1,2,3,4-tetrahydropyrimido[4',5':4,5]thieno[2,3-c]isoquinoline-5,8-diamine

8-Chloro-5-dimethylamino-2,2-dimethyl-1,2,3,4-tetrahydropyrimido[4',5':4,5]thieno[2,3-c]isoquinoline (0.07g, 0.20 mmol, see Preparation 22) is suspended in ethanol (5 ml) and (2-morpholin-4-ylethyl)amine (0.13 ml, 1.01 mmol) is added. The mixture is refluxed overnight and then allowed to cool to room temperature. After evaporation of the solvent under vacuum, the residue is purified by flash chromatography, eluting first with CH₂Cl₂:MeOH 95:5 and then with CH₂Cl₂:MeOH 98:2. 0.07g of the desired final compound are isolated. Yield= 76%.
m.p. 173.4-175.0°C
¹H NMR (300 MHz, CHLOROFORM-D) δ ppm 1.14 (s, 6 H) 1.58 (m, 4 H) 2.55 (m, 4 H) 2.74 (m, 4 H) 2.99 (s, 6 H) 3.45 (m, 2 H) 3.73 (m, 4 H) 5.51 (m, 1 H) 8.71 (s, 1 H)

### EXAMPLE 39

### N⁵,N⁵,2,2-Tetramethyl-N⁸-(pyridin-3-ylmethyl)-1,2,3,4-tetrahydropyrimido[4',5':4,5] thieno[2,3-c]isoquinoline-5,8-diamine

Obtained (73%) from the title compound of Preparation 22 and (2-morpholino-4-ylethyl)amine following the experimental procedure described in Example 38.
m.p. 208.4-209.1°C
¹H NMR (300 MHz, CHLOROFORM-D) δ ppm 1.14 (s, 6 H) 1.59 (m, 2 H) 2.77 (t, *J*=6.46 Hz, 2 H) 3.00 (s, 6 H) 3.43 (s, 2 H) 4.92 (d, *J*=5.77 Hz, 2 H) 5.01 (m, *J*=5.77 Hz, 1 H) 7.28 (m, 1 H) 7.75 (m, 1 H) 8.55 (dd, *J*=4.81, 1.51 Hz, 1 H) 8.68 (d, *J*=2.20 Hz, 1 H) 8.74 (s, 1 H)

### EXAMPLE 40

### N⁸-(2,3-Dimethoxybenzyl)-N⁵,N⁵,2,2-tetramethyl-1,2,3,4-tetrahydropyrimido[4',5':4,5]thieno[2,3-c]isoquinoline-5,8-diamine

Obtained (98%) from the title compound of Preparation 22 and (2,3-dimethoxybenzyl)amine following the experimental procedure described in Example 38.
m.p.91.9-93.0°C
¹H NMR (300 MHz, CHLOROFORM-D) δ ppm 1.13 (s, 6 H) 1.58 (m, 2 H) 2.76 (t, *J*=6.32 Hz, 2 H) 2.97 (d, *J*=4.67 Hz, 6 H) 3.43 (s, 2 H) 3.88 (m, 3 H) 3.93 (s, 3 H) 4.90 (d, *J*=5.77 Hz, 2 H) 5.13 (m, 1 H) 6.90 (m, 1 H) 7.03 (m, 2 H) 8.74 (s, 1 H)

### EXAMPLE 41

### N⁸-[2-(1H-Imidazol-5-yl)ethyl]-N⁵,N⁵,2,2-tetramethyl-1,2,3,4-tetrahydropyrimido[4',5':4,5]thieno[2,3-c]isoquinoline-5,8-diamine

Obtained (49%) from the title compound of Preparation 22 and (2,3-dimethoxybenzyl)amine following the experimental procedure described in Example 38.
m.p. 212.6-213.9°C
¹H NMR (400 MHz, CHLOROFORM-D) d ppm 1.2 (s, 6 H) 1.6 (t, *J*=5.7 Hz, 2 H) 2.8 (d, *J*=6.3 Hz, 2 H) 3.0 (m, 9 H) 3.7 (d, *J*=2.0 Hz, 2 H) 4.0 (d, *J*=6.3 Hz, 2 H) 6.9 (s, 1 H) 7.3 (m, *J*=2.0 Hz, 1 H) 7.7 (s, 1 H) 8.7 (d, *J*=1.6 Hz, 1 H)

### EXAMPLE 42

### 1-(3-{[5-(Dimethytamino)-2,2-dimethyl-1,2,3,4-tetrahydropyrimido[4',5':4,5]thieno[2,3-c]isoquinolin-8-yl]amino}propyl)pyrrolidin-2-one

Obtained (92%) from the title compound of Preparation 22 and 1-(3-aminopropyl)pyrrolidin-2-one following the experimental procedure described in Example 38.
m.p. 214.3-215.0°C
¹H NMR (400 MHz, CHLOROFORM-D) d ppm 1.1 (m, 6 H) 1.6 (m, 2 H) 1.9 (m, 2 H) 2.1 (m, 2 H) 2.5 (t, *J*=8.2 Hz, 2 H) 2.8 (t, *J*=6.3 Hz, 2 H) 3.0 (m, 6 H) 3.4 (q, *J*=7.3 Hz, 6 H) 3.7 (q, *J*=6.3 Hz, 2 H) 6.2 (t, *J*=6.3 Hz, 1 H) 8.7 (s, 1 H)

### EXAMPLE 43

### Ethyl 4-(2-{[5-(dimethylamino)-2,2-dimethyl-1,2,3,4-tetrahydropyrimido[4',5':4,5]thieno[2,3-c]isoquinolin-8-yl]amino}ethyl)piperazine-1-carboxylate

Obtained (89%) from the title compound of Preparation 22 and ethyl 4-(2-aminoethyl)-piperazine-1-carboxylate following the experimental procedure described in Example 38.
m.p. 138.1-140.0°C
¹H NMR (400 MHz, CHLOROFORM-D) δ ppm 1.2 (m, 6 H) 1.3 (t, *J*=7.0 Hz, 3 H) 1.6 (m, 2 H) 2.5 (m, 4 H) 2.7 (m, 4 H) 3.0 (m, 6 H) 3.4 (s, 2 H) 3.5 (m, 4 H) 3.7 (m, 2 H) 4.2 (q, *J*=7.0 Hz, 2 H) 5.5 (t, *J*=4.3 Hz, 1 H) 8.7 (s, 1 H)

### EXAMPLE 44

### N⁵,N⁵,2,2-Tetramethyl-N⁸-(2-piperazin-1-ylethyl)-1,2,3,4-tetrahydropyrimido[4',5':4,5]thieno[2,3-c]isoquinoline-5,8-diamine

Ethyl 4-(2-{[5-(dimethylamino)-2,2-dimethyl-1,2,3,4-tetrahydropyrimido[4',5':4,5]thieno[2,3-c]isoquinolin-8-yl]amino}ethyl)piperazine-1-carboxylate (0.13g, 0.26 mmol, see Example 43) is dissolved in isopropanol (5 ml). Potassium hydroxide 85% (0.17 g, 2.6 mmol) is added and the reaction mixture is refluxed overnight. The solvent is evaporated under reduced pressure and the residue is fractioned between water and chloroform. After the usual work-up, the organic residue is purified through a flash-chromatography column, eluting first with CH₂Cl₂/MeOH 98:2 and then with CH₂Cl₂/MeOH 9:1. 0.03g of the desired final product are obtained. Yield= 26%
m.p. 193.0-194.2°C
¹H NMR (400 MHz, CHLOROFORM-D) d ppm 1.1 (m, *J*=20.0 Hz, 6 H) 1.6 (t, *J*=6.5 Hz, 3 H) 2.6 (s, 4 H) 2.7 (m, 4 H) 3.0 (s, 6 H) 3.02 (m, 4H) 3.4 (s, 2 H) 3.7 (m, *J*=5.6, 5.6, 5.6 Hz, 2 H) 5.5 (t, *J*=4.5 Hz, 1H) 8.7 (s, 1 H)

### EXAMPLE 45

### 3-{[5-(Dimethylamino)-2,2-dimethyl-1,2,3,4-tetrahydropyrimido[4',5':4,5]thieno[2,3-c]isoquinolin-8-yl]amino}propanenitrile

Obtained (9%) from the title compound of Preparation 22 and 3-aminopropionitril following the experimental procedure described in Example 38.
LRMS: m/z 381 (M+1)⁺

### EXAMPLE 46

### 8-Ethoxy-N,N,2,2-tetramethyl-1,2,3,4-tetrahydropyrimido[4',5':4,5]thieno[2,3-c]isoquinolin-5-amine

Obtained (39%) from the title compound of Preparation 22 and ethanol following the experimental procedure described in Example 38.
m. p. 219.6-220.7°C
¹H NMR (400 MHz, CHLOROFORM-D) d ppm 1.1 (s, 6 H) 1.5 (m, 5 H) 2.8 (t, *J*=6.1 Hz, 2 H) 3.0 (s, 6 H) 3.4 (s, 2 H) 4.6 (q, *J*=6.8 Hz, 2 H) 8.8 (m, *J*=1.6 Hz, 1 H)

### EXAMPLE 47

### N⁵-Ethyl-N⁵,2,2-trimethyl-N⁸-(2-morpholin-4-ylethyl)-1,2,3,4-tetrahydropyrimido[4',5':4,5]thieno[2,3-c]isoquinoline-5,8-diamine

8-Chloro-5-ethylmethylamino-2,2-dimethyl-1,2,3,4-tetrahydropyrimido[4',5':4,5]thieno[2,3-c]isoquinoline (0.06g, 0.17 mmol, see Preparation 29) is suspended in ethanol (5 ml) and (2-morpholin-4-ylethyl)amine (0.13 ml, 1.01 mmol) is added. The mixture is refluxed overnight and then allowed to cool to room temperature. After evaporation of the solvent under vacuum, the residue is purified by flash chromatography, eluting first with CH₂Cl₂ and then with CH₂Cl₂:MeOH 99:1. 0.04g of the desired final compound are isolated. Yield= 51%.
m.p. 143.6-144.4°C
¹H NMR (400 MHz, CHLOROFORM-D) d ppm 1.1 (d, *J*=21.6 Hz, 6 H) 1.2 (t, *J*=7.3 Hz, 3 H) 1.6 (m, 5 H) 2.5 (s, 2 H) 2.7 (m, 3 H) 3.0 (s, 3 H) 3.3 (q, *J*=7.2 Hz, 2 H) 3.4 (s, 2 H) 3.7 (m, 5 H) 5.5 (m, 1 H) 8.7 (s, 1 H)

### EXAMPLE 48

### N⁵-Ethyl-N⁵,2,2-trimethyl-N⁸-(pyridin-4-ylmethyl)-1,2,3,4-tetrahydropyrimido[4',5':4,5]thieno[2,3-c]isoquinoline-5,8-diamine

Obtained (47%) from the title compound of Preparation 29 and pyridin-4-ylmethylamine following the experimental procedure described in Example 47.
m.p. 206.7-207.2°C
¹H NMR (400 MHz, CHLOROFORM-D) d ppm 1.1 (s, 6 H) 1.2 (m, 2 H) 1.6 (m, 3 H) 2.7 (t, *J*=6.2 Hz, 2 H) 3.0 (s, 3 H) 3.3 (q, *J*=7.0 Hz, 2 H) 3.5 (m, 2 H) 4.9 (d, *J*=6.2 Hz, 2 H) 5.1 (t, *J*=5.6 Hz, 1 H) 7.3 (d, *J*=6.2 Hz, 2 H) 8.6 (d, *J*=5.8 Hz, 2 H) 8.7 (s, 1H)

### EXAMPLE 49

### N⁵-Ethyl-N⁵,2,2-trimethyl-N⁸-(pyridin-3-ylmethyl)-1,2,3,4-tetrahydropyrimido[4',5':4,5]thieno[2,3-c]isoquinoline-5,8-diamine

Obtained (71 %) from the title compound of Preparation 29 and pyridin-3-ylmethylamine following the experimental procedure described in Example 47.
¹H NMR (400 MHz, CHLOROFORM-D) d ppm 1.1 (s, 6 H) 1.3 (m, 2 H) 1.6 (m, 3 H) 2.7 (t, *J*=5.8 Hz, 2 H) 3.0 (s, 3 H) 3.3 (q, *J*=7.0 Hz, 2 H) 3.4 (s, 2 H) 4.9 (d, *J*=5.4 Hz, 2 H) 5.0 (d, *J*=4.6 Hz, 1 H) 7.3 (m, 1 H) 7.8 (dd, *J*=7.9, 1.7 Hz, 1 H) 8.6 (d, *J*=4.6 Hz, 1 H) 8.7 (s, 1 H) 8.8 (d, *J*=2.1 Hz, 1 H)

### EXAMPLE 50

### N⁵-Ethyl-N⁵,2,2-trimethyl-N⁸-(pyridin-2-ylmethyl)-1,2,3,4-tetrahydropyrimido[4',5':4,5]thieno[2,3-c] isoquinoline-5,8-diamine

Obtained (67%) from the title compound of Preparation 29 and pyridin-2-ylmethylamine following the experimental procedure described in Example 47.
m.p. 151.6-152.1°C
¹H NMR (400 MHz, CHLOROFORM-D) d ppm 1.1 (s, 6 H) 1.2 (t, *J*=7.0 Hz, 2 H) 1.6 (t, 3 H) 2.7 (t, *J*=6.4 Hz, 2 H) 3.0 (s, 3 H) 3.3 (q, *J*=7.0 Hz, 2 H) 3.4 (s, 2 H) 5.0 (d, *J*=4.6 Hz, 2 H) 6.2 (t, *J*=4.6 Hz, 1 H) 7.2 (m, 1 H) 7.4 (d, *J*=7.9 Hz, 1 H) 7.7 (m, 1 H) 8.6 (d, *J*=5.0 Hz, 1 H) 8.7 (s, 1H)

### EXAMPLE 51

### N⁵-Ethyl-N⁸-(2-furylmethyl)-N⁵,2,2-trimethyl-1,2,3,4-tetrahydropyrimido[4',5':4,5]thieno[2,3-c]isoquinoline-5,8-diamine

Obtained (66%) from the title compound of Preparation 29 and furan-2-ylmethylamine following the experimental procedure described in Example 47.
m.p. 116.4-117.7°C
¹H NMR (400 MHz, DMSO-D6) d ppm 1.1 (s, 6 H) 1.5 (t, *J*=6.4 Hz, 3 H) 2.7 (t, *J*=6.2 Hz, 2 H) 2.9 (s, 2 H) 3.2 (q, *J*=7.0 Hz, 2 H) 3.3 (s, 3 H) 3.4 (s, 2 H) 4.7 (d, *J*=5.8 Hz, 2 H) 6.3 (d, *J*=2.9 Hz, 1 H) 6.4 (m, 1 H) 7.6 (s, 1 H) 8.1 (t, *J*=5.6 Hz, 1 H) 8.6 (s, 1H)

### EXAMPLE 52

### N⁵-Ethyl-N⁸-[2-(1H-imidazol-4-yl)ethyl]-N⁵,2,2-trimethyl-1,2,3,4-tetrahydropyrimido[4',5':4,5]thieno[2,3-c]isoquinoline-5,8-diamine

Obtained (32%) from the title compound of Preparation 29 and 2-(1 H-imidazol-4-yl)ethylamine following the experimental procedure described in Example 47.
m.p. 202.4-203.8°C
¹H NMR (400 MHz, DMSO-D6) d ppm 1.1 (s, 6 H) 1.2 (m, *J*=7.0, 7.0 Hz, 3 H) 1.5 (t, *J*=6.2 Hz, 2 H) 2.7 (t, *J*=6.2 Hz, 2 H) 2.9 (m, 2 H) 2.9 (s, 3 H) 3.2 (q, *J*=7.3 Hz, 2 H) 3.4 (s, 2 H) 3.7 (m, 2 H) 6.8 (s, 1H) 7.5 (s, 1 H) 7.7 (t, *J*=5.8 Hz, 1 H) 8.6 (s, 1H) 11.8 (s, 1 H)

### EXAMPLE 53

### 1-[3-({5-[Ethyl(methyl)amino]-2,2-dimethyl-1,2,3,4-tetrahydropyrimido[4',5':4,5]thieno[2,3-c]isoquinolin-8-yl}amino)propyl]pyrrolidin-2-one

Obtained (96%) from the title compound of Preparation 29 and 1-(3-aminopropyl)pyrrolidin-2-one following the experimental procedure described in Example 47.
m.p. 166.5-167.2°C
¹H NMR (400 MHz, CHLOROFORM-D) d ppm 1.1 (s, 6 H) 1.2 (t, *J*=7.0 Hz, 2 H) 1.6 (m, 5 H) 1.9 (m, 2 H) 2.5 (m, 2 H) 2.7 (t, *J*=6.2 Hz, 2 H) 2.9 (s, 3 H) 3.3 (q, *J*=7.0 Hz, 2 H) 3.4 (m, 6 H) 3.7 (q, *J*=6.2 Hz, 2 H) 6.2 (t, *J*=6.4 Hz, 1 H) 8.7 (s, 1 H)

### EXAMPLE 54

### N²-{5-[Ethyl(methyl)amino]-2,2-dimethyl-1,2,3,4-tetrahydropyrimido[4',5':4,5]thieno[2,3-c]isoquinolin-8-yl}-N'-(tetrahydrofuran-2-ylmethyl)glycinamide

Obtained (21 %) from the title compound of Preparation 29 and 2-amino-N-(tetrahydrofuran-2-ylmethyl)acetamide (see Preparation 25) following the experimental procedure described in Example 47.
m.p. 103.1-104.8°C
¹H NMR (400 MHz, DMSO-D6) d ppm 1.1 (s, 6 H) 1.2 (t, *J*=6.8 Hz, 2 H) 1.5 (m, 3 H) 1.8 (m, 2 H) 2.7 (t, *J*=5.8 Hz, 2 H) 2.9 (s, 2 H) 3.2 (t, *J*=5.6 Hz, 2 H) 3.3 (q, *J*=7.0 Hz, 2 H) 3.3 (s, 3 H) 3.4 (s, 2 H) 3.6 (m, 1 H) 3.7 (m, 1 H) 3.8 (m, 1 H) 4.1 (d, *J*=5.8 Hz, 2 H) 7.9 (t, *J*=5.8 Hz, 1 H) 8.0 (t, *J*=5.8 Hz, 1 H) 8.5 (s, 1 H)

### EXAMPLE 55

### 2,2,5-Trimethyl-N-(2-morpholin-4-ylethyl)-1,2,3,4-tetrahydropyrimido[4',5':4,5]thieno[2,3-c]isoquinolin-8-amine

8-Chloro-2,2, 5-trimethyl-1, 2, 3,4-tetrahydropyrimido[4', 5':4,5]thieno[2, 3-c]isoquinoline (0.05g, 0.16 mmol, see Preparation 36) is suspended in ethanol (5 ml) and (2-morpholin-4-ylethyl)amine (0.10 ml, 0.79 mmol) is added. The mixture is refluxed overnight and then allowed to cool to room temperature. After evaporation of the solvent under vacuum, the residue is purified by flash chromatography, eluting with CH₂Cl₂:MeOH 99:1. 0.06g of the desired final compound are isolated. Yield= 95%.
¹H NMR (300 MHz, CHLOROFORM-D) d ppm 1.1 (s, 6 H) 1.6 (s, 2 H) 1.7 (t, *J*=6.6 Hz, 2 H) 2.6 (m, 4 H) 2.6 (s, 3 H) 2.7 (t, *J*=6.0 Hz, 2 H) 2.8 (t, *J*=6.7 Hz, 2 H) 3.5 (s, 2 H) 3.7 (m, 4 H) 5.6 (t, *J*=4.4 Hz, 1 H) 8.8 (s, 1 H)

### EXAMPLE 56

### 2,2,5-Trimethyl-N-(pyridin-3-ylmethyl)-1,2,3,4-tetrahydropyrimido[4',5':4,5]thieno[2,3-c]isoquinolin-8-amine

Obtained (66%) from the title compound of Preparation 36 and pyridin-3-ylmethylamine following the experimental procedure described in Example 55.
¹H NMR (400 MHz, CHLOROFORM-D) d ppm 1.1 (m, 6 H) 1.7 (t, *J*=6.8 Hz, 2 H) 2.6 (s, 3 H) 2.8 (t, *J*=6.7 Hz, 2 H) 3.5 (s, 2 H) 4.9 (d, *J*=6.3 Hz, 2 H) 5.1 (t, *J*=7.0 Hz, 1 H) 7.3 (dd, *J*=8.0, 5.3 Hz, 1 H) 7.8 (d, *J*=8.2 Hz, 1 H) 8.6 (d, *J*=4.3 Hz, 1 H) 8.7 (s, 1 H) 8.8 (s, 1 H)

### EXAMPLE 57

### 1-{3-[(2,2,5-Trimethyl-1,2,3,4-tetrahydropyrimido[4',5':4,5]thieno[2,3-c]isoquinolin-8-yl)amino]propyl}pyrrolidin-2-one

Obtained (72%) from the title compound of Preparation 36 and 1-(3-aminopropyl)pyrrolidin-2-one following the experimental procedure described in Example 55.
m.p. 204. 8-206.3°C
¹H NMR (400 MHz, CHLOROFORM-D) d ppm 1.1 (s, 6 H) 1.6 (s, 3 H) 1.9 (m, 2 H) 2.1 (m, 2 H) 2.5 (t, *J*=8.0 Hz, 2 H) 2.6 (s, 3 H) 2.8 (t, *J*=6.7 Hz, 2 H) 3.4 (m, 5 H) 3.7 (q, *J*=5.9 Hz, 2 H) 6.2 (t, *J*=5.9 Hz, 1 H) 8.7 (m, 1 H)

### EXAMPLE 58

### 5-Isobutyl-2,2-dimethyl-N-(2-morpholin-4-ylethyl)-1,2,3,4-tetrahydropyrimido[4',5':4,5]thieno[2,3-c]isoquinolin-8-amine

8-Chloro-2,2-dimethyl-5-isobutyl-1,2,3,4-tetrahydropyrimido[4',5':4,5]thieno[2,3-c]isoquinoline (0.05g, 0.14 mmol, see Preparation 40) is suspended in ethanol (5 ml) and (2-morpholin-4-ylethyl)amine (0.09 ml, 0.69 mmol) is added. The mixture is refluxed overnight and then allowed to cool to room temperature. After evaporation of the solvent under vacuum, the residue is purified by flash chromatography, eluting with CH₂Cl₂:MeOH 99:1. 0.04 g of the desired final compound are isolated. Yield= 68%.
m.p. 189.9-190.5°C
¹H NMR (400 MHz, CHLOROFORM-D) d ppm 1.0 (d, 6 H) 1.1 (s, 6 H) 1.6 (s, 2 H) 1.7 (t, *J*=6.8 Hz, 2 H) 2.3 (m, 1 H) 2.6 (s, 4 H) 2.7 (t, *J*=6.1 Hz, 2 H) 2.8 (d, *J*=7.0 Hz, 2 H) 2.9 (t, *J*=6.5 Hz, 2 H) 3.5 (s, 2 H) 3.7 (dd, *J*=12.1, 4.7 Hz, 4 H) 5.6 (m, 1 H) 8.8 (s, 1 H)

### EXAMPLE 59

### 5-isobutyl-2,2-dimethyl-N-(pyridin-3-ylmethyl)-1,2,3,4-tetrahydropyrimido[4',5':4,5]thieno[2,3-c]isoquinolin-8-amine

Obtained (89%) from the title compound of Preparation 40 and pyridin-3-ylmethylamine following the experimental procedure described in Example 58.
m.p. 229.9-230.4°C
¹H NMR (400 MHz, CHLOROFORM-D) d ppm 1.0 (d, 6 H) 1.1 (m, 6 H) 1.7 (t, *J*=6.7 Hz, 2 H) 2.3 (m, 1 H) 2.8 (d, *J*=7.0 Hz, 2 H) 2.9 (t, *J*=6.5 Hz, 2 H) 3.5 (s, 2 H) 4.9 (d, *J*=5.9 Hz, 2 H) 5.1 (t, *J*=5.9 Hz, 1 H) 7.3 (m, 1 H) 7.7 (d, *J*=8.2 Hz, 1 H) 8.6 (m, 1 H) 8.7 (s, 1 H) 8.8 (s, 1 H)

### EXAMPLE 60

### 1-{3-[(5-Isobutyl-2,2-dimethyl-1,2,3,4-tetrahydropyrimido[4',5':4,5]thieno[2,3-c]isoquinolin-8-yl)amino]propyl)pyrrolidin-2-one

Obtained (82%) from the title compound of Preparation 40 and 1-(3-aminopropyl)pyrrolidin-2-one following the experimental procedure described in Example 58.
m.p. 175.5-176.1°C
¹H NMR (400 MHz, CHLOROFORM-D) d ppm 1.0 (d, *J*=6.7 Hz, 6 H) 1.1 (m, *J*=7.8 Hz, 6 H) 1.7 (t, *J*=6.7 Hz, 2 H) 1.9 (m, 2 H) 2.1 (m, 3 H) 2.3 (m, 2 H) 2.5 (t, *J*=8.2 Hz, 2 H) 2.8 (d, *J*=7.0 Hz, 2 H) 2.9 (t, *J*=6.7 Hz, 2 H) 3.4 (m, 2 H) 3.5 (s, 2 H) 3.7 (q, *J*=6.3 Hz, 2 H) 6.2 (t, *J*=5.9 Hz; 1 H) 8.7 (s, 1 H)

### EXAMPLE 61

### Ethyl 4-{2-[(5-isobutyl-2,2-dimethyl-1,2,3,4-tetrahydropyrimido[4',5':4,5]thieno[2,3-c]isoquinolin-8-yl)amino]ethyl}piperazine-1-carboxylate

Obtained (39%) from the title compound of Preparation 40 and ethyl 4-(2-aminoethyl)piperazine-1-carboxylate following the experimental procedure described in Example 58.
m.p. 185.3-186.0°C
¹H NMR (400 MHz, DMSO-D6) d ppm 1.0 (d, *J*=6.7 Hz, 6 H) 1.0 (s, 6 H) 1.2 (t, *J*=7.0 Hz, 3 H) 1.6 (t, *J*=6.7 Hz, 2 H) 2.2 (d, *J*=7.0 Hz, 1 H) 2.4 (t, 4 H) 2.6 (t, *J*=6.8 Hz, 2 H) 2.8 (d, *J*=7.0 Hz, 2 H) 2.8 (t, *J*=6.5 Hz, 2 H) 3.3 (d, 3 H) 3.4 (s, 2 H) 3.7 (m, 2 H) 4.0 (q, *J*=7.0 Hz, 2 H) 7.7 (t, *J*=5.3 Hz, 1 H) 8.6 (s, 1 H) 13.1 (m, 1 H)

### EXAMPLE 62

### 5-(2-Furyl)-2,2-dimethyl-N-(2-morpholin-4-ylethyl)-1,2,3,4-tetrahydropyrimido[4',5':4,5]thieno[2,3-c]isoquinolin-8-amine

8-Chloro-5-(2-furyl)-2,2-dimethyl-1,2,3,4-tetrahydropyrimido[4',5':4,5]thieno[2,3-c]isoquinoline (0.05g, 0.14 mmol, see Preparation 44) is suspended in ethanol (5 ml) and (2-morpholin-4-ylethyl)amine (0.09 ml, 0.69 mmol) is added. The mixture is refluxed overnight and then allowed to cool to room temperature. After evaporation of the solvent under vacuum, the residue is purified by flash chromatography, eluting first with CH₂Cl₂ and then with CH₂Cl₂:MeOH 99:1. 0.05 g of the desired final compound are isolated.
Yield= 75%.
m.p. 123.0-125.1°C
¹H NMR (400 MHz, CHLOROFORM-D) d ppm 1.1 (s, 6 H) 1.7 (t, *J*=6.7 Hz, 2 H) 2.6 (s, 4 H) 2.7 (m, *J*=6.1, 6.1 Hz, 2 H) 3.1 (t, *J*=6.7 Hz, 2 H) 3.6 (s, 2 H) 3.8 (m, 6 H) 5.6 (t, *J*=4.3 Hz, 1 H) 6.6 (dd, *J*=3.3, 1.8 Hz, 1 H) 7.1 (d, *J*=3.5 Hz, 1 H) 7.7 (d, *J*=2.0 Hz, 1 H) 8.8 (s, 1 H)

### EXAMPLE 63

### 5-(2-Furyl)-2,2-dimethyl-N-(pyridin-4-ylmethyl)-1,2,3,4-tetrahydropyrimido[4',5':4,5]thieno[2,3-c]isoquinolin-8-amine

Obtained (38%) from the title compound of Preparation 44 and pyridin-4-ylmethylamine following the experimental procedure described in Example 62.
LRMS: m/z 442 (M+1)⁺

### EXAMPLE 64

### 5-(2-Furyl)-2,2-dimethyl-N-(pyridin-3-ylmethyl)-1,2,3,4-tetrahydropyrimido[4',5':4,5]thieno[2,3-c]isoquinolin-8-amine

Obtained (42%) from the title compound of Preparation 44 and pyridin-3-ylmethylamine following the experimental procedure described in Example 62.
¹H NMR (400 MHz, CHLOROFORM-D) d ppm 1.1 (s, 6 H) 1.7 (t, *J*=6.7 Hz, 2 H) 3.1 (t, *J*=6.7 Hz, 2 H) 3.6 (s, 2 H) 4.9 (d, *J*=5.9 Hz, 2 H) 5.1 (t, *J*=5.1 Hz, 1 H) 6.6 (dd, *J*=3.3, 1.8 Hz, 1 H) 7.1 (d, *J*=3.5 Hz, 1 H) 7.3 (dd, *J*=8.2, 5.1 Hz, 1 H) 7.7 (s, 1 H) 7.8 (d, *J*=8.2 Hz, 1 H) 8.6 (d, *J*=5.1 Hz, 1 H) 8.7 (d, *J*=1.6 Hz, 1 H) 8.8 (s, 1 H)

### EXAMPLE 65

### 5-(2-Furyl)-2,2-dimethyl-N-(pyridin-2-ylmethyl)-1,2,3,4-tetrahydropyrimido[4',5':4,5]thieno[2,3-c]isoquinolin-8-amine

Obtained (33%) from the title compound of Preparation 44 and pyridin-2-ylmethylamine following the experimental procedure described in Example 62.
m.p. 232.6-233.4°C
¹H NMR (400 MHz, CHLOROFORM-D) d ppm 1.2 (m, 6 H) 1.7 (t, *J*=6.7 Hz, 2 H) 3.1 (t, *J*=6.7 Hz, 2 H) 3.6 (s, 2 H) 5.0 (d, *J*=5.1 Hz, 2 H) 6.3 (t, *J*=4.7 Hz, 1 H) 6.6 (dd, *J*=3.5, 2.0 Hz, 1 H) 7.1 (d, *J*=3.1 Hz, 1 H) 7.2 (m, 1 H) 7.4 (d, *J*=7.4 Hz, 1 H) 7.7 (m, 2 H) 8.6 (d, *J*=5.1 Hz, 1 H) 8.8 (s, 1 H)

### EXAMPLE 66

### 1-(3-{[5-(2-Furyl)-2,2-dimethyl-1,2,3,4-tetrahydropyrimido[4',5':4,5]thieno[2,3-c]isoquinolin-8-yl]amino}propyl)pyrrolidin-2-one

Obtained (72%) from the title compound of Preparation 44 and 1-(3-aminopropyl)pyrrolidin-2-one following the experimental procedure described in Example 62.
m.p. 213. 7-214.2°C
¹H NMR (400 MHz, CHLOROFORM-D) d ppm 1.1 (d, *J*=16.0 Hz, 6 H) 1.7 (t, *J*=6.7 Hz, 2 H) 1.9 (m, 2 H) 2.1 (m, 2 H) 2.5 (t, *J*=8.0 Hz, 2 H) 3.2 (t, *J*=6.8 Hz, 2 H) 3.4 (m, 4 H) 3.6 (s, 2 H) 3.7 (q, *J*=6.3 Hz, 2 H) 6.3 (m, 1 H) 6.6 (dd, *J*=3.5, 1.6 Hz, 1 H) 7.1 (d, *J*=3.5 Hz, 1 H) 7.7 (s, 1 H) 8.7 (s, 1 H)

### EXAMPLE 67

### Ethyl 4-(2-{[5-(2-furyl)-2,2-dimethyl-1,2,3,4-tetrahydropyrimido[4',5':4,5]thieno[2,3-c]isoquinolin-8-yl]amino}ethyl)piperazine-1-carboxylate

Obtained (42%) from the title compound of Preparation 44 and ethyl 4-(2-aminoethyl)-piperazine-1-carboxylate following the experimental procedure described in Example 62.
m.p. 99.1-100.8°C
¹H NMR (400 MHz, CHLOROFORM-D) d ppm 1.1 (s, 6 H) 1.3 (m, 3 H) 2.5 (s, 4 H) 2.7 (t, *J*=6.1 Hz,2H)3.1 (t, *J*=6.7 Hz, 2 H) 3.5 (m, 8 H) 3.7 (m, 2 H) 4.2 (q, *J*=7.0 Hz, 2 H) 5.6 (m, 1 H) 6.6 (dd, *J*=3.5, 2.0 Hz, 1 H) 7.1 (d, *J*=2.7 Hz, 1 H) 7.7 (d, *J*=1.6 Hz, 1 H) 8.8 (s, 1 H)

### EXAMPLE 68

### 5-(3-Furyl)-2,2-dimethyl-N-(2-morpholin-4-ylethyl)-1,2,3,4-tetrahydropyrimido[4',5':4,5]thieno[2,3-c]isoquinolin-8-amine

8-Chloro-5-(3-furyl)-2,2-dimethyl-1,2,3,4-tetrahydropyrimido[4',5':4,5]thieno[2,3-c]isoquinoline (0.05g, 0.14 mmol, see Preparation 48) is suspended in ethanol (5 ml) and (2-morpholin-4-ylethyl)amine (0.09 ml, 0.69 mmol) is added. The mixture is refluxed overnight and then allowed to cool to room temperature. After evaporation of the solvent under vacuum, the residue is purified by flash chromatography, eluting first with CH₂Cl₂ and then with CH₂Cl₂:MeOH 98:2. 0.05 g of the desired final compound are isolated.
Yield= 79%.
m.p. 181.7-183.1°C
¹H NMR (400 MHz, CHLOROFORM-D) d ppm 1.1 (s, 6 H) 2.6 (m, 4 H) 2.7 (m, 2 H) 3.0 (t, *J*=6.7 Hz, 2 H) 3.5 (q, *J*=6.9 Hz, 2 H) 3.6 (s, 2 H) 3.8 (m, 6 H) 5.6 (t, *J*=3.9 Hz, 1 H) 7.1 (d, *J*=2.0 Hz, 1 H) 7.5 (d, *J*=1.6 Hz, 1 H) 8.0 (s, 1 H) 8.8 (s, 1 H)

### EXAMPLE 69

### 5-(3-Furyl)-2,2-dimethyl-N-(pyridin-2-ylmethyl)-1,2,3,4-tetrahydropyrimido[4',5':4,5]thieno[2,3-c]isoquinolin-8-amine

Obtained (51 %) from the title compound of Preparation 48 and pyridin-2-ylmethylamine following the experimental procedure described in Example 68.
m.p. 234.8-235.6°C
¹H NMR (400 MHz, DMSO-D6) d ppm 1.0 (s, 6 H) 1.7 (t, *J*=6.5 Hz, 2 H) 2.9 (t, *J*=6.3 Hz, 2 H) 3.5 (s, 2 H) 4.8 (d, *J*=5.9 Hz, 2 H) 7.2 (s, 1 H) 7.2 (m, 1 H) 7.3 (d, *J*=7.8 Hz, 1 H) 7.7 (m, 1 H) 7.8 (s, 1 H) 8.3 (s, 1 H) 8.5 (m, 2 H) 8.6 (s, 1 H)

### EXAMPLE 70

### 5-(3-Furyl)-2,2-dimethyl-N-(pyridin-3-ylmethyl)-1,2,3,4-tetrahydropyrimido[4',5':4,5]thieno[2,3-c]isoquinolin-8-amine

Obtained (83%) from the title compound of Preparation 48 and pyridin-3-ylmethylamine following the experimental procedure described in Example 68.
m.p. 247.1-248.3°C
¹H NMR (400 MHz, CHLOROFORM-D) d ppm 1.1 (d, *J*=20.3 Hz, 6 H) 1.7 (t, *J*=6.5 Hz, 2 H) 3.0 (t, *J*=6.5 Hz, 2 H) 3.6 (s, 2 H) 4.9 (d, *J*=6.3 Hz, 2 H) 5.1 (t, *J*=6.3 Hz, 1 H) 7.1 (d, *J*=2.0 Hz, 1 H) 7.3 (m, 1 H) 7.5 (m, 1 H) 7.8 (m, 1 H) 8.0 (s, 1 H) 8.6 (dd, *J*=4.9, 1.8 Hz, 1 H) 8.7 (d, *J*=2.0 Hz, 1 H) 8.8 (s, 1 H)

### EXAMPLE 71

### 5-(3-Furyl)-2,2-dimethyl-N-(pyridin-4-ylmethyl)-1,2,3,4-tetrahydropyrimido[4',5':4,5]thieno[2,3-c]isoquinolin-8-amine

Obtained (59%) from the title compound of Preparation 48 and pyridin-4-ylmethylamine following the experimental procedure described in Example 68.
m.p. 264.6-266.0°C
¹H NMR (400 MHz, DMSO-D6) d ppm 1.0 (s, 6 H) 1.7 (t, *J*=6.5 Hz, 2 H) 3.0 (t, *J*=6.5 Hz, 2 H) 3.5 (s, 2 H) 4.8 (d, *J*=5.9 Hz, 2 H) 5.7 (s, 1 H) 7.2 (s, 1 H) 7.3 (d, *J*=5.9 Hz, 2 H) 7.8 (s, 1 H) 8.3 (s, 1 H) 8.5 (dd, *J*=5.9, 4.3 Hz, 2 H) 8.6 (s, 1 H)

### EXAMPLE 72

### 5-(3-Furyl)-N-[2-(1 H-imidazol-4-yl)ethyl]-2,2-dimethyl-1,2,3,4-tetrahydropyrimido[4',5':4,5]thieno[2,3-c]isoquinolin-8-amine

Obtained (78%) from the title compound of Preparation 48 and 2-(1 H-imidazol-4-yl)-ethylamine following the experimental procedure described in Example 68.
m.p. 265.3-266.6°C
¹H NMR (400 MHz, DMSO-D6) d ppm 1.0 (m, 6 H) 1.7 (t, *J*=6.5 Hz, 2 H) 2.9 (t, *J*=6.5 Hz, 4 H) 3.5 (s, 2 H) 3.7 (m, 2 H) 6.9 (s, 1 H) 7.1 (s, 1 H) 7.5 (s, 1 H) 7.8 (s, 1 H) 7.9 (t, *J*=5.5 Hz, 1 H) 8.3 (s, 1 H) 8.6 (s, 1 H) 11.8 (s, 1 H)

### EXAMPLE 73

### 1-(3-{[5-(3-Furyl)-2,2-dimethyl-1,2,3,4-tetrahydropyrimido[4',5':4,5]thieno[2,3-c]isoquinolin-8-yl]amino}propyl)pyrrolidin-2-one

Obtained (94%) from the title compound of Preparation 48 and 1-(3-aminopropyl)-pyrrolidin-2-one following the experimental procedure described in Example 68.
m.p. 193.5-194.8°C
¹H NMR (400 MHz, CHLOROFORM-D) d ppm 1.1 (s, 6 H) 1.7 (t, *J*=6.7 Hz, 2 H) 1.9 (d, *J*=5.9 Hz, 2 H) 2.1 (m, 2 H) 2.5 (t, *J*=8.2 Hz, 2 H) 3.0 (t, *J*=6.7 Hz, 2 H) 3.5 (m, 4 H) 3.6 (s, 2 H) 3.7 (q, *J*=6.3 Hz, 2 H) 6.4 (m, 1 H) 7.1 (s, 1 H) 7.5 (t, *J*=1.8 Hz, 1 H) 8.0 (s, 1 H) 8.7 (s, 1 H)

### EXAMPLE 74

### Ethyl 4-(2-{[5-(3-furyl)-2,2-dimethyl-1,2,3,4-tetrahydropyrimido[4',5':4,5]thieno[2,3-c]isoquinolin-8-yl]amino}ethyl)piperazine-1-carboxylate

Obtained (69%) from the title compound of Preparation 48 and ethyl 4-(2-aminoethyl)-piperazine-1-carboxylate following the experimental procedure described in Example 68.
m.p. 85.1-86.4°C
¹H NMR (400 MHz, CHLOROFORM-D) d ppm 1.1 (m, 6 H) 1.3 (t, *J*=7.0 Hz, 3 H) 1.7 (t, *J*=6.7 Hz, 2 H) 2.5 (m, 4 H) 2.7 (t, *J*=6.1 Hz, 2 H) 3.0 (t, *J*=6.7 Hz, 2 H) 3.5 (m, 6 H) 3.8 (m, 2 H) 4.2 (q, J=7.2 Hz, 2 H) 5.6 (t, *J*=4.1 Hz, 1 H) 7.1 (d, *J*=2.0 Hz, 1 H) 7.5 (m, 1 H) 8.0 (s, 1 H) 8.8 (s, 1 H)

### EXAMPLE 75

### 2,2,3-Trimethyl-5-morpholin-4-yl-N-(2-morpholin-4-ylethyl)-1,2,3,4-tetrahydropyrimido[4',5':4,5]thieno[2,3-c]-2,7-naphthyridin-8-amine

8-Chloro-2,2,3-trimethyl-5-morpholin-4-yl-1,2,3,4-tetrahydropyrimido[4',5':4,5]thieno[2,3-c]-2,7-naphthyridine (0.07g, 0.17 mmol, see Preparation 23) is suspended in ethanol (5 ml) and (2-morpholin-4-ylethyl)amine (0.11 ml, 0.87 mmol) is added. The mixture is refluxed overnight and then allowed to cool to room temperature. After evaporation of the solvent under vacuum, the residue is purified by flash chromatography, eluting first with dichloromethane and then with CH₂Cl₂:MeOH 99:1. 0.05g of the desired final compound are isolated. Yield= 53%.
m.p. 103.2-105.1°C
¹H NMR (300 MHz, DMSO-D6) δ ppm 1.13 (s, 6 H) 2.31 (m, 2 H) 2.50 (m, 4 H) 2.57 (m, 1 H) 3.20 (s, 4 H) 3.33 (d, *J*=7.02 Hz, 5 H) 3.57 (m, 4 H) 3.63 (s, 4 H) 3.77 (d, *J*=5.19 Hz, 4 H) 8.59 (s, 1 H)

### EXAMPLE 76

### 2,2,3-Trimethyl-5-morpholin-4-yl-N-(pyridin-3-ylmethyl)-1,2,3,4-tetrahydropyrimido[4',5':4,5]thieno[2,3-c]-2,7-naphthyridin-8-amine

Obtained (50%) following the experimental procedure described in Example 75 using (pyridine-3-ylmethyl)amine instead of (2-morpholino-4-ylethyl)amine.
m.p. 224.6-225.0°C
¹H NMR (300 MHz, DMSO-D6) δ ppm 1.10 (m, 6 H) 2.31 (s, 3 H) 3.21 (m, 4 H) 3.41 (s, 2 H) 3.63 (s, 2 H) 3.77 (m, 4 H) 4.77 (d, *J*=5.19 Hz, 2 H) 7.35 (dd, *J*=7.78, 4.43 Hz, 1 H) 7.77 (m, 1 H) 8.36 (d, *J*=5.80 Hz, 1 H) 8.46 (d, *J*=1.83 Hz, 1 H) 8.60 (m, 2 H)

### EXAMPLE 77

### N-(2-Methoxybenzyl)-2,2,3-trimethyl-5-morpholin-4-yl-1,2,3,4-tetrahydropyrimido[4',5':4,5]thieno[2,3-c]-2,7-naphthyridin-8-amine

Obtained (67%) following the experimental procedure described in Example 75 using (2-methoxybenzyl)amine instead of (2-morpholino-4-ylethyl)amine.
m.p. 73.8-75.9°C
¹H NMR (300 MHz, CHLOROFORM-D) δ ppm 1.28 (d, *J*=13.12 Hz, 6 H) 2.48 (d, *J*=5.49 Hz, 2 H) 3.26 (m, 4 H) 3.64 (s, 2 H) 3.85 (m, 7 H) 3.90 (m, 3 H) 4.86 (dd, *J*=11.14, 6.26 Hz, 2 H) 5.33 (m, *J*=5.49 Hz, 1 H) 6.92 (m, 2 H) 7.30 (m, 1 H) 7.39 (d, *J*=7.63 Hz, 1 H) 8.73 (s, 1 H)

### EXAMPLE 78

### N4,N4,2,2-Tetramethyl-N'-(2-morpholin-4-ylethyl)-2,3-dihydro-1 H-cyclopenta[4',5']pyrido[3',2':4,5]thieno[3,2-d]pyrimidin-4,7-diamine

7-Chloro-N,N,2,2-tetramethyl-2,3-dihydro-1 H-cyclopenta[4',5']pyrido[3',2':4,5]thieno[3,2-d]pyrimidin-4-amine (0.1g, 0.30 mmol, see Preparation 54) is suspended in ethanol (7 ml) and (2-morpholin-4-ylethyl)amine (0.20 ml, 1.50 mmol) is added. The mixture is refluxed overnight and then allowed to cool to room temperature. After evaporation of the solvent under vacuum, the residue is purified by flash chromatography, eluting first with CH₂Cl₂:MeOH 99.5:0.5 and then with CH₂Cl₂:MeOH 98:2. 0.10g of the desired final compound are isolated. Yield= 77%.
m.p. 174.9-176.0°C
¹H NMR (400 MHz, DMSO-D6) d ppm 1.1 (s, 6 H) 2.4 (m, 4 H) 2.5 (m, 2 H) 2.9 (s, 2 H) 3.19 (s, 2H) 3.3 (s, 6 H) 3.6 (m, 6 H) 7.5 (s, 1 H) 8.5 (s, 1 H)

### EXAMPLE 79

### N⁴, N⁴, 2, 2-Tetramethyl-N⁷-(pyridin-3-ylmethyl)-2,3-dihydro-1H-cyclopenta[4',5']pyrido[3',2':4,5]thieno[3,2-d]pyirimidin-4,7-diamine

Obtained (83%) from the title compound of Preparation 54 and pyridin-3-ylmethylamine following the experimental procedure described in Example 78.
m.p. 248.6-249.4°C
¹H NMR (400 MHz, DMSO-D6) d ppm 1.2 (s, 6 H) 2.9 (s, 2 H) 3.1 (s, 6 H) 3.2 (d, J=7.0 Hz, 2 H) 4.7 (d, *J*=5.9 Hz, 2 H) 7.3 (dd, *J*=7.8, 4.7 Hz, 1 H) 7.8 (m, 1 H) 8.2 (d, *J*=5.5 Hz, 1 H) 8.4 (dd, *J*=4.7, 1.6 Hz, 1 H) 8.5 (s, 1 H) 8.6 (s, 1 H)

### EXAMPLE 80

### 1-(3-{[4-(Dimethylamino)-2,2-dimethyl-2,3-dihydro-1H-cyclopenta[4',5']pyrido[3',2':4,5]thieno[3,2-d]pyrimidin-7-yl]amino}propyl)pyrrolidin-2-one

Obtained (93%) from the title compound of Preparation 54 and 1-(3-aminopropyl)-pyrrolidin-2-one following the experimental procedure described in Example 78.
m.p. 184.4-185.7°C
¹H NMR (400 MHz, DMSO-D6) d ppm 1.2 (s, 6 H) 1.8 (m, 2 H) 1.9 (m, 2 H) 2.2 (t, *J*=8.0 Hz,2H)3.0(m,2H)3.1 (m, 6 H) 3.2 (m, 2 H) 3.3 (t, *J*=7.0 Hz, 2 H) 3.4 (m, 2 H) 3.5 (t, 2 H) 7.5 (t, *J*=5.7 Hz, 1 H) 8.5 (s, 1 H)

### EXAMPLE 81

### N⁷-(2-Furylmethyl)-N⁴, N⁴, 2, 2-tetramethyl-2,3-dihydro-1H-cyclopenta[4',5']pyrido[3',2':4,5]thieno[3,2-d]pyrimidin-4,7-diamine

Obtained (64%) from the title compound of Preparation 54 and furyl-2-ylmethylamine following the experimental procedure described in Example 78.
m.p. 216.6-217.3°C
¹H NMR (400 MHz, DMSO-D6) d ppm 1.1 (d, *J*=20.0 Hz, 6 H) 2.9 (s, 2 H) 3.1 (m, *J*=20.0 Hz, 6 H) 3.2 (m, *J*=10.6 Hz, 2 H) 4.7 (d, *J*=5.5 Hz, 2 H) 6.3 (d, *J*=2.7 Hz, 1 H) 6.4 (m, 1 H) 7.6 (s, 1 H) 8.1 (t, *J*=5.9 Hz, 1 H) 8.5 (s, 1 H)

### EXAMPLE 82

### 2,2-Dimethyl-4-morpholin-4-yl-7-(pyridin-3-ylmethyl)-2,3-dihydro-1H-cyclopenta[4',5']pyrido[3',2':4,5]thieno[3,2-d]pyrimidin-7-amine

Obtained (46%) from the title compound of Preparation 58 and (pyridin-3-ylmethyl)amine following the experimental procedure described in Example 1.
m.p. 212.7-214.0°C
¹H NMR (400 MHz, DMSO-D6) d ppm 1.2 (s, 6 H) 2.8 (s, 2 H) 3.3 (s, 2 H) 3.5 (m, 4 H) 3.7 (m, 4 H) 4.8 (d, *J*=5.9 Hz, 2 H) 7.3 (dd, *J*=7.8, 4.7 Hz, 1 H) 7.8 (d, *J*=7.8 Hz, 1 H) 8.3 (t, *J*=6.1 Hz, 1 H) 8.4 (dd, *J*=4.7, 1.6 Hz, 1 H) 8.5 (s, 1 H) 8.6 (d, *J*=2.3 Hz, 1 H)

### EXAMPLE 83

### N⁴N⁴,1,1-Tetramethyl-N⁷-(2-morpholin-4-ylethyl)-2,3-dihydro-1H-cyclopenta[4',5']pyrido[3',2':4,5]thieno[3,2-d]pyrimidin-4,7-diamine

7-Chloro-N,N,1,1-tetramethyl-2,3-dihydro-1H-cyclopenta[4',5']pyrido[3',2':4,5]thieno[3,2-d]pyrimidin-4-amine (0.2g, 0.60 mmol, see Preparation 64) is suspended in ethanol (14 ml) and (2-morpholin-4.-ylethyl)amine (0.39 ml, 3.00 mmol) is added. The mixture is refluxed overnight and then allowed to cool to room temperature. After evaporation of the solvent under vacuum, the residue is purified by flash chromatography, eluting first with CH₂Cl₂/MeOH 99.5:0.5 and then with CH₂Cl₂/MeOH 99:1. 0.06g of the desired final compound are isolated. Yield= 88%.
m.p. 197.3-198.5°C
¹H NMR (400 MHz, DMSO-D6) d ppm 1.7 (m, 6 H) 2.0 (t, *J*=7.4 Hz, 2 H) 2.5 (m, 4 H) 2.6 (m, 2H)3.1 (m, 6 H) 3.3 (m, *J*=18.0 Hz, 2 H) 3.6 (m, 6 H) 7.4 (t, *J*=5.7 Hz, 1 H) 8.5 (s, 1 H)

### EXAMPLE 84

### N⁴,N⁴,1,1-Tetramethyl-N⁷-(pyridin-3-ylmethyl)-2,3-dihydro-1H-cyclopenta[4',5']pyrido[3',2':4,5]thieno[3,2-d]pyrimidin-4,7-diamine

Obtained (88%) from the title compound of Preparation 64 and (pyridin-3-ylmethyl)amine following the experimental procedure described in Example 83.
m.p. 237.1-237.7°C
¹H NMR (400 MHz, DMSO-D6) d ppm 1.6 (d, *J*=20.0 Hz, 6 H) 2.0 (t, *J*=7.4 Hz, 2 H) 3.3 (s, 2 H) 3.3 (s, 6 H) 4.8 (d, *J*=5.9 Hz, 2 H) 7.3 (dd, *J*=7.8, 4.7 Hz, 1 H) 7.8 (m, 1 H) 8.1 (t, *J*=5.9 Hz, 1 H) 8.4 (m, 1 H) 8.5 (s, 1 H) 8.6 (s, 1 H)

### EXAMPLE 85

### Ethyl 4-(2-{[4-(dimethylamino)-1,1-dimethyl-2,3-dihydro-9H-cyclopenta[4',5']pyrido[3',2':4,5]thieno[3,2-d]pyrimidin-7-yl]amino}ethyl)pyperazincarboxilate

Obtained (37%) from the title compound of Preparation 64 and ethyl 4-(2-aminoethyl)-piperazine-1-carboxylate following the experimental procedure described in Example 83.
m.p. 214. 7-215.6°C
¹H NMR (400 MHz, DMSO-D6) d ppm 1.2 (m, 6 H) 2.0 (m, 2 H) 2.4 (m, 6 H) 2.6 (t, *J*=6.8 Hz,2H)3.1 (m, 6 H) 3.3 (m, 6 H) 3.6 (q, *J*=6.3 Hz, 2 H) 4.0 (q, *J*=7.2 Hz, 2 H) 7.4 (t, *J*=5.7 Hz, 1 H) 8.5 (s, 1 H) 13.1 (s, 1 H)

### EXAMPLE 86

### 1-(3-{[4-(Dimehylamino)-1,1-dimethyl-2,3-dihydro-1H-cyclopenta[4',5']pyrido[3',2';4,5]thieno[3,2-d]pyrimidin-7-yl]amino}propyl)pyrrolidin-2-one

Obtained (42%) from the title compound of Preparation 64 and 1-(3-aminopropyl)pyrrolidin-2-one following the experimental procedure described in Example 83.
m.p. 194.1-195.7°C
¹H NMR (400 MHz, DMSO-D6) d ppm 1.7 (s, 6 H) 1.8 (m, 2 H) 1.9 (m, 4 H) 2.2 (t, *J*=8.0 Hz,2H)3.1 (m, 6 H) 3.3 (t, *J*=7.0 Hz, 2 H) 3.4 (m, 3 H) 3.5 (m, 2 H) 7.5 (t, *J*=5.7 Hz, 1 H) 8.5 (s, 1 H) 13.1 (s, 1 H)

### EXAMPLE 87

### N⁷-[2-(1H-Imidazol-4-yl)ethyl]-N⁴,N⁴,1,1-tetramethyl-2,3-dihydro-1H-cyclopenta[4',5']pyrido[3',2':4,5]thieno[3,2-d]pyrimidin-4,7-diamine

Obtained (88%) from the title compound of Preparation 64 and 2-(1 H-imidazol-4-yl)-ethylamine following the experimental procedure described in Example 83.
m.p. 260.8-261.5°C
¹H NMR (400 MHz, DMSO-D6) d ppm 1.7 (s, 6 H) 1.9 (t, *J*=7.4 Hz, 2 H) 2.8 (m, 2 H) 3.1 (m, *J*=5.1 Hz, 2 H) 3.3 (s, 6 H) 3.7 (m, 2 H) 6.8 (s, 1 H) 7.5 (s, 1 H) 7.6 (t, *J*=5.5 Hz, 1 H) 8.5 (s, 1H) 11.8(s, 1H)

### EXAMPLE 88

### N⁷-(2-Furylmethyl)-N⁴,N⁴,1,1-tetramethyl-2,3-dihydro-1H-cyclopenta[4',5']pyrido[3',2':4,5]thieno[3,2-d]pyrimidin-4,7-diamine

Obtained (67%) from the title compound of Preparation 64 and furyl-2-ylmethylamine following the experimental procedure described in Example 83.
m.p. 89.3-91.0°C
¹H NMR (400 MHz, DMSO-D6) d ppm 1.6 (m, 6 H) 2.0 (m, 2 H) 3.1 (m, 6 H) 3.3 (d, *J*=17.0 Hz, 2 H) 4.7 (d, *J*=5.8 Hz, 2 H) 6.3 (d, *J*=3.3 Hz, 1 H) 6.4 (m, 1 H) 7.6 (s, 1 H) 8.0 (m, *J*=5.8, 5.8 Hz, 1 H) 8.6 (s, 1 H)

### EXAMPLE 89

### N⁷-(2,3-Dimethoxybenzyl)-N⁴,N⁴,1,1-tetramethyl-2,3-dihydro-1H-cyclopenta[4',5']pyrido[3',2':4,5]thieno[3,2-d]pyrimidin-4,7-diamine

Obtained (42%) from the title compound of Preparation 64 and 2,3-dimethoxybenzylamine following the experimental procedure described in Example 83. m.p. 91.9-93.6°C
¹H NMR (400 MHz, DMSO-D6) d ppm 1.7 (s, 6 H) 2.0 (t, *J*=7.4 Hz, 2 H) 3.1 (m, 2 H) 3.1 (s, 6 H) 3.8 (s, 3 H) 3.8 (s, 3 H) 4.8 (d, *J*=5.1 Hz, 2 H) 6.9 (m, *J*=5.9 Hz, 4 H) 8.5 (s, 1 H)

### EXAMPLE 90

### 1,1-Dimethyl-4-morpholin-4-yl-N⁷-(2-morpholin-4-ylmethyl)-2,3-dihydro-1H-cyclopenta[4',5']pyrido[3',2':4,5]thieno[3,2-d]pyrimidin-7-amine

7-Chloro-1,1-dimethyl-4-morpholin-4-yl-2,3-dihydro-1 H-cyclopenta[4',5']pyrido[3',2':4,5]thieno[3,2-d]pyrimidin-4-amine (0.11g, 0.29 mmol, see Preparation 68) is suspended in ethanol (7 ml) and (2-morpholin-4-ylethyl)amine (0.19 ml, 1.45 mmol) is added. The mixture is refluxed overnight and then allowed to cool to room temperature. After evaporation of the solvent under vacuum, the residue is purified by flash chromatography, eluting first with CH₂Cl₂/MeOH 99.5:0.5 and then with CH₂Cl₂/MeOH 99:1 and finally with CH₂Cl₂/MeOH 98:2. 0.11g of the desired final compound are isolated as a solid. Yield= 81%.
m.p. 100.0-100.9°C
¹H NMR (400 MHz, DMSO-D6) d ppm 1.7 (s, 6 H) 2.0 (t, *J*=7.3 Hz, 2 H) 2.4 (d, *J*=4.1 Hz, 2 H) 2.6 (t, 2 H) 3.0 (t, *J*=7.3 Hz, 2 H) 3.4 (m, 4 H) 3.6 (m, 4 H) 3.6 (m, 4 H) 3.7 (m, 4 H) 7.5 (t, *J*=5.4 Hz, 1 H) 8.6 (s, 1 H)

### EXAMPLE 91

### 1,1-Dimethyl-4-morpholin-4-yl-N7-(pyridin-3-ylmethyl)-2,3-dihydro-1H-cyclopenta[4',5']pyrido[3',2':4,5]thieno[3,2-d]pyrimidin-7-amine

Obtained (100%) from the title compound of Preparation 68 and pyridin-3-ylmethylamine following the experimental procedure described in Example 90.
m.p. 185.5-186.8°C
¹H NMR (400 MHz, DMSO-D6) d ppm 1.7 (s, 6 H) 2.0 (t, *J*=7.5 Hz, 2 H) 3.0 (t, *J*=7.3 Hz, 2 H) 3.4 (m, 4 H) 3.7 (m, 4 H) 4.8 (d, *J*=6.2 Hz, 2 H) 7.3 (dd, *J*=7.9, 5.0 Hz, 1 H) 7.8 (d, *J*=7.9 Hz, 1 H) 8.3 (t, *J*=6.2 Hz, 1 H) 8.5 (m, 1 H) 8.6 (s, 1 H) 8.6 (s, 1 H)

### EXAMPLE 92

### 1-(3-{[4-(Morpholin-4-yl)-1,1-dimethyl-2,3-dihydro-1H-cyclopenta[4',5']pyrido[3',2':4,5]thieno[3,2-d]pyrimidin-7-yl]amino}propyl)pyrrolidin-2-one

Obtained (64%) from the title compound of Preparation 68 and 1-(3-aminopropyl)-pyrrolidin-2-one following the experimental procedure described in Example 90.
m.p. 141.8-143.0°C
¹H NMR (400 MHz, DMSO-D6) d ppm 1.7 (s, 6 H) 1.8 (m, 2 H) 1.9 (m, 2 H) 2.0 (t, *J*=7.3 Hz, 2 H) 2.2 (t, *J*=8.1 Hz, 2 H) 3.0 (t, *J*=7.3 Hz, 2 H) 3.3 (t, *J*=7.3 Hz, 2 H) 3.4 (m, 2 H) 3.4 (m, 4 H) 3.5 (m, 2 H) 3.7 (t, 4 H) 7.6 (t, *J*=5.6 Hz, 1 H) 8.6 (s, 1 H)

### EXAMPLE 93

### 2,2-Dimethyl-7-(2-morpholin-4-ylethyl)-4-pyrrolidin-1-yl-2,3-dihydro-1H-cyclopenta[4',5']pyrido[3',2':4,5]thieno[3,2-d]pyrimidin-7-amine

Obtained (71%) from the title compound of Preparation 72 and (2-morpholine-4-ylethyl)amine following the experimental procedure described in Example 78.
**LAS100506**
¹H NMR (300 MHz, CHLOROFORM-D) δ ppm 1.2 (s, 6 H) 2.0 (m, 4 H) 2.5 (m, 4 H) 2.7 (t, *J*=5.9 Hz, 2 H) 3.0 (s, 2 H) 3.3 (s, 2 H) 3.7 (m, 10 H) 5.5 (t, *J*=4.5 Hz, 1H) 8.7 (s, 1H)

### EXAMPLE 94

### 2,2-Dimethyl-7-(pyridin-3-ylmethyl)-4-pyrrolidin-1-yl-2,3-dihydro-1H-cyclopenta[4',5']pyrido[3',2':4,5]thieno[3,2-d]pyrimidin-7-amine

Obtained (25%) from the title compound of Preparation 72 and pyridine-3-ylmethylamine following the experimental procedure described in Example 78. **LAS100513**
¹H NMR (300 MHz, CHLOROFORM-D) δ ppm 1.2 (m, 6 H) 2.0 (t, *J*=6.5 Hz, 4 H) 3.1 (s, 2 H) 3.3 (s, 2 H) 3.8 (m, 4 H) 4.9 (d, *J*=5.8 Hz, 2 H) 5.0 (m, 1 H) 7.3 (m, 1 H) 7.8 (d, *J*=8.2 Hz, 1 H) 8.6 (d, *J*=4.1 Hz, 1 H) 8.7 (m, 1 H) 8.7 (s, 1 H)

### EXAMPLE 95

### 2-[(2,2-Dimethyl-4-pyrrolidin-1-yl-2,3-dihydro-1H-cyclopenta[4',5']pyrido[3',2':4,5]thieno[3,2-d]pyrimidin-7-yl)(2-morpholin-4-ylethyl)amino]ethanol

Obtained (12%) from the title compound of Preparation 72 and 2-(2-morpholin-4-ylethylamino)ethanol following the experimental procedure described in Example 78. **LAS100529**
¹H NMR (300 MHz, CHLOROFORM-D) δ ppm 1.2 (s, 6 H) 2.0 (m, 4 H) 2.5 (m, 6 H) 2.8 (s, 2H)3.1 (s, 2 H) 3.3 (s, 2 H) 3.6 (m, 2 H) 3.7 (m, 7H) 4.0 (m, 2 H) 4.0 (s, 2 H) 8.6 (s, 1 H)

### EXAMPLE 96

### 2-[(2,2-Dimethyl-5-pyrrolidin-1-yl-1,2,3,4-tetrahydropyrimido[4',5':4,5]thieno[2,3-c]isoquinolin-8-yl)-(2-morpholin-4-ylethyl)amino]ethanol

Obtained (39%) from the title compound of Preparation 18 and 2-(2-morpholin-4-ylethylamino)ethanol following the experimental procedure described in Example 23. **LAS100545**
¹H NMR (300 MHz, CHLOROFORM-D) δ ppm 1.1 (s, 6 H) 1.6 (m, *J*=6.9 Hz, 2 H) 2.0 (m, 4 H) 2.6 (m, 4 H) 2.8 (t, *J*=5.9 Hz, 2 H) 2.9 (m, 2 H) 3.4 (s, 2 H) 3.7 (m, 4 H) 3.7 (m, 4 H) 4.0 (m, 6 H) 8.6 (s, 1 H)

### EXAMPLE 97

### N,N,2,2-Tetramethyl-4-pyrrolidin-1-yl-2,3-dihydro-1H-cyclopenta[4',5']pyrido[3',2':4,5]thieno[3,2-d]pyrimidin-7-amine

Obtained (15%) from the title compound of Preparation 72 and dimethylamine (dissolved in dimethylformamide) following the experimental procedure described in Example 78.
**LAS 100547**
¹H NMR (300 MHz, CHLOROFORM-D) δ ppm 1.2 (s, 6 H) 2.0 (t, *J*=6.3 Hz, 4 H) 3.0 (s, 2 H) 3.3 (s, 2 H) 3.4 (s, 6 H) 3.7 (t, *J*=6.3 Hz, 4 H) 8.6 (s, 1 H)

### EXAMPLE 98

### 2,2-Dimethyl-N-(2-morpholin-4-ylethyl)-N-(pyridin-3-ylmethyl)-4-pyrrolidin-1-yl-2,3-dihydro-1 H-cyclopenta[4',5']pyrido[3',2':4,5]thieno[3,2-d]pyrimidin-7-amine

Obtained (10%) from the title compound of Preparation 72 and (2-morpholin-4-ylethyl)-pyridin-3-ylmethylamine following the experimental procedure described in Example 78.
**LAS100550**
¹H NMR (300 MHz, CHLOROFORM-D) δ ppm 1.3 (m, 6 H) 2.0 (t, *J*=6.3 Hz, 4 H) 2.5 (d, *J*=3.8 Hz, 4 H) 2.7 (t, *J*=6.7 Hz, 2 H) 3.1 (s, 2 H) 3.3 (s, 2 H) 3.7 (m, 8 H) 3.9 (t, *J*=6.7 Hz, 2 H) 5.2 (s, 2 H) 7.2 (m, 1 H) 7.7 (d, *J*=8.0 Hz, 1 H) 8.6 (m, 3 H)

### EXAMPLE 99

### N⁴-Ethyl-N⁴,2,2-trimethyl-N'-(2-morpholin-4-ylethyl)-2,3-dihydro-1H-cyclopenta[4',5']pyrido[3',2':4,5]thieno[3,2-d]pyrimidin-4,7-diamine

Obtained (43%) from the title compound of Preparation 76 and (2-morpholin-4-ylethyl)amine following the experimental procedure described in Example 78.
**LAS100582**
¹H NMR (300 MHz, CHLOROFORM-D) δ ppm 1.2 (m, 9 H) 2.5 (m, 4 H) 2.7 (t, *J*=6.0 Hz, 2 H) 2.9 (s, 2 H) 3.2 (s, 3 H) 3.3 (s, 2 H) 3.6 (q, *J*=7.0 Hz, 2 H) 3.7 (m, 6 H) 5.5 (m, 1 H) 8.7 (s, 1 H)

### EXAMPLE 100

### N⁴-Ethyl-N⁴,2,2-trimethyl-N⁷-(pyridin-3-ylmethyl)-2,3-dihydro-1H-cyclopenta[4',5']pyrido[3',2':4,5]thieno[3,2-d]pyrimidin-4,7-diamine

Obtained (21%) from the title compound of Preparation 76 and pyridine-3-ylmethylamine following the experimental procedure described in Example 78.
**LAS100586**
¹H NMR (300 MHz, CHLOROFORM-D) δ ppm 1.2 (s, 9 H) 2.9 (s, 2 H) 3.2 (s, 3 H) 3.3 (s, 2 H) 3.6 (q, *J*=7.1 Hz, 2 H) 4.9 (d, *J*=5.8 Hz, 2 H) 5.1 (d, *J*=5.2 Hz, 1 H) 7.3 (m, 2 H) 7.8 (d, *J*=8.2 Hz, 1 H) 8.6 (s, 1 H) 8.7 (s, 1 H)

### EXAMPLE 101

### 1-[3-({4-[Ethyl(methyl)amino]-2,2-dimethyl-2,3-dihydro-1H-cyclopenta[4',5']pyrido[3',2':4,5]thieno[3,2-d]pyrimidin-7-yl}amino)propyl]pyrrolidin-2-one

Obtained (71 %) from the title compound of Preparation 76 and 1-(3-aminopropyl)-pyrrolidine-2-one following the experimental procedure described in Example 78. **LAS100589**
¹H NMR (300 MHz, CHLOROFORM-D) δ ppm 1.2 (m, 9 H) 1.9 (dd, *J*=11.5, 5.5 Hz, 2 H) 2.1 (m, 2 H) 2.5 (t, *J*=8.1 Hz, 2 H) 2.9 (s, 2 H) 3.1 (s, 3 H) 3.3 (s, 2 H) 3.4 (m, 4 H) 3.6 (m, 4 H) 6.2 (m, 1 H) 8.7 (s, 1 H)

### EXAMPLE 102

### N⁷-(2,3-Dimethoxybenzyl)-N⁴-ethyl-N⁴,2,2-trimethyl-2,3-dihydro-1H-cyclopenta[4',5']pyrido[3',2':4,5]thieno[3,2-d]pyrimidin-4,7-diamine

Obtained (51 %) from the title compound of Preparation 76 and 2,3-dimethoxybenzylamine following the experimental procedure described in Example 78. **LAS100596**
¹H NMR (300 MHz, CHLOROFORM-D) δ ppm 1.2 (m, 9 H) 2.9 (s, 2 H) 3.1 (s, 3 H) 3.3 (s, 2 H) 3.6 (q, *J*=7.0 Hz, 2 H) 3.9 (s, 3 H) 3.9 (s, 3 H) 4.9 (d, *J*=5.5 Hz, 2 H) 5.1 (t, *J*=5.6 Hz, 1 H) 6.9 (dd, *J*=7.1, 2.7 Hz, 1 H) 7.0 (m, 2 H) 8.7 (s, 1H)

### EXAMPLE 103

### 2-[{4-[Ethyl(methyl)amino+-2,2-dimethyl-2,3-dihydro-1H-cyclopenta[4',5']pyrido[3',2':4,5]thieno[3,2-d]pyrimidin-7-yl}(2-morpholin-4-ylethyl)amino]ethanol

Obtained (26%) from the title compound of Preparation 76 and 2-(2-morpholin-4-ylethylamino)ethanol following the experimental procedure described in Example 78.
**LAS100599**
¹H NMR (300 MHz, DMSO-D6) δ ppm 1.2 (s, 9 H) 2.5 (s, 4 H) 2.6 (t, *J*=7.0 Hz, 2 H) 2.9 (s, 2H)3.1 (s, 3 H) 3.2 (s, 2 H) 3.6 (m, 5 H) 3.7 (m, 4 H) 3.8 (m, 2 H) 3.9 (m, 2 H) 8.5 (s, 1 H)

### EXAMPLE 104

### N⁴-Ethyl-N⁴,2,2-trimethyl-N⁷-(2-morpholin-4-ylethyl)-N⁷-(pyridin-3-ylmethyl)-2,3-dihydro-1 H-cyclopenta[4',5']pyrido[3',2':4,5]thieno[3,2-d]pyrimidin-4,7-diamine

Obtained (8%) from the title compound of Preparation 76 and (2-morpholin-4-ylethyl)-pyridin-3-ylmethylamine following the experimental procedure described in Example 78.
**LAS100600**
¹H NMR (300 MHz, DMSO-D6) δ ppm 1.1 (m, 9 H) 2.4 (m, 4 H) 2.5 (m, 2 H) 2.6 (t, *J*=6.9 Hz, 2 H) 2.9 (s, 2 H) 3.1 (s, 3 H) 3.2 (s, 2 H) 3.5 (m, 4 H) 3.9 (m, 2 H) 5.1 (s, 2 H) 7.3 (dd, *J*=7.8, 4.5 Hz, 1 H) 7.7 (d, *J*=8.0 Hz, 1 H) 8.5 (dd, *J*=4.8, 1.8 Hz, 1 H) 8.5 (s, 1 H) 8.5 (d, *J*=1.6 Hz, 1 H)

### EXAMPLE 105

### 2,2-Dimethyl-4-morpholin-4-yl-N-(2-morpholin-4-ylethyl)-2,3-dihydro-1H-cyclopenta[4',5']pyrido[3',2':4,5]thieno[3,2-d]pyrimidin-7-amine

Obtained (39%) from the title compound of Preparation 58 and (2-morpholin-4-ylethyl)amine following the experimental procedure described in Example 78.
**LAS100629**
¹H NMR (300 MHz, CHLOROFORM-D) δ ppm 1.2 (m, 6 H) 2.6 (s, 4 H) 2.7 (t, *J*=5.9 Hz, 2 H) 2.8 (s, 2 H) 3.4 (s, 2 H) 3.6 (m, 4 H) 3.7 (m, 6 H) 3.9 (m, 4 H) 5.6 (m, 1 H) 8.7 (s, 1 H)

### EXAMPLE 106

### 2-[(2,2-Dimethyl-4-morpholin-4-yl-2,3-dihydro-1H-cyclopenta[4',5']pyrido[3',2':4,5]thieno[3,2-d]pyrimidin-7-yl)(2-morpholin-4-ylethyl)amino]ethanol

Obtained (51%) from the title compound of Preparation 58 and 2-(2-morpholin-4-ylethylamino)ethanol following the experimental procedure described in Example 78.
**LAS100637**
¹H NMR (300 MHz, CHLOROFORM-D) δ ppm 1.2 (s, 6 H) 2.6 (s, 5 H) 2.8 (s, 2 H) 2.9 (t, *J*=5.1 Hz, 2 H) 3.4 (s, 2 H) 3.6 (m, 4 H) 3.7 (m, 4 H) 3.9 (m, 4 H) 4.0 (m, 6 H) 8.6 (s, 1 H)

### EXAMPLE 107

### N-(2,3-Dimethoxybenzyl)-2,2-dimethyl-4-morpholin-4-yl-2,3-dihydro-1H-cyclopenta[4',5']pyrido[3',2':4,5]thieno[3,2-d]pyrimidin-7-amine

Obtained (73%) from the title compound of Preparation 58 and 2,3-dimethoxybenzylamine following the experimental procedure described in Example 78.
**LAS100644**
¹H NMR (300 MHz, CHLOROFORM-D) δ ppm 1.2 (s, 6 H) 2.8 (s, 2 H) 3.4 (s, 2 H) 3.5 (m, 3 H) 3.9 (m, 5 H) 3.9 (s, 3 H) 3.9 (s, 3 H) 4.9 (d, *J*=5.5 Hz, 2 H) 5.2 (t, *J*=5.6 Hz, 1 H) 6.9 (dd, *J*=7.1, 2.5 Hz, 1 H) 7.0 (m, 2 H) 8.7 (s, 1 H)

### EXAMPLE 108

### 1-{3-[(2,2-Dimethyl-4-morpholin-4-yl-2,3-dihydro-1H-cyclopenta[4',5']pyrido[3',2':4,5]thieno[3,2-d]pyrimidin-7-yl)amino]propyl}pyrrolidin-2-one

Obtained (73%) from the title compound of Preparation 58 and 1-(3-aminopropyl)-pyrrolidin-2-one following the experimental procedure described in Example 78.
**LAS100649**
¹H NMR (300 MHz, CHLOROFORM-D) δ ppm 1.2 (s, 6 H) 1.9 (m, 2 H) 2.1 (m, 2 H) 2.5 (t, *J*=8.1 Hz, 2 H) 2.8 (s, 2 H) 3.4 (s, 2 H) 3.4 (m, 4 H) 3.5 (m, 4 H) 3.6 (m, 2 H) 3.9 (m, 4 H) 6.4 (t, *J*=6.0 Hz, 1 H) 8.7 (s, 1 H)

### EXAMPLE 109

### 2-[(2,2-Dimethyl-5-morpholin-4-yl-1,2,3,4-tetrahydropyrimido[4',5':4,5]thieno[2,3-c]isoquinolin-8-yl)(2-morpholin-4-ylethyl)amino]ethanol

Obtained (57%) from the title compound of Preparation 6 and 2-(2-morpholin-4-ylethylamino)ethanol following the experimental procedure described in Example 1.
**LAS100690**
¹H NMR (300 MHz, CHLOROFORM-D) δ ppm 1.1 (s, 6 H) 1.6 (t, *J*=6.3 Hz, 2 H) 2.6 (s, 4 H) 2.8 (t, *J*=6.3 Hz, 2 H) 2.9 (m, 2 H) 3.3 (m, 4 H) 3.5 (s, 3 H) 3.7 (m, 4 H) 3.9 (m, 4 H) 4.0 (m, 6 H) 8.6 (s, 1 H)

### EXAMPLE 110

### 2-[(2,2-Dimethyl-5-morpholin-4yl-1,2,3,4-tetrahydropyrimido[4',5':4,5]thieno[2,3-c]isoquinolin-8-yl)(pyridin-3-ylmethyl)amino]ethanol

Obtained (46%) from the title compound of Preparation 6 and [(pyridin-3-ylmethyl)amino]ethanol following the experimental procedure described in Example 1.
**LAS100718**
¹H NMR (300 MHz, CHLOROFORM-D) δ ppm 1.1 (s, 6 H) 1.6 (t, *J*=6.2 Hz, 2 H) 2.4 (s, 2 H) 2.8 (t, *J*=5.9 Hz, 2 H) 3.3 (m, 4 H) 3.5 (s, 2 H) 3.9 (m, 3 H) 3.9 (m, 3 H) 4.4 (s, 1 H) 5.2 (s, 2 H) 7.3 (m, 1 H) 7.7 (m, 1 H) 8.5 (m, 2 H) 8.7 (s, 1 H)

### EXAMPLE 111

### N⁵,2,2-Trimethyl-N⁸-(2-morpholin-4-ylethyl)-1,2,3,4-tetrahydropyrimido[4',5':4,5]thieno[2,3-c]isoquinoline-5,8-diamine

N⁵-Benzyl-N⁵,2,2-trimethyl-N⁸-(2-morpholin-4-ylethyl)-1,2,3,4-tetrahydropyrimido [4',5':4,5]thieno[2,3-c]isoquinoline-5,8-diamine (173mg, 0.33 mmol, see Preparation 81) is dissolved in toluene and aluminum chloride is portionwise added. This reaction mixture is refluxed for 2h. Once the reaction, is over, the reaction mixture is diluted with ethyl acetate and washed twice with water, once with brine, dried over magnesium sulfate, filtered and evaporated under reduced pressure. The residue is purified by flash chromatography, eluting first with dichloromethane, then with dichloromethane/methanol 99:1 and finally with dichloromethane/methanol 98:2. 73 mg of the final compound are isolated. Its ¹HNMR is consistent with the desired final compound. Yield= 51%.
**LAS100851**
¹H NMR (300 MHz, CHLOROFORM-D) δ ppm 1.1 (s, 6 H) 1.7 (t, *J*=5.9 Hz, 2 H) 2.4 (t, *J*=6.3 Hz, 2 H) 2.5 (s, 4 H) 2.7 (t, *J*=4.7 Hz, 2 H) 3.2 (d, *J*=3.3 Hz, 3 H) 3.4 (s, 2 H) 3.7 (m, *J*=16.5 Hz, 6 H) 4.7 (s, 1 H) 5.5 (s, 1 H) 8.7 (s, 1 H)

### EXAMPLE 112

### N⁵,2,2-Trimethyl-N⁸-(pyridin-3-ylmethyl)-1,2,3,4-tetrahydropyrimido[4',5':4,5]thieno[2,3-c]isoquinoline-5,8-diamine

Obtained (74%) from the title compound of Preparation 82 following the experimental procedure described in Example 111.
**LAS100852**
¹H NMR (300 MHz, CHLOROFORM-D) δ ppm 1.1 (s, 6 H) 1.7 (t, *J*=6.3 Hz, 2 H) 2.4 (t, *J*=6.2 Hz, 2 H) 3.2 (d, *J*=4.7 Hz, 3 H) 3.4 (s, 2 H) 4.7 (d, *J*=4.4 Hz, 1 H) 4.9 (d, *J*=5.5 Hz, 2 H) 5.0 (d, *J*=8.2 Hz, 1 H) 7.3 (m, 1 H) 7.8 (d, *J*=7.7 Hz, 1 H) 8.6 (d, *J*=4.4 Hz, 1 H) 8.7 (s, 1 H) 8.7 (s, 1 H)

### EXAMPLE 113

### 1-(3-{[2,2-Dimethyl-5-(methylamino)-1,2,3,4-tetrahydropyrimido[4',5':4,5]thieno[2,3-c] isoquinolin-8-yl]amino}propyl)pyrrolidin-2-one

Obtained (61 %) from the title compound of Preparation 83 following the experimental procedure described in Example 111.
**LAS100883**
¹H NMR (300 MHz, CHLOROFORM-D) δ ppm 1.1 (s, 6 H) 1.7 (t, *J*=6.6 Hz, 2 H) 1.9 (m, 2 H) 2.1 (m, 2 H) 2.4 (m, 4 H) 3.1 (d, *J*=4.9 Hz, 3 H) 3.4 (s, 2 H) 3.4 (m, 4 H) 3.6 (q, *J*=6.0 Hz, 2 H) 4.6 (m, *J*=4.4 Hz, 1 H) 6.0 (m, 1 H) 8.7 (s, 1 H)

The following examples illustrate pharmaceutical compositions according to the present invention.

### COMPOSITION EXAMPLES:

### COMPOSITION EXAMPLE 1

### Preparation of tablets

| Formulation: | |
|---|---|
| Compound of the present invention | 5.0 mg |
| Lactose | 113.6 mg |
| Microcrystalline cellulose | 28.4 mg |
| Light silicic anhydride | 1.5 mg |
| Magnesium stearate | 1.5 mg |

Using a mixer machine, 15 g of the compound of the present invention are mixed with 340.8 g of lactose and 85.2 g of microcrystalline cellulose. The mixture is subjected to compression moulding using a roller compactor to give a flake-like compressed material. The flake-like compressed material is pulverised using a hammer mill, and the pulverised material is screened through a 20 mesh screen. A 4.5 g portion of light silicic anhydride and 4.5 g of magnesium stearate are added to the screened material and mixed. The mixed product is subjected to a tablet making machine equipped with a die/punch system of 7.5 mm in diameter, thereby obtaining 3,000 tablets each having 150 mg in weight.

### COMPOSITION EXAMPLE 2

### Preparation of coated tablets

| | |
|---|---|
| Formulation: | |
| Compound of the present invention | 5.0 mg |
| Lactose | 95.2 mg |
| Corn starch | 40.8 mg |
| Polyvinylpyrrolidone K25 | 7.5 mg |
| Magnesium stearate | 1.5 mg |
| Hydroxypropylcellulose | 2.3 mg |
| Polyethylene glycol 6000 | 0.4 mg |
| Titanium dioxide | 1.1 mg |
| Purified talc | 0.7 mg |

Using a fluidised bed granulating machine, 15 g of the compound of the present invention are mixed with 285.6 g of lactose and 122.4 g of corn starch. Separately, 22.5 g of polyvinylpyrrolidone is dissolved in 127.5 g of water to prepare a binding solution. Using a fluidised bed granulating machine, the binding solution is sprayed on the above mixture to give granulates. A 4.5 g portion of magnesium stearate is added to the obtained granulates and mixed. The obtained mixture is subjected to a tablet making machine equipped with a die/punch biconcave system of 6.5 mm in diameter, thereby obtaining 3,000 tablets, each having 150 mg in weight.

Separately, a coating solution is prepared by suspending 6.9 g of .hydroxypropylmethylcellulose 2910, 1.2 g of polyethylene glycol 6000, 3.3 g of titanium dioxide and 2.1 g of purified talc in 72.6 g of water. Using a High Coated, the 3,000 tablets prepared above are coated with the coating solution to give film-coated tablets, each having 154.5 mg in weight.

### COMPOSITION EXAMPLE 3

### Preparation of capsules

| Formulation: | |
|---|---|
| Compound of the present invention | 5.0 mg |
| Lactose monohydrate | 200 mg |
| Colloidal silicon dioxide | 2 mg |
| Corn starch | 20 mg |
| Magnesium stearate | 4 mg |

25 g of active compound, 1 Kg of lactose monohydrate, 10 g of colloidal silicon dioxide, 100 g of corn starch and 20 g of magnesium stearate are mixed. The mixture is sieved through a 60 mesh sieve, and then filled into 5,000 gelatine capsules.

### COMPOSITION EXAMPLE 4

### Preparation of a cream

| Formulation: | |
|---|---|
| Compound of the present invention | 1 % |
| Cetyl alcohol | 3 % |
| Stearyl alcohol | 4 % |
| Gliceryl monostearate | 4 % |
| Sorbitan monostearate | 0.8 % |
| Sorbitan monostearate POE | 0.8 % |
| Liquid vaseline | 5 % |
| Methylparaben | 0.18 % |
| Propylparaben | 0.02 % |
| Glycerine | 15 % |
| Purified water csp. | 100 % |

An oil-in-water emulsion cream is prepared with the ingredients listed above, using conventional methods.

## Claims

1. Use of a pyridothienopyrimidine derivative of formula (I) 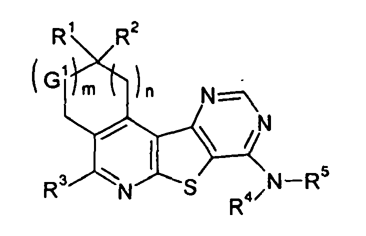 and the pharmaceutically acceptable salts and N-oxides thereof, wherein
G¹ represents a group selected from -CR⁶R⁷- or -NR⁶, R⁶ and R⁷ being independently selected from hydrogen atoms and C₁₋₄ alkyl groups
m and n are integers selected from 0 or 1
R¹ and R² are independently selected from hydrogen atoms and C₁₋₄ alkyl groups
R³ represents a group selected from C₁ to C₂₀ alkyl, amino, mono(C₁ to C₁₀ alkyl)amino, di(C₁ to C₁₀ alkyl)amino, C₅ to C₁₄ aryl, 5- to 14- membered heteroaryl and saturated N-containing 3- to 10- membered heterocyclyl groups bound through the nitrogen atom to the pyridine ring, all of them being optionally substituted by one or more substituents selected from the group consisting of halogen atoms and C₁ to C₂₀ alkyl, (C₁ to C₁₀ alkoxy)(C₁ to C₂₀ alkyl), (C₅ to C₁₄ aryl)(C₁ to C₂₀ alkyl), R⁸OCO-, C₁ to C₁₀ alkoxy, R⁸R⁹N-CO-, -CN, -CF₃, -NR⁸R⁹, -SR⁸ and -SO₂NH₂ groups wherein R⁸ and R⁹ are independently selected from hydrogen atoms and C₁₋₄ alkyl groups
R⁴ and R⁵ are independently selected from the group consisting of hydrogen atoms, C₁ to C₂₀ alkyl groups and groups of formula (II): 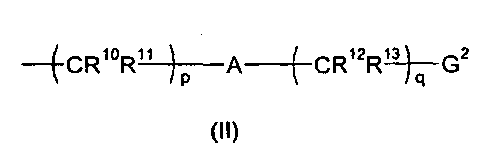 wherein p and q are integers selected from 1, 2 and 3; A is either a direct bond or a group selected from -CONR¹⁴-, -NR¹⁴CO-, -O-, -COO-, -OCO-, -NR¹⁴COO-, - OCONR¹⁴-,-NR¹⁴CONR¹⁵-, -S-, -SO-, -SO₂-, -COS- and -SCO-; and G² is a group selected from C₅ to C₁₄ aryl, 5- to 14- membered heteroaryl or 3- to 10- membered heterocyclyl; wherein the C₁ to C₂₀ alkyl groups and the group G² are optionally substituted by one or more substituents selected from group consisting of halogen atoms and C₁ to C₂₀ alkyl, (C₁ to C₁₀ alkoxy)(C₁ to C₂₀ alkyl), (C₅ to C₁₄ aryl)(C₁ to C₂₀ alkyl), R¹⁶OCO-, hydroxy, C₁ to C₁₀ alkoxy, oxo, R¹⁶R¹⁷NCO-, -CN, -CF₃, -NR¹⁶R¹⁷, - SR¹⁶ and -SO₂NH₂ groups; wherein R¹⁰ to R¹⁷ are independently selected from hydrogen atoms and C₁₋₄ alkyl groups;
in the manufacture of a medicament for the treatment or prevention of a pathological condition or disease which is asthma, chronic obstructive pulmonary disease, rheumatoid arthritis, atopic dermatitis, psoriasis or irritable bowel disease.

2. Use according to claim 1 wherein G¹ is a group -CR⁶R⁷- wherein R⁶ and R⁷ are as hereinabove defined.

3. Use according to claim 2 wherein G¹ is a group -CH₂-.

4. Use according to any preceding claim wherein R¹ and R² are both methyl groups.

5. Use according to according to any preceding claim wherein m and n have both the value of 1.

6. Use according to any preceding claim wherein R³ is selected from mono(C₁ to C₁₀ alkyl)amino, di(C₁ to C₁₀ alkyl)amino and saturated N-containing 3- to 10- membered heterocyclyl groups bound through the nitrogen atom to the pyridine ring, all of them being optionally substituted by one or more substituents selected from the group consisting of halogen atoms and C₁ to C₂₀ alkyl, (C₁ to C₁₀ alkoxy)(C₁ to C₂₀ alkyl), (C₅ to C₁₄ aryl)(C₁ to C₂₀ alkyl), R⁸OCO-, C₁ to C₁₀ alkoxy, R⁸R⁹NCO-, -CN, -CF₃, -NR⁸R⁹, - SR⁸ and -SO₂NH₂ groups wherein R³ and R⁹ are independently selected from hydrogen atoms and C₁₋₄ alkyl groups.

7. Use according to claim 6 wherein R³ is selected from mono(C₁ to C₁₀ alkyl)amino, di(C₁ to C₁₀ alkyl)amino and saturated N-containing 3- to 10- membered heterocyclyl groups bound through the nitrogen atom to the pyridine ring, all of them being non substituted.

8. Use according to any preceding claim wherein R⁴ is a hydrogen atom.

9. Use according to any preceding claim wherein R⁵ is a group of formula (III) 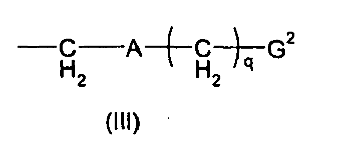 herein q is an integer selected from 1 or 2, A represents a direct bond or a group - CONH- and G² is a group selected from C₅ to C₁₄ aryl, 5- to 14- membered heteroaryl or 3- to 10- membered heterocyclyl; wherein the group G² is optionally substituted by one or more substituents selected from group consisting of halogen atoms and C₁ to C₂₀ alkyl, (C₁ to C₁₀ alkoxy)(C₁ to C₂₀ alkyl), (C₅ to C₁₄ aryl)(C₁ to C₂₀ alkyl), R¹⁶OCO-, C₁ to C₁₀ alkoxy, R¹⁶R¹⁷NCO-, -CN, -CF₃, -NR¹⁶R¹⁷, -SR¹⁶ and -SO₂NH₂ groups; wherein R¹⁶ and R¹⁷ are independently selected from hydrogen atoms and C₁₋₄ alkyl groups.

10. Use according to claim 9 wherein the group G² is optionally substituted by one or more substituents selected from group consisting of halogen atoms and C₁ to C₁₀ alkoxy and R¹⁶OCO- groups; wherein R¹⁶ is selected from hydrogen atoms and C₁₋₄ alkyl groups.

11. Use according to any preceding claim wherein G¹ is a -CH₂- group, R¹ and R² are both methyl groups, m and n have both the value of 1, R³ is selected from mono(C₁ to C₁₀ alkyl)amino, di(C₁ to C₁₀ alkyl)amino and saturated N-containing 3- to 10-membered heterocyclyl groups bound through the nitrogen atom to the pyridine ring, all of them being non substituted, R⁴ is a hydrogen atom and R⁵ is a group of formula (III) 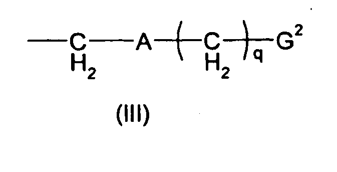 wherein q is an integer selected from 1 or 2, A represents a direct bond or a group - CONH- and G² is a group selected from C₅ to C₁₄ aryl, 5- to 14- membered heteroaryl or 3- to 10- membered heterocyclyl; wherein the group G² is optionally substituted by one or more substituents selected from group consisting of halogen atoms and C₁ to C₈ alkoxy, oxo and R¹⁶OCO- groups; wherein R¹⁶ is as hereinabove defined.

12. A pyridothienopyrimidine derivative of formula (I) 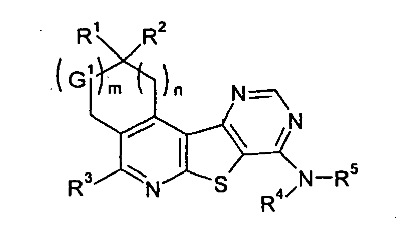 and the pharmaceutically acceptable salts and N-oxides thereof, wherein
G¹ represents a group selected from -CR⁶R⁷- or-NR⁶, R⁶ and R⁷ being independently selected from hydrogen atoms or C₁₋₄ alkyl groups
m and n are integers selected from 0 or 1
R¹ is selected from C₁₋₄ alkyl groups
R² is selected from hydrogen atoms and C₁₋₄ alkyl groups
R³ represents a group selected from C₁ to C₂₀ alkyl, amino, mono(C₁ to C₁₀ alkyl)amino, di(C₁ to C₁₀ alkyl)amino, C₅ to C₁₄ aryl, 5- to 14- membered heteroaryl and saturated N-containing 3- to 10- membered heterocyclyl groups bound through the nitrogen atom to the pyridine ring, all of them being optionally substituted by one or more substituents selected from the group consisting of halogen atoms and C₁ to C₂₀ alkyl, (C₁ to C₁₀ alkoxy)(C₁ to C₂₀ alkyl), (C₅ to C₁₄ aryl)(C₁ to C₂₀ alkyl), R⁸OCO-, C₁ to C₁₀ alkoxy, R⁸R⁹NCO-, -CN, -CF₃, -NR⁸R⁹, -SR⁸ and -SO₂NH₂ groups wherein R⁸ and R⁹ are independently selected from hydrogen atoms or C₁₄ alkyl groups
R⁴ and R⁵ are independently selected from the group consisting of hydrogen atoms alkyl groups and groups of formula (II): 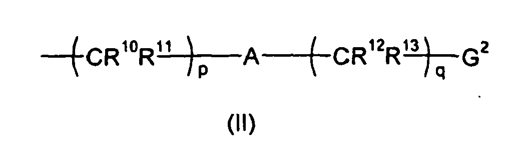 wherein p and q are integers selected from 1, 2 and 3; A is either a direct bond or a group selected from -CONR¹⁴-, -NR¹⁴CO-, -O-, -COO-, -OCO-, -NR¹⁴COO-, - OCONR¹⁴-, -NR¹⁴CONR¹⁵-, -S-, -SO-, -SO₂-, -COS- and -SCO-; and G² is a group selected from C₅ to C₁₄ aryl, 5- to 14- membered heteroaryl or 3- to 10- membered heterocyclyl; wherein the C₁ to C₂₀ alkyl groups and the group G² are optionally substituted by one or more substituents selected from group consisting of halogen atoms and C₁ to C₂₀ alkyl, (C₁ to C₁₀ alkoxy)(C₁ to C₂₀ alkyl), (C₅ to C₁₄ aryl)(C₁ to C₂₀ alkyl), R¹⁶OCO-, hydroxy, C₁ to C₁₀ alkoxy, oxo, R¹⁶R¹⁷NCO-, -CN, -CF₃, -NR¹⁶R¹⁷, - SR¹⁶ and -SO₂NH₂ groups; wherein R¹⁰ to R¹⁷ are independently selected from hydrogen atoms or C₁₋₄ alkyl groups
and the pharmaceutically acceptable salts and N-oxides thereof.

13. A compound according to claim 12 wherein G¹ is a group -CR⁶R⁷⁻.

14. A compound according to claim 13 wherein G¹ is a group -CH₂-.

15. A compound according to anyone of claims 12 to 14 wherein R¹ and R² are both methyl groups.

16. A compound according to anyone of claims 12 to 15 wherein m and n have both the value of 1.

17. A compound according to anyone of claims 12 to 16 wherein R³ is selected from mono(C₁ to C₁₀ alkyl)amino, di(C₁ to C₁₀ alkyl)amino and saturated N-containing 3- to 10- membered heterocyclyl groups bound through the nitrogen atom to the pyridine ring, all of them being optionally substituted by one or more substituents selected from the group consisting of halogen atoms and C₁ to C₂₀ alkyl, (C₁ to C₁₀ alkoxy)(C₁ to C₂₀ alkyl), (C₅ to C₁₄ aryl)(C₁ to C₂₀ alkyl), R⁸OCO-, alkoxy, R⁸R⁹NCO-, -CN, -CF₃, -NR⁸R⁹, -SR⁸ and -SO₂NH₂ groups wherein R⁸ and R⁹ are independently selected from hydrogen atoms or C₁₋₄ alkyl groups.

18. A compound according to anyone of claims 12 to 17 wherein R³ is selected from mono(C₁ to C₁₀ alkyl)amino, di(C₁ to C₁₀ alkyl)amino and saturated N-containing 3- to 10- membered heterocyclyl groups bound through the nitrogen atom to the pyridine ring, all of them being non substituted.

19. A compound according to anyone of claims 12 to 18 wherein R⁴ is a hydrogen atom.

20. A compound according to anyone of claims 12 to 19 wherein R⁵ is a group of formula (III) 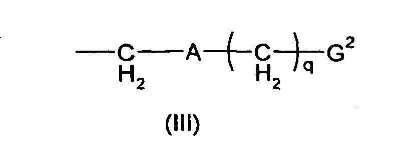 wherein q is an integer selected from 1 or 2, A represents a direct bond or a group - CONH- and G² is a group selected from C₅ to C₁₄ aryl, 5- to 14- membered heteroaryl or 3- to 10- membered heterocyclyl; wherein the group G² is optionally substituted by one or more substituents selected from group consisting of halogen atoms and C₁ to C₂₀ alkyl, (C₁ to C₁₀ alkoxy)(C₁ to C₂₀ alkyl), (C₅ to C₁₄ aryl)(C₁ to C₂₀ alkyl), R¹⁶OCO-hydroxy, C₁ to C₁₀ alkoxy, oxo, R¹⁶R¹⁷N-CO-, -CN, -CF₃, -NR¹⁶R¹⁷, -SR¹⁶ and -SO₂NH₂ groups; wherein R¹⁸ and R¹⁷ are independently selected from hydrogen atoms or C₁₋₄ alkyl groups.

21. A compound according to claim 20 wherein the group G² is optionally substituted by one or more substituents selected from group consisting of halogen atoms and C₁ to C₁₀ alkoxy and R¹⁶OCO- groups; wherein R¹⁸ is selected from hydrogen atoms or C₁₋₄ alkyl groups.

22. A compound according to anyone of claims 12 to 21 wherein G¹ is a -CH₂- group, R¹ and R² are both methyl groups, m and n have both the value of 1, R³ is selected from mono(C₁ to C₁₀ alkyl)amino, di(C₁ to C₁₀ alkyl)amino and saturated N-containing 3- to 10- membered heterocyclyl groups bound through the nitrogen atom to the pyridine ring, all of them being non substituted, R⁴ is a hydrogen atom and R⁵ is a group of formula (III) 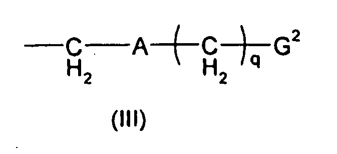 wherein q is an integer selected from 1 or 2, A represents a direct bond or a group - CONH- and G² is a group selected from C₅ to C₁₄ aryl, 5- to 14- membered heteroaryl or 3- to 10- membered heterocyclyl; wherein the group G² is optionally substituted by one or more substituents selected from group consisting of halogen atoms and C₁ to C₁₀ alkoxy, oxo and R¹⁶OCO- groups; wherein R¹⁶ is selected from hydrogen atoms or C₁₋₄ alkyl groups.

23. A compound according to anyone of claims 12 to 22 which is one of:
2,2-Dimethyl-5-morpholin-4-yl-N-(2-morpholin-4-ylethyl)-1,2,3,4-tetrahydropyrimido[4',5':4,5]thieno[2,3-c]isoquinolin-8-amine
2,2-Dimethyl-5-morpholin-4-yl-N-(pyridin-4-ylmethyl)-1,2,3,4-tetrahydropyrimido[4',5':4,5]thieno[2,3-c]isoquinolin-8-amine
2,2-Dimethyl-5-morpholin-4-yl-N-(pyridin-3-ylmethyl)-1,2,3,4-tetrahydropyrimido[4',5':4,5]thieno[2,3-c]isoquinolin-8-amine
2,2-Dimethyl-5-morpholin-4-yl-N-(pyridin-2-ylmethyl)-1,2,3,4-tetrahydropyrimido[4',5':4,5]thieno[2,3-c]isoquinolin-8-amine
N-(2-Methoxybenzyl)-2,2-dimethyl-5-morpholin-4-yl-1,2,3,4-tetrahydropyrimido[4',5':4;5]thieno[2,3-c]isoquinolin-8-amine
N-(2,3-Dimethoxybenzyl)-2,2-dimethyl-5-morpholin-4-yl-1,2,3,4-tetrahydropyrimido[4',5':4,5]thieno[2,3-c]isoquinolin-8-amine
4-{[(2,2-Dimethyl-5-morpholin-4-yl-1,2,3,4-tetrahydropyrimido[4',5':4,5]thieno[2,3-c]isoquinolin-8-yl)amino]methyl}benzenesulfonamide
2,2-Dimethyl-5-morpholin-4-yl-N-(2-pyridin-2-ylethyl)-1,2,3,4-tetrahydropyrimido[4',5':4,5]thieno[2,3-c]isoquinolin-8-amine
2,2-Dimethyl-5-morpholin-4-yl-N-(1-naphthylmethyl)-1,2,3,4-tetrahydropyrimido[4',5':4,5]thieno[2,3-c]isoquinolin-8-amine
N-(2-Furylmethyl)-2,2-dimethyl-5-morpholin-4-yl-1,2,3,4-tetrahydropyrimido[4',5':4,5]thieno[2,3-c]isoquinolin-8-amine
N-[2-(1H-Imidazol-4-yl)ethyl]-2,2-dimethyl-5-morpholin-4-yl-1,2,3,4-tetrahydropyrimido[4',5':4,5]thieno[2,3-c]isoquinolin-8-amine
1-{3-[(2,2-Dimethyl-5-morpholin-4-yl-1,2,3,4-tetrahydropyrimido[4',5':4,5]thieno[2,3-c]isoquinolin-8-yl)amino]propyl}pyrrolidin-2-one
2,2-Dimethyl-N-(2-morpholin-4-ylethyl)-5-thiomorpholin-4-yl-1,2,3,4-tetrahydropyrimido[4',5':4,5]thieno[2,3-c]isoquinolin-8-amine
2,2-Dimethyl-N-(pyridin-4-ylmethyl)-5-thiomorpholin-4-yl-1,2,3,4-tetrahydropyrimido[4',5':4,5]thieno[2,3-c]isoquinolin-8-amine
2,2-Dimethyl-N-(pyridin-3-ylmethyl)-5-thiomorpholin-4-yl-1,2,3,4-tetrahydropyrimido[4',5':4,5]thieno[2,3-c]isoquinolin-8-amine
2,2-Dimethyl-N-(pyridin-2-ylmethyl)-5-thiomorpholin-4-yl-1,2,3,4-tetrahydropyrimido[4',5':4,5]thieno[2,3-c]isoquinolin-8-amine
N-(2,3-Dimethoxybenzyl)-2,2-dimethyl-5-thiomorpholin-4-yl-1,2,3,4-tetrahydropyrimido[4',5':4,5]thieno[2,3-c]isoquinolin-8-amine
1-{3-[(2,2-Dimethyl-5-thiomorpholin-4-yl-1,2,3,4-tetrahydropyrimido[4',5':4,5]thieno[2,3-c]isoquinolin-8-yl)amino]propyl}pyrrolidin-2-one
Ethyl 4-{2-[(2,2-dimethyl-5-thiomorpholin-4-yl-1,2,3,4-tetrahydropyrimido[4',5':4,5]thieno[2,3-c]isoquinolin-8-yl)amino]ethyl}piperazine-1-carboxylate
2,2-Dimethyl-5-(4-methylpiperazin-1-yl)-N-(2-morpholin-4-ylethyl)-1,2,3,4-tetrahydropyrimido[4',5':4,5]thieno[2,3-c]isoquinolin-8-amine
2,2-Dimethyl-5-(4-methylpiperazin-1-yl)-N-(pyridin-3-ylmethyl)-1,2,3,4-tetrahydropyrimido[4',5':4,5]thieno[2,3-c]isoquinolin-8-amine
2,2-Dimethyl-5-(4-methylpiperazin-1-yl)-N-(pyridin-2-ylmethyl)-1,2,3,4-tetrahydropyrimido[4',5':4,5]thieno[2,3-c]isoquinolin-8-amine
2,2-Dimethyl-N-(2-morpholin-4-ylethyl)-5-pyrrolidin-1-yl-1,2,3,4-tetrahydropyrimido[4',5':4,5]thieno[2,3-c]isoquinolin-8-amine
2,2-Dimethyl-N-(pyridin-4-ylmethyl)-5-pyrrolidin-1-yl-1,2,3,4-tetrahydropyrimido[4',5':4,5]thieno[2,3-c]isoquinolin-8-amine
(2,2-Dimethyl-N-(pyridin-3-ylmethyl)-5-pyrrolidin-1-yl-1,2,3,4-tetrahydropyrimido[4',5':4,5]thieno[2,3-c]isoquinolin-8-amine
2,2-Dimethyl-N-(pyridin-2-ylmethyl)-5-pyrrolidin-1-yl-1,2,3,4-tetrahydropyrimido[4',5':4,5]thieno[2,3-c]isoquinolin-8-amine
N-(2-Methoxybenzyl)-2,2-dimethyl-5-pyrrolidin-1-yl-1,2,3,4-tetrahydropyrimido[4',5':4,5]thieno[2,3-c]isoquinolin-8-amine
N-(2,3-Dimethoxybenzyl)-2,2-dimethyl-5-pyrrolidin-1-yl-1,2,3,4-tetrahydropyrimido[4',5':4,5]thieno[2,3-c]isoquinolin-8-amine
N-(2-Furylmethyl)-2,2-dimethyl-5-pyrrolidin-1-yl-1,2,3,4-tetrahydropyrimido[4',5':4,5]thieno[2,3-c]isoquinolin-8-amine
N-[2-(1H-Imidazol-4-yl)ethyl]-2,2-dimethyl-5-pyrrotidin-1-yl-1,2,3,4-tetrahydropyrimido[4',5':4,5]thieno[2,3-c]isoquinolin-8-amine
1-{3-[(2,2-Dimethyl-5-pyrrolidin-1-yl-1,2,3,4-tetrahydropyrimido[4',5':4,5]thieno[2,3-c]isoquinolin-8-yl)amino]propyl}pyrrolidin-2-one
Ethyl 4-{2-[(2,2-dimethyl-5-pyrrolidin-1-yl-1,2,3,4-tetrahydropyrimido[4',5':4,5]thieno[2,3-c]isoquinolin-8-yl)amino]ethyl}piperazine-1-carboxylate
2,2-Dimethyl-N-(2-piperazin-1-ylethyl)-5-pyrrolidin-1-yl-1,2,3,4-tetrahydropyrimido[4',5':4,5]thieno[2,3-c]isoquinolin-8-amine
2,2-Dimethyl-N-[2-(4-methylpiperazin-1-yl)ethyl]-5-pyrrolidin-1-yl-1,2,3,4-tetrahydropyrimido[4',5':4,5]thieno[2,3-c]isoquinolin-8-amine
N2-(2,2-Dimethyl-5-pyrrolidin-1-yl-1,2,3,4-tetrahydropyrimido[4',5':4,5]thieno[2,3-c]isoquinolin-8-yl)-N1-(tetrahydrofuran-2-ylmethyl)glycinamide
2,2-Dimethyl-5-pyrrolidin-1-yl-N-(quinolin-3-ylmethyl)-1,2,3,4-hydropyrimido[4',5':4,5]thieno[2,3-c]isoquinolin-8-amine
2,2-Dimethyl-5-pyrrolidin-1-yl-N-(isoquinolin-4-ylmethyl)-1,2,3,4-tetrahydropyrimido[4',5':4,5]thieno[2,3-c]isoquinolin-8-amine
N⁵,N⁵,2,2-tetramethyl-N⁸-(2-morpholin-4-ylethyl)-1,2,3,4-tetrahydropyrimido[4',5':4,5]thieno[2,3-c]isoquinoline-5,8-diamine
N⁵,N⁵,2,2-Tetramethyl-N⁸-(pyridin-3-ylmethyl)-1,2,3,4-tetrahydropyrimido[4',5':4,5]thieno[2,3-c]isoquinoline-5,8-diamine
N⁸-(2,3-Dimethoxybenzyl)-N⁵, N⁵,2,2-tetramethyl-1,2,3,4-tetrahydropyrimido[4',5':4,5]thieno[2,3-c]isoquinoline-5,8-diamine
N⁸-[2-(1H-Imidazol-5-yl)ethyl]-N⁵, N⁵,2,2-tetramethyl-1,2,3,4-tetrahydropyrimido[4',5':4,5]thieno[2,3-c]isoquinoline-5,8-diamine
1-(3-{[5-(Dimethylamino)-2,2-dimethyl-1,2,3,4-tetrahydropyrimido[4',5':4,5]thieno[2,3-c]isoquinofin-8-yl]amino}propyl)pyrrolidin-2-one
Ethyl 4-(2-{[5-(dimethylamino)-2,2-dimethyl-1,2,3,4-tetrahydropyrimido[4',5':4,5]thieno[2,3-c]isoquinolin-8-yl]amino}ethyl)piperazine-1-carboxylate
N⁵,N⁵,2,2-Tetramethyl-N⁸-(2-piperazin-1-ylethyl)-1,2,3,4-tetrahydropyrimido[4',5':4,5]thieno[2,3-c]isoquinoline-5,8-diamine
3-{[5-(Dimethylamino)-2,2-dimethyl-1,2,3,4-tetrahydropyrimido[4',5':4,5]thieno[2,3-c]isoquinolin-8-yl]amino}propanenitrile
8-Ethoxy-N,N,2,2-tetramethyl-1,2,3,4-tetrahydropyrimido[4',5':4,5]thieno[2,3-c]isoquinolin-5-amine
N⁵-Ethyl-N⁵,2,2-trimethyl-N⁸-(2-morpholin-4-ylethyl)-1,2,3,4-tetrahydropyrimido[4',5':4,5]thieno[2,3-c]isoquinoline-5,8-diamine
N⁵-Ethyl-N⁵,2,2-trimethyl-N⁸-(pyridin-4-ylmethyl)-1,2,3,4-tetrahydropyrimido[4',5':4,5]thieno[2,3-c]isoquinoline-5,8-diamine
N⁵-Ethyl-N⁵,2,2-trimethyl-N⁸-(pyridin-3-ylmethyl)-1,2,3,4-tetrahydropyrimido[4',5':4,5]thieno[2,3-c]isoquinoline-5,8-diamine
N⁵-Ethyl-N⁵,2,2-trimethyl-N⁸-(pyridin-2-ylmethyl)-1,2,3,4-tetrahydropyrimido[4',5':4,5]thieno[2,3-c]isoquinoline-5,8-diamine
N⁵-Ethyl-N⁸-(2-furylmethyl)-N⁵,2,2-trimethyl-1,2,3,4-tetrahydropyrimido[4',5':4,5]thieno[2,3-c]isoquinoline-5,8-diamine
N⁵-Ethyl-N⁸-[2-(1H-imidazol-4-yl)ethyl]-N⁵,2,2-trimethyl-1,2,3,4-tetrahydropyrimido[4',5':4,5]thieno[2,3-c]isoquinoline-5,8-diamine
1-[3-({5-[Ethyl(methyl)amino]-2,2-dimethyl-1,2,3,4-tetrahydropyrimido[4',5':4,5]thieno[2,3-c]isoquinolin-8-yl}amino)propyl]pyrrolidin-2-one
N²-{5-[Ethyl(methyl)amino]-2,2-dimethyl-1,2,3,4-tetrahydropyrimido[4',5':4,5]thieno[2,3-c]isoquinolin-8-yl}-N'-(tetrahydrofuran-2-ylmethyl)glycinamide
2,2,5-Trimethyl-N-(2-morpholin-4-ylethyl)-1,2,3,4-tetrahydropyrimido[4',5':4,5]thieno[2,3-c]isoquinolin-8-amine
2,2,5-Trimethyl-N-(pyridin-3-ylmethyl)-1,2,3,4-tetrahydropyrimido[4',5':4,5]thieno[2,3-c]isoquinolin-8-amine
1-{3-[(2,2,5-Trimethyl-1,2,3,4-tetrahydropyrimido[4',5':4,5]thieno[2,3-c]isoquinolin-8-yl)amino]propyl}pyrrolidin-2-one
5-Isobutyl-2,2-dimethyl-N-(2-morpholin-4-ylethyl)-1,2,3,4-tetrahydropyrimido[4',5':4,5]thieno[2,3-c]isoquinolin-8-amine
5-Isobutyl-2,2-dimethyl-N-(pyridin-3-ylmethyl)-1,2,3,4-tetrahydropyrimido[4',5':4,5]thieno[2,3-c]isoquinolin-8-amine
1-{3-[(5-Isobutyl-2,2-dimethyl-1,2,3,4-tetrahydropyrimido[4',5':4,5]thieno[2,3-c]isoquinolin-8-yl)amino]propyl}pyrrolidin-2-one
Ethyl 4-{2-[(5-isobutyl-2,2-dimethyl-1,2,3,4-tetrahydropyrimido[4',5':4,5]thieno[2,3-c]isoquinolin-8-yl)amino]ethyl}piperazine-1-carboxylate
5-(2-Furyl)-2,2-dimethyl-N-(2-morpholin-4-ylethyl)-1,2,3,4-tetrahydropyrimido[4',5':4,5]thieno[2,3-c]isoquinolin-8-amine
5-(2-Furyl)-2,2-dimethyl-N-(pyridin-4-ylmethyl)-1,2,3,4-tetrahydropyrimido[4',5':4,5]thieno[2,3-c]isoquinolin-8-amine
5-(2-Furyl)-2,2-dimethyl-N-(pyridin-3-ylmethyl)-1,2,3,4-tetrahydropyrimido[4',5':4,5]thieno[2,3-c]isoquinolin-8-amine
5-(2-Furyl)-2,2-dimethyl-N-(pyridin-2-ylmethyl)-1,2,3,4-tetrahydropyrimido[4',5':4,5]thieno[2,3-c]isoquinolin-8-amine
1-(3-{[5-(2-Furyl)-2,2-dimethyl-1,2,3,4-tetrahydropyrimido[4', 5':4,5]thieno[2,3-c]isoquinolin-8-yl]amino}propyl)pyrrolidin-2-one
Ethyl 4-(2-{[5-(2-furyl)-2,2-dimethyl-1,2,3,4-tetrahydropyrimido[4',5':4,5]thieno[2,3-c]isoquinolin-8-yl]amino}ethyl)piperazine-1-carboxylate
5-(3-Furyl)-2,2-dimethyl-N-(2-morpholin-4-ylethyl)-1,2,3,4-tetrahydropyrimido[4',5':4,5]thieno[2,3-c]isoquinolin-8-amine
5-(3-Furyl)-2,2-dimethyl-N-(pyridin-2-ylmethyl)-1,2,3,4-tetrahydropyrimido[4',5':4,5]thieno[2,3-c]isoquinolin-8-amine
5-(3-Furyl)-2,2-dimethyl-N-(pyridin-3-ylmethyl)-1,2,3,4-tetrahydropyrimido[4',5':4,5]thieno[2,3-c]isoquinolin-8-amine
5-(3-Furyl)-2,2-dimethyl-N-(pyridin-4-ylmethyl)-1,2,3,4-tetrahydropyrimido[4',5':4,5]thieno[2,3-c]isoquinolin-8-amine
5-(3-Furyl)-N-[2-(1H-imidazol-4-yl)ethyl]-2,2-dimethyl-1,2,3,4-tetrahydropyrimido[4',5':4,5]thieno[2,3-c]isoquinolin-8-amine
1-(3-{[5-(3-Furyl)-2,2-dimethyl-1,2,3,4-tetrahydropyrimido[4',5':4,5]thieno[2,3-c]isoquinolin-8-yl]amino}propyl)pyrrolidin-2-one
Ethyl 4-(2-{[5-(3-furyl)-2,2-dimethyl-1,2,3,4-tetrahydropyrimido[4',5':4,5]thieno[2,3-c]isoquinolin-8-yl]amino}ethyl)piperazine-1-carboxylate
2,2,3-Trimethyl-5-morpholin-4-yl-N-(2-morpholin-4-ylethyl)-1,2,3,4-tetrahydropyrimido[4',5':4,5]thieno[2,3-c]-2,7-naphthyridin-8-amine
2,2,3-Trimethyl-5-morpholin-4-yl-N-(pyridin-3-ylmethyl)-1,2,3,4-tetrahydropyrimido[4',5':4,5]thieno[2,3-c]-2,7-naphthyridin-8-amine
N-(2-Methoxybenzyl)-2,2,3-trimethyl-5-morpholin-4-yl-1,2,3,4-tetrahydropyrimido[4',5':4,5]thieno[2,3-c]-2,7-naphthyridin-8-amine
N⁴,N⁴,2,2-Tetramethyl-N⁷-(2-morpholin-4-ylethyl)-2,3-dihydro-1H-cyclopenta[4',5']pyrido[3',2':4,5]thieno[3,2-d]pyrimidin-4,7-diamine
N⁴, N⁴,2, 2-Tetramethyl-N⁷-(pyridin-3-ylmethyl)-2,3-dihydro-1H-cyclopenta[4',5']pyrido[3',2':4,5]thieno[3,2-d]pyirimidin-4,7-diamine
1-(3-{[4-(Dimethylamino)-2,2-dimethyl-2,3-dihydro-1H-cyclopenta[4',5']pyrido[3',2':4,5]thieno[3,2-d]pyrimidin-7-yl]amino}propyl)pyrrolidin-2-one
N⁷-(2-Furylmethyl)-N⁴, N⁴, 2, 2-tetramethyl-2,3-dihydro-1H-cyclopenta[4',5']pyrido[3',2':4,5]thieno[3,2-d]pyrimidin-4,7-diamine
2,2-Dimethyl-4-morpholin-4-yl-7-(pyridin-3-ylmethyl)-2,3-dihydro-1 H-cyclopenta[4',5']pyrido[3',2':4,5]thieno[3,2-d]pyrimidin-7-amine
N⁴,N⁴,1,1-Tetramethyl-N⁷-(2-morpholin-4-ylethyl)-2,3-dihydro-1H-cyclopenta[4',5']pyrido[3',2':4,5]thieno[3,2-d]pyrimidin-4,7-diamine
N⁴,N⁴,1,1-Tetramethyl-N⁷-(pyridin-3-ylmethyl)-2,3-dihydro-1H-cyclopenta[4',5']pyrido[3',2':4,5]thieno[3,2-d]pyrimidin-4,7-diamine
Ethyl 4-(2-{[4-(dimethylamino)-1,1-dimethyl-2,3-dihydro-1H-cyclopenta[4',5']pyrido[3',2':4,5]thieno[3,2-d]pyrimidin-7-yl]amino}ethyl)pyperazincarboxilate
1-(3-{[4-(Dimehylamino)-1,1-dimethyl-2,3-dihydro-1H-cyclopenta[4',5']pyrido[3',2':4,5]thieno[3,2-d]pyrimidin-7-yl]amino}propyl)pyrrolidin-2-one
N⁷-[2-(1H-Imidazol-4-yl)ethyl]-N⁴,N⁴,1,1-tetramethyl-2,3-dihydro-1H-cyclopenta[4',5']pyrido[3',2':4,5]thieno[3,2-d]pyrimidin-4,7-diamine
N⁷-(2-Furylmethyl)-N⁴,N⁴,1,1-tetramethyl-2,3-dihydro-1H-cyclopenta[4',5']pyrido[3',2':4,5]thieno[3,2-d]pyrimidin-4,7-diamine
N'-(2,3-Dimethoxybenzyl)-N⁴,N⁴,1,1-tetramethyl-2,3-dihydro-1H-cyclopenta[4',5']pyrido[3',2':4,5]thieno[3,2-d]pyrimidin-4,7-diamine
1,1-Dimethyl-4-morpholin-4-yl-N⁷-(2-morpholin-4-ylethyl)-2,3-dihydro-1H-cyclopenta[4',5']pyrido[3',2':4,5]thieno[3,2-d]pyrimidin-7-amine
1,1-Dimethyl-4-morpholin-4-yl-N⁷-(pyridin-3-ylmethyl)-2,3-dihydro-1H-cyclopenta[4',5']pyrido[3',2':4,5]thieno[3,2-d]pyrimidin-7-amine
1-(3-{[4-(Morpholin-4-yl)-1,1-dimethyl-2,3-dihydro-1H-cyclopenta[4',5']pyrido[3',2':4,5]thieno[3,2-d]pyrimidin-7-yl]amino}propyl)pyrrolidin-2-one
2,2-Dimethyl-7-(2-morpholin-4-ylethyl)-4-pyrrolidin-1-yl-2,3-dihydro-1 H-cyclopenta[4',5']pyrido[3',2':4,5]thieno[3,2-d]pyrimidin-7-amine
2,2-Dimethyl-7-(pyridin-3-ylmethyl)-4-pyrrolidin-1-yl-2,3-dihydro-1H-cyclopenta[4',5']pyrido[3',2':4,5]thieno[3,2-d]pyrimidin-7-amine
2-[(2,2-Dimethyl-4-pyrrolidin-1-yl-2,3-dihydro-1H-cyclopenta[4',5']pyrido[3',2':4,5]thieno[3,2-d]pyrimidin-7-yl)(2-morpholin-4-ylethyl)amino]ethanol
2-[(2,2-Dimethyl-5-pyrrolidin-1-yl-1,2,3,4-tetrahydropyrimido[4',5':4,5]thieno[2,3-c]isoquinolin-8-yl)-(2-morpholin-4-ylethyl)amino]ethanol
N,N,2,2-Tetramethyl-4-pyrrolidin-1-yl-2,3-dihydro-1 H-cyclopenta[4',5']pyrido[3',2':4,5]thieno[3,2-d]pyrimidin-7-amine
2,2-Dimethyl-N-(2-morpholin-4-ylethyl)-N-(pyridin-3-ylmethyl)-4-pyrrolidin-1-yl-2,3-dihydro-1 H-cyclopenta[4',5']pyrido[3',2':4,5]thieno[3,2-d]pyrimidin-7-amine
N⁴-Ethyl-N⁴,2,2-trimethyl-N⁷-(2-morpholin-4-ylethyl)-2,3-dihydro-1H-cyclopenta[4',5']pyrido[3',2':4,5]thieno[3,2-d]pyrimidin-4,7-diamine
N⁴-Ethyl-N⁴,2,2-trimethyl-N⁷-(pyridin-3-ylmethyl)-2,3-dihydro-1H-cyclopenta[4',5']pyrido[3',2':4,5]thieno[3,2-d]pyrimidin-4,7-diamine.
1-[3-({4-[Ethyl(methyl)amino]-2,2-dimethyl-2,3-dihydro-1H-cyclopenta[4',5']pyrido[3',2':4,5]thieno[3,2-d]pyrimidin-7-yl}amino)propyl]pyrrolidin-2-one
N⁷-(2,3-Dimethoxybenzyl)-N⁴-ethyl-N⁴,2,2-trimethyl-2,3-dihydro-1H-cyclopenta[4',5']pyrido[3',2':4,5]thieno[3,2-d]pyrimidin-4,7-diamine
2-[{4-[Ethyl(methyl)amino+-2,2-dimethyl-2,3-dihydro-1H-cyclopenta[4',5']pyrido[3',2':4,5]thieno[3,2-d]pyrimidin-7-yl}(2-morpholin-4-ylethyl)amino]ethanol
N⁴-Ethyl-N⁴,2,2-trimethyl-N⁷-(2-morpholin-4-ylethyl)-N⁷-(pyridin-3-ylmethyl)-2,3-dihydro-1H-cyclopenta[4',5']pyrido[3',2':4,5]thieno[3,2-d]pyrimidin-4,7-diamine
2,2-Dimethyl-4-morpholin-4-yl-N-(2-morpholin-4-ylethyl)-2,3-dihydro- 1H-cyclopenta[4',5']pyrido[3',2':4,5]thieno[3,2-d]pyrimidin-7-amine
2-[(2,2-Dimethyl-4-morpholin-4-yl-2,3-dihydro-1 H-cyclopenta[4',5']pyrido[3',2':4,5]thieno[3,2-d]pyrimidin-7-yl)(2-morpholin-4-ylethyl)amino]ethanol
N-(2,3-Dimethoxybenzyl)-2,2-dimethyl-4-morpholin-4-yl-2,3-dihydro-1H-cyclopenta[4',5']pyrido[3',2':4,5]thieno[3,2-d]pyrimidin-7-amine
1-{3-[(2,2-Dimethyl-4-morpholin-4-yl-2,3-dihydro-1 H-cyclopenta[4',5']pyrido[3',2':4,5]thieno[3,2-d]pyrimidin-7-yl)amino]propyl}pyrrolidin-2-one
2-[(2,2-Dimethyl-5-morpholin-4-yl-1,2,3,4-tetrahydropyrimido[4',5':4,5]thieno[2,3-c]isoquinolin-8-yl)(2-morpholin-4-ylethyl)amino]ethanol
2-[(2,2-Dimethyl-5-morpholin-4yl-1,2,3,4-tetrahydropyrimido[4',5':4,5]thieno[2,3-c]isoquinolin-8-yl)(pyridin-3-ylmethyl)amino]ethanol
N⁵,2,2-Trimethyl-N⁸-(2-morpholin-4-ylethyl)-1,2,3,4-tetrahydropyrimido[4',5':4,5]thieno[2,3-c]isoquinoline-5,8-diamine
N⁵,2,2-Trimethyl-N⁸-(pyridin-3-ylmethyl)-1,2,3,4-tetrahydropyrimido[4',5':4,5]thieno[2,3-c]isoquinoline-5,8-diamine
1-(3-{[2,2-Dimethyl-5-(methylamino)-1,2,3,4-tetrahydropyrimido[4',5':4,5]thieno[2,3-c]isoquinolin-8-yl]amino}propyl)pyrrolidin-2-one
and pharmaceutically acceptable salts thereof.

24. A pharmaceutical composition comprising a compound according to any one of claims 12 to 23 in admixture with a pharmaceutically acceptable diluent or carrier.

25. A compound as defined in any one of claims 1 to 23 for use in treating a subject afflicted with a pathological condition or disease which is asthma, chronic obstructive pulmonary disease, rheumatoid arthritis, atopic dermatitis, psoriasis or irritable bowel syndrome.

26. A combination product comprising:
(i) a compound according to any one of claims 12 to 23; and
(ii) another compound selected from (a) steroids, (b) immunosuppressive agents, (c) T-cell receptor blockers and (d) antiinflammatory drugs
for simultaneous, separate or sequential use in the treatment of the human or animal body.

## Patentansprüche

1. Verwendung eines Pyridothienopyrimidinderivats der Formel (I) 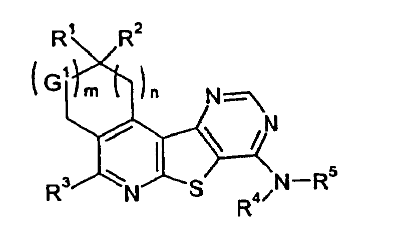 und der pharmazeutisch verträglichen Salze und N-Oxide davon, worin
G¹ eine Gruppe, ausgewählt aus -CR⁶R⁷- oder -NR⁶, darstellt, wobei R⁶ und R⁷ unabhängig ausgewählt sind aus Wasserstoffatomen und C₁₋₄-Alkylgruppen,
m und n ganze Zahlen sind, ausgewählt aus 0 oder 1,
R¹ und R² unabhängig ausgewählt sind aus Wasserstoffatomen und C₁₋₄-Alkylgruppen,
R³ eine Gruppe darstellt, ausgewählt aus C₁- bis C₂₀-Alkyl-, Amino-, Mono (C₁- bis C₁₀-alkyl) amino-, Di (C₁- bis C₁₀-alkyl) - amino, C₅ bis C₁₄Aryl-, 5- bis 14-gliedrigen Heteroaryl- und gesättigten N-haltigen 3- bis 10-gliedrigen Heterocyclylgruppen, gebunden über das Stickstoffatom an den Pyridinring, wobei alle davon gegebenenfalls mit einem oder mehreren Substituenten substituiert sind, ausgewählt aus der Gruppe, bestehend aus Halogenatomen und C₁- bis C₂₀-Alkyl-, (C₁- bis C₁₀-Alkoxy) (C₁- bis C₂₀-alkyl)-, (C₅- bis C₁₄-Aryl) (C₁- bis C₂₀-Alkyl) -, (R⁸OCO-)-, (C₁- bis C₁₀-Alkoxy) -, (R⁸R⁹N-CO-)-, (-CN) -, (-CF₃) -, (-NR⁸R⁹) -, (-SR⁸) - und (-SO₂NH₂) -Gruppen, wobei R⁸ und R⁹ unabhängig ausgewählt sind aus Wasserstoffatomen und C₁₋₄Alkylgruppen,
R⁴ und R⁵ unabhängig ausgewählt sind aus der Gruppe, bestehend aus Wasserstoffatomen, C₁- bis C₂₀-Alkylgruppen und Gruppen der Formel (II): 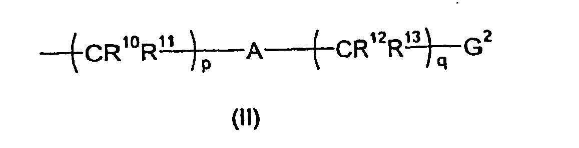 worin p und q ganze Zahlen sind, ausgewählt aus 1, 2 und 3; A entweder eine direkte Bindung oder eine Gruppe ist, ausgewählt aus -CONR¹⁴-, -DR¹⁴CO-, -O-, -COO-, -OCO-, -NR¹⁴COO-, -OCNR¹⁴-, -NR¹⁴ CONR¹⁵-, -S-, -SO-, -SO₂-, -COS- und -SCO-; und G² eine Gruppe ist, ausgewählt aus C₅- bis C₁₄-Aryl, 5- bis 14-gliedrigem Heteroaryl und 3- bis 10-gliedrigem Heterocyclyl; wobei die C₁- bis C₂₀-Alkylgruppen und die Gruppe G² gegebenenfalls substituiert sind mit einem oder mehreren Substituenten, ausgewählt aus der Gruppe, bestehend aus Halogenatomen und C₁- bis C₂₀-Alkyl-, (C₁- bis C₁₀-Alkoxy) (C₁- bis C₂₀-Alkyl)-, (C₅- bis C₁₄-Aryl) (C₁- bis C₂₀-alkyl)-, (R¹⁶OCO-)-, Hydroxy-, C₁- bis C₁₀-Alkoxy-, Oxo-, (R¹⁶R¹⁷NCO-)-, (-CN)-, (-CF₃) -, (-NR¹⁶R¹⁷) -, (SR¹⁶) - und (-SO₂NH₂) -Gruppen; wobei R¹⁰ bis R¹⁷ unabhängig ausgewählt sind aus Wasserstoffatomen und C₁₋₄-Alkylgruppen;
bei der Herstellung eines Medikaments für die Behandlung oder Prävention eines pathologischen Zustands oder einer Erkrankung, der/die Asthma, chronisch-obstruktive Lungenerkrankung, rheumatoide Arthritis, atopische Dermatitis, Psoriasis oder Reizdarmerkrankung ist.

2. Verwendung nach Anspruch 1, worin G¹ eine Gruppe -CR⁶R⁷ ist, wobei R⁶ und R⁷ wie hierin obenstehend definiert sind.

3. Verwendung gemäß Anspruch 2, wobei G¹ eine Gruppe -CH₂-ist.

4. Verwendung gemäß einem vorhergehenden Anspruch, wobei R¹ und R² beide Methylgruppen sind.

5. Verwendung gemäß einem vorhergehenden Anspruch, wobei m und n beide den Wert 1 aufweisen.

6. Verwendung gemäß einem vorhergehenden Anspruch, wobei R³ ausgewählt ist aus Mono(C₁- bis C₁₀-alkyl)amino-, Di(C₁- bis C₁₀-alkyl)amino- und gesättigten N-haltigen 3- bis 10-gliedrigen Heterocyclylgruppen, gebunden über das Stickstoffatom an den Pyridinring, wobei alle davon gegebenenfalls substituiert sind mit einem oder mehreren Substituenten, ausgewählt aus der Gruppe, bestehend aus Halogenatomen und C₁- bis C₂₀-Alkyl-, (C₁- bis C₁₀-Alkoxy) (C₁- bis C₂₀-alkyl) -, (C₅- bis C₁₄-Aryl) (C₁- bis C₂₀-alkyl)-, (R⁸OCO-)-, C₁- bis C₁₀-Alkoxy-, (R⁸R⁹NCO-) -, (-CN) -, (-CF₃) -, (-NR⁸R⁹) -, (SR⁸) - und (-SO₂NH₂)-Gruppen, wobei R⁸ und R⁹ unabhängig ausgewählt sind aus Wasserstoffatomen und C₁₋₄-Alkylgruppen.

7. Verwendung gemäß Anspruch 6, wobei R³ ausgewählt ist aus Mono (C₁- bis C₁₀-alkyl) amino-, Di (C₁- bis C₁₀-alkyl)amino- und gesättigten N-haltigen 3- bis 10-gliedrigen Heterocyclylgruppen, gebunden über das Stickstoffatom an den Pyridinring, wobei alle davon nicht substituiert sind.

8. Verwendung gemäß einem vorhergehenden Anspruch, wobei R⁴ ein Wasserstoffatom ist.

9. Verwendung gemäß einem vorhergehenden Anspruch, wobei R⁵ eine Gruppe der Formel (III) ist 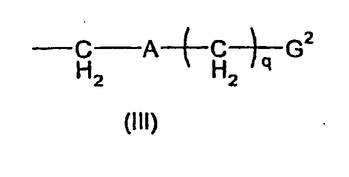 worin q eine ganze Zahl ist, ausgewählt aus 1 oder 2, A eine direkte Bindung oder eine Gruppe -CONH- darstellt, und G² eine Gruppe ist, ausgewählt aus C₅- bis C₁₄-Aryl, 5- bis 14-gliedrigem Heteroaryl und 3- bis 10-gliedrigem Heterocyclyl;
wobei die Gruppe G² gegebenenfalls substituiert ist mit einem oder mehreren Substituenten, ausgewählt aus der Gruppe, bestehend aus Halogenatomen und C₁- bis C₂₀-Alkyl-, (C₁- bis C₁₀-Alkoxy) (C₁- bis C₂₀-alkyl)-, (C₅- bis C₁₄-Aryl)(C₁- bis C₂₀-alkyl)-, (R¹⁶OCO-)-, C₁- bis C₁₀-Alkoxy-, (R¹⁶R¹⁷NCO-)-, (-CN)-, (-CF₃)-, (-NR¹⁶R¹⁷) (-SR¹⁶) - und (-SO₂NH₂) -Gruppen;
wobei R¹⁶ und R¹⁷ unabhängig ausgewählt sind aus Wasserstoffatomen und C₁₋₄-Alkylgruppen.

10. Verwendung gemäß Anspruch 9, wobei die Gruppe G² gegebenenfalls substituiert ist mit einem oder mehreren Substituenten, ausgewählt aus der Gruppe, bestehend aus Halogenatomen und C₁-C₁₀-Alkoxy- und (R¹⁶OCO)-Gruppen; wobei R¹⁶ ausgewählt ist aus Wasserstoffatomen und C₁₋₄-Alkylgruppen.

11. Verwendung gemäß einem vorhergehenden Anspruch, wobei G¹ eine Gruppe -CH₂- ist, R¹ und R² beide Methylgruppen sind, m und n beide den Wert 1 aufweisen, R³ ausgewählt ist aus Mono (C₁- bis C₁₀-alkyl)amino-, Di (C₁- bis C₁₀-alkyl)amino- und gesättigten N-haltigen 3- bis 10-gliedrigen Heterocyclylgruppen, gebunden über das Stickstoffatom an den Pyridinring, wobei alle davon nicht substituiert sind, R⁴ ein Wasserstoffatom ist und R⁵ eine Gruppe der Formel (III) ist 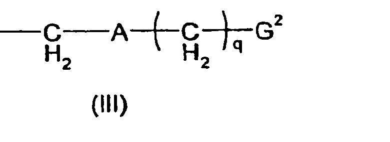 worin q eine ganze Zahl ist, ausgewählt aus 1 oder 2, A eine direkte Bindung oder eine Gruppe -CONH- darstellt und G² eine Gruppe ist, ausgewählt aus C₅- bis C₁₄-Aryl, 5- bis 14-gliedrigem Heteroaryl und 3- bis 10-gliedrigem Heterocyclyl;
wobei die Gruppe G² gegebenenfalls substituiert ist mit einem oder mehreren Substituenten, ausgewählt aus der Gruppe, bestehend aus Halogenatomen und C₁- bis C₈-Alkoxy-, Oxo- und (R¹⁶OCO-) -Gruppen; wobei R¹⁶ wie hierin obenstehend definiert ist.

12. Pyridothienopyrimidinderivat der Formel (I) 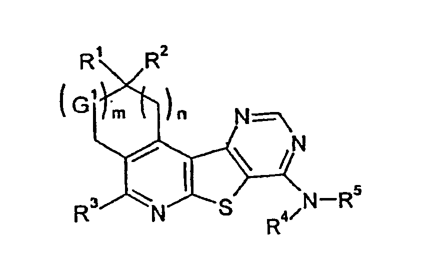 und die pharmazeutisch verträglichen Salze und N-Oxide davon, worin
G¹ eine Gruppe, ausgewählt aus -CR⁶R⁷- oder -NR⁶, darstellt, wobei R⁶ und R⁷ unabhängig ausgewählt sind aus Wasserstoffatomen und C₁₋₄-Alkylgruppen,
m und n ganze Zahlen sind, ausgewählt aus 0 oder 1,
R¹ ausgewählt ist aus C₁₋₄-Alkylgruppen,
R² ausgewählt ist aus Wasserstoffatomen und C₁₋₄-Alkylgruppen,
R³ eine Gruppe darstellt, ausgewählt aus C₁- bis C₂₀-Alkyl-, Amino-, Mono (C₁- bis C₁₀-alkyl) amino-, Di (C₁- bis C₁₀-alkyl) amino, C₅- bis C₁₄-Aryl-, 5- bis 14-gliedrigen Heteroaryl- und gesättigten N-haltigen 3- bis 10-gliedrigen Heterocyclylgruppen, gebunden über das Stickstoffatom an den Pyridinring, wobei alle davon gegebenenfalls mit einem oder mehreren Substituenten substituiert sind, ausgewählt aus der Gruppe, bestehend aus Halogenatomen und C₁- bis C₂₀-Alkyl-, (C₁- bis C₁₀-Alkoxy) (C₁- bis C₂₀-alkyl) -, (C₅- bis C₁₄-Aryl) (C₁-bis C₂₀-alkyl) -, (R⁸OCO-)-, (C₁- bis C₁₀-Alkoxy) -, (R⁸R⁹NCO-)-, (-CN) -, (-CF₃) -, (-NR⁸R⁹)-, (-SR⁸) - und (-SO₂NH₂)-Gruppen, wobei R⁸ und R⁹ unabhängig ausgewählt sind aus Wasserstoffatomen und C₁₋₉-Alkylgruppen,
R⁴ und R⁵ unabhängig ausgewählt sind aus der Gruppe, bestehend aus Wasserstoffatomen, Alkylgruppen und Gruppen der Formel (II): 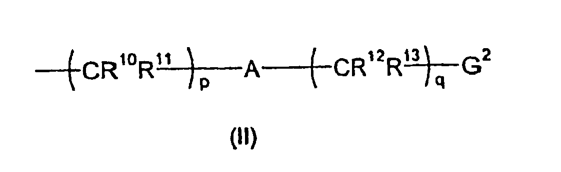 worin p und q ganze Zahlen sind, ausgewählt aus 1, 2 und 3; A entweder eine direkte Bindung oder eine Gruppe ist, ausgewählt aus -CONR¹⁴-, -NR¹⁴CO-, -O-, -COO-, -OCO-, -NR¹⁴COO-, -OCNR¹⁴-, -NR¹⁴CONR¹⁵-, -S-, -SO-, -SO₂-, -COS- und -SCO-; und G² eine Gruppe ist, ausgewählt aus C₅ bis C₁₄Aryl, 5- bis 14-gliedrigem Heteroaryl und 3- bis 10-gliedrigem Heterocyclyl; wobei die C₁- bis C₂₀-Alkylgruppen und die Gruppe G² gegebenenfalls substituiert sind mit einem oder mehreren Substituenten, ausgewählt aus der Gruppe, bestehend aus Halogenatomen und C₁- bis C₂₀-Alkyl-, (C₁- bis C₁₀-Alkoxy) (C₁- bis C₂₀-alkyl) -, (C₅- bis C₁₄-Aryl) (C₁- bis C₂₀-alkyl) -, (R¹⁶OCO-)-, Hydroxy-, C₁- bis C₁₀-Alkoxy-, Oxo-, (R¹⁶R¹⁷NCO-) -, (-CN) -, (-CF₃) -, (-NR¹⁶R¹⁷) -, (SR¹⁶) - und (-SO₂NH₂) -Gruppen; wobei R¹⁰ bis R¹⁷ unabhängig ausgewählt sind aus Wasserstoffatomen und C₁₋₄-Alkylgruppen;
und die pharmazeutisch verträglichen Salze und N-Oxide davon.

13. Verbindung gemäß Anspruch 12, worin G¹ eine Gruppe -CR⁶R⁷- ist.

14. Verbindung gemäß Anspruch 13, worin G¹ eine Gruppe -CH₂-ist.

15. Verbindung gemäß einem der Ansprüche 12 bis 14, worin R¹ und R² beide Methylgruppen sind.

16. Verbindung gemäß einem der Ansprüche 12 bis 15, worin m und n beide den Wert 1 aufweisen.

17. Verbindung gemäß einem der Ansprüche 12 bis 16, wobei R³ ausgewählt ist aus Mono (C₁- bis C₁₀-alkyl)amino-, Di (C₁- bis C₁₀-alkyl)amino- und gesättigten N-haltigen 3- bis 10-gliedrigen Heterocyclylgruppen, gebunden über das Stickstoffatom an den Pyridinring, wobei alle davon gegebenenfalls substituiert sind mit einem oder mehreren Substituenten, ausgewählt aus der Gruppe, bestehend aus Halogenatomen und C₁- bis C₂₀-Alkyl-, (C₁- bis C₁₀-Alkoxy) (C₁- bis C₂₀-alkyl)-, (C₅- bis C₁₄-Aryl) (C₁- bis C₂₀-alkyl)-, (R⁸OCO-)-, Alkoxy-, (R⁸R⁹NCO-) -, (-CN)-, (-CF₃) -, (-NR⁸R⁹)-, (SR⁸) - und (-SO₂NH₂) -Gruppen, wobei R⁸ und R⁹ unabhängig ausgewählt sind aus Wasserstoffatomen und C₁₋₄-Alkylgruppen.

18. Verbindung gemäß einem der Ansprüche 12 bis 17, wobei R³ ausgewählt ist aus Mono (C₁- bis C₁₀-alkyl) amino-, Di (C₁- bis C₁₀-alkyl)amino- und gesättigten N-haltigen 3- bis 10-gliedrigen Heterocyclylgruppen, gebunden über das Stickstoffatom an den Pyridinring, wobei alle davon nicht substituiert sind.

19. Verbindung gemäß einem der Ansprüche 12 bis 18, worin R⁴ ein Wasserstoffatom ist.

20. Verbindung gemäß einem der Ansprüche 12 bis 19, worin R⁵ eine Gruppe der Formel (III) ist 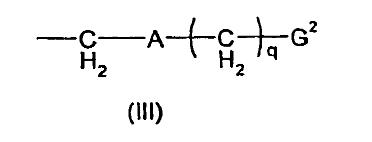 worin q eine ganze Zahl ist, ausgewählt aus 1 oder 2, A eine direkte Bindung oder eine Gruppe -CONH- darstellt, und G² eine Gruppe ist, ausgewählt aus C₅- bis C₁₄-Aryl, 5- bis 14-gliedrigem Heteroaryl und 3- bis 10-gliedrigem Heterocyclyl; wobei die Gruppe G² gegebenenfalls substituiert ist mit einem oder mehreren Substituenten, ausgewählt aus der Gruppe, bestehend aus Halogenatomen und C₁- bis C₂₀-Alkyl-, (C₁- bis C₁₀-Alkoxy) (C₁- bis C₂₀-alkyl)-, (C₅- bis C₁₄-Aryl) (C₁- bis C₂₀-alkyl) -, (R¹⁶OCO-)-, Hydroxy-, C₁- bis C₁₀-Alkoxy-, Oxo-, (R¹⁶R¹⁷N-CO-) -, (-CN) -, (-CF₃) -, (-NR¹⁶R¹⁷) -, (-SR¹⁶) - und (-SO₂NH₂) -Gruppen; wobei R¹⁶ und R¹⁷ unabhängig ausgewählt sind aus Wasserstoffatomen und C₁₋₄-Alkylgruppen.

21. Verbindung gemäß Anspruch 20, wobei die Gruppe G² gegebenenfalls substituiert ist mit einem oder mehreren Substituenten, ausgewählt aus der Gruppe, bestehend aus Halogenatomen und C₁-C₁₀-Alkoxy- und (R¹⁶OCO) -Gruppen; wobei R¹⁶ ausgewählt ist aus Wasserstoffatomen und C₁₋₄-Alkylgruppen.

22. Verbindung gemäß einem der Ansprüche 12 bis 21, wobei G¹ eine Gruppe -CH₂- ist, R¹ und R² beide Methylgruppen sind, m und n beide den Wert 1 aufweisen, R³ ausgewählt ist aus Mono (C₁- bis C₁₀-alkyl) amino-, Di (C₁- bis C₁₀-alkyl) amino- und gesättigten N-haltigen 3- bis 10-gliedrigen Heterocyclylgruppen, gebunden über das Stickstoffatom an den Pyridinring, wobei alle davon nicht substituiert sind, R⁴ ein Wasserstoffatom ist und R⁵ eine Gruppe der Formel (III) ist 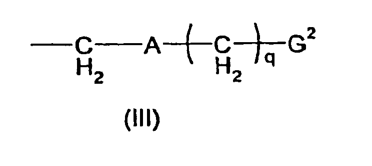 worin q eine ganze Zahl ist, ausgewählt aus 1 oder 2, A eine direkte Bindung oder eine Gruppe -CONH- darstellt und G² eine Gruppe ist, ausgewählt aus C₅- bis C₁₄-Aryl, 5- bis 14-gliedrigem Heteroaryl oder 3- bis 10-gliedrigem Heterocyclyl;
wobei die Gruppe G² gegebenenfalls substituiert ist mit einem oder mehreren Substituenten, ausgewählt aus der Gruppe, bestehend aus Halogenatomen und C₁- bis C₁₀-Alkoxy-, OXO- und (R¹⁶OCO-)-Gruppen; wobei R¹⁶ ausgewählt ist aus Wasserstoffatomen und C₁₋₄-Alkylgruppen.

23. Verbindung gemäß einem der Ansprüche 12 bis 22, welche eine der folgenden ist:
2,2-Dimethyl-5-morpholin-4-yl-N-(2-morpholin-4-ylethyl)-1,2,3,4-tetrahydropyrimido[4',5':4,5]thieno[2,3-c]isochinolin-8-amin
2,2-Dimethyl-5-morpholin-4-yl-N-(pyridin-4-ylmethyl)-1,2,3,4-tetrahydropyrimido[4',5':4,5]thieno[2,3-c]isochinolin-8-amin
2,2-Dimethyl-5-morpholin-4-yl-N-(pyridin-3-ylmethyl)-1,2,3,4-tetrahydropyrimido[4',5':4,5]thieno[2,3-c]isochinolin-8-amin
2,2-Dimethyl-5-morpholin-4-yl-N-(pyridin-2-ylmethyl)-1,2,3,4-tetrahydropyrimido[4',5':4,5]thieno[2,3-c]isochinolin-8-amin
N-(2-Methoxybenzyl)-2,2-dimethyl-5-morpholin-4-yl-1,2,3,4-tetrahydropyrimido[4',5':4,5]thieno[2,3-c]isochinolin-8-amin
N-(2,3-Dimethoxybenzyl)-2,2-dimethyl-5-morpholin-4-yl-1,2,3,4-tetrahydropyrimido[4',5':4,5]thieno[2,3-c]isochinolin-8-amin
4-{[(2,2-Dimethyl-5-morpholin-4-yl-1,2;3,4-tetrahydropyrimido[4',5':4,5]thieno[2,3-c]isochinolin-8-yl)amino]methyl}benzolsulfonamid
2,2-Dimethyl-5-morpholin-4-yl-N-(2-pyridin-2-ylethyl)-1,2,3,4-tetrahydropyrimido[4',5':4,5]thieno[2,3-c]isochinolin-8-amin
2,2-Dimethyl-5-morpholin-4-yl-N-(1-naphthylmethyl)-1,2,3,4-tetrahydropyrimido[4',5':4,5]thieno[2,3-c]isochinolin-8-amin
N-(2-Furylmethyl)-2,2-dimethyl-5-morpholin-4-yl-1,2,3,4-tetrahydropyrimido[4',5':4,5]thieno[2,3-c]isochinolin-8-amin
N-[2-(1H-Imidazol-4-yl)ethyl]-2,2-dimethyl-5-morpholin-4-yl-1,2,3,4-tetrahydropyrimido[4',5':4,5]thieno[2,3-c]isochinolin-8-amin
1-{3-[(2,2-Dimethyl-5-morpholin-4-yl-1,2,3,4-tetrahydropyrimido[4',5':4,5]thieno[2,3-c]isochinolin-8-yl)amino]propyl}pyrrolidin-2-on
2,2-Dimethyl-N-(2-morpholin-4-ylethyl)-5-thiomorpholin-4-yl-1,2,3,4-tetrahydropyrimido[4',5':4,5]thieno[2,3-c]isochinolin-8-amin
2,2-Dimethyl-N-(pyridin-4-ylmethyl)-5-thiomorpholin-4-yl-1,2,3,4-tetrahydropyrimido[4',5':4,5]thieno[2,3-c]isochinolin-8-amin
2,2-Dimethyl-N-(pyridin-3-ylmethyl)-5-thiomorpholin-4-yl-1,2,3,4-tetrahydropyrimido[4',5':4,5]thieno[2,3-c]isochinolin-8-amin
2,2-Dimethyl-N-(pyridin-2-ylmethyl)-5-thiomorpholin-4-yl-1,2,3,4-tetrahydropyrimido[4',5':4,5]thieno[2,3-c]isochinolin-8-amin
N-(2,3-Dimethoxybenzyl)-2,2-dimethyl-5-thiomorpholin-4-yl-1,2,3,4-tetrahydropyrimido[4',5':4,5]thieno[2,3-c]isochinolin-8-amin
1-{3-[(2,2-Dimethyl-5-thiomorpholin-4-yl-1,2,3,4-tetrahydropyrimido[4',5':4,5]thieno[2,3-c]isochinolin-8-yl)amino]propyl}pyrrolidin-2-on
4-{2-[(2,2-Dimethyl-5-thiomorpholin-4-yl-1,2,3,4-tetrahydropyrimido[4',5':4,5]thieno[2,3-c]isochinolin-8-yl)amino]ethyl}piperazin-1-carbonsäurethylester
2,2-Dimethyl-5-(4-methylpiperazin-1-yl)-N-(2-morpholin-4-ylethyl)-1,2,3,4-tetrahydropyrimido[4',5':4,5]thieno[2,3-c]isochinolin-8-amin
2,2-Dimethyl-5-(4-methylpiperazin-1-yl)-N-(pyridin-3-ylmethyl)-1,2,3,4-tetrahydropyrimido[4',5':4,5]thieno[2,3-c]isochinolin-8-amin
2,2-Dimethyl-5-(4-methylpiperazin-1-yl)-N-(pyridin-2-ylmethyl)-1,2,3,4-tetrahydropyrimido[4',5':4,5]thieno[2,3-c]isochinolin-8-amin
2,2-Dimethyl-N-(2-morpholin-4-ylethyl)-5-pyrrolidin-1-yl-1,2,3,4-tetrahydropyrimido[4',5':4,5]thieno[2,3-c]isochinolin-8-amin
2,2-Dimethyl-N-(pyridin-4-ylmethyl)-5-pyrrolidin-1-yl-1,2,3,4-tetrahydropyrimido[4',5':4,5]thieno[2,3-c]isochinolin-8-amin
(2,2-Dimethyl-N-(pyridin-3-ylmethyl)-5-pyrrolidin-1-yl-1,2,3,4-tetrahydropyrimido[4',5':4,5]thieno[2,3-c]isochinolin-8-amin
2,2-Dimethyl-N-(pyridin-2-ylmethyl)-5-pyrrolidin-1-yl-1,2,3,4-tetrahydropyrimido[4',5':4,5]thieno[2,3-c]isochinolin-8-amin
N-(2-Methoxybenzyl)-2,2-dimethyl-5-pyrrolidin-1-yl-1,2,3,4-tetrahydropyrimido[4',5':4,5]thieno[2,3-c]isochinolin-8-amin
N-(2,3-Dimethoxybenzyl)-2,2-dimethyl-5-pyrrolidin-1-yl-1,2,3,4-tetrahydropyrimido[4',5':4,5]thieno[2,3-c]isochinolin-8-amine
N-(2-Furylmethyl)-2,2-dimethyl-5-pyrrolidin-1-yl-1,2,3,4-tetrahydropyrimido[4',5':4,5]thieno[2,3-c]isochinolin-8-amin
N-[2-(1H-Imidazol-4-yl)ethyl]-2,2-dimethyl-5-pyrrolidin-1-yl-1,2,3,4-tetrahydropyrimido[4',5':4,5]thieno[2,3-c]isochinolin-8-amin
1-{3-[(2,2-Dimethyl-5-pyrrolidin-1-yl-1,2,3,4-tetrahydropyrimido[4',5':4,5]thieno[2,3-c]isochinolin-8-yl)amino]propyl}pyrrolidin-2-on
4-{2-[(2,2-Dimethyl-5-pyrrolidin-1-yl-1,2,3,4-tetrahydropyrimido[4',5':4,5]thieno[2,3-c]isochinolin-8-yl)amino]ethyl}piperazin-1-carbonsäureethylester
2,2-Dimethyl-N-(2-piperazin-1-ylethyl)-5-pyrrolidin-1-yl-1,2,3,4-tetrahydropyrimido[4',5':4,5]thieno[2,3-c]isochinolin-8-amin
2,2-Dimethyl-N-[2-(4-methylpiperazin-1-yl)ethyl]-5-pyrrolidin-1-yl-1,2,3,4-tetrahydropyrimido[4',5':4,5]thieno[2,3-c]isochinolin-8-amin
N²-(2,2-Dimethyl-5-pyrrolidin-1-yl-1,2,3,4-tetrahydropyrimido[4',5':4,5]thieno[2,3-c]isochinolin-8-yl)-N¹-(tetrahydrofuran-2-ylmethyl)glycinamid
2,2-Dimethyl-5-pyrrolidin-1-yl-N-(chinolin-3-ylmethyl)-1,2,3,4-hydropyrimido[4',5':4,5]thieno[2,3-c]isochinolin-8-amin
2,2-Dimethyl-5-pyrrolidin-1-yl-N-(isochinolin-4-ylmethyl)-1,2,3,4-tetrahydropyrimido[4',5':4,5]thieno[2,3-c]isochinolin-8-amin
N⁵,N⁵,2,2-Tetramethyl-N⁸-(2-morpholin-4-ylethyl)-1,2,3,4-tetrahydropyrimido[4',5':4,5]thieno[2,3-c]isochinolin-5,8-diamin
N⁵,N⁵,2,2-Tetramethyl-N⁸-(pyridin-3-ylmethyl)-1,2,3,4-tetrahydropyrimido[4',5':4,5]thieno[2,3-c]isochinolin-5,8-diamin
N⁸-(2,3-Dimethoxybenzyl)-N⁵,N⁵,2,2-tetramethyl-1,2,3,4-tetrahydropyrimido[4',5':4,5]thieno[2,3-c]isochinolin-5,8-diamin
N⁸-[2-(1H-Imidazol-5-yl)ethyl]-N⁵,N⁵,2,2-tetramethyl-1,2,3,4-tetrahydropyrimido[4',5':4,5]thieno[2,3-c]isochinolin-5,8-diamin
1-(3-{[5-(Dimethylamino)-2,2-dimethyl-1,2,3,4-tetrahydropyrimido[4',5':4,5]thieno[2,3-c]isochinolin-8-yl]amino}propyl)pyrrolidin-2-on
4-(2-{[5-(Dimethylamino)-2,2-dimethyl-1,2,3,4-tetrahydropyrimido[4',5':4,5]thieno[2,3-c]isochinolin-8-yl]amino}ethyl)piperazin-1-carbonsäureethylester
N⁵,N⁵,2,2-Tetramethyl-N⁸-(2-piperazin-1-ylethyl)-1,2,3,4-tetrahydropyrimido[4',5':4,5]thieno[2,3-c]isochinolin-5,8-diamin
3-{[5-(Dimethylamino)-2,2-dimethyl-1,2,3,4-tetrahydropyrimido[4',5':4,5]thieno[2,3-c]isochinolin-8-yl]amino)propannitril
8-Ethoxy-N,N,2,2-tetramethyl-1,2,3,4-tetrahydropyrimido[4',5':4,5]thieno[2,3-c]isochinolin-5-amin
N⁵-Ethyl-N⁵,2,2-trimethyl-N⁸-(2-morpholin-4-ylethyl)-1,2,3,4-tetrahydropyrimido[4',5':4,5]thieno[2,3-c]isochinolin-5,8-diamin
N⁵-Ethyl-N⁵,2,2-trimethyl-N⁸-(pyridin-4-ylmethyl)-1,2,3,4-tetrahydropyrimido[4',5':4,5]thieno[2,3-c]isochinolin-5,8-diamin
N⁵-Ethyl-N⁵,2,2-trimethyl-N⁸-(pyridin-3-ylmethyl)-1,2,3,4-tetrahydropyrimido[4',5':4,5]thieno[2,3-c]isochinolin-5,8-diamin
N⁵-Ethyl-N⁵,2,2-trimethyl-N⁸-(pyridin-2-ylmethyl)-1,2,3,4-tetrahydropyrimido[4',5':4,5]thieno[2,3-c]isochinolin-5,8-diamin
N⁵-Ethyl-N⁸-(2-furylmethyl)-N⁵,2,2-trimethyl-1,2,3,4-tetrahydropyrimido[4',5':4,5]thieno[2,3-c]isochinolin-5,8-diamin
N⁵-Ethyl-N⁸-[2-(1H-imidazol-4-yl)ethyl]-N⁵,2,2-trimethyl-1,2,3,4-tetrahydropyrimido[4',5':4,5]thieno[2,3-c]isochinolin-5,8-diamin
1-[3-({5-[Ethyl(methyl)amino]-2,2-dimethyl-1,2,3,4-tetrahydropyrimido[4',5':4,5]thieno[2,3-c]isochinolin-8-yl}amino)propyl]pyrrolidin-2-on
N²-{5-[Ethyl(methyl)amino]-2,2-dimethyl-1,2,3,4-tetrahydropyrimido[4',5':4,5]thieno[2,3-c]isochinolin-8-yl}-N¹-(tetrahydrofuran-2-ylmethyl)glycinamid
2,2,5-Trimethyl-N-(2-morpholin-4-ylethyl)-1,2,3,4-tetrahydropyrimido[4',5':4,5]thieno[2,3-c]isochinolin-8-amin
2,2,5-Trimethyl-N-(pyridin-3-ylmethyl)-1,2,3,4-tetrahydropyrimido[4',5':4,5]thieno[2,3-c]isochinolin-8-amin
1-{3-[(2,2,5-Trimethyl-1,2,3,4-tetrahydropyrimido[4',5':4,5]thieno[2,3-c]isochinolin-8-yl)amino]propyl}pyrrolidin-2-on
5-Isobutyl-2,2-dimethyl-N-(2-morpholin-4-ylethyl)-1,2,3,4-tetrahydropyrimido[4',5':4,5]thieno[2,3-c]isochinolin-8-amin
5-Isobutyl-2,2-dimethyl-N-(pyridin-3-ylmethyl)-1,2,3,4-tetrahydropyrimido[4',5':4,5]thieno[2,3-c]isochinolin-8-amin
1-{3-[(5-Isobutyl-2,2-dimethyl-1,2,3,4-tetrahydropyrimido[4',5':4,5]thieno[2,3-c]isochinolin-8-yl)amino]propyl}pyrrolidin-2-on
4-{2-[(5-Isobutyl-2,2-dimethyl-1,2,3,4-tetrahydropyrimido[4',5':4,5]thieno[2,3-c]isochinolin-8-yl)amino]ethyl}piperazin-1-carbonsäureethylester
5-(2-Furyl)-2,2-dimethyl-N-(2-morpholin-4-ylethyl)-1,2,3,4-tetrahydropyrimido[4',5':4,5]thieno[2,3-c]isochinolin-8-amin
5-(2-Furyl)-2,2-dimethyl-N-(pyridin-4-ylmethyl)-1,2,3,4-tetrahydropyrimido[4',5':4,5]thieno[2,3-c]isochinolin-8-amin
5-(2-Furyl)-2,2-dimethyl-N-(pyridin-3-ylmethyl)-1,2,3,4-tetrahydropyrimido[4',5':4,5]thieno[2,3-c]isochinolin-8-amin
5-(2-Furyl)-2,2-dimethyl-N-(pyridin-2-ylmethyl)-1,2,3,4-tetrahydropyrimido[4',5':4,5]thieno[2,3-c]isochinolin-8-amin
1-(3-{[5-(2-Furyl)-2,2-dimethyl-1,2,3,4-tetrahydropyrimido[4',5':4,5]thieno[2,3-c]isochinolin-8-yl]amin}propyl)pyrrolidin-2-on
4-(2-{[5-(2-Furyl)-2,2-dimethyl-1,2,3,4-tetrahydropyrimido[4',5':4,5]thieno[2,3-c]isochinolin-8-yl]amino}ethyl)piperazin-1-carbonsäureethylester
5-(3-Furyl)-2,2-dimethyl-N-(2-morpholin-4-ylethyl)-1,2,3,4-tetrahydropyrimido[4',5':4,5]thieno[2,3-c]isochinolin-8-amin
5-(3-Furyl)-2,2-dimethyl-N-(pyridin-2-ylmethyl)-1,2,3,4-tetrahydropyrimido[4',5':4,5]thieno[2,3-c]isochinolin-8-amin
5-(3-Furyl)-2,2-dimethyl-N-(pyridin-3-ylmethyl)-1,2,3,4-tetrahydropyrimido[4',5':4,5]thieno[2,3-c]isochinolin-8-amin
5-(3-Furyl)-2,2-dimethyl-N-(pyridin-4-ylmethyl)-1,2,3,4-tetrahydropyrimido[4',5':4,5]thieno[2,3-c]isochinolin-8-amin
5-(3-Furyl)-N-[2-(1H-imidazol-4-yl)ethyl]-2,2-dimethyl-1,2,3,4-tetrahydropyrimido[4',5':4,5]thieno[2,3-c]isochinolin-8-amin
1-(3-{[5-{3-Furyl)-2,2-dimethyl-1,2,3,4-tetrahydropyrimido[4',5':4,5]thieno[2,3-c]isochinolin-8-yl]amino}propyl)pyrrolidin-2-on
4-(2-{[5-(3-Furyl)-2,2-dimethyl-1,2,3,4-tetrahydropyrimido[4',5':4,5]thieno[2,3-c]isochinolin-8-yl]amino}ethyl)piperazin-1-carbonsäureethylester
2,2,3-Trimethyl-5-morpholin-4-yl-N-(2-morpholin-4-ylethyl)-1,2,3,4-tetrahydropyrimido[4',5':4,5]thieno[2,3-c]-2,7-naphthyridin-8-amin
2,2,3-Trimethyl-5-morpholin-4-yl-N-(pyridin-3-ylmethyl)-1,2,3,4-tetrahydropyrimido[4',5':4,5]thieno[2,3-c]-2,7-naphthyridin-8-amin
N-(2-Methoxybenzyl)-2,2,3-trimethyl-5-morpholin-4-yl-1,2,3,4-tetrahydropyrimido[4',5':4,5]thieno[2,3-c]-2,7-naphthyridin-8-amin
N⁴,N⁴,2,2-Tetramethyl-N⁷-(2-morpholin-4-ylethyl)-2,3-dihydro-1H-cyclopenta[4',5']pyrido[3',2':4,5]thieno[3,2-d]pyrimidin-4,7-diamin
N⁴,N⁴,2,2-Tetramethyl-N⁷-(pyridin-3-ylmethyl)-2,3-dihydro-1H-cyclopenta[4',5']pyrido[3',2':4,5]thieno[3,2-d]pyrimidin-4,7-diamin
1-(3-{[4-(Dimethylamino)-2,2-dimethyl-2,3-dihydro-1H-cyclopenta[4',5']pyrido[3',2':4,5]thieno[3,2-d]pyrimidin-7-yl]amino}propyl)pyrrolidin-2-on
N'-(2-Furylmethyl)-N⁴,N⁴,2,2-tetramethyl-2,3-dihydro-1H-cyclopenta[4',5']pyrido[3',2':4,5]thieno[3,2-d]pyrimidin-4,7-diamin
2,2-Dimethyl-4-morpholin-4-yl-7-(pyridin-3-ylmethyl)-2,3-dihydro-1H-cyclopenta[4',5']pyrido[3',2':4,5]thieno[3,2-d]pyrimidin-7-amin
N⁴,N⁴,1,1-Tetramethyl-N⁷-(2-morpholin-4-ylethyl)-2,3-dihydro-1H-cyclopenta[4',5']pyrido[3',2':4,5]thieno[3,2-d]pyrimidin-4,7-diamin
N⁴,N⁴,1,1-Tetramethyl-N⁷-(pyridin-3-ylmethyl)-2,3-dihydro-1H-cyclopenta[4',5']pyrido[3',2':4,5]thieno[3,2-d]pyrimidin-4,7-diamin
4-(2-{[4-(Dimethylamino)-1,1-dimethyl-2,3-dihydro-1H-cyclopenta[4',5']pyrido[3',2':4,5]thieno[3,2-d]pyrimidin-7-yl]amino}ethyl)piperazincarbonsäureethylester
1-(3-{[4-(Dimethylamino)-1,1-dimethyl-2,3-dihydro-1H-cyclopenta[4',5']pyrido[3',2':4,5]thieno[3,2-d]pyrimidin-7-yl]amino}propyl)pyrrolidin-2-on
N⁷-[2-(1H-Imidazol-4-yl)ethyl]-N⁴,N⁴,1,1-tetramethyl-2,3-dihydro-1H-cyclopenta[4',5']pyrido[3',2':4,5]thieno[3,2-d]pyrimidin-4,7-diamin
N⁷-(2-Furylmethyl) -N⁴,N⁴,1,1-tetramethyl-2,3-dihydro-1H-cyclopenta[4',5']pyrido[3',2':4,5]thieno[3,2-d]pyrimidin-4,7-diamin
N⁷-(2,3-Dimethoxybenzyl)-N⁴,N⁴,1,1-tetramethyl-2,3-dihydro-1H-cyclopenta[4',5']pyrido[3',2':4,5]thieno[3,2-d]pyrimidin-4,7-diamin
1,1-Dimethyl-4-morpholin-4-yl-N'-(2-morpholin-4-ylethyl)-2,3-dihydro-1H-cyclopenta[4',5']pyrido[3',2':4,5]thieno[3,2-d]pyrimidin-7-amin
1,1-Dimethyl-4-morpholin-4-yl-N⁷-(pyridin-3-ylmethyl)-2,3-dihydro-1H-cyclopenta[4',5']pyrido[3',2':4,5]thieno[3,2-d]pyrimidin-7-amin
1-(3-{[4-(Morpholin-4-yl)-1,1-dimethyl-2,3-dihydro-1H-cyclopenta[4',5']pyrido[3',2':4,5]thieno[3,2-d]pyrimidin-7-yl]amino}propyl)pyrrolidin-2-on
2,2-Dimethyl-7-(2-morpholin-4-ylethyl)-4-pyrrolidin-1-yl-2,3-dihydro-1H-cyclopenta[4',5']pyrido[3',2':4,5]thieno[3,2-d]pyrimidin-7-amin
2,2-Dimethyl-7-(pyridin-3-ylmethyl)-4-pyrrolidin-1-yl-2,3-dihydro-1H-cyclopenta[4',5']pyrido[3',2':4,5]thieno[3,2-d]pyrimidin-7-amin
2-[(2,2-Dimethyl-4-pyrrolidin-1-yl-2,3-dihydro-1H-cyclopenta[4',5']pyrido[3',2':4,5]thieno[3,2-d]pyrimidin-7-yl)(2-morpholin-4-ylethyl)amino]ethanol
2-[(2,2-Dimethyl-5-pyrrolidin-1-yl-1,2,3,4-tetrahydropyrimido[4',5':4,5]thieno[2,3-c]isochinolin-8-yl)-(2-morpholin-4-ylethyl)amino]ethanol
N,N,2,2-Tetramethyl-4-pyrrolidin-1-yl-2,3-dihydro-1H-cyclopenta[4',5']pyrido[3',2':4,5]thieno[3,2-d]pyrimidin-7-amin
2,2-Dimethyl-N-(2-morpholin-4-ylethyl)-N-(pyridin-3-ylmethyl)-4-pyrrolidin-1-yl-2,3-dihydro-1H-cyclopenta[4',5']pyrido[3',2':4,5]thieno[3,2-d]pyrimidin-7-amin
N⁴-Ethyl-N⁴,2,2-trimethyl-N⁷-(2-morpholin-4-ylethyl)-2,3-dihydro-1H-cyclopenta[4',5']pyrido[3',2':4,5]thieno[3,2-d]pyrimidin-4,7-diamin
N⁴-Ethyl-N⁴,2,2-trimethyl-N⁷-(pyridin-3-ylmethyl)-2,3-dihydro-1H-cyclopenta[4',5']pyrido[3',2':4,5]thieno[3,2-d]pyrimidin-4,7-diamin
1-[3-({4-[Ethyl(methyl)amino]-2,2-dimethyl-2,3-dihydro-1H-cyclopenta[4',5']pyrido[3',2':4,5]thieno[3,2-d]pyrimidin-7-yl}amino)propyl]pyrrolidin-2-on
N⁷-(2,3-Dimethoxybenzyl)-N⁴-ethyl-N⁴,2,2-trimethyl-2,3-dihydro-1H-cyclopenta[4',5']pyrido[3',2':4,5]thieno[3,2-d]pyrimidin-4,7-diamin
2-[{4-[Ethyl(methyl)amino+-2,2-dimethyl-2,3-dihydro-1H-cyclopenta[4',5']pyrido[3',2':4,5]thieno[3,2-d]pyrimidin-7-yl}(2-morpholin-4-ylethyl)amino]ethanol
N⁴-Ethyl-N⁴, 2,2-trimethyl-N⁷-(2-morpholin-4-ylethyl)-N⁷-(pyridin-3-ylmethyl)-2,3-dihydro-1H-cyclopenta[4',5']pyrido[3',2':4,5]thieno[3,2-d]pyrimidin-4,7-diamin
2,2-Dimethyl-4-morpholin-4-yl-N-(2-morpholin-4-ylethyl)-2,3-dihydro-1H-cyclopenta[4',5']pyrido[3',2':4,5]thieno[3,2-d]pyrimidin-7-amin
2-[(2,2-Dimethyl-4-morpholin-4-yl-2,3-dihydro-1H-cyclopenta[4',5']pyrido[3',2':4,5]thieno[3,2-d]pyrimidin-7-yl)(2-morpholin-4-ylethyl)amino]ethanol
N-(2,3-Dimethoxybenzyl)-2,2-dimethyl-4-morpholin-4-yl-2,3-dihydro-1H-cyclopenta[9',5']pyrido[3',2':4,5]thieno[3,2-d]pyrimidin-7-amin
1-{3-[(2,2-Dimethyl-4-morpholin-4-yl-2,3-dihydro-1H-cyclopenta[4',5']pyrido[3',2':4,5]thieno[3,2-d]pyrimidin-7-yl)amino]propyl}pyrrolidin-2-on
2-[(2,2-Dimethyl-5-morpholin-4-yl-1,2,3,4-tetrahydropyrimido[4',5':4,5]thieno[2,3-c]isochinolin-8-yl)(2-morpholin-4-ylethyl)amino]ethanol
2-[(2,2-Dimethyl-5-morpholin-4-yl-1,2,3,4-tetrahydropyrimido[4',5':4,5]thieno[2,3-c]isochinolin-8-yl)(pyridin-3-ylmethyl)amino]ethanol
N⁵,2,2-Trimethyl-N⁸-(2-morpholin-4-ylethyl)-1,2,3,4-tetrahydropyrimido[4',5':4,5]thieno[2,3-c]isochinolin-5,8-diamin
N⁵,2,2-Trimethyl-N⁸-(pyridin-3-ylmethyl)-1,2,3,4-tetrahydropyrimido[4',5':4,5]thieno[2,3-c]isochinolin-5,8-diamin
1-(3-{[2,2-Dimethyl-5-(methylamino)-1,2,3,4-tetrahydropyrimido[4',5':4,5]thieno[2,3-c]isochinolin-8-yl]amino}propyl)pyrrolidin-2-on
und pharmazeutisch verträgliche Salze davon.

24. Pharmazeutische Zusammensetzung, umfassend eine Verbindung gemäß einem der Ansprüche 12 bis 23 in Mischung mit einem pharmazeutisch verträglichen Verdünnungsmittel oder Träger.

25. Verbindung, wie in einem der Ansprüche 1 bis 23 definiert, zur Verwendung bei der Behandlung eines Subjekts, das von einem pathologischen Zustand oder einer Erkrankung betroffen ist, der/die Asthma, chronisch-obstruktive Lungenerkrankung, rheumatoide Arthritis, atopische Dermatitis, Psoriasis oder Reizdarmsyndrom ist.

26. Kombinationsprodukt, umfassend:
(i) eine Verbindung gemäß einem der Ansprüche 12 bis 23, und
(ii) eine andere Verbindung, ausgewählt aus (a) Steroiden, (b) Immunsuppressiva, (c) T-Zell-Rezeptor-Blockern und (d) entzündungshemmenden Wirkstoffen,
zur simultanen, getrennten oder sequentiellen Verwendung bei der Behandlung des menschlichen oder tierischen Körpers.

## Revendications

1. Utilisation d'un dérivé de pyridothiénopyrimidine de formule (I) 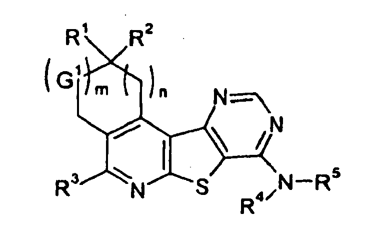 et des sels et N-oxydes pharmaceutiquement acceptables de celui-ci, dans laquelle
G¹ représente un groupe choisi parmi -CR⁶R⁷- ou -NR⁶, R⁶ et R⁷ étant indépendamment choisis parmi les atomes d'hydrogène et les groupes alkyles en C₁₋₄
m et n sont des entiers choisis parmi 0 et 1
R¹ et R² sont indépendamment choisis parmi les atomes d'hydrogène et les groupes alkyles en C₁₋₄
R³ représente un groupe choisi parmi les groupes alkyle en C₁ à C₂₀, amino, mono(alkyl en C₁ à C₁₀) amino, di(alkyl en C₁ à C₁₀) amino, aryle en C₅ à C₁₄, hétéroaryle à 5 à 14 chaînons et hétérocyclyle saturé à 3 à 10 chaînons contenant N, liés par l'atome d'azote au cycle pyridine, tous étant facultativement substitués par un ou plusieurs substituants choisis dans le groupe constitué par les atomes d'halogène et les groupes alkyle en C₁ à C₂₀, (alcoxy en C₁ à C₁₀) (alkyle en C₁ à C₂₀), (aryl en C₅ à C₁₄) (alkyle en C₁ à C₂₀), R⁸OCO-, alcoxy en C₁ à C₁₀, R⁸R⁹N-CO-, -CN, -CF₃, -NR⁸R⁹, -SR⁸ et -SO₂NH₂ où R⁸ et R⁹ sont indépendamment choisis parmi les atomes d'hydrogène et les groupes alkyles en C₁₋₄
R⁴ et R⁵ sont indépendamment choisis dans le groupe constitué par les atomes d'hydrogène, les groupes alkyles en C₁ à C₂₀ et les groupes de formule (II) _{:} 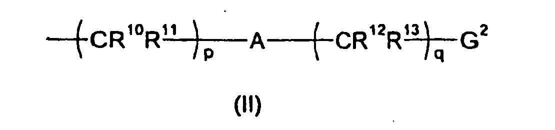 dans laquelle p et q sont des entiers choisis parmi 1, 2 et 3 ; A est soit une liaison directe soit un groupe choisi parmi -CONR¹⁴ -, -NR¹⁴CO-, -O-, -COO-, -OCO-, -NR¹⁴COO-, -OCONR¹⁴-, -NR¹⁴CONR¹⁵-, -S-, -SO-, -SO₂-, -COS- et -SCO- ; et G² est un groupe choisi parmi un aryle en C₅ à C₁₄, un hétéroaryle à 5 à 14 chaînons ou un hétérocyclyle à 3 à 10 chaînons ; dans laquelle les groupes alkyles en C₁ à C₂₀ et le groupe G² sont facultativement substitués par un ou plusieurs substituants choisis dans le groupe constitué par les atomes d'halogène et les groupes alkyle en C₁ à C₂₀, (alcoxy en C₁ à C₁₀) (alkyle en C₁ à C₂₀), (aryl en C₅ à C₁₄) (alkyle en C₁ à C₂₀), R¹⁶OCO-, hydroxy, alcoxy en C₁ à C₁₀, oxo, R¹⁶R¹⁷NCO-, -CN, -CF₃, -NR¹⁶R¹⁷, -SR¹⁶ et -SO₂NH₂; où R¹⁰ à R¹⁷ sont indépendamment choisis parmi les atomes d'hydrogène et les groupes alkyles en C₁₋₄ ;
dans la fabrication d'un médicament pour le traitement ou la prévention d'une affection pathologique ou d'une maladie qui est un asthme, une bronchopneumopathie chronique obstructive, une polyarthrite rhumatoïde, une dermatite atopique, un psoriasis ou une maladie du côlon irritable.

2. Utilisation selon la revendication 1, dans laquelle G¹ est un groupe -CR⁶R⁷-, où R⁶ et R⁷ sont tels que définis ci-dessus.

3. Utilisation selon la revendication 2, dans laquelle le groupe G¹ est un groupe -CH₂-.

4. Utilisation selon l'une quelconque des revendications précédentes, dans laquelle R¹ et R² sont tous les deux des groupes méthyles.

5. Utilisation selon l'une quelconque des revendications précédentes, dans laquelle m et n ont tous les deux la valeur de 1.

6. Utilisation selon l'une quelconque des revendications précédentes, dans laquelle R³ est choisi parmi un mono(alkyl en C₁ à C₁₀) amino, un di(alkyl en C₁ à C₁₀)amino et des groupes hétérocyclyles saturés à 3 à 10 chaînons contenant N, liés par l'atome d'azote au cycle pyridine, tous ceux-ci étant facultativement substitués par un ou plusieurs substituants choisis dans le groupe constitué par les atomes d'halogène et les groupes alkyle en C₁ à C₂₀, (alcoxy en C₁ à C₁₀) (alkyle en C₁ à C₂₀) , (aryl en C₅ à C₁₉) (alkyle en C₁ à C₂₀), R⁸OCO-, alcoxy en C₁ à C₁₀, R⁸R⁹NCO-, -CN, -CF₃, -NR⁸R⁹, -SR⁸ et -SO₂NH₂ où R⁸ et R⁹ sont indépendamment choisis parmi les atomes d'hydrogène et les groupes alkyles en C₁₋₄.

7. Utilisation selon la revendication 6, dans laquelle R³ est choisi parmi les groupes mono(alkyl en C₁ à C₁₀) amino, di (alkyl en C₁ à C₁₀) amino et hétérocyclyle saturé à 3 à 10 chaînons contenant N, liés par l'atome d'azote au cycle pyridine, tous ceux-ci étant non substitués.

8. Utilisation selon l'une quelconque des revendications précédentes, dans laquelle R⁴ est un atome d'hydrogène.

9. Utilisation selon l'une quelconque des revendications précédentes, dans laquelle R⁵ est un groupe de formule (III) 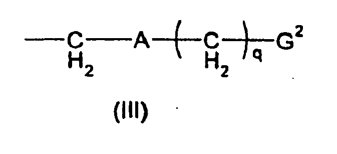 dans laquelle q est un entier choisi parmi 1 et 2, A représente une liaison directe ou un groupe -CONH- et G² est un groupe choisi parmi un aryle en C₅ à C₁₄, un hétéroaryle à 5 à 14 chaînons et un hétérocyclyle à 3 à 10 chaînons ; dans laquelle le groupe G² est facultativement substitué par un ou plusieurs substituants choisis dans le groupe constitué par les atomes d'halogène et les groupes alkyle en C₁ à C₂₀, (alcoxy en C₁ à C₁₀) (alkyle en C₁ à C₂₀), (aryl en C₅ à C₁₄) (alkyle en C₁ à C₂₀) , R¹⁶OCO-, alcoxy en C₁ à C₁₀, R¹⁶R¹⁷NCO-, -CN, -CF₃, -NR¹⁶R¹⁷, -SR¹⁶ et -SO₂NH₂ ; où R¹⁶ et R¹⁷ sont indépendamment choisis parmi les atomes d'hydrogène et les groupes alkyles en C₁₋₄.

10. Utilisation selon la revendication 9, dans laquelle le groupe G² est facultativement substitué par un ou plusieurs substituants choisis dans le groupe constitué par les atomes d'halogène et les groupes alcoxy en C₁ à C₁₀ et R¹⁶OCO- : où R¹⁶ est choisi parmi les atomes d'hydrogène et les groupes alkyles en C₁₋₄.

11. Utilisation selon l'une quelconque des revendications précédentes, dans laquelle G¹ est un groupe -CH₂-, R¹ et R² sont tous les deux des groupes méthyles, m et n ont tous les deux la valeur de 1, R³ est choisi parmi les groupes mono(alkyl en C₁ à C₁₀) amino, di(alkyl en C₁ à C₁₀)amino et hétérocyclyle saturé à 3 à 10 chaînons contenant N, liés par l'atome d'azote au cycle pyridine, tous ceux-ci étant non substitués, R⁴ est un atome d'hydrogène et R⁵ est un groupe de formule (III) 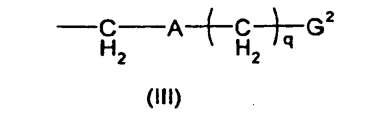 dans laquelle q est un entier choisi parmi 1 et 2, A représente une liaison directe ou un groupe -CONH- et G² est un groupe choisi parmi un aryle en C₅ à C₁₄, un hétéroaryle à 5 à 14 chaînons et un hétérocyclyle à 3 à 10 chaînons ; dans laquelle le groupe G² est facultativement substitué par un ou plusieurs substituants choisis dans le groupe constitué par les atomes d'halogène et les groupes alcoxy en C₁ à C₈, oxo et R¹⁶OCO- ; où R¹⁶ est tel que défini ci-dessus.

12. Dérivé de pyridothiénopyrimidine de formule (I) 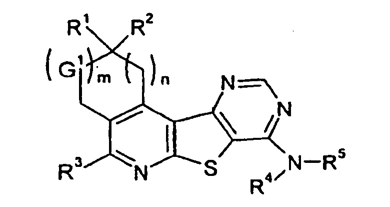 et les sels et N-oxydes pharmaceutiquement acceptables de celui-ci, dans laquelle
G¹ représente un groupe choisi parmi -CR⁶R⁷- ou -NR⁶, R⁶ et R⁷ étant indépendamment choisis parmi les atomes d'hydrogène et les groupes alkyles en C₁₋₄
m et n sont des entiers choisis parmi 0 et 1
R¹ est choisi parmi les groupes alkyles en C₁₋₄
R² est choisi parmi les atomes d'hydrogène et les groupes alkyles en C₁₋₄
R³ représente un groupe choisi parmi les groupes alkyle en C₁ à C₂₀, amino, mono(alkyl en C₁ à C₁₀) amino, di(alkyl en C₁ à C₁₀) amino, aryle en C₅ à C₁₄, hétéroaryle à 5 à 14 chaînons et hétérocyclyle saturé à 3 à 10 chaînons contenant N, liés par l'atome d'azote au cycle pyridine, tous ceux-ci étant facultativement substitués par un ou plusieurs substituants choisis dans le groupe constitué par les atomes d'halogène et les groupes alkyle en C₁ à C₂₀, (alcoxy en C₁ à C₁₀) (alkyle en C₁ à C₂₀), (aryl en C₅ à C₁₄) (alkyle en C₁ à C₂₀), R⁸OCO-, alcoxy en C₁ à C₁₀, R⁸R⁹NCO-, -CN, -CF₃, -NR⁸R⁹, -SR⁸ et -SO₂NH₂ où R⁸ et R⁹ sont indépendamment choisis parmi les atomes d'hydrogène et les groupes alkyles en C₁₋₄
R⁴ et R⁵ sont indépendamment choisis dans le groupe constitué par les atomes d'hydrogène, les groupes alkyles et les groupes de formule (II) : 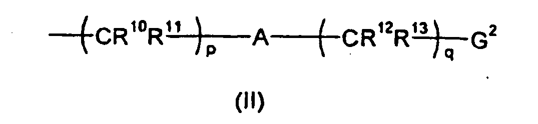 dans laquelle p et q sont des entiers choisis parmi 1, 2 et 3 ; A est soit une liaison directe soit un groupe choisi parmi -CONR¹⁴ -, -NR¹⁴CO-, -O-, -COO-, -OCO-, -NR¹⁴COO-, -OCONR¹⁴-, -NR¹⁴CONR¹⁵-, -S-, -SO-, -SO₂-, -COS- et -SCO- ; et G² est un groupe choisi parmi un aryle en C₅ à C₁₄, un hétéroaryle à 5 à 14 chaînons ou un hétérocyclyle à 3 à 10 chaînons ; dans laquelle les groupes alkyles en C₁ à C₂₀ et le groupe G² sont facultativement substitués par un ou plusieurs substituants choisis dans le groupe constitué par les atomes d'halogène et les groupes alkyle en C₁ à C₂₀, (alcoxy en C₁ à C₁₀) (alkyle en C₁ à C₂₀), (aryl en C₅ à C₁₄) (alkyle en C₁ à C₂₀) , R¹⁶OCO-, hydroxy, alcoxy en C₁ à C₁₀, oxo, R¹⁶R¹⁷NCO-, -CN, -CF₃, -NR¹⁶R¹⁷, -SR¹⁶ et -SO₂NH₂ ; où R¹⁰ à R¹⁷ sont indépendamment choisis parmi les atomes d'hydrogène et les groupes alkyles en C₁₋₄
et les sels et N-oxydes pharmaceutiquement acceptables de celui-ci.

13. Composé selon la revendication 12, dans lequel G¹ est un groupe -CR⁶R⁷-.

14. Composé selon la revendication 13, dans lequel G¹ est un groupe -CH₂-.

15. Composé selon l'une quelconque des revendications 12 à 14, dans lequel R¹ et R² sont tous les deux des groupes méthyles.

16. Composé selon l'une quelconque des revendications 12 à 15, dans lequel m et n ont tous les deux la valeur de 1.

17. Composé selon l'une quelconque des revendications 12 à 16, dans lequel R³ est choisi parmi les groupes mono(alkyl en C₁ à C₁₀) amino, di(alkyl en C₁ à C₁₀) amino et hétérocyclyle saturé à 3 à 10 chaînons contenant N, liés par l'atome d'azote au cycle pyridine, tous étant facultativement substitués par un ou plusieurs substituants choisis dans le groupe constitué par les atomes d'halogène et les groupes alkyle en C₁ à C₂₀, (alcoxy en C₁ à C₁₀) (alkyle en C₁ à C₂₀), (aryl en C₅ à C₁₉) (alkyle en C₁ à C₂₀), R⁸OCO-, alcoxy en C₁ à C₁₀, R⁸R⁹NCO-, -CN, -CF₃, -NR⁸R⁹, -SR⁸ et -SO₂NH₂ où R⁸ et R⁹ sont indépendamment choisis parmi les atomes d'hydrogène et les groupes alkyles en C₁₋₄.

18. Composé selon l'une quelconque des revendications 12 à 17, dans lequel R³ est choisi parmi un mono (alkyl en C₁ à C₁₀) amino, un di(alkyl en C₁ à C₁₀) amino et des groupes hétérocyclyles saturés à 3 à 10 chaînons contenant N, liés par l'atome d'azote au cycle pyridine, tous ceux-ci étant non substitués.

19. Composé selon l'une quelconque des revendications 12 à 18, dans lequel R⁴ est un atome d'hydrogène.

20. Composé selon l'une quelconque des revendications 12 à 19, dans lequel R⁵ est un groupe de formule (III) 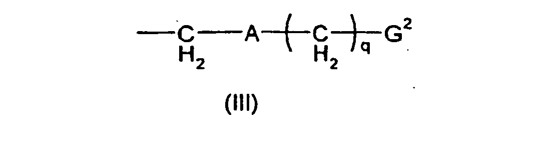 dans laquelle q est un entier choisi parmi 1 et 2, A représente une liaison directe ou un groupe -CONH- et G² est un groupe choisi parmi un aryle en C₅ à C₁₄, un hétéroaryle à 5 à 14 chaînons et un hétérocyclyle à 3 à 10 chaînons ; dans laquelle le groupe G² est facultativement substitué par un ou plusieurs substituants choisis dans le groupe constitué par les atomes d'halogène et les groupes alkyle en C₁ à C₂₀, (alcoxy en C₁ à C₁₀) (alkyle en C₁ à C₂₀), (aryl en C₅ à C₁₄) (alkyle en C₁ à C₂₀), R¹⁶OCO-, hydroxy, alcoxy en C₁ à C₁₀, oxo, R¹⁶R¹⁷N-CO-, -CN, -CF₃, -NR¹⁶R¹⁷, -SR¹⁶ et -SO₂NH₂ ; où R¹⁶ et R¹⁷ sont indépendamment choisis parmi les atomes d'hydrogène et les groupes alkyles en C₁₋₄.

21. Composé selon la revendication 20, dans lequel le groupe G² est facultativement substitué par un ou plusieurs substituants choisis dans le groupe constitué par les atomes d'halogène et les groupes alcoxy en C₁ à C₁₀ et R¹⁶OCO ; où R¹⁶ est choisi parmi les atomes d'hydrogène et les groupes alkyles en C₁₋₄.

22. Composé selon l'une quelconque des revendications 12 à 21, dans lequel G¹ est un groupe -CH₂-, R¹ et R² sont tous les deux des groupes méthyles, m et n ont tous les deux la valeur de 1, R³ est choisi parmi les groupes mono(alkyl en C₁ à C₁₀) amino, di(alkyl en C₁ à C₁₀) amino et hétérocyclyle saturé à 3 à 10 chaînons contenant N, liés par l'atome d'azote au cycle pyridine, tous ceux-ci étant non substitués, R⁴ est un atome d'hydrogène et R⁵ est un groupe de formule (III) 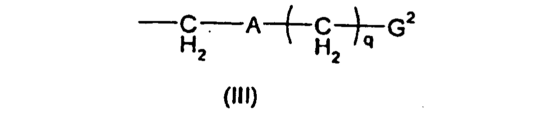 dans laquelle q est un entier choisi parmi 1 et 2, A représente une liaison directe ou un groupe -CONH- et G² est un groupe choisi parmi un aryle en C₅ à C₁₄, un hétéroaryle à 5 à 14 chaînons et un hétérocyclyle à 3 à 10 chaînons ; dans laquelle le groupe G² est facultativement substitué par un ou plusieurs substituants choisis dans le groupe constitué par les atomes d'halogène et les groupes alcoxy en C₁ à C₁₀, oxo et R¹⁶OCO- ; où R¹⁶ est choisi parmi les atomes d'hydrogène et les groupes alkyles en C₁₋₄.

23. Composé selon l'une quelconque des revendications 12 à 22, qui est l'un parmi :
la 2,2-diméthyl-5-morpholin-4-yl-N-(2-morpholin-4-yléthyl)-1,2,3,4-tétrahydropyrimido[4',5':4,5]thiéno[2,3-c]isoquinoléine-8-amine
la 2,2-diméthyl-5-morpholin-4-yl-N-(pyridin-4-ylméthyl)-1,2,3,4-tétrahydropyrimido[4',5':4,5]thiéno[2,3-c]isoquinoléine-8-amine
la 2,2-diméthyl-5-morpholin-4-yl-N-(pyridin-3-ylméthyl)-1,2,3,4-tétrahydropyrimido[4',5':4,5]thiéno[2,3-c]isoquinoléine-8-amine
la 2,2-diméthyl-5-morpholin-4-yl-N-(pyridin-2-ylméthyl)-1,2,3,4-tétrahydropyrimido[4',5':4,5]thiéno[2,3-c]isoquinoléine-8-amine
la N-(2-méthoxybenzyl)-2,2-diméthyl-5-morpholin-4-yl-1,2,3,4-tétrahydropyrimido[4',5':4,5]thiéno[2,3-c]-isoquinoléine-8-amine
la N-(2,3-diméthoxybenzyl)-2,2-diméthyl-5-morpholin-4-yl-1,2,3,4-tétrahydropyrimido[4',5':4,5]thiéno[2,3-c]-isoquinoléine-8-amine
le N-{[(2,2-diméthyl-5-morpholin-4-yl-1,2,3,4-tétra-hydropyrimido[4',5':4,5]thiéno[2,3-c]isoquinoléin-8-yl)-amino]méthyl}benzènesulfonamide
la 2,2-diméthyl-5-morpholin-4-yl-N-(2-pyridin-2-yléthyl)-1,2,3,4-tétrahydropyrimido[4',5':4,5]thiéno[2,3-c]isoquinoléine-8-amine
la 2,2-diméthyl-5-morpholin-4-yl-N-(1-naphtylméthyl)-1,2,3,4-tétrahydropyrimido[4',5':4,5]thiéno[2,3-c]-isoquinoléine-8-amine
la N-(2-furylméthyl)-2,2-diméthyl-5-morpholin-4-yl-1,2,3,4-tétrahydropyrimido[4',5':4,5]thiéno[2,3-c]-isoquinoléine-8-amine
la N-[2-(1H-imidazol-4-yl)éthyl]-2,2-diméthyl-5-morpholin-4-yl-1,2,3,4-tétrahydropyrimido[4',5':4,5]thiéno-[2,3-c]isoquinoléine-8-amine
la 1-{3-[(2,2-diméthyl-5-morpholin-4-yl-1,2,3,4-tétra-hydropyrimido[4',5':4,5]thiéno[2,3-c]isoquinoléin-8-yl)amino]propyl}pyrrolidin-2-one
la 2,2-diméthyl-N-(2-morpholin-4-yléthyl)-5-thio-morpholin-4-yl-1,2,3,4-tétrahydropyrimido[4',5':4,5]thiéno-[2,3-c]isoquinoléine-8-amine
la 2,2-diméthyl-N-(pyridin-4-ylméthyl)-5-thiomorpholin-4-yl-1,2,3,4-tétrahydropyrimido[4',5':4,5]thiéno[2,3-c]isoquinoléine-8-amine
la 2,2-diméthyl-N-(pyridin-3-ylméthyl)-5-thiomorpholin-4-yl-1,2,3,4-tétrahydropyrimido[4',5':4,5]thiéno[2,3-c]isoquinoléine-8-amine
la 2,2-diméthyl-N-(pyridin-2-ylméthyl)-5-thiomorpholin-4-yl-1,2,3,4-tétrahydropyrimido[4',5':4,5]thiéno[2,3-c]isoquinoléine-8-amine
la N-(2,3-diméthoxybenzyl)-2,2-diméthyl-5-thiomorpholin-4-yl-1,2,3,4-tétrahydropyrimido[4',5':4,5]thiéno[2,3-c]-isoquinoléine-8-amine
la 1-{3-[(2,2-diméthyl-5-thiomorpholin-4-yl-1,2,3,4-tétrahydropyrimido[4',5':4,5]thiéno[2,3-c]isoquinoléin-8-yl)amino]propyl}pyrrolidin-2-one
le 4-{2-[(2,2-diméthyl-5-thiomorpholin-4-yl-1,2,3,4-tétrahydropyrimido[4',5':4,5]thiéno[2,3-c]isoquinoléin-8-yl)amino]éthyl}pipérazine-1-carboxylate d'éthyle
la 2,2-diméthyl-5-(4-méthylpipérazin-1-yl)-N-(2-morpholin-4-yléthyl)-1,2,3,4-tétrahydropyrimido[4',5':4,5]-thiéno[2,3-c]isoquinoléine-8-amine
la 2,2-diméthyl-5-(4-méthylpipérazin-1-yl)-N-(pyridin-3-ylméthyl)-1,2,3,4-tétrahydropyrimido[4',5':4,5]thiéno[2,3-c]isoquinoléine-8-amine
la 2,2-diméthyl-5-(4-méthylpipérazin-1-yl)-N-(pyridin-2-ylméthyl)-1,2,3,4-tétrahydropyrimido[4',5':4,5]thiéno[2,3-c]isoquinoléine-8-amine
la 2,2-diméthyl-N-(2-morpholin-4-yléthyl)-5-pyrrolidin-1-yl-1,2,3,4-tétrahydropyrimido[4',5':4,5]thiéno[2,3-c]isoquinoléine-8-amine
la 2,2-diméthyl-N-(pyridin-4-ylméthyl)-5-pyrrolidin-1-yl-1,2,3,4-tétrahydropyrimido[4',5':4,5]thiéno[2,3-c]isoquinoléine-8-amine
la 2,2-diméthyl-N-(pyridin-3-ylméthyl)-5-pyrrolidin-1-yl-1,2,3,4-tétrahydropyrimido[4',5':4,5]thiéno[2,3-c]isoquinoléine-8-amine
la 2,2-diméthyl-N-(pyridin-2-ylméthyl)-5-pyrrolidin-1-yl-1,2,3,4-tétrahydropyrimido[4',5':4,5]thiéno[2,3-c]isoquinoléine-8-amine
la N-(2-méthoxybenzyl)-2,2-diméthyl-5-pyrrolidin-1-yl-1,2,3,4-tétrahydropyrimido[4',5':4,5]thiéno[2,3-c]-isoquinoléine-8-amine
la N-(2,3-diméthoxybenzyl)-2,2-diméthyl-5-pyrrolidin-1-yl-1,2,3,4-tétrahydropyrimido[4',5':4,5]thiéno[2,3-c]-isoquinoléine-8-amine
la N-(2-furylméthyl)-2,2-diméthyl-5-pyrrolidin-1-yl-1,2,3,4-tétrahydropyrimido[4',5':4,5]thiéno[2,3-c]-isoquinoléine-8-amine
la N-[2-(1H-imidazol-4-yl)éthyl]-2,2-diméthyl-5-pyrrolidin-1-yl-1,2,3,4-tétrahydropyrimido[4',5':4,5]thiéno-[2,3-c]isoquinoléine-8-amine
la 1-{3-[(2,2-diméthyl-5-pyrrolidin-1-yl-1,2,3,4-tétrahydropyrimido[4',5':4,5]thiéno[2,3-c]isoquinoléine-8-yl)amino]propyl}pyrrolidin-2-one
le 4-{2-[(2,2-diméthyl-5-pyrrolidin-1-yl-1,2,3,4-tétra-hydropyrimido[4',5':4,5]thiéno[2,3-c]isoquinoléin-8-yl)amino]éthyl}pipérazine-1-carboxylate d'éthyle
la 2,2-diméthyl-N-(2-pipérazin-1-yléthyl)-5-pyrrolidin-1-yl-1,2,3,4-tétrahydropyrimido[4',5':4,5]thiéno[2,3-c]isoquinoléine-8-amine
la 2,2-diméthyl-N-[2-(4-méthylpipérazin-1-yl)éthyl]-5-pyrrolidin-1-yl-1,2,3,4-tétrahydropyrimido[4',5':4,5]thiéno-[2,3-c]isoquinoléine-8-amine
le N2-(2,2-diméthyl-5-pyrrolidin-1-yl-1,2,3,4-tétra-hydropyrimido[4',5':4,5]thiéno[2,3-c]isoquinoléine-8-yl)-N1-(tétrahydrofuran-2-ylméthyl)glycinamide
la 2,2-diméthyl-5-pyrrolidin-1-yl-N-(quinoléin-3-ylméthyl)-1,2,3,4-hydropyrimido[4',5':4,5]thiéno[2,3-c]isoquinoléine-8-amine
la 2,2-diméthyl-5-pyrrolidin-1-yl-N-(isoquinoléin-4-ylméthyl)-1,2,3,4-tétrahydropyrimido[4',5':4,5]thiéno[2,3-c]isoquinoléine-8-amine
la N⁵,N⁵,2,2-tétraméthyl-N⁸-(2-morpholin-4-yléthyl)-1,2,3,4-tétrahydropyrimido[4',5':4,5]thiéno[2,3-c]isoquinoléine-5,8-diamine
la N⁵,N⁵,2,2-tétraméthyl-N⁸-(pyridin-3-ylméthyl)-1,2,3,4-tétrahydropyrimido[4',5':4,5]thiéno[2,3-c]isoquinoléine-5,8-diamine
la N⁸-(2,3-diméthoxybenzyl)-N⁵,N⁵,2,2-tétraméthyl-1,2,3,4-tétrahydropyrimido[4',5':4,5]thiéno[2,3-c]isoquinoléine-5,8-diamine
la N⁸-[2-(1H-imidazol-5-yl)éthyl]-N⁵,N⁵,2,2-tétraméthyl-1,2,3,4-tétrahydropyrimido[4',5':4,5]thiéno[2,3-c]isoquinoléine-5,8-diamine
la 1-(3-{[5-(diméthylamino)-2,2-diméthyl-1,2,3,4-tétra-hydropyrimido[4',5':4,5]thiéno[2,3-c]isoquinoléin-8-yl]-amino}propyl)pyrrolidin-2-one
le 4-(2-{[5-(diméthylamino)-2,2-diméthyl-1,2,3,4-tétra-hydropyrimido[4',5':4,5]thiéno[2,3-c]isoquinoléin-8-yl]-amino}éthyl)pipérazine-1-carboxylate d'éthyle
la N⁵,N⁵,2,2-tétraméthyl-N⁸-(2-pipérazin-1-yléthyl)-1,2,3,4-tétrahydropyrimido[4',5':4,5]thiéno[2,3-c]isoquinoléine-5,8-diamine
le 3-{[5-(diméthylamino)-2,2-diméthyl-1,2,3,4-tétra-hydropyrimido[4',5':4,5]thiéno[2,3-c]isoquinoléin-8-yl]-amino}propanenitrile
la 8-éthoxy-N,N,2,2-tétraméthyl-1,2,3,4-tétrahydro-pyrimido[4',5':4,5]thiéno[2,3-c]isoquinoléine-5-amine
la N⁵-éthyl-N⁵,2,2-triméthyl-N⁸-(2-morpholin-4-yléthyl)-1,2,3,4-tétrahydropyrimido[4',5':4,5]thiéno[2,3-c]isoquinoléine-5,8-diamine
la N⁵-éthyl-N⁵,2,2-triméthyl-N⁸-(pyridin-4-ylméthyl)-1,2,3,4-tétrahydropyrimido[4',5':4,5]thiéno[2,3-c]isoquinoléine-5,8-diamine
la N⁵-éthyl-N⁵,2,2-triméthyl-N⁸-(pyridin-3-ylméthyl)-1,2,3,4-tétrahydropyrimido[4',5':4,5]thiéno[2,3-c]isoquinoléine-5,8-diamine
la N⁵-éthyl-N⁵,2,2-triméthyl-N⁸-(pyridin-2-ylméthyl)-1,2,3,4-tétrahydropyrimido[4',5':4,5]thiéno[2,3-c]isoquinoléine-5,8-diamine
la N⁵-éthyl-N⁸-(2-furylméthyl)-N⁵,2,2-triméthyl-1,2,3,4-tétrahydropyrimido[4',5':4,5]thiéno[2,3-c]isoquinoléine-5,8-diamine
la N⁵-éthyl-N⁸-[2-(1H-imidazol-4-yl)éthyl]-N⁵,2,2-triméthyl-1,2,3,4-tétrahydropyrimido[4',5':4,5]thiéno[2,3-c]isoquinoléine-5,8-diamine
la 1-[3-({5-[éthyl(méthyl)amino]-2,2-diméthyl-1,2,3,4-tétrahydropyrimido[4',5':4,5]thiéno[2,3-c]isoquinoléin-8-yl}amino)propyl]pyrrolidin-2-one
le N²-{5-[éthyl(méthyl)amino]-2,2-diméthyl-1,2,3,4-tétrahydropyrimido[4',5':4,5]thiéno[2,3-c]isoquinoléin-8-yl}-N¹-(tétrahydrofuran-2-ylméthyl)glycinamide
la 2,2,5-triméthyl-N-(2-morpholin-4-yléthyl)-1,2,3,4-tétrahydropyrimido[4',5':4,5]thiéno[2,3-c]isoquinoléine-8-amine
la 2,2,5-triméthyl-N-(pyridin-3-ylméthyl)-1,2,3,4-tétra-hydropyrimido[4',5':4,5]thiéno[2,3-c]isoquinoléine-8-amine
la 1-{3-[(2,2,5-triméthyl-1,2,3,4-tétrahydropyrimido-[4',5':4,5]thiéno[2,3-c]isoquinoléin-8-yl)amino]propyl}-pyrrolidin-2-one
la 5-isobutyl-2,2-diméthyl-N-(2-morpholin-4-yléthyl)-1,2,3,4-tétrahydropyrimido[4',5':4,5]thiéno[2,3-c]isoquinoléine-8-amine
la 5-isobutyl-2,2-diméthyl-N-(pyridin-3-ylméthyl)-1,2,3,4-tétrahydropyrimido[4',5':4,5]thiéno[2,3-c]isoquinoléine-8-amine
la 1-{3-[(5-isobutyl-2,2-diméthyl-1,2,3,4-tétrahydro-pyrimido[4',5':4,5]thiéno[2,3-c]isoquinoléin-8-yl)amino]-propyl}pyrrolidin-2-one
le 4-{2-[(5-isobutyl-2,2-diméthyl-1,2,3,4-tétrahydro-pyrimido[4',5':4,5]thiéno[2,3-c]isoquinoléin-8-yl)amino]-éthyl}pipérazine-1-carboxylate d'éthyle
la 5-(2-furyl)-2,2-diméthyl-N-(2-morpholin-4-yléthyl)-1,2,3,4-tétrahydropyrimido[4',5':4,5]thiéno[2,3-c]-isoquinoléine-8-amine
la 5-(2-furyl)-2,2-diméthyl-N-(pyridin-4-ylméthyl)-1,2,3,4-tétrahydropyrimido[4',5':4,5]thiéno[2,3-c]-isoquinoléine-8-amine
la 5-(2-furyl)-2,2-diméthyl-N-(pyridin-3-ylméthyl)-1,2,3,4-tétrahydropyrimido[4',5':4,5]thiéno[2,3-c]-isoquinoléine-8-amine
la 5-(2-furyl)-2,2-diméthyl-N-(pyridin-2-ylméthyl)-1,2,3,4-tétrahydropyrimido[4',5':4,5]thiéno[2,3-c]-isoquinoléine-8-amine
la 1-(3-{[5-(2-furyl)-2,2-diméthyl-1,2,3,4-tétrahydro-pyrimido[4',5':4,5]thiéno[2,3-c]isoquinoléin-8-yl]amino}-propyl)pyrrolidin-2-one
le 4-(2-{[5-(2-furyl)-2,2-diméthyl-1,2,3,4-tétrahydro-pyrimido[4',5':4,5]thiéno[2,3-c]isoquinoléin-8-yl]amino}-éthyl)pipérazine-1-carboxylate d'éthyle
la 5-(3-furyl)-2,2-diméthyl-N-(2-morpholin-4-yléthyl)-1,2,3,4-tétrahydropyrimido[4',5':4,5]thiéno[2,3-c]-isoquinoléine-8-amine
la 5-(3-furyl)-2,2-diméthyl-N-(pyridin-2-ylméthyl)-1,2,3,4-tétrahydropyrimido[4',5':4,5]thiéno[2,3-c]-isoquinoléine-8-amine
la 5-(3-furyl)-2,2-diméthyl-N-(pyridin-3-ylméthyl)-1,2,3,4-tétrahydropyrimido[4',5':4,5]thiéno[2,3-c]-isoquinoléine-8-amine
la 5-(3-furyl)-2,2-diméthyl-N-(pyridin-4-ylméthyl)-1,2,3,4-tétrahydropyrimido[4',5':4,5]thiéno[2,3-c]-isoquinoléine-8-amine
la 5-(3-furyl)-N-[2-(1H-imidazol-4-yl)éthyl]-2,2-diméthyl-1,2,3,4-tétrahydropyrimido[4',5':4,5]thiéno[2,3-c]-isoquinoléine-8-amine
la 1-(3-{[5-(3-furyl)-2,2-diméthyl-1,2,3,4-tétrahydro-pyrimido[4',5':4,5]thiéno[2,3-c]isoquinoléin-8-yl]amino}-propyl)pyrrolidin-2-one
le 4-(2-{[5-(3-furyl)-2,2-diméthyl-1,2,3,4-tétrahydro-pyrimido[4',5':4,5]thiéno[2,3-c]isoquinoléin-8-yl]amino}-éthyl)pipérazine-1-carboxylate d'éthyle
la 2,2,3-triméthyl-5-morpholin-4-yl-N-(2-morpholin-4-yléthyl)-1,2,3,4-tétrahydropyrimido[4',5':4,5]thiéno[2,3-c]-2,7-naphtyridine-8-amine
la 2,2,3-triméthyl-5-morpholin-4-yl-N-(pyridin-3-ylméthyl)-1,2,3,4-tétrahydropyrimido[4',5':4,5]thiéno[2,3-c]-2,7-naphtyridine-8-amine
la N-(2-méthoxybenzyl)-2,2,3-triméthyl-5-morpholin-4-yl-1,2,3,4-tétrahydropyrimido[4',5':4,5]thiéno[2,3-c]-2,7-naphtyridine-8-amine
la N⁴,N⁴,2,2-tétraméthyl-N⁷-(2-morpholin-4-yléthyl)-2,3-dihydro-1H-cyclopenta[4',5']pyrido[3',2':4,5]thiéno[3,2-d]-pyrimidine-4,7-diamine
la N⁴,N⁴,2,2-tétraméthyl-N⁷-(pyridin-3-ylméthyl)-2,3-dihydro-1H-cyclopenta[4',5']pyrido[3',2':4,5]thiéno[3,2-d]-pyrimidine-4,7-diamine
la 1-(3-{[4-(diméthylamino)-2,2-diméthyl-2,3-dihydro-1H-cyclopenta[4',5']pyrido[3',2':4,5]thiéno[3,2-d]pyrimidin-7-yl]amino}propyl)pyrrolidin-2-one
la N⁷-(2-furylméthyl)-N⁴,N⁴,2,2-tétraméthyl-2,3-dihydro-1H-cyclopenta[4',5']pyrido[3',2':4,5]thiéno[3,2-d]-pyrimidine-4,7-diamine
la 2,2-diméthyl-4-morpholin-4-yl-7-(pyridin-3-ylméthyl)-2,3-dihydro-1H-cyclopenta[4',5']pyrido[3',2':4,5]thiéno[3,2-d]pyrimidine-7-amine
la N⁴,N⁴,1,1-tétraméthyl-N⁷-(2-morpholin-4-yléthyl)-2,3-dihydro-1H-cyclopenta[4',5']pyrido[3',2':4,5]thiéno[3,2-d]-pyrimidine-4,7-diamine
la N⁴,N⁴,1,1-tétraméthyl-N⁷-(pyridin-3-ylméthyl)-2,3-dihydro-1H-cyclopenta[4',5']pyrido[3',2':4,5]thiéno[3,2-d]-pyrimidine-4,7-diamine
le 4-(2-([4-(diméthylamino)-1,1-diméthyl-2,3-dihydro-1H-cyclopenta[4',5']pyrido[3',2':4,5]thiéno[3,2-d]pyrimidin-7-yl]amino}éthyl)pipérazinecarboxylate d'éthyle
la 1-(3-{[4-(diméthylamino)-1,1-diméthyl-2,3-dihydro-1H-cyclopenta[4',5']pyrido[3',2':4,5]thiéno[3,2-d]pyrimidin-7-yl]amino}propyl)pyrrolidin-2-one
la N⁷-[2-(1H-imidazol-4-yl)éthyl]-N⁴,N⁴,1,1-tétraméthyl-2,3-dihydro-1H-cyclopenta[4',5']pyrido[3',2':4,5]thiéno[3,2-d]pyrimidine-4,7-diamine
la N⁷-(2-furylméthyl)-N⁴,N⁴,1,1-tétraméthyl-2,3-dihydro-1H-cyclopenta[4',5']pyrido[3',2':4,5]thiéno[3,2-d]-pyrimidine-4,7-diamine
la N⁷-(2,3-diméthoxybenzyl)-N⁴,N⁴,1,1-tétraméthyl-2,3-dihydro-1H-cyclopenta[4',5']pyrido[3',2':4,5]thiéno[3,2-d]-pyrimidine-4,7-diamine
la 1,1-diméthyl-4-morpholin-4-yl-N⁷-(2-morpholin-4-yléthyl)-2,3-dihydro-1H-cyclopenta[4',5']pyrido[3',2':4,5]-thiéno[3,2-d]pyrimidine-7-amine
la 1,1-diméthyl-4-morpholin-4-yl-N⁷-(pyridin-3-ylméthyl)-2,3-dihydro-1H-cyclopenta[4',5']pyrido[3',2':4,5]-thiéno[3,2-d]pyrimidine-7-amine
la 1-(3-{[4-(morpholin-4-yl)-1,1-diméthyl-2,3-dihydro-1H-cyclopenta[4',5']pyrido[3',2':4,5]thiéno[3,2-d]pyrimidin-7-yl]amino}propyl)pyrrolidin-2-one
la 2,2-diméthyl-7-(2-morpholin-4-yléthyl)-4-pyrrolidin-1-yl-2,3-dihydro-1H-cyclopenta[4',5']pyrido[3',2':4,5]-thiéno[3,2-d]pyrimidine-7-amine
la 2,2-diméthyl-7-(pyridin-3-ylméthyl)-4-pyrrolidin-1-yl-2,3-dihydro-1H-cyclopenta[4',5']pyrido[3',2':4,5]thiéno-[3,2-d]pyrimidine-7-amine
le 2-[(2,2-diméthyl-4-pyrrolidin-1-yl-2,3-dihydro-1H-cyclopenta[4',5']pyrido[3',2':4,5]thiéno[3,2-d]pyrimidin-7-yl)(2-morpholin-4-yléthyl)amino]éthanol
le 2-[(2,2-diméthyl-5-pyrrolidin-1-yl-1,2,3,4-tétra-hydropyrimido[4',5':4,5]thiéno[2,3-c]isoquinoléin-8-yl)-(2-morpholin-4-yléthyl)amino]éthanol
la N,N,2,2-tétraméthyl-4-pyrrolidin-1-yl-2,3-dihydro-1H-cyclopenta[4',5']pyrido[3',2':4,5]thiéno[3,2-d]pyrimidine-7-amine
la 2,2-diméthyl-N-(2-morpholin-4-yléthyl)-N-(pyridin-3-ylméthyl)-4-pyrrolidin-1-yl-2,3-dihydro-1H-cyclopenta-[4',5']pyrido[3',2':4,5]thiéno[3,2-d]pyrimidine-7-amine
la N⁴-éthyl-N⁴,2,2-triméthyl-N⁷-(2-morpholin-4-yléthyl)-2,3-dihydro-1H-cyclopenta[4',5']pyrido[3',2':4,5]thiéno[3,2-d]pyrimidine-4,7-diamine
la N⁴-éthyl-N⁴,2,2-triméthyl-N⁷-(pyridin-3-ylméthyl)-2,3-dihydro-1H-cyclopenta[4',5']pyrido[3',2':4,5]thiéno[3,2-d]-pyrimidine-4,7-diamine
la 1-[3-({4-[éthyl(méthyl)amino]-2,2-diméthyl-2,3-dihydro-1H-cyclopenta[4',5']pyrido [3',2':4,5]thiéno[3,2-d]-pyrimidin-7-yl}amino)propyl]pyrrolidin-2-one
la N⁷-(2,3-diméthoxybenzyl)-N⁴-éthyl-N⁴,2,2-triméthyl-2,3-dihydro-1H-cyclopenta[4',5']pyrido[3',2':4,5]thiéno[3,2-d]pyrimidine-4,7-diamine
le 2-[{4-[éthyl(méthyl)amino+-2,2-diméthyl-2,3-dihydro-1H-cyclopenta[4',5']pyrido[3',2':4,5]thiéno[3,2-d]pyrimidin-7-yl}(2-morpholin-4-yléthyl)amino]éthanol
la N⁴-éthyl-N⁴,2,2-triméthyl-N⁷-(2-morpholin-4-yléthyl)-N⁷-(pyridin-3-ylméthyl)-2,3-dihydro-1H-cyclopenta[4',5']-pyrido[3',2':4,5]thiéno[3,2-d]pyrimidine-4,7-diamine
la 2,2-diméthyl-4-morpholin-4-yl-N-(2-morpholin-4-yléthyl)-2,3-dihydro-1H-cyclopenta[4',5']pyrido[3',2':4,5]-thiéno[3,2-d]pyrimidine-7-amine
le 2-[(2,2-diméthyl-4-morpholin-4-yl-2,3-dihydro-1H-cyclopenta[4',5']pyrido[3',2':4,5]thiéno[3,2-d]pyrimidin-7-yl)(2-morpholin-4-yléthyl)amino]éthanol
la N-(2,3-diméthoxybenzyl)-2,2-diméthyl-4-morpholin-4-yl-2,3-dihydro-1H-cyclopenta[4',5']pyrido[3',2':4,5]thiéno-[3,2-d]pyrimidine-7-amine
la 1-{3-[(2,2-diméthyl-4-morpholin-4-yl-2,3-dihydro-1H-cyclopenta-[4',5']pyrido [3',2':4,5]thiéno[3,2-d]pyrimidin-7-yl)amino]propyl}pyrrolidin-2-one
le 2-[(2,2-diméthyl-5-morpholin-4-yl-1,2,3,4-tétrahydro-pyrimido[4',5':4,5]thiéno[2,3-c]isoquinoléin-8-yl)(2-morpholin-4-yléthyl)amino]éthanol
le 2-[(2,2-diméthyl-5-morpholin-4-yl-1,2,3,4-tétrahydro-pyrimido[4',5':4,5]thiéno[2,3-c]isoquinoléin-8-yl)(pyridin-3-ylméthyl)amino]éthanol
la N⁵,2,2-triméthyl-N⁸-(2-morpholin-4-yléthyl)-1,2,3,4-tétrahydropyrimido[4',5':4,5]thiéno[2,3-c]isoquinoléine-5,8-diamine
la N⁵,2,2-triméthyl-N⁸-(pyridin-3-ylméthyl)-1,2,3,4-tétrahydropyrimido[4',5':4,5]thiéno[2,3-c]isoquinoléine-5,8-diamine
la 1-(3-{[2,2-diméthyl-5-(méthylamino)-1,2,3,4-tétra-hydropyrimido[4',5':4,5]thiéno[2,3-c]isoquinoléin-8-yl]-amino}propyl)pyrrolidin-2-one
et les sels pharmaceutiquement acceptables de celui-ci.

24. Composition pharmaceutique comprenant un composé selon l'une quelconque des revendications 12 à 23 en mélange avec un diluant ou véhicule pharmaceutiquement acceptable.

25. Composé selon l'une quelconque des revendications 1 à 23, pour l'utilisation dans le traitement d'un sujet souffrant d'une affection pathologique ou d'une maladie qui est un asthme, une bronchopneumopathie chronique obstructive, une polyarthrite rhumatoïde, une dermatite atopique, un psoriasis ou une maladie du côlon irritable.

26. Produit de combinaison comprenant :
(i) un composé selon l'une quelconque des revendications 12 à 23 ; et
(ii) un autre composé choisis parmi (a) les stéroïdes, (b) les agents immunosuppresseurs, (c) les agents bloquant les récepteurs des lymphocytes T et (d) les substances médicamenteuses anti-inflammatoires
pour une utilisation simultanée, séparée ou séquentielle dans le traitement du corps humain ou animal.
